(19) Europäisches Patentamt
European Patent Office
Office européen des brevets

(11) EP 4 047 016 A1

(12) **EUROPEAN PATENT APPLICATION**

(43) Date of publication:
**24.08.2022 Bulletin 2022/34**

(21) Application number: **21305210.3**

(22) Date of filing: **19.02.2021**

(51) International Patent Classification (IPC):
***C07K 16/18*** *(2006.01)* ***G01N 33/574*** *(2006.01)*
*A61K 39/395* *(2006.01)* *A61P 35/00* *(2006.01)*
*A61P 35/04* *(2006.01)*

(52) Cooperative Patent Classification (CPC):
**C07K 16/18; A61K 51/10;** C07K 2317/22;
C07K 2317/569; C07K 2317/76; C07K 2317/92

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Université de Strasbourg**
  **67000 Strasbourg (FR)**
- **INSERM - Institut National de la Santé et de la Recherche Médicale**
  **75013 Paris (FR)**
- **INSTITUT PASTEUR DE TUNIS**
  **Tunis (TN)**
- **Friedrich Miescher Institute for Biomedical Research**
  **4058 Basel (CH)**

(72) Inventors:
- **OREND, Gertraud**
  **77694 Kehl (DE)**
- **BOUHAOUALA-ZAHAR, Balkiss**
  **Tunis (TN)**
- **DHAOUADI, Sayda**
  **Tunis (TN)**
- **CHIQUET-EHRISMANN, Ruth**
  **deceased (CH)**
- **HENDAOUI, Ismaïl**
  **68128 Rosenau (FR)**

(74) Representative: **Novagraaf Technologies**
**Bâtiment O2**
**2, rue Sarah Bernhardt**
**CS90017**
**92665 Asnières-sur-Seine Cedex (FR)**

# (54) ANTI-TENASCIN-C (TNC) SINGLE-DOMAIN ANTIBODIES (NANOBODIES) AND USE THEREOF

(57) The present invention refers to an isolated single-domain antibody directed against Tenascin-C (TNC) wherein said single-domain antibody comprises the amino acid sequences: i) GYTNSIYT (SEQ ID NO: 1) as variable heavy (VHH) chain complementarity determining region (CDR)1, ii) IXaSRNGNT (SEQ ID NO: 2) as variable heavy (VHH) chain complementarity determining region (CDR)2, wherein $X_a$ is G or A iii) AAGSSWDLILQAYAYDY (SEQ ID NO: 3) as variable heavy (VHH) chain complementarity determining region (CDR)3. The invention further relates to a said single-domain antibody directed against Tenascin-C (TNC) for use as medicament and to a pharmaceutical composition comprising said single-domain antibody directed against Tenascin-C (TNC).

The present invention finds application in the therapeutic and diagnostic medical technical fields.

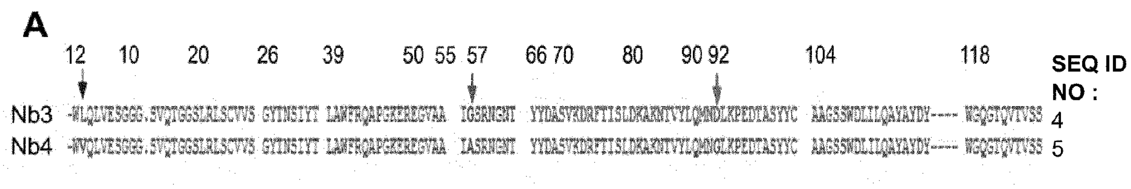


FIGURE 1

EP 4 047 016 A1

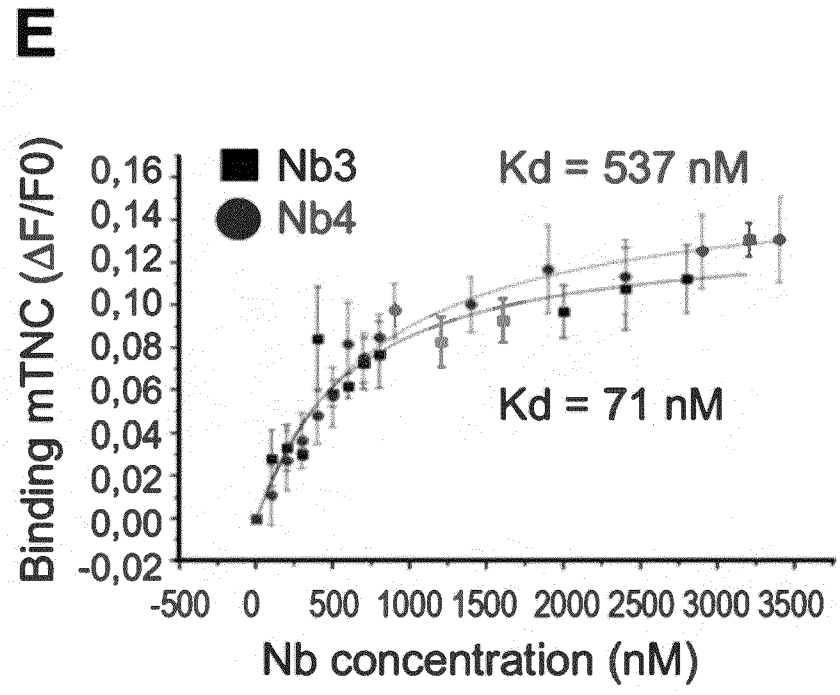
E
Nb3
Nb4
Kd = 537 nM
Kd = 71 nM
Binding mTNC (ΔF/F0)
0,16
0,14
0,12
0,10
0,08
0,06
0,04
0,02
0,00
-0,02
-500 0 500 1000 1500 2000 2500 3000 3500
Nb concentration (nM)

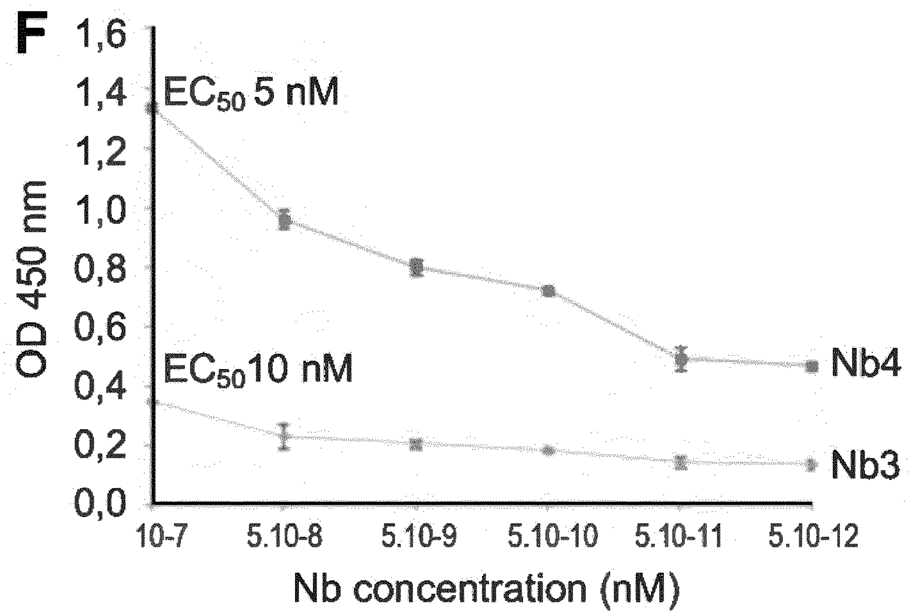
F
OD 450 nm
1,6
1,4
1,2
1,0
0,8
0,6
0,4
0,2
0,0
EC50 5 nM
EC50 10 nM
Nb4
Nb3
10-7 5.10-8 5.10-9 5.10-10 5.10-11 5.10-12
Nb concentration (nM)

FIGURE 1(2/2)

SEQUENCE LISTING

<110> Université de Strasbourg
INSERM - Institut National de la Santé et de la Recherche Médicale
Institut Pasteur de Tunis
Friedrich Miescher Institute for Biomedical Research

<120> Anti-tenascin-C (TNC) single-domain antibodies (nanobodies) and use thereof

<130> BNT230579EP00

<160> 20

<170> PatentIn version 3.5

<210> 1
<211> 8
<212> PRT
<213> Artificial Sequence

<220>
<223> CDR1

<400> 1

```
Gly Tyr Thr Asn Ser Ile Tyr Thr
1               5
```

<210> 2
<211> 8
<212> PRT
<213> Artificial Sequence

<220>
<223> CDR2

<220>
<221> MISC_FEATURE
<222> (2)..(2)
<223> Xaa can be G or A

<400> 2

```
Ile Xaa Ser Arg Asn Gly Asn Thr
1               5
```

<210> 3
<211> 17
<212> PRT
<213> Artificial Sequence

<220>
<223> CDR3

<400> 3

```
Ala Ala Gly Ser Ser Trp Asp Leu Ile Leu Gln Ala Tyr Ala Tyr Asp
1               5                   10                  15
```

Tyr

<210> 4
<211> 124
<212> PRT
<213> Artificial Sequence

<220>
<223> single-domain antibodies directed against Tenascin-C (TNC)

<400> 4

Trp Leu Gln Leu Val Glu Ser Gly Gly Gly Ser Val Gln Thr Gly Gly
1 5 10 15

Ser Leu Arg Leu Ser Cys Val Val Ser Gly Tyr Thr Asn Ser Ile Tyr
20 25 30

Thr Leu Ala Trp Phe Arg Gln Ala Pro Gly Lys Glu Arg Glu Gly Val
35 40 45

Ala Ala Ile Gly Ser Arg Asn Gly Asn Thr Tyr Tyr Asp Ala Ser Val
50 55 60

Lys Asp Arg Phe Thr Ile Ser Leu Asp Lys Ala Lys Asn Thr Val Tyr
65 70 75 80

Leu Gln Met Asn Asp Leu Lys Pro Glu Asp Thr Ala Ser Tyr Tyr Cys
85 90 95

Ala Ala Gly Ser Ser Trp Asp Leu Ile Leu Gln Ala Tyr Ala Tyr Asp
100 105 110

Tyr Trp Gly Gln Gly Thr Gln Val Thr Val Ser Ser
115 120

<210> 5
<211> 124
<212> PRT
<213> Artificial Sequence

<220>
<223> single-domain antibody directed against Tenascin-C (TNC)

<400> 5

Trp Val Gln Leu Val Glu Ser Gly Gly Gly Ser Val Gln Thr Gly Gly
1 5 10 15

Ser Leu Arg Leu Ser Cys Val Val Ser Gly Tyr Thr Asn Ser Ile Tyr

```
            20                  25                  30

Thr Leu Ala Trp Phe Arg Gln Ala Pro Gly Lys Glu Arg Glu Gly Val
        35                  40                  45

Ala Ala Ile Ala Ser Arg Asn Gly Asn Thr Tyr Tyr Asp Ala Ser Val
    50                  55                  60

Lys Asp Arg Phe Thr Ile Ser Leu Asp Lys Ala Lys Asn Thr Val Tyr
65                  70                  75                  80

Leu Gln Met Asn Gly Leu Lys Pro Glu Asp Thr Ala Ser Tyr Tyr Cys
                85                  90                  95

Ala Ala Gly Ser Ser Trp Asp Leu Ile Leu Gln Ala Tyr Ala Tyr Asp
            100                 105                 110

Tyr Trp Gly Gln Gly Thr Gln Val Thr Val Ser Ser
        115                 120


<210>  6
<211>  554
<212>  DNA
<213>  Artificial Sequence

<220>
<223>  Nucleic acid encoding a single-domain antibody directed against
       Tenascin-C (TNC)

<400>  6
atggctgcat ggttgcagct ggtggagtct gggggaggct cggtgcagac tggagggtct      60

ctgagactct cctgtgtagt ctctggatac accaacagta tctacacgct ggcctggttc     120

cgccaggctc cagggaagga gcgtgagggg gtagcggcta ttggtagtcg taatggtaac     180

acatactacg acgcctccgt gaaggaccga ttcaccatct ccctagacaa ggccaagaac     240

acggtgtatc tgcaaatgaa cgacctgaaa cctgaggaca cggccagcta ttactgtgcg     300

gcaggatcct cttgggacct catcttacag gcatatgcgt atgactactg gggccagggg     360

acccaggtca ccgtctcctc agcggccgca tacccgtacg acgttccgga ctacggttcc     420

caccaccatc accatcacta gactgttgaa agttgtttag caaaacctca tacagaaaat     480

tcatttacta acgtctggaa agacgacaaa actttagatc gttacgctaa ctatgagggt     540

tgtctgtgga atgc                                                       554


<210>  7
<211>  555
<212>  DNA
<213>  Artificial Sequence
```

<220>
<223> nucleic acid encoding a single-domain antibody directed against Tenascin-C (TNC)

<400> 7
atggctgcat gggtgcagct ggtggagtct gggggaggct cggtgcagac tggagggtct 60

ctgagactct cctgtgtagt ctctggatac accaacagta tctacawgct ggcctggttc 120

cgccaggctc cagggaagga gcgtgagggg gtagcggcta ttgctagtcg taatggtaac 180

acatactacg acgcctccgt gaaggaccga ttcaccatct ccctagacaa ggccaagaac 240

acggtgtatc tgcaaatgaa cggcctgaaa cctgaggaca cggccagcta ttactgtgcg 300

gcaggatcct cttgggacct catcttacag gcatatgcgt atgactactg gggccagggg 360

acccaggtca ccgtctcctc agcggccgca tacccgtacg acgttccgga ctacggttcc 420

caccaccatc accatcacta gactgttgaa agttgtttag caaaacctca tacagaaaat 480

tcatttacta acgtctggaa agacgacaaa actttagatc gttacgctaa ctatgagggc 540

tggtctgtgg aatgc 555

<210> 8
<211> 23
<212> DNA
<213> Artificial Sequence

<220>
<223> Call 001 primer

<400> 8
gtcctggctg ctcttctaca agg 23

<210> 9
<211> 23
<212> DNA
<213> Artificial Sequence

<220>
<223> CALL002 primer

<400> 9
ggtacgtgct gttgaactgt tcc 23

<210> 10
<211> 41
<212> DNA
<213> Artificial Sequence

<220>
<223> SM017 primer

<400> 10
ccagccggcc atggctgcat ggtgcagctg gtggagtctg g 41

<210> 11

<211> 34
<212> DNA
<213> Artificial Sequence

<220>
<223> PMCF primer

<400> 11
ctagtgcggc cgctgaggag acggtgacct gggt 34

<210> 12
<211> 8
<212> PRT
<213> Artificial Sequence

<220>
<223> CDR2

<400> 12

Ile Gly Ser Arg Asn Gly Asn Thr
1 5

<210> 13
<211> 8
<212> PRT
<213> Artificial Sequence

<220>
<223> CDR2

<400> 13

Ile Ala Ser Arg Asn Gly Asn Thr
1 5

<210> 14
<211> 21
<212> DNA
<213> Artificial Sequence

<220>
<223> sequence coding the (CDR)1 of isolated single-domain antibody directed against Tenascin-C (TNC)

<400> 14
ggatacacca acagtatcta c 21

<210> 15
<211> 21
<212> DNA
<213> Artificial Sequence

<220>
<223> sequence coding (CDR)2 of the isolated single-domain antibody directed against Tenascin-C (TNC)

<400> 15
attggtagtc gtaatggtaa c 21

<210> 16
<211> 21
<212> DNA
<213> Artificial Sequence

<220>
<223> sequence coding (CDR)2 of isolated single-domain antibody directed against Tenascin-C (TNC)

<400> 16
attgctagtc gtaatggtaa c 21

<210> 17
<211> 31
<212> DNA
<213> Artificial Sequence

<220>
<223> sequence coding (CDR)3 of isolated single-domain antibody directed against Tenascin-C (TNC)

<400> 17
gcggcaggat cctcttggga cctcatctta c 31

<210> 18
<211> 2201
<212> PRT
<213> Homo sapiens

<400> 18

```
Met Gly Ala Met Thr Gln Leu Leu Ala Gly Val Phe Leu Ala Phe Leu
1               5                   10                  15

Ala Leu Ala Thr Glu Gly Gly Val Leu Lys Lys Val Ile Arg His Lys
            20                  25                  30

Arg Gln Ser Gly Val Asn Ala Thr Leu Pro Glu Glu Asn Gln Pro Val
        35                  40                  45

Val Phe Asn His Val Tyr Asn Ile Lys Leu Pro Val Gly Ser Gln Cys
    50                  55                  60

Ser Val Asp Leu Glu Ser Ala Ser Gly Glu Lys Asp Leu Ala Pro Pro
65                  70                  75                  80

Ser Glu Pro Ser Glu Ser Phe Gln Glu His Thr Val Asp Gly Glu Asn
                85                  90                  95

Gln Ile Val Phe Thr His Arg Ile Asn Ile Pro Arg Arg Ala Cys Gly
            100                 105                 110
```

Cys Ala Ala Ala Pro Asp Val Lys Glu Leu Leu Ser Arg Leu Glu Glu
115 120 125

Leu Glu Asn Leu Val Ser Ser Leu Arg Glu Gln Cys Thr Ala Gly Ala
130 135 140

Gly Cys Cys Leu Gln Pro Ala Thr Gly Arg Leu Asp Thr Arg Pro Phe
145 150 155 160

Cys Ser Gly Arg Gly Asn Phe Ser Thr Glu Gly Cys Gly Cys Val Cys
165 170 175

Glu Pro Gly Trp Lys Gly Pro Asn Cys Ser Glu Pro Glu Cys Pro Gly
180 185 190

Asn Cys His Leu Arg Gly Arg Cys Ile Asp Gly Gln Cys Ile Cys Asp
195 200 205

Asp Gly Phe Thr Gly Glu Asp Cys Ser Gln Leu Ala Cys Pro Ser Asp
210 215 220

Cys Asn Asp Gln Gly Lys Cys Val Asn Gly Val Cys Ile Cys Phe Glu
225 230 235 240

Gly Tyr Ala Gly Ala Asp Cys Ser Arg Glu Ile Cys Pro Val Pro Cys
245 250 255

Ser Glu Glu His Gly Thr Cys Val Asp Gly Leu Cys Val Cys His Asp
260 265 270

Gly Phe Ala Gly Asp Asp Cys Asn Lys Pro Leu Cys Leu Asn Asn Cys
275 280 285

Tyr Asn Arg Gly Arg Cys Val Glu Asn Glu Cys Val Cys Asp Glu Gly
290 295 300

Phe Thr Gly Glu Asp Cys Ser Glu Leu Ile Cys Pro Asn Asp Cys Phe
305 310 315 320

Asp Arg Gly Arg Cys Ile Asn Gly Thr Cys Tyr Cys Glu Glu Gly Phe
325 330 335

Thr Gly Glu Asp Cys Gly Lys Pro Thr Cys Pro His Ala Cys His Thr
340 345 350

Gln Gly Arg Cys Glu Glu Gly Gln Cys Val Cys Asp Glu Gly Phe Ala
355 360 365

```
Gly Val Asp Cys Ser Glu Lys Arg Cys Pro Ala Asp Cys His Asn Arg
    370                 375                 380

Gly Arg Cys Val Asp Gly Arg Cys Glu Cys Asp Asp Gly Phe Thr Gly
385                 390                 395                 400

Ala Asp Cys Gly Glu Leu Lys Cys Pro Asn Gly Cys Ser Gly His Gly
                405                 410                 415

Arg Cys Val Asn Gly Gln Cys Val Cys Asp Glu Gly Tyr Thr Gly Glu
            420                 425                 430

Asp Cys Ser Gln Leu Arg Cys Pro Asn Asp Cys His Ser Arg Gly Arg
        435                 440                 445

Cys Val Glu Gly Lys Cys Val Cys Glu Gln Gly Phe Lys Gly Tyr Asp
    450                 455                 460

Cys Ser Asp Met Ser Cys Pro Asn Asp Cys His Gln His Gly Arg Cys
465                 470                 475                 480

Val Asn Gly Met Cys Val Cys Asp Asp Gly Tyr Thr Gly Glu Asp Cys
                485                 490                 495

Arg Asp Arg Gln Cys Pro Arg Asp Cys Ser Asn Arg Gly Leu Cys Val
            500                 505                 510

Asp Gly Gln Cys Val Cys Glu Asp Gly Phe Thr Gly Pro Asp Cys Ala
        515                 520                 525

Glu Leu Ser Cys Pro Asn Asp Cys His Gly Gln Gly Arg Cys Val Asn
    530                 535                 540

Gly Gln Cys Val Cys His Glu Gly Phe Met Gly Lys Asp Cys Lys Glu
545                 550                 555                 560

Gln Arg Cys Pro Ser Asp Cys His Gly Gln Gly Arg Cys Val Asp Gly
                565                 570                 575

Gln Cys Ile Cys His Glu Gly Phe Thr Gly Leu Asp Cys Gly Gln His
            580                 585                 590

Ser Cys Pro Ser Asp Cys Asn Asn Leu Gly Gln Cys Val Ser Gly Arg
        595                 600                 605

Cys Ile Cys Asn Glu Gly Tyr Ser Gly Glu Asp Cys Ser Glu Val Ser
    610                 615                 620
```

Pro Pro Lys Asp Leu Val Val Thr Glu Val Thr Glu Glu Thr Val Asn
625 630 635 640

Leu Ala Trp Asp Asn Glu Met Arg Val Thr Glu Tyr Leu Val Val Tyr
645 650 655

Thr Pro Thr His Glu Gly Gly Leu Glu Met Gln Phe Arg Val Pro Gly
660 665 670

Asp Gln Thr Ser Thr Ile Ile Gln Glu Leu Glu Pro Gly Val Glu Tyr
675 680 685

Phe Ile Arg Val Phe Ala Ile Leu Glu Asn Lys Lys Ser Ile Pro Val
690 695 700

Ser Ala Arg Val Ala Thr Tyr Leu Pro Ala Pro Glu Gly Leu Lys Phe
705 710 715 720

Lys Ser Ile Lys Glu Thr Ser Val Glu Val Glu Trp Asp Pro Leu Asp
725 730 735

Ile Ala Phe Glu Thr Trp Glu Ile Ile Phe Arg Asn Met Asn Lys Glu
740 745 750

Asp Glu Gly Glu Ile Thr Lys Ser Leu Arg Arg Pro Glu Thr Ser Tyr
755 760 765

Arg Gln Thr Gly Leu Ala Pro Gly Gln Glu Tyr Glu Ile Ser Leu His
770 775 780

Ile Val Lys Asn Asn Thr Arg Gly Pro Gly Leu Lys Arg Val Thr Thr
785 790 795 800

Thr Arg Leu Asp Ala Pro Ser Gln Ile Glu Val Lys Asp Val Thr Asp
805 810 815

Thr Thr Ala Leu Ile Thr Trp Phe Lys Pro Leu Ala Glu Ile Asp Gly
820 825 830

Ile Glu Leu Thr Tyr Gly Ile Lys Asp Val Pro Gly Asp Arg Thr Thr
835 840 845

Ile Asp Leu Thr Glu Asp Glu Asn Gln Tyr Ser Ile Gly Asn Leu Lys
850 855 860

Pro Asp Thr Glu Tyr Glu Val Ser Leu Ile Ser Arg Arg Gly Asp Met

865 870 875 880

Ser Ser Asn Pro Ala Lys Glu Thr Phe Thr Thr Gly Leu Asp Ala Pro
885 890 895

Arg Asn Leu Arg Arg Val Ser Gln Thr Asp Asn Ser Ile Thr Leu Glu
900 905 910

Trp Arg Asn Gly Lys Ala Ala Ile Asp Ser Tyr Arg Ile Lys Tyr Ala
915 920 925

Pro Ile Ser Gly Gly Asp His Ala Glu Val Asp Val Pro Lys Ser Gln
930 935 940

Gln Ala Thr Thr Lys Thr Thr Leu Thr Gly Leu Arg Pro Gly Thr Glu
945 950 955 960

Tyr Gly Ile Gly Val Ser Ala Val Lys Glu Asp Lys Glu Ser Asn Pro
965 970 975

Ala Thr Ile Asn Ala Ala Thr Glu Leu Asp Thr Pro Lys Asp Leu Gln
980 985 990

Val Ser Glu Thr Ala Glu Thr Ser Leu Thr Leu Leu Trp Lys Thr Pro
995 1000 1005

Leu Ala Lys Phe Asp Arg Tyr Arg Leu Asn Tyr Ser Leu Pro Thr
1010 1015 1020

Gly Gln Trp Val Gly Val Gln Leu Pro Arg Asn Thr Thr Ser Tyr
1025 1030 1035

Val Leu Arg Gly Leu Glu Pro Gly Gln Glu Tyr Asn Val Leu Leu
1040 1045 1050

Thr Ala Glu Lys Gly Arg His Lys Ser Lys Pro Ala Arg Val Lys
1055 1060 1065

Ala Ser Thr Glu Gln Ala Pro Glu Leu Glu Asn Leu Thr Val Thr
1070 1075 1080

Glu Val Gly Trp Asp Gly Leu Arg Leu Asn Trp Thr Ala Ala Asp
1085 1090 1095

Gln Ala Tyr Glu His Phe Ile Ile Gln Val Gln Glu Ala Asn Lys
1100 1105 1110

```
Val Glu  Ala Ala Arg Asn Leu  Thr Val Pro Gly Ser  Leu Arg Ala
    1115                 1120                 1125

Val Asp  Ile Pro Gly Leu Lys  Ala Ala Thr Pro Tyr  Thr Val Ser
    1130                 1135                 1140

Ile Tyr  Gly Val Ile Gln Gly  Tyr Arg Thr Pro Val  Leu Ser Ala
    1145                 1150                 1155

Glu Ala  Ser Thr Gly Glu Thr  Pro Asn Leu Gly Glu  Val Val Val
    1160                 1165                 1170

Ala Glu  Val Gly Trp Asp Ala  Leu Lys Leu Asn Trp  Thr Ala Pro
    1175                 1180                 1185

Glu Gly  Ala Tyr Glu Tyr Phe  Phe Ile Gln Val Gln  Glu Ala Asp
    1190                 1195                 1200

Thr Val  Glu Ala Ala Gln Asn  Leu Thr Val Pro Gly  Gly Leu Arg
    1205                 1210                 1215

Ser Thr  Asp Leu Pro Gly Leu  Lys Ala Ala Thr His  Tyr Thr Ile
    1220                 1225                 1230

Thr Ile  Arg Gly Val Thr Gln  Asp Phe Ser Thr Thr  Pro Leu Ser
    1235                 1240                 1245

Val Glu  Val Leu Thr Glu Glu  Val Pro Asp Met Gly  Asn Leu Thr
    1250                 1255                 1260

Val Thr  Glu Val Ser Trp Asp  Ala Leu Arg Leu Asn  Trp Thr Thr
    1265                 1270                 1275

Pro Asp  Gly Thr Tyr Asp Gln  Phe Thr Ile Gln Val  Gln Glu Ala
    1280                 1285                 1290

Asp Gln  Val Glu Glu Ala His  Asn Leu Thr Val Pro  Gly Ser Leu
    1295                 1300                 1305

Arg Ser  Met Glu Ile Pro Gly  Leu Arg Ala Gly Thr  Pro Tyr Thr
    1310                 1315                 1320

Val Thr  Leu His Gly Glu Val  Arg Gly His Ser Thr  Arg Pro Leu
    1325                 1330                 1335

Ala Val  Glu Val Val Thr Glu  Asp Leu Pro Gln Leu  Gly Asp Leu
    1340                 1345                 1350
```

```
Ala Val  Ser Glu Val Gly Trp  Asp Gly Leu Arg Leu  Asn Trp Thr
    1355                 1360                 1365

Ala Ala  Asp Asn Ala Tyr Glu  His Phe Val Ile Gln  Val Gln Glu
    1370                 1375                 1380

Val Asn  Lys Val Glu Ala Ala  Gln Asn Leu Thr Leu  Pro Gly Ser
    1385                 1390                 1395

Leu Arg  Ala Val Asp Ile Pro  Gly Leu Glu Ala Ala  Thr Pro Tyr
    1400                 1405                 1410

Arg Val  Ser Ile Tyr Gly Val  Ile Arg Gly Tyr Arg  Thr Pro Val
    1415                 1420                 1425

Leu Ser  Ala Glu Ala Ser Thr  Ala Lys Glu Pro Glu  Ile Gly Asn
    1430                 1435                 1440

Leu Asn  Val Ser Asp Ile Thr  Pro Glu Ser Phe Asn  Leu Ser Trp
    1445                 1450                 1455

Met Ala  Thr Asp Gly Ile Phe  Glu Thr Phe Thr Ile  Glu Ile Ile
    1460                 1465                 1470

Asp Ser  Asn Arg Leu Leu Glu  Thr Val Glu Tyr Asn  Ile Ser Gly
    1475                 1480                 1485

Ala Glu  Arg Thr Ala His Ile  Ser Gly Leu Pro Pro  Ser Thr Asp
    1490                 1495                 1500

Phe Ile  Val Tyr Leu Ser Gly  Leu Ala Pro Ser Ile  Arg Thr Lys
    1505                 1510                 1515

Thr Ile  Ser Ala Thr Ala Thr  Thr Glu Ala Leu Pro  Leu Leu Glu
    1520                 1525                 1530

Asn Leu  Thr Ile Ser Asp Ile  Asn Pro Tyr Gly Phe  Thr Val Ser
    1535                 1540                 1545

Trp Met  Ala Ser Glu Asn Ala  Phe Asp Ser Phe Leu  Val Thr Val
    1550                 1555                 1560

Val Asp  Ser Gly Lys Leu Leu  Asp Pro Gln Glu Phe  Thr Leu Ser
    1565                 1570                 1575

Gly Thr  Gln Arg Lys Leu Glu  Leu Arg Gly Leu Ile  Thr Gly Ile
    1580                 1585                 1590
```

Gly Tyr Glu Val Met Val Ser Gly Phe Thr Gln Gly His Gln Thr
    1595                1600                1605

Lys Pro Leu Arg Ala Glu Ile Val Thr Glu Ala Glu Pro Glu Val
    1610                1615                1620

Asp Asn Leu Leu Val Ser Asp Ala Thr Pro Asp Gly Phe Arg Leu
    1625                1630                1635

Ser Trp Thr Ala Asp Glu Gly Val Phe Asp Asn Phe Val Leu Lys
    1640                1645                1650

Ile Arg Asp Thr Lys Lys Gln Ser Glu Pro Leu Glu Ile Thr Leu
    1655                1660                1665

Leu Ala Pro Glu Arg Thr Arg Asp Ile Thr Gly Leu Arg Glu Ala
    1670                1675                1680

Thr Glu Tyr Glu Ile Glu Leu Tyr Gly Ile Ser Lys Gly Arg Arg
    1685                1690                1695

Ser Gln Thr Val Ser Ala Ile Ala Thr Thr Ala Met Gly Ser Pro
    1700                1705                1710

Lys Glu Val Ile Phe Ser Asp Ile Thr Glu Asn Ser Ala Thr Val
    1715                1720                1725

Ser Trp Arg Ala Pro Thr Ala Gln Val Glu Ser Phe Arg Ile Thr
    1730                1735                1740

Tyr Val Pro Ile Thr Gly Gly Thr Pro Ser Met Val Thr Val Asp
    1745                1750                1755

Gly Thr Lys Thr Gln Thr Arg Leu Val Lys Leu Ile Pro Gly Val
    1760                1765                1770

Glu Tyr Leu Val Ser Ile Ile Ala Met Lys Gly Phe Glu Glu Ser
    1775                1780                1785

Glu Pro Val Ser Gly Ser Phe Thr Thr Ala Leu Asp Gly Pro Ser
    1790                1795                1800

Gly Leu Val Thr Ala Asn Ile Thr Asp Ser Glu Ala Leu Ala Arg
    1805                1810                1815

Trp Gln Pro Ala Ile Ala Thr Val Asp Ser Tyr Val Ile Ser Tyr

1820 1825 1830

Thr Gly Glu Lys Val Pro Glu Ile Thr Arg Thr Val Ser Gly Asn
1835 1840 1845

Thr Val Glu Tyr Ala Leu Thr Asp Leu Glu Pro Ala Thr Glu Tyr
1850 1855 1860

Thr Leu Arg Ile Phe Ala Glu Lys Gly Pro Gln Lys Ser Ser Thr
1865 1870 1875

Ile Thr Ala Lys Phe Thr Thr Asp Leu Asp Ser Pro Arg Asp Leu
1880 1885 1890

Thr Ala Thr Glu Val Gln Ser Glu Thr Ala Leu Leu Thr Trp Arg
1895 1900 1905

Pro Pro Arg Ala Ser Val Thr Gly Tyr Leu Leu Val Tyr Glu Ser
1910 1915 1920

Val Asp Gly Thr Val Lys Glu Val Ile Val Gly Pro Asp Thr Thr
1925 1930 1935

Ser Tyr Ser Leu Ala Asp Leu Ser Pro Ser Thr His Tyr Thr Ala
1940 1945 1950

Lys Ile Gln Ala Leu Asn Gly Pro Leu Arg Ser Asn Met Ile Gln
1955 1960 1965

Thr Ile Phe Thr Thr Ile Gly Leu Leu Tyr Pro Phe Pro Lys Asp
1970 1975 1980

Cys Ser Gln Ala Met Leu Asn Gly Asp Thr Thr Ser Gly Leu Tyr
1985 1990 1995

Thr Ile Tyr Leu Asn Gly Asp Lys Ala Glu Ala Leu Glu Val Phe
2000 2005 2010

Cys Asp Met Thr Ser Asp Gly Gly Gly Trp Ile Val Phe Leu Arg
2015 2020 2025

Arg Lys Asn Gly Arg Glu Asn Phe Tyr Gln Asn Trp Lys Ala Tyr
2030 2035 2040

Ala Ala Gly Phe Gly Asp Arg Arg Glu Glu Phe Trp Leu Gly Leu
2045 2050 2055

Asp Asn Leu Asn Lys Ile Thr Ala Gln Gly Gln Tyr Glu Leu Arg
2060 2065 2070

Val Asp Leu Arg Asp His Gly Glu Thr Ala Phe Ala Val Tyr Asp
2075 2080 2085

Lys Phe Ser Val Gly Asp Ala Lys Thr Arg Tyr Lys Leu Lys Val
2090 2095 2100

Glu Gly Tyr Ser Gly Thr Ala Gly Asp Ser Met Ala Tyr His Asn
2105 2110 2115

Gly Arg Ser Phe Ser Thr Phe Asp Lys Asp Thr Asp Ser Ala Ile
2120 2125 2130

Thr Asn Cys Ala Leu Ser Tyr Lys Gly Ala Phe Trp Tyr Arg Asn
2135 2140 2145

Cys His Arg Val Asn Leu Met Gly Arg Tyr Gly Asp Asn Asn His
2150 2155 2160

Ser Gln Gly Val Asn Trp Phe His Trp Lys Gly His Glu His Ser
2165 2170 2175

Ile Gln Phe Ala Glu Met Lys Leu Arg Pro Ser Asn Phe Arg Asn
2180 2185 2190

Leu Glu Gly Arg Arg Lys Arg Ala
2195 2200

<210> 19
<211> 98670
<212> DNA
<213> Homo sapiens

<400> 19
aaaaagtggg aaaggatgtc tggaggcgag gcgtcccatt acagaggaag gagctcgcta 60

tataagccag ccaaagttgg ctgcaccggc cacagcctgc ctactgtcac ccgcctctcc 120

cgcgcgcaga tacacgcccc cgcctccgtg ggcacaaagg cagcgctgct ggggaactcg 180

ggggaacgcg cacgtgggaa ccgccgcagc tccacactcc aggtacttct tccaaggacc 240

taggtctctc gcccatcgga aaggtaagtc tgttttgccc tcatcactag agatccttat 300

gatgtaagga ttgcattcct ggtaccaaat gagttgtgat tttatattat aattacaaaa 360

catatggggt tttttttcc accgctggaa aaaaaaggag tgtgtattgg ttatatcaga 420

tgcaccttga cttttctggt cttgtaattt tgtttgtttt aactttatga atagtcagac 480

ttggaaggaa cttttcgtaa agttgttatc ttctgctgtt ctgcagagga aaagtaaaaa 540

```
ttttctcttc aactgctctc ctacttctag tctggctgat tcacctgtat atatctttta      600
ttttgagggt tagggcagta gagttttaag gcagactttg ctcatagcct gcccctggga      660
tgggttagag ggagtttgat agaggagtgc agggagaata taaaagttta tatctaaaca      720
gcacttacat aaagtttaca aagagttttg tacccattcc ctcatttggt cctcaaaata      780
ttcaccctgg aaatgagagc tgggggacag aagagaggag gtggctcctg ggccagatga      840
ataactgaca ggcagctaga gagtggtgga gaacagcttt actggggtgc ctagagccag      900
cagcgtccca catgtctcta gaatcacctc ctcctcctca cctctcatca cagaaagatc      960
tgagcaacaa catcccccag gtatgggaac cctgctgccc ctggtgtact ttggaaagaa     1020
cacaggggtt cttctcaccc atcagtgttg taagaggaat aagagcttag gtttgaagcc     1080
agaggtgttt ggtattctcc ccaccaccat acatatgacc ctgagaaaga tacatcactt     1140
ccttaatcct cagttttcac atctctaaaa tggggacaat agcgtctgct gtcgttttga     1200
ggagaaaatg aggtatagaa agatcagcaa tgactgctta ataactggaa gtttggataa     1260
tcattgtcat tgccaaaaac tgccatcact ctggaagtta aaagaggcat tgagccagtc     1320
ttggattgtt tcttgtgtgt tgttcctcag cccgccccca gcccccgctt tttatctcac     1380
ccaatctgag ggtgtctcac taggctggca ggtctgagac ttggctgtat tttacaagca     1440
ggaggttgtg ttggcacagg ttggagaaaa ggtagtagcc cggggctgga agttaatgtc     1500
aaattttgat tccagaatgt caggaggttt tgagtttagg catctgcaga ttctaaagct     1560
gacagctatg aagtacgatg ctgttggaaa ctctttggaa aagctttctg tccggaggga     1620
gggcaggaca gggagcacca cttgaagaat gaatgcctct ttttcatgtt tttgtagact     1680
ttttccccca tttttacaga gctttcccat atgtgtttta gaaaggaaaa ctactatcca     1740
tcccagccag gcaagcccaa ttcaggactg aagaagattt attcagtcct agacaaccaa     1800
gtgaagagtt tctggctttg tgcgcataca agtcatttgc aggagatgta agtcagcgtg     1860
ctttggccct tgcatttcag gaaggtgact gctttgtggg gaagaaaaag gtgcacaata     1920
tgagtattct ctggtttgct caaacagggt aaattggaca aggattgaaa gggatgttgc     1980
caagctctac agtcattaaa tatttttttc ctctatgctt tgttttaaaa acctagcttc     2040
tctattttat cccacactaa cctcctcctt ccccactcta agaagtgact aacgtttcag     2100
ccctcaactt tgtcattcat ataatggaga tgacaaacta tgtctcacag ggctcttgca     2160
acattcaaat gaggtaatat atggcaaaca cttggcatag taccttccac atattaaggg     2220
ttcaataaat ggaaactttt cttagtttct ctgtttatca ttttacaagg acttgtccaa     2280
gattaatatt ctaagatcat gatttttaaa cttattttta atgatgaaaa tattcattac     2340
atgaatttag ggcaaacccc cagtatatga caggtaaatg cagggctgct ttggttgaag     2400
```

tggggtcaca aggtctaaaa cttacacctt tgctcttagc ctctgggaca ttgccacaga 2460
atcttcaatg acaaaaagca catttacaaa tcatgaaacc aggctgtgtg cattctggtt 2520
gccattctgc ccttcaacaa ggatttttgc ccctctgagt caccatctgt agcagaagta 2580
agagcaagtc cacaaatagt gtaatagaca ggaatatgaa tgagaaataa aagacttaac 2640
aaaggctatt ttaattaatt ctaatgacaa tgatagccaa aatatatgat gagcctactc 2700
tctaaactgt aggcttttgt gggatcatcc acagcatgtc tctgaggtat ggactatatt 2760
tgttggacaa agagatggct tcaagaacag ggagactgaa taactttacc aaggtcctag 2820
aactcagagt gatacagcca ggtttcacac ccaggtcccc gtgatcacca agtcagaggt 2880
acattctgcc gtgttccttc tgaatgcttc tctgcttctt gcctgccttt agaaggaaaa 2940
cacttccttt ctgactaaat atgcccctcc aggcagactg tggccaccca taaatacagc 3000
agtagcctga tcatataacc agaatggaat ttggaaagag aattcaagct gacagaatga 3060
aaaacatatc cccttaatc ctaccctttc taatgctaac aataaattaa gagcacatat 3120
tctctcctct ctctctagat ttctatagac acctagctgt gctttcttac aagaaaacat 3180
atccactgga ccaaagcgca gccccttca tgctgaagcc acttcagttt gaaacgtaaa 3240
aagtaggttg caaacattta gttggctcga aatatatgtc cttttgtgat ggtaaaacac 3300
acaaacacaa agttaatagt gtcacatatg taataggagg gtttgccaat ggtttctcat 3360
ctcattctca ctcagacact acaaattata aattactagc tcaattttaa agaaatcgac 3420
actcaaggaa gttaggtgac tcatctgagg tctttccgta attgaggggc agagctggct 3480
ttgacccaag ctttctaact aataccccac tgttctttct gctgtgctat attgccttct 3540
gaagtcaggc tgtgctgacc aagtttagaa ccttaaatgg tgtctttcag tgaatgatca 3600
agatggtgga gaagccccca gctggcattg gtgcctattg attttacaga atagccctgc 3660
atgtatcatt tttcatggtt ctcagcaaag gtcagctctc aatggtgcaa ctctctgcct 3720
ggaaacagga aagttcaaat ttcagccctg gaagagagaa gaatgaaact agaggtagca 3780
gtcaggtcac tcctgctgat tttcatctca gaaaagtagg cacacaggtg agccaaacat 3840
ctggttttaa aagggctgat tggtaggtaa agtgaaatcg gccttggggc catgaatccc 3900
aggacagctt ggaaatggtt gtaggctccg agccacaaat tgagccacaa attcagcgga 3960
agcctcagag aggaagatta tgattcaaat acctctcact ctgagaagat tgtagggaat 4020
ccccttgaat cttatcccgg gccagtttga acaccttcaa catgtgggaa acattagatg 4080
aaggctccct tccaaggagg aaaacccccc tcaaagtgtg acatgaccag cctcctgcaa 4140
accccaggcc ataattccat tcaatgtaaa agacagagat ggtgaactta cctaccttcg 4200
ggggctatac atgctttgtg acttctctgt aaaggagaaa agtctgcaaa ccaacctgat 4260
tctaatcctg gtggccagac atttttcagt tatgccctta agcctaaact cctttccctc 4320

caccttgtct cacatttcca gcttctcttc cactttagaa acaaccctgt cactatcatt 4380

actctctttg gcccttcatc atggaataaa agcaaaatgc ccaagtcaaa agcagatcgc 4440

gaacggaaaa tcccttccat gtgcattttt agtctatctg tgaatggaga ttttaaaaat 4500

atgtatttca tgccacgtcg tctcagaatt ttgaagtttc ctgaagttca taggtccttc 4560

atgatatagc catggtaact taaatagctt tgtttggtcc tttaaccctc ctcaaatacc 4620

ctataccaag agccagagat atatcatgag ttctgaacat agatagcttt gcttcagaat 4680

tgaccgttgt tcacctctaa ccctaaagct ggctgctgct aacttcgaca agaatgtgtc 4740

tgaaatgttc tcctaggaga atcctagggg ataatcgtaa tagctaatat ttactgagga 4800

ctctataggc atcatgccat ttagtcaacc atagcttcta gtaagtctta tcacccaccc 4860

tattcaaata tgcacaatgt aaggctgaaa acgtttaagt agcttaccca ataccatgca 4920

gaaagtactt ggtggagtta aaaatcaaac ccagttccca gtcctgagag gctagttata 4980

accatttcag taaggaagtc atgctcagct ccctaattct attcatcttc tgacaagaga 5040

gagggctaga cacagcaaag ctaagaaaga cacaaaagct tgtctttttg gccctggtgt 5100

tctttgacag actggttaat tttgctatct ccagcttgct ccttcaggct tcacctcttg 5160

aaaaaaactg taattaaatg attagcatgt gacccataat gtaatacgtt ttctctacta 5220

gactttaagc tacatgaggg caaagcccga tccacatcac atccctgcaa ccagcctgag 5280

acctgaagca tagacacaca taagcaagtg ctgcccaagg gaagccatga gtgccctgca 5340

ggctgcctgg aatccccctg ctccaggttc caactgagct ctgcacgtcc cccactgtct 5400

gtgtcataat taccccctatg attgccttcc ctgccacact ggactgtaca ccactgaggt 5460

caagaatgtt agggaggcgg gagctttgtc tgctggagtt cattttcccc aaaaacggac 5520

ccaccatggg cagacgcacc attagcacag tccccaaagg agatgcccag gactgtggga 5580

tggtcttgtg aaggtcgatg cacagatatt gcagaggaat gctttaactt ctaggtaggt 5640

tactattttc ttaaatctcc ttagggaata cagaattttc aaactaaaag ggcttacggg 5700

cagcctctaa gccagaatct tgcagactgg attttaaaaa gaatttaaaa agatttgggg 5760

gctgctgtgg agagacaggg aggccaagca ggggattccc ctcaacctta tttcaatgaa 5820

agcccctcca cttatctatt ttgtacactg agcctcctca tgaaatttta tttgatgaaa 5880

gggctctgtg cagaaaaaac agaatgtttt tgtaaattgc tggcctaggt tagcatacct 5940

cattttaaag agggtgaaac tgagacatag agaagggaaa gatctttcta agatcttccg 6000

ccagttggca acagggtcag gttggaatcc taggtcttct gactcttgct cttggaagcc 6060

agggacaatc ttaaaagttt cagaattaag acattctttt agaaaagaaa aatgagcaga 6120

gtcttattct ctcaacagcc ttaactcaca cggaccgagc caaggatgac tagtatctcc 6180

gtattcatta tctattactg cataccaaat tactttaaat caacactcat ttattatctc 6240
atggtttcta gaggtctaag cacagcttag ttggattttc tgcctcatgg tctctcatga 6300
ggttattgtc tgtgttggac agggttgatg tctcatctaa aggctgaatt gggggatgat 6360
tagcttccaa gcttgttgat gtggttgttg gctggactcc atttcttgag agatattgaa 6420
ctgagggcct cagttccttc aaagctgttg gtccaaggct accctcagtt ccttgccaca 6480
tgggcttctc caacaaggcc atttacatta acaaagccaa tgagaaagag agttaccggg 6540
catggtcgct cacacctctt aatcccagca ctttgggagc ccgaggcggg tggatcactt 6600
gaggttagga gttcgagacc agcctggcca atatgctgaa accccgtatc tactaaaaat 6660
acaaaaaaat tagcctggca tggtggtggg cacctgtaat cccagctact caggaagctg 6720
aagcaggaga atcacttgaa cttgggagtc gtaggttgca gtgagccaag atcgagccat 6780
tccactccag cctgtgcaac aagagcgaga ttccatctca aaaaaaaaaa aaaaaaaaaa 6840
aaaaagagag agagagagaa agtctataag caagaggcac atcacaatcc tgtgtaacct 6900
aatcatggaa gggatattcc atcaccttag tcatattctg tttgttagaa gaaagtcact 6960
gggccatccc acactcaagg ggaggtaatt gcacacgtat gagaatgccg ggaggcagta 7020
tcaatggggc caccttagag tctgcctgcc acagcctcca gcactccagt gtcaccaatt 7080
tctggcaacc tcttatcaat gacaagttca ctcaacccctt cagtaccctc actgatgagt 7140
aataaaaaaa ttgatcgaga ccatggtgaa accccgtctc tactaaaaat acaaaaaatt 7200
agctgggtgt ggtggcgggc acctgtagtc ccagctactc aggaggctga ggcaggagac 7260
tggcgtgaac ctgggaggcg gagcttgcag ttagccgaga tcacaccact gcactccagc 7320
ctgggcaaca cagtgagact ctgtctcaaa aaaaaaaaaa aaaaaattct ggcagccact 7380
aaagtggact agtttttatt taacatcctc ccatgtatat agtggagcca agtgttttga 7440
ttagttgttt taactcattg gttagccata atttctcttg aatttcaccc cagggagggg 7500
cctttcacgg caatgattcc aaatgtattt tattctaaag caagacagtc agtgtgacca 7560
aaactaaaac tcatccattg ttcctccctc aatctattcc tctcagcaga tataatgaat 7620
agtaacatct tcgattaatt ctttttctt tactaacccc acacacaaaa tgttgtagtc 7680
tctactgcct tgacaagcaa atctgttcac tgcctcccca ctttcttgat caccaccccc 7740
tgccccacct ccattacccc tgtagttcag atcttagcat ctcacagatg ggccactgca 7800
acagctttcc cactggcccc cagcaaccca gccttcccct tcttcaacct gagacataga 7860
ggttaacatt cctgggcctc aagatttcca aatgcttttc ttctgcctgc aaaaaagtcc 7920
agatattctg acattcaaga ttctccatga tcttgcttct ggtctctaaa cagatcctgt 7980
acttcagctg tcattcatac tgttcacatc aaccctttta ttctttttat atccttgtaa 8040
atgccattcc ctcgtgccta gaatatcctt ttttatgttt ttcacctatt catatttttc 8100

tctcaaaatt cagttcaggc accacttcct ccaggtaaat tagatgccct tttactgtca 8160
tctcattcta cctatgtttc tttttttaaa tctatcatct cctttatcat cctggaatgg 8220
cctgtctgcc cctcttgaca atatagttcc taaaagaaaa aattgtatct ggtttatctg 8280
aaacctagca caactggaat aaagttaatc tttagcaatt aataggagtc cttgctgtgt 8340
cccagatttg gacatcctct tccttagcag tttgctcaga aagcccagag cttgaagttg 8400
ttttcacgct tcacccactg gcttgcaagt atgcagtgtg atgacacaac atgtgatagg 8460
aggaaaccat cccagcccta ggacaggaaa ctgtctcagg acacagacct caaggaatgt 8520
gtttgctttc agaggtggcc ttcaggataa aaacacaaat tgctttggat ttggaaaaac 8580
cagtaccaag gacagaggag tcctgtctgg ggatttgagg ctgagtcaca agagatagct 8640
gaaaatctgg aggttataac aaagattagt caacaactaa taggaggaaa gaacaggctc 8700
aagggtggga tggagacaaa gagctctcta tcttaggaca tggagaagct cacagcccac 8760
aagaagaaat gaataatggt taagagtatg actttggcat aagaatcctc tgggtttgag 8820
aggtatttgc ttgctatgtg atcttggcca agttacttag cttctttaag ctaatttctt 8880
cttatccata ttaaaagcaa agtagtagca cctagtttaa agtttattgt gatgattaaa 8940
tgatgtagtt catgaacact gcttagcaca gaaactggca ttcaatataa atgttttgtt 9000
gttgtcattg ttttgttacc tgttgatcaa atagttcttg atgataagat cccactagag 9060
aagcagttcc caaaagtgtg tcatgtggaa aagcattctc atgggaatca cctaaaaaag 9120
ggttttgtgg ccaaatgagt ttgggaaaca cttggtactc ttctgttctt ggaaatgcac 9180
agctcatatg tccattcaat acatagtttt agggctggtg atacaataga aagcaataca 9240
gtcaagatcc ttttattcat ggtgtttaca ttatggtggc atatcttagt ttgtacaagc 9300
tgctacaaca aaataccata aactgagtga cttacaaaca acagaaatta ctttctcaca 9360
gttctggagg ctgggaagtc caagatcaag atggcacctt atatgtcctc acatggcaga 9420
agacataagg cagctctatg ggtctctttt taataagggc acaaattcta tttatgaagc 9480
ctctgccctc atgatctaag tacctcccaa agactctgcc aactaatatt gtcacactgg 9540
tgattatgtt ccaatgtgtg gattttgagg gaacacaaac attcagacca tagcaggggg 9600
aaggcagaca ataaaaagga atcacataaa cttatagcac aatttcaggt agtgatattt 9660
gctaagaaga aagacaaaac aaggtaaggg cataaacaat gactggtggt gagaggagtg 9720
ctcttttaga tggggtgaga ggtgaatgtg ccattgtgca gatgtctgaa tgaaatgaga 9780
gaaggagctc tgtaggcaat ggctaggagt ttgaatttca ttctgagtgt ggttagaagc 9840
caatggatgg ttcagggaag tagagtgcat catctgagat atcttgctgt aattaatctg 9900
ttcaactttc gttagcccag tatctccaaa catacctgac cataggacat ctacctaaaa 9960

acatcttgca acgtagtctg ggaaattctt cactggagca caaggcttca tagattttaa 10020

agggactgca aatttgctct tgttcatcta gatctgcact aaaaatccaa acatactgct 10080

tgggacaaaa taggcatgcc caggaaggaa aacctagctc tagaggtaga gacaagcagg 10140

atgtcatcct aattagcagc tctttttggc aaagacaatc aatgagcaca gccaggcctc 10200

agcagattct ggaagcccat ttgatgccaa cggaaactcg ccaatatcta cccaaggcta 10260

ataagtgttt gcaaacagta caccttggct taagtattct ccaaaaagga agtacataaa 10320

acttgagatc cagccagagg ttctgggaga aaaacagagg tcaaatattt tggtgcttac 10380

atgtgcacag ccaccaaaga gctgctgaag gtctaggaaa tggaaagtga gttcatccac 10440

ttgaatgctg atctctttct ttatggagca gtaaaaattt aagtttttac cttatctgaa 10500

ctaagaaaat tcctttagga gtcagcccag tgtagctcaa tgtagttgaa agcagacatt 10560

ttagaaccac aaataaatca ggattcaaaa gctggctctg cctcttaagc cagtcatgga 10620

aactccctga gtctcaaatt aattttcaaa taagcataat aatacctaac tcaccaggtt 10680

gtcatgacaa atgacaagat aaatgatagc atgaataaaa agcatttagt gtaagatttg 10740

gcataaaaat gtatataata ggcactcaac aaaatgttag tttccttatt gttcaattca 10800

atctgtattt agtttcataa tagaagctgt catttattga ataactacta tattccagca 10860

gaggttagca aaccataggg tggcagccta tttttgtaaa taaagtttta ttggaacaca 10920

gccatgctca tttgtttgtg tattatctat agctgctgcc tctacaatag cagagttgaa 10980

tagttgtgac tgaaatcatc taactgcaaa gctttacata ttttctattt ggtcatttaa 11040

ggaaatgttt actgatccct tccagtcaac cttactaaat ttttatactt actttccagt 11100

caccttaacc taacaaggtg gatattatca tacttgtgta tatagagatt gaagcagaaa 11160

aaaaaaatca tctagtaagt tcccctccaa aaacataatg ccaaactcta tacctaaaag 11220

aagttcagaa taatgattaa aagttattca tttgtatgat tcatcttcat ttgcctctgc 11280

cttcctctga ctagtatttc tctctggcta atttcttgaa tttcctgcca tatgctgaat 11340

taaatgatcc tatttagaag gaattttccc tcctttcctt acttatacaa ataagttgtt 11400

tgttcataga agtccatgct ttcaaaactg aaagtggtaa tttccagccc ctctgaaacg 11460

ctctctgatc ttgactgatt tcttggaaac cacacgtttt ttagatagta catagtgtga 11520

agcccagttt tccagaaatg atagattttt agctccccca tctgctactt ctcactgtat 11580

cttcctctca cttattaacc ttctcttcag gtcagactca aggagagcag gcttttctcc 11640

aggggcttta aagatttgcc taagacacag atattatagt ccacctggcc tatgccctcc 11700

cctcaagttc acaacagtaa ataaaatagt tatgtaaacc ccaattccca aacaccatcc 11760

taccttgttc cattttgcaa atccaacaac tctattcata ttaatgatat tgtgtttctc 11820

tctgatagtt agtagataaa tattaattct ttaacttgaa caatgaatct tacttcaaag 11880

gtaacttgat atagcagaaa gagcatagac tttggaatta taagactgtt cttaattctg 11940
attcaaataa ttgctgacta ggccacttac ttaaccttct taagccttca gttctcttac 12000
cagtaaaata caaacaatgc tccctaattc ccagagttgg taacatgatt aaacagcatt 12060
tgtaaagtgc cacgcacagt ataaatattg acctacttgc ttcactctta gtttaaattg 12120
ctcaagatcc tctaacatag agcttcacat tttatcccag agaggtcata gttaggtata 12180
tctctacttc cttctaatac aggaagaaat gaaaggagta agggtctagg atttgttctc 12240
tatcagttct tcccccatcg tgtgccatcc aacctaatcc tgtgactcac ttcaaacaga 12300
ataatcaagt agatttggga aacactctat aaactgtccc cttcttcgag atttccaacg 12360
agcatgaacc tattaaacac cctggaaaaa cctgaagtaa agaaagttgt gtaatttgtt 12420
tggcttaaaa tcatatttgg tttaaaataa cattttccaa acgtgtttaa ttaggaaatc 12480
tttttccctc agattagtac ctatcaacgt gttgtagatt acactttaga aaatgctgtt 12540
taaagttaca cagctaagtt aaagccaagt taaggggggtt agacatatct ctatttttca 12600
gatttttctg cagtttttcc tgtcctgcct gtagatcata aagatatttc attaccagta 12660
gtttctctgg accccgacct tagttatgca aaccacccctt taacataatt aaaacaagtg 12720
aatcagttca cctgatttaa atttctgttc cccgtttcta gctatgtgat cttgagctgt 12780
gtgagcctca gttttctcct ccataaactg tgaaaaataa agacaactac ctcagaagtc 12840
ttggggagga ttcaatgaga taatccacag aagacacaaa aagcagtgtc tggcacagac 12900
gacatatgca atcaatgttc attattatta tagcatttag atggtcccat tttgggaaag 12960
ggaaaataga caatgaaagt atgtttgctt tgcctgaggt cacacaaatt aattacggga 13020
gtagtcttag ttttccctga gagtctggtt tcctgcttcc agtcctagag acgtattttt 13080
gtttcatgag tcatattcta tcaaaatgta tgagacttta ttgaacttga tttaaacagg 13140
attttgtaat tgtcaatggc tccatctggc tccttctgat gcaaatccca ctcaagcagg 13200
attccattca tttgaatatt agcacattgg atcgggaaag atttccaaag ctgtctagtt 13260
ttgctatgcc aaatatttgg tgtacacgtc aacactctcc actcctgtac ccatggtgga 13320
tattgctaat ccatcacagc acctttcccc gaaagcctaa tcatagccac agaatctttc 13380
tcaaggcagt tccaggtagc tatgtcagct ctttgagttg aaacataatt gatcccaaac 13440
caattctact tcttaaccag tgcttgaacc acctctacac tattactctt ttattttta 13500
atatgcccaa tgtcatggag ctctctcata aagggagaca ggaaaaggaa agaagaggag 13560
aagatatact aacatatgtg taacgtttat tattaatatg tcaggcactt atagctttgt 13620
tgtcccattc actgcctcaa acaaccctgg agagatttta tagttgaata agccacaatt 13680
cagaaggtat aggacgttgt ccaaggctcc ccagcagata agtagaaatt ggtcttaagt 13740

cttcctgact ctaaaggcaa tgcccctgtc tctcccatca caatgccttc ttaaaatttt 13800
tcagggtctt tcgggactaa atttctgact ttgggtggct gataacagtt tcacaaaggc 13860
aaccatctca cagaccccta ttcagaagtt ctcttattta gggttgtgtt gtaagtctgt 13920
ctgtaatcgg ttcttcctag ataaatctct tccatttctt aaccttggct caagactttg 13980
agaactcact attcaatgct tctgaaagag ccaactagat ttcttaaaat tctggatgaa 14040
gaccaaaata gaagaggtca ttgttccatt gggaaatgaa ttcaaattta tttttgaaat 14100
aattttccag aattttacag gaatctatat tgttgccact tgtccagaac cactttttat 14160
tttcttttct gtctaggcaa gaaatagaag agagaaagca agtgctctgc agttttattt 14220
caaaaattga aaccagacca caccagggca gtttgtggtc tgaatgtatc aatggatcta 14280
gtcatatttc atctgagcat ttcttaggta tactaaacca caaatatgaa tctcattatt 14340
gtgtctggat ggaaatattt tatggattaa gatttgtgga tttaatttgg ttggtaatgg 14400
aacttctgtt caaggggcag gagggaggaa gtcgtgaggc accaccaatg cttaagccaa 14460
actatggaaa gaaacactat ttggagggtt tgtacttaat gttggccagt tgacttgggt 14520
ctaaagtgaa attcaaaatt aagttcaaca aaaatattgt gaaattagca atggaccaca 14580
gatcactgga accaaaggga tcttagaatt ggaaaccata cacagttgct cattttacaa 14640
gagaaactga gacctcaaga gtggatgcga cttgcccaag gtcacacaat gaccctgttg 14700
gtcagacctg cttggcagaa gaggagctca gagcttctcc tccagatctt ccatttattg 14760
cacttcctgt tatatggcct cataagaggc caagtatctg ggaacttaaa aaaaagtaat 14820
gaaggtaaaa atttaaaatg agaaagggag acctatattt aacaacagta caacaaaatg 14880
ataaatactg caatgaaatt gaaagggtta ctaactaata acccaggatg cttccttcac 14940
accaaaacca aattttcatg ctgagtgcat cttaggcctg gccaattcca ccttctgcca 15000
aaggagttta cttgatcata gagaaatctt gtctcaaagt agaaagtact gagctcttaa 15060
aagcccttgg gttgatgtct agaatctcat gggaaggagc taggtctgtc tttgtcactt 15120
ttgtaccttc tgcacttagt gtggtgtctg gaacgtagta aatactcact gaataactga 15180
atgaattaaa acgaactaat tacagaactg aggctgtgac aaattccttg atacatctta 15240
ctgtagaaga gattacagga gagctaatta cattagggcc atcaggaaag taagagtatt 15300
tgttcattca ttaattccac aatataagaa cctgctatgt gtaagttcaa accaagaaat 15360
acattcagct tcatttcatt taatataaac atattgtgca tttactatgt tctagataaa 15420
gtgttttgtg ttgtgcattc agagatgaat gtctcagata tatccctgcc ctcatagagc 15480
ttatagtcta catacaatct aactgcacat atgcaaacca actgtttata gactaatata 15540
aatacaatct agaattagct aatttcctaa gagaaggagt tatgcatgca taataaagcc 15600
attccaaatc tttttttaaa tgataaatca aataatatca tatcaattcc ccagacaatt 15660

cagaagagag aaagataaat ttaggccaaa gttacctgat aaacctttca atagtagatt 15720
tctttaagca gcatcttgaa ggactgatag atttattcat gcagagatgg caaaaaggct 15780
tctagaagcc ttcaaggcat ctggaaccct cgctgaatga tcactgagaa atggaagcat 15840
cccaaatgta tctgtcagct tgcttcatct ccacagcagc cacaggctgt ctggcatctt 15900
aatggtctac ttctggagga ttgatagtat tctgctttga gccaggtgtt gggaagttat 15960
atagtcagag gttggggctg gtcttgactc atagctcctt tgcttggccc tccagcacaa 16020
aaggctgtat aaaatcatgg ttcagagccc tgactttgga ctcagatggg atttggttca 16080
aatcctgact ccataactta ataatgtctg aacttgcaca agccatgtaa agactctatg 16140
cctcagtttt ttcagctgca aaatgagggt acaaatacta tctcataatt gtatgtgaat 16200
ttgaaattgg aaatgtaaaa tgctaactaa gcacaggctc tggcaaaatc aacacttaat 16260
aaattatagt taatgatatc tgtattactt atgatggtga tgatgatgat gtcaacttcc 16320
tgattattat tattatgtca acttcttaat gatcttccaa ggccttttca accactattt 16380
ggtgattgta attctttcct tttctaggaa gccccccatg aacttcccag tgcaaagtgt 16440
cacataatta tctcattcag gctggcatta ataccagacc ttgcttgtgt atgatattct 16500
agtttctgag atctctagga acaaaaaata gctccttact tggcctattt tccaaagtac 16560
ccagcacatt gggaggtcca tactaagtaa atagcatttc gctcttgaaa gaatgttaac 16620
atttatactc ttgctattgg cttgggagag tgcaccatgt tcccaacctg cagtggcact 16680
taagaatatc acaaatacta aatcaactgt acatggagtt aaaatcaatt gcggccaggt 16740
gcagtggctc acgcctgtaa ccctagcatt ttgggaggca gaggtgggca gatcacctga 16800
ggtcaggagt tcgagaccag cctggtcaac atgatgaaat cctgtctcta ctaaaaatac 16860
aaaaagttag ctgagcgtgg tggtgggcac ctgtaatcac agctactcag gaggctgaga 16920
caagagaatc gcttgaacct ggaaggtaga agttgcaatg agctgagatc gcaccactgc 16980
actccagcct gggcaacaga gcgagacttc atctcaaaaa ataaataaat tgtattttgt 17040
gactctttgg gaggaaatta tgagctttgc gattatacag aacatgttta tcttctttgg 17100
tgttgtatcg tttatactta actcagggct tggcacgtat gtactgaaaa agtagatagt 17160
tggatgaatt gatggataga tagacagaaa agtaaaaaac tcatcagggc caagaagtct 17220
agaaatactt taacacaaat gctctccagg atcccaccac tgatatttct ctctggagat 17280
cattacaatg acctcagact aagaattact gaaagacttt catatgcatt tttaaaggtt 17340
tttttccccc agctttattg aggtataatt gacaaattaa aattaaaatt ttatacattt 17400
aaggtataca acgtgataat ttaatatatg tatacattgt gatatgatta ctagattcaa 17460
gctaattaac acatccatcg ctttacatag ttacctctgt gtgtcaatga taaagacact 17520

taaggtctac tactatctta gcaaatttga agtatacaat gcattattat taactgtagt 17580
caccatgccg tacatatgca ctcctttttg aaggcctccc aatggtctag aggaagagat 17640
agatcttaat cgaattcctt gacccaaagg caaaatctct ggattttaat atcagcttta 17700
ggagttaatt agtaaagtca ccactgatta gctagtgact ctgggtgttt caattcagtt 17760
attctcttaa ataactaggt attatggcaa ataagtttca tgtgatatac tacctctatt 17820
tatatactac ttaattggct gcctgaagtg atgatttaga atgattctta agtggggctc 17880
ctcagaattg tgttgttatc gattagctgt gtctgcccca tgcactagaa tgaagtgttt 17940
tgtgccacag acttgccatg cctataccaa atgatcttct acagttgctt ctaagtcctg 18000
ctcaagcatg attttgatcc tataatttga tgtctgtctg tgtctttgga aagtactgag 18060
aagctgggcc aaatgtttcc cgttaggaac acagtcttga aagactaaac cacaggcagt 18120
ctattcagta gaaagccctg gctcagcagc atggacagcc atcccaacag cagcactgcc 18180
cctgaggaac cttggccagg gtcctgacct cccagaaaag agccccacac caggctaatt 18240
catttgcttt tcttgctact gagttagaag agataggata acaggaaaac cagggctgta 18300
gccacagcct cctcattttc ctaaaaattt tagagtgtcc ctgctacttg acaaattgaa 18360
atactaagat ttatacattt ccatggaaaa agcaacagtg ggaaagagag ggcttcccag 18420
atttgtctta tagatctcat ccttcagaga ctagccttct gttagaaatg ctgtctccaa 18480
gcacaagaca gaataatcat ataataccaa tacacaccag ttgctaaggt ctccatcctt 18540
ttaagtattt gttactgagt gttttgcctg tattatctca tttttctcac tttcagttga 18600
aagaacttta ttgtgcagtt gatattagtg ttaccatttt acagatgtga aaattaaggc 18660
ccagacaggc aatgttacac aaccaaaaca gctactggat ggaacccttt ggatttgcta 18720
ctacccaact atttctgacc taaaagagtg gtgattccat gtggttcaaa ttaatagcaa 18780
gtggcagagt aaagatctaa cagtgtttga ctgatgtaga gcagatatcc tcaaacattg 18840
cacagatgtt tcttggatat ttgtccatgt tagtatttat cattcttcac aacaagtatc 18900
tgtttacatg tctgctttct atacctgact gtgagctcat taagggcaag acctatccat 18960
cattggcttc tgaaacaccc caccaccact aggctatccc atagcccgtt cttacacttg 19020
ttcaacacat ctctgttgaa tgcaagaata aaccatttag aaattaattc tcagtcctct 19080
tggataatca ctggctcccc cagcatctta actgggcacc agctaagcca ctgtgctgcc 19140
taaatggaca accacttctc agttctggaa ttcttcaaac acctagggtt aggagggtct 19200
tcgctttggc agttagagtt ttctcaattc ttttatgaca caggttgcac cggactcact 19260
caactatgga gatcagatgt cctcaggaaa aaaaaaaaaa aaaaaataca cacttaaggc 19320
tgtttcctgg ctcctctctc ttcctgacac attccaaaaa ggaatttatg gatgttggga 19380
aggacaaagg gtgaggaatt tctaggacat ttaagaacgc ccatgtaagc aactctatgc 19440

ttgacaggcc atgcacaata aaactctttg gcctgtaagc gagaagaaaa atttggtgta 19500
agctgcctca tgggttgcca aaatgatgtg gaaacaaatt catctgcatt tctaaacaat 19560
aggaattctc ctcgtcctcc caaacctaga atatttcatg tatgtgctta agtcaacaag 19620
ataaatatat tccatgagct actaaaaatg aaatcataag taactaattc tgaaatatga 19680
ttctagccag ttggaagttt catctttatc taaaaatgaa gttgtttatt ctccagctgt 19740
gaccagggaa aaaatcagat tgttgatttc ttcgtctagc aatgatcttt gtcctgcagt 19800
gaggtgccca aaaataactg ttagcctatt ccctgctgct tccaaaaggt tagctaccaa 19860
acagctctac tagtaaaaga gaaaaaaaga aggatttgac agcaagccaa gaggcagctt 19920
aatgtgtaaa attatgagct aaaaagccat tacgactggc atgacactgg ccttggtttc 19980
ccaacctggt aaataaattt ttatctcaca gtaatgctct gaataaaagt ctataatgcc 20040
aaaatctgaa gaaatgattc aacactgata acatttaaca ccaaggcaat tgaaataaga 20100
ttaaagctta acatttaacc cttcttccac cccacactcc ccacaaaagc tttcaggacc 20160
attgttttta tcttcttatt ttttgtagcg ggctggggcc ataaatgtac caggtatttg 20220
gagaatgggg gactaaatat taaagcctat attctaccca gccttctcaa cagatgacta 20280
actgtgagca gttaggcacc tagccttaca gaggtgccat attgcttact gagttaagta 20340
ccagctcttc atattgatct tttatatcct tcccaatatg gcttctgcca ataccatgta 20400
gtctctacct ttctgctttc ctatgcatta ttttataccc tagtagaaca agacctctcc 20460
atattccctg catgctacac caccttggca tgtatctggc acccacaagg agatgccact 20520
attattacag ctatcttctt aattctcctc accacccatg cattccaact tctttgcctt 20580
ttctcatggt gtttcttttt tctagggtgc tgtaggcttc aatttgaacc cacctactgg 20640
agttgtatcc aaccattttt cagaatcttg cccatctttc aagtacatct caaataccac 20700
ttttttgaga tgtctttctt gggaccttca atggatatga gttggccaat ctcttttgtt 20760
acttgttata atcatgaatg tatctccctg attccccccat cccttctgga tacaaggttt 20820
tctttctgat ctattctcta ttggatcagt gttgttatta ccgctgctgc tatcattagc 20880
taccacatat taagcacacc ttaatgtgtg ccaggcactg tgccagtagc ttaacatatg 20940
tcttctcatt tcatcccact ctcctgcaac actatgagat agacatatgt atctccaggg 21000
agaggaggcg gggaaattga ggttcagagg agaaagggaa tggccatagg gagcagctgc 21060
taaagtgata gaactggatt cgaacacagg tttattttac accaaagacc agactctttc 21120
ccccacacgc aactgcttca ttaatggaaa tgtttagatg gctgaaggat atggactgag 21180
tgccctcaaa actctggagc acagcagtgc aggacagcag agaaacggga gtcatgagtt 21240
gtctttgaca agctcctcca gcttctaagg agaggctgaa gcagacagaa agagacatta 21300

gacttggcca gagtggttcc tggagcaact tggttttagt ttaacgtggg gagaagattc 21360

ttacgagact ctaaggtgtg cagagggagg aagaaaaaaa tatttcttaa cccaaggaca 21420

aggctggggg aaaagatgct gatctgacac agggtagtta tctcttcaga tctgcaaagg 21480

acaatagcat tgaggattgc ttctcacttg tacctggata tgttcaaaca tgtaaggtat 21540

ggaaactaca atgcacatac atgcttgcta tagttaagtg agttagttgc taaaaagaag 21600

ccattttttc tccctaatcc ccgggctctt gtgtctgagg tactcaccca ttttcattag 21660

tcaagaaact taacaacatt gaaactttct tatagactct tcttgatcgc tttgagtctc 21720

agtttcctca ttttgtacca tgcgcttagt aaaaacaagc aatcctctaa accagtactg 21780

ctcaataaaa cttctttgga tgatagagat gttcttcatc tgcactgtct catattgtaa 21840

ccacaagccc catgggctct taagtacttg aaatgtgagt gatacaactg agcaatgaga 21900

tttttaattg tatttaattt tcattaattt taataatcac atgcggaata tgggacagag 21960

cacttcgaat tcaagaccaa tgaattcgaa gtgttttatc tctttttaaa ttttctatct 22020

cacggggaag tttttattgt ggattttatg ttcaccagat taactagctt ttgagacatt 22080

gacagggatg gagatacaca gaaaatctca caaaaacaat ttcataagtc acaggtattt 22140

gtttgactct aaaaattaat tttaataaag aaagcaaagt ctgacacaag tgtgtcttgt 22200

tttgcgttag atacataagc tgaggcggag caaaaagcag cacaacatgc ttagcttgga 22260

aatagatttg tcctggatat gggaccccaa aaatctgcgt tttgtgccaa tgcaaagact 22320

atgtctcttc ctgattccct tctttgcaat cattgactta ccagatcctt aaatatcacc 22380

atcaaagata tacacacact tacatatata atatgatatg taataatata ttatctttca 22440

ttgtattata atatgtaata ttatgttaca tcacatatta taatattata tgtaatataa 22500

ttggagcaac tattatatta tatatggaga tatatatata catatatata catatatata 22560

tacatatata tacatatata tatacatata tatacatata tatatacata tatatacata 22620

tatatataca tatatacata tatatataca tatatataca tatatataca tatatataca 22680

tatatataca tatatataca tatatataca tatatatata catatatata catatatata 22740

tacatatata tacatatata tatacatata tatacatata tatatacata tatatataca 22800

cataccacga catgcatatt ttctgggatc actgagagag gaaaagttct cactttgctg 22860

tagaactctt gtctttgctt aaaaacagac atatgccaaa gtaggggttt caaaggttag 22920

gagaaagaaa atcctagaaa agaaaaacat tcattaaagt ccatattttt cattacaaat 22980

gtttcctttt attaagctct tactgctacc aaagactgtt tgccatttct ttcgcataca 23040

ttatctcatg cacttttcac aatgatccta tgaggactcc cttaacacat ttattgatga 23100

cagtactctg gcttatggtc acagataata accttgcaca aggaacagga agtgatcaaa 23160

cagtgattca aacctaagac aatttgccta taaaacttgt gcccttcaat gccattctat 23220

ttacattctg gtgctttgga acctgctaca ttctcaaaga agacctttct attgggtacc 23280

aattccaact agaaaagtct ttctcctgtt gtgctgaaat ttgtcttctc ataaccaacc 23340

ctccctccct gcatcatccc cagcacataa acgcacacac acacacacac acacacacac 23400

acacacacac acacacacac acacacagag gcacttgttc tgtccttagg ggctacaaga 23460

ggcaagccag tgcctgcttt gccaggattt ctacatggta accctgaccc actcaacctt 23520

ggttttctca actacaaaat aaacctgccc aggtctgtag tccttcctcc tacaagatat 23580

tttccagacc ttttattgct gatcaatttt ctataaagat tagaatctgt gttttctaaa 23640

gagtctctgt agaataagtt gctcattctg aaattataaa agtcctacta tcagaacatg 23700

attcttcatg tctgacctaa atacctccaa ctgcaatctc aagtaagttt ccctgggttc 23760

atccacaaag gagacttaaa acagccagtt gtctgcatga gaatatttta cattcttaaa 23820

ggtcattatt tgcttcctat tttttccctc tctcatataa atatttctag ttctagtaaa 23880

tttacctcct cacccaccag tgatttctgc agctcctctt tgatctagat ccaagttgct 23940

gctgagttta ggactgtatt caactaggcg gtcaagctca ggcttaagta aaaggaggct 24000

attgacatgg actcttgtta ggttttgttc cccaggagcg caacaaaata ggaactactg 24060

tttaatgagt gcctactacg agcttgacac catgctgggt actttatgta caggtctcta 24120

atccttaatt ttctctggaa aggttttgac tcctttgtta tatatgaggt catggtttag 24180

agaggataag tagcttgtca aagttgcagg gctagtgagt gacatttgaa cccaggtatg 24240

cctaattcca aagcctaacc attttctaca acactgaaaa tataatggat ttttcccaaa 24300

gtacaaaaac acccactgcc ctcagaccac catgagccaa taggaaatac acaatgctca 24360

agaataacat actctttccc ccaacgatgc aacatttggt ggtaccagtt tacgtctctt 24420

aaacacacag tgagaaatcc tcactctact cacactctgg tcattattac cctaaactct 24480

ttgccttgga gtctcaatca gcaactataa tggcttttta gatggaaatt taagtcattt 24540

agcaacacac agaaaagtgt tggggagaag tccaaaaaca tttcaaactt ggcccatggc 24600

cctttctaaa aaaaaaaaaa aaaagctaag aattgatgga aaaggggtag gaaagtagaa 24660

ctttgtgaag gtgaagagag ttagagcagc cattctagta gactatgcag ccaataaata 24720

tttgtccagt aaacacataa gatggccaaa ttggtttgaa aggagagaac atgccaagtc 24780

tcctttttta taataatctt cattcagtgg ccttacctac attacagacc cacacattgt 24840

tagttaagaa gctaatttca atgagaaagc atgctcaaaa gagggttggg tatttttttgt 24900

ttctttgttt ttgttgttgt tgttgttttt gttttgtttt gtttttgcag atattacagg 24960

gacagttggt aaagtgtcaa taagaatata attagaatgt ggtaggtatg tggatacgac 25020

cttcttagtt aaattcacta gtgctttctc agtccttagg cccctgatca gtcatataga 25080

atgcttccca gttcaatttt ccttgccaca attcctctct aagttttgca aaggctttct 25140
gtcatgagga aggagatggt cagttgggat catgttgatg cattctatca ttaggataaa 25200
atctttccag aaaaaaagtt agaaaccttc ttataggctg aataatccaa gcaccaccac 25260
taattgacat aaataactaa cttacagtgt gcgagtataa gaatatcatg ggcataggga 25320
aatgtcaatt tgtaaatgac atgctaaaaa ccaaatagca gccaggcacg gtggctcatg 25380
cctgtaatcc tagcactttg ggaagttgag gcaagtggat tgcctgagct caggagttcg 25440
ggaccagcct ggggaacatg gtgaaactcc aactctacta aaaaaaaaaa aaaaatacaa 25500
aaaatcagcc aggcatggca gtgtgcacct gaaatcccag ctacttggga ggctgaggca 25560
ggaggatcac ttgaacctgg gaggcggaag ttgcagagag tcaagatcat gccattgcac 25620
tccagcctgg acaacagagt gagactctgt ctcaaaaata aataaataaa taaaatagct 25680
cttcaattat catttgaatt ttactcgggt gcatctccta attaaagaaa accatgaatg 25740
acttcatatc gtgtatagat cattctcaca tgcctccata gcattgtttt agtaaatatt 25800
ataactgcca tttcttcctg tctttattta gtttctcaac tgcctctaac aagtcccctt 25860
ttctctgcta cctgattgct catacaggtg aatagctcta agagagatga ataagataac 25920
ttggcaaagt gagaattgct tcatggtagc atattctcct ccatattctg ttcccatcta 25980
attcttctca ctatttaagg ctcagcttaa attttaccct ccctttccca taagctatgc 26040
cccacctttc tcttgaccat tcctttttat tttcaaaccc cataggcagt tatacactat 26100
accacaaaac aaatcctata cattctttct tgctttgctc cttaagtact ggatggatga 26160
ttccattttc ttcttaattc tattgttaag tcccctgatg gtatgtatac acttggtctt 26220
atatttattt ctaccaccct cagacctctg taaaaatgct ataagtactt gctgattgga 26280
gaagacatca gaggctctta gacatccttt ctgagctgtg agcctcagtt tagacaactg 26340
gttcaagaat attctgtcag gctcttctaa aaactggact tttgaaggtt ttggaaataa 26400
atcaaggagc agggaaactg aggtgcagag gagtagactg aatgctatac tgacagtcat 26460
ataactagta agaagctcgg ctgggattaa aactgtttgc ctcctagagt cctttatttt 26520
aacctctgcc tctcatgaca gaagagaacc agcagatggt gtggaaatca gttttgaaaa 26580
tcaataagag gcaagagtta ctgaacatct accgggacca gggcctttat aacactataa 26640
taattcactc agaagacata gtaaatactg aaggaattaa tgaatacaca aataacttct 26700
acatcattct tttaaccctg tcattagcaa ctatgatctt cactatacag aaagtgaaat 26760
aaggcaaaga aaaattacat aacgtatcca agggaccaga gctacagcca agatacaaac 26820
ctatgtctat tttatttcaa tattttacct ttttctctct atttttctgt ctccttaaat 26880
ttacagaagt tgaatagtga gtgaaatcat tccttcaagt gacaaagttc ttttagagac 26940
cattgtatgt tatttcaagg gtatataatg aaaaattatc aggagagctt caaatacagg 27000

gatataaaat caaaattcac ttggaattgt agatattcaa tatattcaat agtagatact 27060
cataattttt ttgttcatct ttgcttgtag aaaataattc tttcaagaag atcagggaca 27120
actgatttga agtctactct gtgcttctaa atccccaatt ctgctgaaag tgagataccc 27180
tagagcccta gagccccagc agcacccagc caaacccacc tccaccatgg gggccatgac 27240
tcagctgttg gcaggtgtct ttcttgcttt ccttgccctc gctaccgaag gtggggtcct 27300
caagaaagtc atccggcaca agcgacagag tggggtgaac gccaccctgc cagaagagaa 27360
ccagccagtg gtgtttaacc acgtttacaa catcaagctg ccagtgggat cccagtgttc 27420
ggtggatctg gagtcagcca gtggggagaa agacctggca ccgccttcag agcccagcga 27480
aagctttcag gagcacacag tggatgggga aaaccagatt gtcttcacac atcgcatcaa 27540
catccccgc cgggcctgtg gctgtgccgc agcccctgat gttaaggagc tgctgagcag 27600
actggaggag ctggagaacc tggtgtcttc cctgagggag caatgtactg caggagcagg 27660
ctgctgtctc cagcctgcca caggtatgag ctggtggccc gcccagtccc acactgtgca 27720
aacatggaga aatgaagagc atagcagcat ccatcacttg cttccaaagt gggctcacta 27780
gaaaactagc ttcccagagt gttcattaca aagagatttt atgttcaaat aagtgtggag 27840
actgcagcca tctctctcct gcttggagga gtcacggcga caactgcttc tcagaggaat 27900
tttcaggctt ctgataagtc ctgctttgaa aaaccctatt ttaccttaat tataagtttc 27960
taaaacgtat ttgaccatga aatctaccca ctttctaccc caaaacctat gagtacgcca 28020
tgaaatttct accccagaga gcactctctg ataggtactg ctgtggcccc atcacctcat 28080
tttacaaata gagaaactga ggcctggaaa gaggatggga ttcctgaagg tcctccaata 28140
aggagtagag ttcctcacat ttcagtattc ttacaaatgc acagtgctgc ctgtgatagt 28200
gctgagttat aagaattaga aaaaagttcc aatttaatag caattacaat gattacaatc 28260
atataagctc cctctgtgta acaggcactg ttgtattatc tcatttaatc ctcccaacag 28320
ccctttaagg taaagattag atgaggggaa gataagtcct cttctacaga tcaaagaaat 28380
gaatctcaag ggattaacc cactggcaca agatggcaca aagagcaata agagataggg 28440
tctttggcta ggcgcagtgg ttcacgcctg taatcccagc actttgggag gctaaggcgg 28500
gcggatcacc tgaagttagg agttcaagac cagcctggcc aacatggtga aaccccatct 28560
ctactaaaaa tacaaaatta gcagggtgtg gtggtgcatg cctgtaatcc cagctactca 28620
ggaggctgag gcaggagaat cacttgaaac cgaggggcag aggttgcagt gagctgagat 28680
catgccattg cactccagcc tgagtgacaa gagcaaaaca ccatccgaaa aaaaaagaga 28740
tagagagaga gagagagaga gagagatagg gattttgcat gtgcctgtgt gtctccagag 28800
ctcatactct cccatggatg ccctgtttcc acatgcatcc tatttcaagg aattttgctg 28860

ctttggaatt atgtctttat ctccaataac taaagttgat aaagctacat gagtttcaac 28920
aacttcaaat cagagctgaa aatctgtttc tgccactcag aaaaaaatta ccaaaatgtt 28980
cttcaacaat tatttcaaga gtaaacctaa ccaccctaag tgaaatagtc ttacggctct 29040
gcaggacagg ctctaggttt ctaaggcaat ttttaaaaac attttcttta ggcataagtt 29100
agtttatctt ccacttctgt caactccctg gaaaaatgca gatgagaata cacagtagga 29160
gtaatgctaa gtagtcagtt aaaatgattg tttagagcag tgctgtctaa tgaaaatatg 29220
actcacacat gtaatcttaa attttctagt agtcatgtta aagaaaggag aaagaaacaa 29280
gtaaacataa ttttagtcat ctcttattta attcaacaga gccaaaatat tggtttttca 29340
gcctgaaatc aatatcaaaa ttattaatgg atacttctca gtctattatt ttatactatt 29400
tggaatctga tgtgtatttt acacttacag aacatctcaa tttagactag ccacgtttca 29460
atgcctaata accacatgtg gccaagggcc accatactag atagtacata cttagactct 29520
cactttacaa atcagctttt tttaaaaaat tctttcttcc tgctgtcaag aatgagataa 29580
aatatattaa tacaagctgt ttctacgtac agagactaca gagtgaatat actctagtgc 29640
agagaatata gaactgggtt ttgtttcttc tccttaagct ccatggagat cagaccaatg 29700
acttctgcaa actgagctaa ggcaatattt aggcctcaag gggctcgagt aatgtcaacc 29760
ttgaccccac attctttaag ttgacctcca cttagccttc aggatagaaa gaaataaaag 29820
ataaaggcac tatacttttc tggggacaga gccaaactga aaggtgtcct tatagaatta 29880
gaaagccatt agctacctca ttttagttgc ggaaaattag aatgttttgg ttatataaac 29940
ccacatacca acttgagcca accttccaca aaaaactttg gaaaataaaa cagaaaaaaa 30000
taacaaggac acaagagtga gctcttaagg ggtttactta actcggatat accccaggga 30060
agtgggctat tggcccaact tcacgtataa gaaaactaag ctccagatag tctaaatccc 30120
ttgccaaatg tggcatagat attgaatcaa aaagtcagaa ttacattcag ttcaacaact 30180
atttcttggg catcttttat gtgcctggcc ttgatgaggc actaaagaaa taataacata 30240
gtggccagtc gtggtggctc acacctgtaa tcccagcact ttgggaggcc aaggcaggca 30300
gatcacgagg tcaggatatt gagaccatcc tggctaacat ggtgaaaccc catctctaca 30360
aaaaatgcaa aaaaaattag ccgggcatgg tggcgggcgc ctggagtccc agctactcag 30420
gaggctgagg caggagaatg gcatgaaccc aggaggcgga gcttgcagtg agccaagatg 30480
gcgccgctgc actccagcct gggcgacaga gcaagactct gtctcaaaaa aaaaaaaaaa 30540
aaaaaagaaa agaaagaaat agtaacatag ccccagccta atgcatttca caaggcagtg 30600
actcataatc atcataggaa tagtaagact tcttgaaaat gtgacaaaaa ctatgaatcc 30660
gctccccagg ggtggaaata cacacacaca cacacacaca cacacacaca cacacacccc 30720
tatgcataat cacacactgt tttggcatac aagcctataa tttcagggaa ttcaggttat 30780

gaattcctac ctggtataac aggatgtgag tctcatctga tttcacacac tacaacaaat 30840
ggttctcaag ccagatggag cctgagggtg caaccgtcct tcagctgaat ttgaatctgg 30900
gtgccctggg tagatacaga aaaatgtggc tgcttaagcc tggctgagaa cttctgtttc 30960
ttttgttgca ggccgcttgg acaccaggcc cttctgtagc ggtcggggca acttcagcac 31020
tgaaggatgt ggctgtgtct gcgaacctgg ctggaaaggc cccaactgct ctgagcccga 31080
atgtccaggc aactgtcacc ttcgaggccg gtgcattgat gggcagtgca tctgtgacga 31140
cggcttcacg ggcgaggact gcagccagct ggcttgcccc agcgactgca atgaccaggg 31200
caagtgcgta aatggagtct gcatctgttt cgaaggctac gccggggctg actgcagccg 31260
tgaaatctgc ccagtgccct gcagtgagga gcacggcaca tgtgtagatg gcttgtgtgt 31320
gtgccacgat ggctttgcag gcgatgactg caacaagcct ctgtgtctca acaattgcta 31380
caaccgtgga cgatgcgtgg agaatgagtg cgtgtgtgat gagggtttca cgggcgaaga 31440
ctgcagtgag ctcatctgcc ccaatgactg cttcgaccgg ggccgctgca tcaatggcac 31500
ctgctactgc gaagaaggct tcacaggtga agactgcggg aaacccacct gcccacatgc 31560
ctgccacacc cagggccggt gtgaggaggg gcagtgtgta tgtgatgagg gctttgccgg 31620
tgtggactgc agcgagaaga ggtgtcctgc tgactgtcac aatcgtggcc gctgtgtaga 31680
cgggcggtgt gagtgtgatg atggtttcac tggagctgac tgtggggagc tcaagtgtcc 31740
caatggctgc agtggccatg gccgctgtgt caatgggcag tgtgtgtgtg atgagggcta 31800
tactggggag gactgcagcc agctacggtg ccccaatgac tgtcacagtc ggggccgctg 31860
tgtcgagggc aaatgtgtat gtgagcaagg cttcaagggc tatgactgca gtgacatgag 31920
ctgccctaat gactgtcacc agcacggccg ctgtgtgaat ggcatgtgtg tttgtgatga 31980
cggctacaca ggggaagact gccgggatcg ccaatgcccc agggactgca gcaacagggg 32040
cctctgtgtg gacggacagt gcgtctgtga ggacggcttc accggccctg actgtgcaga 32100
actctcctgt ccaaatgact gccatggcca gggtcgctgt gtgaatgggc agtgcgtgtg 32160
ccatgaagga tttatgggca aagactgcaa ggagcaaaga tgtcccagtg actgtcatgg 32220
ccagggccgc tgcgtggacg gccagtgcat ctgccacgag ggcttcacag gcctggactg 32280
tggccagcac tcctgcccca gtgactgcaa caacttagga caatgcgtct cgggccgctg 32340
catctgcaac gagggctaca gcggagaaga ctgctcagag ggtgagtgcc agggcgcaga 32400
ccagcatata atggccatga tggagtggac tcctagtatg ggttggaaaa ctacgtttat 32460
atatataaac attcatgcac actgattctt tgtacgtatt aatatatcca tttaaacaca 32520
cacccataat gactttttaa gggggccctg tcattccgtt gttacaaggt gacctattta 32580
agggctgaag ggctcagaga ggttatataa ttgtcccagg gacacacagc tgacacatca 32640

cagagctggt attcaagtct gcctaactca tctacataga gtttaaaata aagttggaga 32700
ggccagaaca catctagtgt tcaagatgag gtagataaag ggatactctc tcttttgcta 32760
aatatcaact ttatttccta ctctccactg acatcaaacc aacagtaaac aaaatctctg 32820
tatttggatg gatgtaaaaa ttcagctaaa ttgaatacgc agtgagatta cataaaaaca 32880
actgtttcaa atgcatactt ggctaaagac gaaatgccta ttatctacaa gattgacctg 32940
tcttttcatt gtcttttgta actggttttg ttttttttgat tgttttgttt ttttaatcct 33000
aaagactgca agctcctatc cttaaaaggg cactatttct agcagttagt atgggacctg 33060
gtacatatta agtgcttagt aaaccctggt taagtgaaaa cacaggagag aaaattaatg 33120
ctcatattta atgagtgtct tctttgtact cctctcatct acccatatta tcattgtcat 33180
aatggagaat tttttccttt actaaagtta ttggtatcta aacataaata aatggctttg 33240
tgttcaatgt ggcgcaagca gggaagcctg gttggaaaga acaccaggct ggcagggagg 33300
agatacaagt tctaaacttg atgaggtgag caagcttggt caaggcgttc tgtcttactc 33360
acctctataa tataagaaag ttggaccaga tatgcccaaa ataccctccc agtgctaggg 33420
acacgtaact caaagttcag agcagggagt gaggctcaga ctgcactttc ccatgccaac 33480
agcctgcatc tgagctaaga cccaggaagc acagaaagtt ggagaggcca gaaacaccca 33540
agggcacact gctgacctgg gactccagcc tgaaggaaat ggactaagga acagccacca 33600
caggtcttaa gatggatcac agcagggggt ccctgatgcc atctgcattt tagaggcaac 33660
agaagatctt cagacctctg tggctgacag tggaaaagac aggtcaaaca aacctagaga 33720
gattttagag gacacactgt tgacaccaga tgtcctttct tattcctgcc agtgtctcct 33780
cccaaagacc tcgttgtgac agaagtgacg gaagagacgg tcaacctggc ctgggacaat 33840
gagatgcggg tcacagagta ccttgtcgtg tacacgccca cccacgaggg tggtctggaa 33900
atgcagttcc gtgtgcctgg ggaccagacg tccaccatca tccaggagct ggagcctggt 33960
gtggagtact ttatccgtgt atttgccatc ctggagaaca agaagagcat tcctgtcagc 34020
gccagggtgg ccacgtgtga gtgagcagaa ctcttttggg cagcctcacc tgatgtcagc 34080
ccacccagtg tataaccctc cctcgccacg gcccctacaa tcctatagtt gttctttaca 34140
gttataccaa ataagagcct agaattaagt ccatagaatc acttattaaa gaaatccctc 34200
attttccatc aaatctttct ccttgctctc ctatatactt cctgccagag aaaatcatta 34260
caagtaacat ttagcaagtg tttactgtgt atcagacttt gttgtaagca gtctacatat 34320
tagtataaga gtcctcattt ggccgggcat ggtggctcat gcccgtaatc ctagcacttt 34380
gggaggccaa agtaggtgga tcacctgggg tcacgagttc aagaccagcc tggccagcat 34440
ggcgaaaccc tgtctctact aaaaatacaa aaattagtcg ggcctggtgg tgcatgcctg 34500
taatcccggc tactcaggag gctgaggcag agaatagctt gaaaccagga ggcagagttt 34560

gtagtgagct gagatcatgc cactgtactc cagcctgggt gacagattga gactctgtct 34620
caagaaaaaa aaaaaaaaaa aagagtcctc attatatgag ttagtatatg tatgaaatag 34680
gagctattgt taaccccatt ttacagatga ggaaactgag actgcatggt taagcatctt 34740
atcccaagtc atgcagctaa caaggagtag aggcaaaact ttaaccctgg cagtcctggc 34800
aggtttatgt tctaaaccac tctattactt ttcagtggag atcactgact atttttcttt 34860
aagtggaaac attctggttt atgagtttag catcagtata attaccttca tatctaaaaa 34920
gcaagctctg gctagaaatt gacccttttt tccttaataa taacattggc tgccatttat 34980
tcaacttgtt ctgcatgcca cgaaccaggc ccagcagtct acacactgga gtggagtata 35040
tcactcactc ctcaatgcat tcccaagaag tagacatatt taccttcttt ttccaagtga 35100
agaaactcag gcttagagca ctgtgtgcaa ttgctcaagt tcacattgat agtagagctt 35160
agattctaac cccaagtctg tctgattcca aagcctgcgt tcattttaga tttaaacata 35220
tgaaattgcc aaaattaatc ataccatttt tcctaatatt aatttttata cagcctcata 35280
gagacctcat cccagaagaa gctttaaaag gacacctgct ctggttgtca gcccctgact 35340
gttgcagaca tccagtctgg tttagtggaa tcgtggccgc gttgcccaat cacacacctg 35400
ccctatcctt acgttctatt tatgttgtta cttacagact tacctgcacc tgaaggcctg 35460
aaattcaagt ccatcaagga gacatctgtg gaagtggagt gggatcctct agacattgct 35520
tttgaaacct gggagatcat cttccggaat atggtaagga gtacaaaaga gcagatccat 35580
ttgatctcaa ggatttgaaa gatgaccaaa gggttctgaa gacttctgta tcaactaatt 35640
ttccttagtt tttggtggag gacaaggctc tcattagcat catttgtgtt tgcatctata 35700
ttttaaggga tgttggtttt tccctgattt taataagtaa gtgtagcact tacggctgac 35760
accctagaca aatagaagtt gtcacctcaa ggttccaatt gtccatcatc accttagaaa 35820
tctctactca gaggcaaaga agagaagaaa cagtctctcc ctcaaaataa aggtacatct 35880
cagatttgag caacccttct ctccaggccc aaaagtacat gtgaagcagg gaacttgttc 35940
tctgaattta agcccaggct ctgccaccaa ccagctgtgt gactatccaa caatcaccct 36000
actcctctga tgctggcctc agtctaccca aggagggatt gagcttgtgc cttcagaatt 36060
cctttggctc tcacattctg tcatttgact tgcatgacaa aggggtaaaa gtttacgaat 36120
gggagaaaca aactagagaa atgtctttat catctctgta tcctcagtag cttacatact 36180
gatctgatgc atagtaagtg cctaatgaat cgatgaaaga ataacagagg aatgaatgaa 36240
tgcagagtga atgaatcaca taaattaaga gactggcacc tcccctaact cttaccaagc 36300
tgcctctatc tctcagaata aagaagatga gggagagatc accaaaagcc tgaggaggcc 36360
agagacctct taccggcaaa ctggtctagc tcctgggcaa gagtatgaga tatctctgca 36420

catagtgaaa aacaataccc ggggccctgg cctgaagagg gtgaccacca cacgtgagta 36480
tcacctttaa tacttataga atgataaggc tgattgtacc tcatagcacc tcctggttga 36540
gaaagtgcct tctcatatag catctacatc atttaacttt tgctgcattt ttaaaaatcc 36600
agatacagtg gtttaaaata acaattcttt gcatgttggt gatttgggtt tgggctcagc 36660
tgagcagctt atctgttctt gactgcactc ctcatgtatc tgtgtgctta aggggtggat 36720
tggcctagga tggcctcagc caggatggtg tctttctgtt tcgcagagcc tctcattctc 36780
caggatgctg gtcctgttca cacagctatt gaacaggttc aaaattggca ttagatcact 36840
tctatcaaag cacatcccac ggccagttta tatttaagga atagagaaac agatccatct 36900
cttgatgaaa ggagtttcaa aatctccttt caaaggggtg tgactacctg aagataatta 36960
agcccattgc taacaatcta attagcatca tgtcactagt ttaatattag cacccagttg 37020
taggacagag gaagggaaaa tattaatgtt aaggcttaaa gaagctaaat cactttaaca 37080
tcccatagtt aacaccatca ggggcataac tttaagccag ggtaagtttt ataactttaa 37140
ccccatttcc actaacattg actaggctct ggtttatgta gaatgttgat tccatctctt 37200
caatgggaac ctaaataaaa tttcattcaa tactaatgat gtcagaagtt cttgggagta 37260
gaataggatt tagcagtact agatcatttg gtgagaaaaa gacctgaatt tggagttgct 37320
ccaccaggaa acattttttt tttttttttc tgtcacccag gctggagtgc agtggaacga 37380
tctcagttca ctgcaatctc tgcctcccag gttcaagtga ttctcctgcc ttagtctccc 37440
aagtagctgg gattacaggc tcccaccacc acatctggct aatttttttg tattttagta 37500
gagatggggt ttcagcatgt tggccaggct ggtttccaac tcctgacctc aagtgatcca 37560
cccgcctcgg cctcctaaag tgctaggatt acaggcgtga gccaccgtgc acagctgcaa 37620
ttttttaaaa agaaaaaaat gcataatttt gcacttccat ataggaaaac ctctttattt 37680
tcttcaagat aacatatgcg ccaactcttt aagtaatcga ctcttcttgg caggggggttg 37740
ttggaaaaat taaatcagtt gctaatgaca agaaagaaca tcttgcattt aaatatttgc 37800
acattttgcc tagctcagag ccaattcaaa ggattttctc tttcataagc aagtgttttc 37860
catcatcctg acaccacctc tctgtggtag gtagaaaagc aggtaccaac aggaaaaaaa 37920
aatcattaga tcaccagaaa gacaaagaaa agacaaagca aatgcttagt tgctatggag 37980
gaggtgcact gaactttttt tttcactgct cttttgttta tctaaatttg ggaattgtct 38040
gctttatttc ttcttatttt agaacaaaca ttccaacatt tgaagttgca taatcataaa 38100
tttcaacata gggggacaaa tatttacctg ttcattttgt atttttagta gagacaggat 38160
ttcaccatgt tggccaggct ggttttgaac tcctgatctc aagtgatcca cccgcctcgg 38220
cctcctaagg tgctggcatt acaggcatga gccgccacgg ccggccacaa tttaccacat 38280
atacctgaaa cctttaaacc tttaaccttt tttccagatg taaagggcct taaacttcta 38340

gttcagaaca catttggctc cttctgtctg gcatctatat aaaggtggta ttatacttac 38400
ttttgaggtc aatatgtttg agaggtgttg tcatgtaaag ggggaaaagg atgggctttg 38460
gattcaatca gttctgagtt tgaattccat ctttaccatt caccatcacc ttggaaaaat 38520
ctatttcttt aagctccaga tccaacagct gaaaaatggg tttatagtat tctccttgtg 38580
gggtcgtcat aagactcaag acataaataa caaagcacca tctttgcacc tgataaatac 38640
agagcattgt tagtttctgc tattcaaggg tcccaaatct agggggatat cagtgtcctt 38700
gacggaaacc caacttcttt aatgcaaggg gtactaagga aggtagtagg tacggtttgg 38760
gtgcttgcta gcacctataa gaacacagag caggcagctt ctgtccattt ttgcgacatc 38820
attttcattg tgcattttac atcatctgtg agctgggact gatgtgtcaa cacctagact 38880
tcaaaaacag actctgcagt tttctcacaa tctgctgtaa attattgttc cttttcattc 38940
cctgtataaa gcccacttcc tgtcacttgt tattgtttac tgaaccaatg ctgaaaacac 39000
atttaaaaaa aaaaaaagga gaagaagaag aaagaaagat tcaaagattc actgaatacc 39060
cagaatatgc ctgtctctgc tgggaattta aaagactcac tctctcaatc aatgaataac 39120
cctacctaaa ttgactcaaa ctgtacagaa atctgttatg aaacaatgag acacgtatga 39180
tattagaatg ctcttcaagg acagtggcag ccacagggag ggaatatgtg tttacctgac 39240
tgaagccggg ttaaaaaaag gcttctcatt aggagacaac atttaagtgg gattttgaag 39300
gatgcatagg agatttttag agggaagtat gatcttagaa agaagaaatg agagaacagc 39360
atgtacagag accaggcggt gcccaacagc ataatgtgct tccagagctc tgagcatcta 39420
tcagagtaca acaccagagt aggtggggca gaaaaagtca agatgagcct gcaaaaacag 39480
gcaaggcttg aaatagcaat gactttcttg cgtgaattct ttctatcaaa aataagtttc 39540
catacactgt caagacagtc tattgcctca cttttctgct tggtaattct gatggaaaag 39600
ttttcaggaa acatccagga agttttgcct aattaaaaat ttaaagagac taagttcatc 39660
ttttcaaata aagtatcccc aaagcatgtt tcctcaactt atgagaacag taataataat 39720
aataataaca aaagaacaac cttttttttt ttcatgtgac atccatgggc cagtctctgt 39780
gctagatgct ttgcaagctt tatctctctt aagatttgaa aaaaacaaaa acaaaaaaca 39840
ctagaggtag ctgctgttat ccctacttat agcttatcaa accaaaaact aaggttcaga 39900
aaggcaaagt aaagtcaagc agttagtata agtattcacc cacaggtctg gagagttaca 39960
aagtcctctc tctgctaagc ctcaggcaat ggccctaacc tctgaagcct ctctgtcctc 40020
tgcccatcat aggcttggat gcccccagcc agatcgaggt gaaagatgtc acagacacca 40080
ctgccttgat cacctggttc aagcccctgg ctgagatcga tggcattgag ctgacctacg 40140
gcatcaaaga cgtgccagga gaccgtacca ccatcgatct cacagaggac gagaaccagt 40200

actccatcgg gaacctgaag cctgacactg agtacgaggt gtccctcatc tcccgcagag 40260
gtgacatgtc aagcaaccca gccaaagaga ccttcacaac aggtaaaagg cctccccctg 40320
ttaacagata tagtaaagga aagattgaaa gatttccatc ccactccatg ttggggtatc 40380
ttccaggctc attttacagt acgaaaaatg aaaggggtat ttctactcct gtaaatttta 40440
aaatattgta gcaatttatt aattaatttt tcaaaaaaaa ttccaaataa ccttccaaag 40500
cacttttggg ggtgggggac atatgtagaa gatgctggtc tgatattctc agacaaaagt 40560
tctggttaaa ataaagttca aataaatatt tttaaagtaa aatatggtat atggaaaaca 40620
tatatgacaa atgagcctcc taacacttta gcctacggca ttattttaaa agttcaaaag 40680
tgtcaatttt tcattcagta aatatttttc agagctcagt aagtactttg tgagttatta 40740
gaaatatgat gaggaggccg ggcgtggtgg ctcacgcctg taatcccagc acttggcagg 40800
ctgatgcagg cagatcatga ggtcaagaga tggagaccat cctggccaac atggcaaaac 40860
cctgtctcta ctaaaagtac aaaaattggg gtcaggcgca gtggctcacg cctgtaatcc 40920
cagcactttg ggaggccaag gcgggcgaat cacgaggtca ggagattgag accattctgg 40980
ctaacacagt gaaaccccgt ctctactaaa aatacaaaaa aaattagcca ggcgtggtgg 41040
cgggtgcctg tagtcccagc tacaggctga ggcaggagaa tggcatgaac ccaggaggtg 41100
gagcttgcag tgagccgaga tcgcaccact gcattccaac ctgggtgaag agcgagactc 41160
cgtctcaaaa aaataaataa ataactgggc gtggtggtgt gctcctgcaa tcccaggtac 41220
tcgggaggct gaggcaggag aatcacttga acctgggaag caggggttgc agtgagccga 41280
gatcgcacca acacactcca gcctggtgac agagcgagac tcttgtctca aaaaaataaa 41340
ataaagaatg aaatatgatg agaaaatgca aaagaccagt ttttacactc atgagcttat 41400
ttttaagtct ttgcattgtt tctttaacca caaagtctga gaaaaacaca aatgactaaa 41460
gtgtcgcact catccataat agggaaaggt gccaaatctg tagcacttgt ttagatttgc 41520
acatccacta tggggacaga tgagtgtgct gagaattttg taatttctct ccagttccag 41580
gcacgtttca tatgtcagct tgagctgtca aagactcctg ctctcttgtg actgatatgt 41640
tcaatacaaa tgttccttct ctcaaacagg cctcgatgct cccaggaatc ttcgacgtgt 41700
ttcccagaca gataacagca tcaccctgga atggaggaat ggcaaggcag ctattgacag 41760
ttacagaatt aagtatgccc ccatctctgg aggggaccac gctgaggttg atgttccaaa 41820
gagccaacaa gccacaacca aaaccacact cacaggtgag ctctgcatct ctgcaagcct 41880
gagccagcca gccctagagg gcccttaaag gatgggcacc tgctcccaca tgtccccatt 41940
tgcaaccctc agaaataagt ccaatttcct gaaatactgc ttattggatt gcagtgagtt 42000
cttttgtaaa acattccgaa aatttgttgt agcctccaga gaccaactca aagccatttt 42060
tatcatcctc tgaaagtctc tctgatttca ggatgaatct caacttgcac attctctcct 42120

taggtctgag gccgggaact gaatatggga ttggagtttc tgctgtgaag gaagacaagg 42180
agagcaatcc agcgaccatc aacgcagcca caggtggggc acacctggaa ttcccatccc 42240
actgagctgg tgctgggcag atgagacaga gtcaatgtgg ccaaaaccta tttaaagaga 42300
aaaagtagaa gcctggagga aaaagggccc tgtagtacca tctagcccca atgttgaaat 42360
tcaaaaggaa cataagtacg gtccatctgt atgatgccaa catcatcccc tgtatggaaa 42420
aggggactgg ggattttgtg tttgttgttg ttgttgttgt tgagatggag tatcgctctg 42480
ccaccaggct ggagtgtagt ggcatgatct cagctcactg caacctccgc ctccagggtt 42540
caagagattc tcctgcctca gcctcccgag tagctgggac tacaggtgca taccaccacg 42600
cccagctaat tttgtatttt ttagtagaaa tggggtttca ccatgttggc caggatggtc 42660
tcaatctctt gacctcatga tccgcccacc tcggcctccc aaagtgctgg gattacaggt 42720
gtgagtcact atgtccagcc gggatttttt aaagaaaaat tggaaccagg ctttgtaaga 42780
aattcatgaa catgtgtaag ttggtaatca gttgcatctt ttaaataaac cattatctag 42840
tccaaacaca actgtggggt tttctctgag aaaacaccta acttttatac ccctgtcaag 42900
cagattataa gtaacaggat tgtagtttaa gtccaggccc tgcacttacc tgttgtatcc 42960
tgtagccgca gcttgtaggt gaggatgctg aggggctacc tgatttgagg tcattgattc 43020
tttcctcaag attttttttt ccatttttct gggaagtttg cactgcaaaa agaaaactcc 43080
tcctctgcct ctcccccagc ccccagctta cctccccaca ggcactatga ggaagaagga 43140
ggctagtgtg taaagaggag atggatcatc attgagcttc cacattatgc gggatgcaag 43200
gccggacatt tacatgccaa ttcttttttt ttaatctttt gattttgaga taattgtaga 43260
ttcacatgca attgtgagaa agcatacaga cagatcccct gtgcccatca cccaggttcc 43320
cccaagggta gcgtcttgca taaatgtaga ctaatataac aactgagaaa ttgacattgg 43380
cgtaatctgt ccactctatt tatatttcac cagttttgca tacactcatt cgtatgtgtg 43440
tgtatatact tagttctatg caattttatc acacatgtag atttgtgaat gcaccactag 43500
agtcaagata cagaacagtc ttactacaag aatcccacat gctacccttt gcagccacag 43560
ccacctccct ccctttgccc ctctgcctaa ccctgggcat tcatttatct gttcttcatc 43620
tctatgattt tgccatttca aggaatgctg cataaatggg atcctacaca tattagtcca 43680
tttgagttac tgtaacaaga ataagaatac cttagaccaa gtaacttata aacagaagaa 43740
atttatttct cacagttctg aaggctgcga agtccaagat caagatgcca gcagatttgg 43800
tgtctgatga aggcctgctt tctggctgac agcaggccat ctttttactg tgtcctcaca 43860
ggacagaagg ggcaaggtct cttttataag ggcaccactc ccattcatga ggcctcccaa 43920
aggtccctag atccaaatgt cttcacattg gggattaggt ttcaacataa gaactttaga 43980

gaggcacaga cattcagtgc atagcaatac agcatgtaac attttgaaac tggctttttt 44040
tccttcagca taattctctc gagattcatc taaattgctt cttttatcaa tagttggttc 44100
cttttaactg ctttaaatct gagcagcatt ttatggaatg gatattccat gacattattt 44160
tcaatcttct cagactccga tggggtcatg tgggtgatgt ataattagac ctattttaca 44220
aaagtaaaaa gagagaccca gagatgtgac tctcctgtgg tccctcagct cctctgtggc 44280
agacctagga ttccagccca gatgcagttg actttcagcc ccagaagcag ccctgtcttg 44340
cagcctgaag agcattcatc tctctgttct cctcccagag ttggacacgc ccaaggacct 44400
tcaggtttct gaaactgcag agaccagcct gaccctgctc tggaagacac cgttggccaa 44460
atttgaccgc taccgcctca attacagtct ccccacaggc cagtgggtgg gagtgcagct 44520
tccaagaaac accacttcct atgtcctgag aggcctggaa ccaggacagg agtacaatgt 44580
cctcctgaca gccgagaaag gcagacacaa gagcaagccc gcacgtgtga aggcatccac 44640
tggtgagtct gccctgagct tcctccaaac tctaggttga ttctgaggtt agcagatctc 44700
ctcagccagg gtgtcctcac atgagaaagc caaatgcaaa agcaagccac tttctccatc 44760
tctgaagccc ctgaacacca ctttgaaagg gctgcttact cgtcttttct acctcctgtc 44820
aactgataca gcaaatttcc cccactggga aataggggtt ttaaaatatt tcttcctagg 44880
agaactttaa acatcagggt ttgtgtttgc atttacctat gatttttgtt ttcttttctt 44940
aactgtgcat cacagcatct tttcaattgt ttcttgttta tttccttctt ttagcctaat 45000
aattaattta cttttcattt tacttcattt aaagatgacc aagatgttgg taataatatt 45060
ttttttgttg ttgttatttt ctacaaactg tttttattcc aggtcttttg ctttctgcca 45120
aggttacccc tgaagagtgg cacacggccc tttgagactt ccatggtgcc ctctcatttt 45180
ctcttttctc ttagtcatta agacattaac ctagtttgtc tatgacgaca gcttaatcct 45240
gacattgtgg cccatgcaac ttgtgtccat tctggcccat gcaacctgtt cactgaacct 45300
gaggccatgg ttactcggtg gctaaactga agagacaacc aggtttttaa aatatttctt 45360
cctttatgaa aaataagcct attcaatttt ggccaccaaa tattcgaaat gtataggtgg 45420
aaaatgttac ttttccaggg tttggcaagt aatagtcttt acttccaatt taattagtaa 45480
gcatgtattg agtacctact gcatacattg taccgtactg atatttgagg aaaagtagtt 45540
cccaaagtca gcattgttat gtggactcat ttgtgcctgc tcatagtgct tgcggaagac 45600
tagatactca gaatgctaga cagaactcga gaaaaagagt gatccaagga aatcaggaga 45660
gctggcaaag gtgttatggc aagggatgtt attccagttg aagcttgata tgcaagaaaa 45720
gggttagaag tgtggacata tttggaaatg gcttgaagag tctttctcac aatatcttca 45780
gtctcaaatc acagtcactt acttaagata gaagttggtt ttctttctta taaaatgaat 45840
ctggagctgg gaggtacagg gataacttag tagtttcaaa ttgtcaggca cccaggctgc 45900

ttctttcttt tccaccattt ttagtccttg gcttccattc tcaaagtcat ctcagtgtca 45960

caacatggct gctgtaatac agccattcca tctgtgttct aggcagcata agggaggaag 46020

aaccaaaact cccaaagagg agcaccttct gggtgagtct ttctggaaac cccaccaatc 46080

acttctcccc atagttcatt ggccactcct agctgtaagg gaggcaggga aaggtagtct 46140

tctgtctgaa catgttactt ccacataaaa ttgaggtcat ttgtaagaga gaaatttggc 46200

agacaatgag cagtccctgc cagagtctga aatgagttta cgtggtcaat tctgttgact 46260

ctgcattgac tgcttcccac ccaactataa gatgtttata aaggatgctc tgaaagaata 46320

gtttaattaa aaaataaggt gggggcgggg gatagaacca agaagaaata aatctatagt 46380

agagatttaa atttcagctt tataaacatg agcttgttat aaagtgtcaa ggggcaacaa 46440

atgaatcaga aatgcccaaa agatgaaatc cctctctttt ctttctcttt ctctctcttt 46500

cttccctctt ccctcactcc tgctcttctt cctcctcctc cttcctctgc ccctctccct 46560

tagctttccc ttgagtttga taaatgctgt atctaagaga gattcctatt gcccctcaat 46620

cattccacct tctggtgatg gccacattgc ctccttcctc cagtgagaaa acctcacacc 46680

tgcgcagtca ctgaagaagt tttatctccc taagatcctg aggtgtgggg cttctccact 46740

agagaaagga ggtaatgtgg ctataaccag gagatttact agctagaatt attctgtttc 46800

tgaatgagag ccccatattg gcattccaaa gcctcagcag ttgcatttcc ccaccacgcc 46860

tccttaatgc catttcaaag atatcatgtt tcgtatttaa agacattctt cagtgtggga 46920

aaaatgtgca aagttctttt tattggttct tctccactgt ccacagacta tttcccccat 46980

ccctttctcc acaggaaaaa atatatatgt ttcatttaag gtgaagcaga cgcttctttc 47040

cctccttgaa ttctaacttt taaagccttc tgcattcact cagtcaataa aatactatca 47100

ccatgctctg tgctaagttc tggggttact agagcaaaga agacgggtat ttaattccaa 47160

ggaactcact gtgtagcgaa caaacagaca ctgagataac attctgctca ctacgatcga 47220

gggtacagag ggtgcagcca gtgctcagga gacactggat ctaacttcag agaatcagga 47280

aaggcttaca atgggaagac gcttttggac agagacataa gaggcaaaca tagatgtgtt 47340

aaatggaata gctaaagaag tagaagccca ggcacctgac cttggtaagc atagaggaag 47400

aaagaaaaca ggaaaagcga gagctcttgg gtgaaggagc catgtggcta taagtcctga 47460

aatgttgcag agttttaggt tcaaccatca gaagcaagaa ccttccagct ctgtatcact 47520

ggattcttaa tagcagaagc ctgccaggcg ccactcaatg tctgtggggc ttacagagaa 47580

aaatgaagaa tcagtggccc tgaagaattt ttcctgggga attccagggt tttactgatg 47640

gattgggcca taatgtaagt ctttttcttg atgtggcacc atactaaaaa tatgcataac 47700

ctggccacct atcacacctc tcgcccaaag acccagccat gggttacagg gccctacaga 47760

atctggcacg gccagcccat cctctgacct cagtcctgcc actcattccc ttgctcactt 47820
caccatggcc tcttgggcct tcacactgtt ccttaagctt tctgagcctg ctcccacccct 47880
cgggctttgg agtgtgctgc tcccactccc tggataccac tcttttccca gcacttcaca 47940
gagcgtgctc cctcatttca ctcagcagct cagatatgac ccagaacaga gggcttctct 48000
gactaccagc ctaaaaaggc acccccatcc tgatctaccc cttcaagctg ctctaggttt 48060
ccttgtgaaa cttatctgcc ttgatattat gcaagtattg gtttactata atatctatgg 48120
cctgcctccc catcagtgag cagaatataa ggactgattc catcatccac tgctgaatcc 48180
cagtgccctg aaggcagaca agtatgtggt atgagcttca tgagtgttta gggaccctgg 48240
ctacttcaga gtcagcattt ccatagaatc ttctgattct tcccttccac ctctgcatta 48300
acaccccaat tctttcctta aggcataagg gaaatcattc ccttggaaag tcttcaattc 48360
cgcttaatct ggatagaaaa ggaaggaagg aaggaaagaa ggaaggaagg gaggaaggaa 48420
gggagggagg gagggaggaa gggaggaagg aaggaaggaa ggaaggaagg gagggaggga 48480
gggagggagg gagggaggga gggagggagg aagggaggaa ggaaggaagg aaggaaggga 48540
gggagggagg gagggaggga gggagggagg gaggaaggga gggagggagg gagggaggga 48600
ggaagggagg gagggaggga gggagagagg gagggaggga gggaggaagg gaaggaaaga 48660
agggaggagg gaagaacgaa aggtaccgtg tctgttggca tttaaaaaaa ttagtgtgta 48720
ccaggcatca ttccctctcc tcctctccca ttccctccct tctctttccc tctcctccac 48780
tctattctcc tcccctctcc ttcctctctt ctcccctctt ctctctcctc tcctctctct 48840
ccctttctc tctgcttttg tactcttata aacccaacta aaccctgctt aaggtcaaag 48900
ccatgaattt gctgcatgcc ataagagaca ggaggcaggg gtgataggca gaaacataaa 48960
gccagcaaag catattttca tagcaacata agccttgaca gtgacaaccc agtagctctc 49020
aggaattttg gaagaagaaa ggtggaattt tgtttttagc cacagctacc agtaatacag 49080
aacattctgc tcttatttgc tattttgatt ttctcacaga ccaaactgaa atttattcaa 49140
atatttattc atccagcaaa tatttatttc atacctactg tgggtcaggc gatgccggtg 49200
tatagaagag aaagaaacag cttctcttct caagaaatgg tgcacgttgc tataagagag 49260
agaaccaaaa cccaaattat aacactgtag cttatctgtt ttagagggat ttgtgggagg 49320
ctgcaggagc atacagacag gtacccatca cagacctaag caaagggaat gaaaagcact 49380
ggagaaggct ttttggaaga agtgatgttt ggttgagttc tgaaaaagaa aaggggctgg 49440
gcaagtggag aaagagtaat gacatttcca gcaaagggaa tagcatctga aaagctgaaa 49500
agcatgagaa actttacctt agtgaactgc aagctataca gtcctgctat atgatagagt 49560
gggaaaacag tagggtagga ggctagagac atcatcaggg gcatctgcaa aggggcttgg 49620
ttcttggcag ttacaaagaa atcaccgaat gttttaaggc agccaaggct tctcatttca 49680

attgtcttat ctctgagaga tgagagtaaa gcgactgaag ataccctttg gggttgtcag 49740
gcttggatgt gaccatgccc ctgctgtggg agaatcactc tttctcccct aagtggacgc 49800
tgaagtgagt tcctcatcaa gccagcactt ggtaatcact atctagaaga tggtaacaat 49860
ctacagattt ttaagttggc actgtcagaa attcctccag accagctgct caacccacca 49920
ggagcacagc aatgggtttc aaaattctga tttcccaggc ccactgacat tgttcaaggg 49980
tagattccag tgcacagctg taagaagaag acaacagcct tttgataaag aaatgccaag 50040
aaattcagtg tggaatctat aatggaaaca cttggcttct agggcagtaa gtccacagta 50100
tgtcatttgc agttgaaatg tggagccagc atttatgtga agcctcttgg tcctcatctc 50160
aactattgtc cactgtattt tttattcata atattccccc ttctttcata attctgaccc 50220
cctctctctt taatttagct actgatttta tttgtggaag ttgtaccttt tactcacttg 50280
gctagcattt atttaataaa gggatctgga tggggcacac gtagcatcca ctactgacat 50340
ggccacagta ctgcctctct gagttataaa ccaatgctct ttcttgattt gcctaccaaa 50400
aaaaaaaaat agtagagaaa tagccaaaag tgttagcaga agtaccaaca ccatcactat 50460
tactatttac tgaacaacat gacattatgt aaaaaaaaaa tcactctatc aactatatga 50520
acagatgaaa caggatgtgg ggacaattag atgaaagtat cagtggcata ctaaatatcc 50580
gattcaaaga gataagatga ttctcattag gaagagacat tttccaacgt gcatcttttc 50640
tctccatcca tcttttgcca cagcttaatg ggcaatccac aatctcattc ccccaccccc 50700
cgtaaggtaa ctttcccaat atctctttca agggggcatt taaaatttat tttatagaaa 50760
aaaatgtttt atgtaacaat tagacataag gattaaaaga tagacacccg tagaaaaaag 50820
acagtaactt aattcacacg aaggggtacg ttgtcaatga cagattagtt cctgacaggt 50880
ggacagcaga gaaacaaaaa caggaaagtg ggataagaag gagagctaag aagacatagg 50940
gttgatttaa ttttcactta gctctgtttt tgtcctatat ctctttctgt atcaggactg 51000
ctgaaatatt tgagaaggac atgctcttga gtttagttgt gcttaaatcc caatgtcaag 51060
ggtataatca ctatatccag ggaactgttt ctggcctcac aaggcccagc aggattaaag 51120
gtagttgaca cctcctttct tttcctaaat ctagatacat cacttgcagt ttggggtcct 51180
ccctttctca taaagaccat gtttaggtgg ttaagaactg ctttggagcc tctggtgtaa 51240
gctttaggat atgagtcggg gttaaagagg agtctgtatt caagatcaag tattgcaaac 51300
attttctgta aagggccaga tggtaaatat tttagacttt ataggccaga gagtttccat 51360
tacaattatt caactgcaca ttttgtaaca caaaagcagc catcaacaat atataagtga 51420
acaagcatgg ctatgctcca ataaaacttt atctgtggca ccagaattgg aattttatat 51480
gacttgcatg caccatgaaa taatattctt ctttggattt atttcccgac catgtaaaaa 51540

agtaagaaaa actgttgtta gcttgctgac taccaaaata agcagctggc cagatgtggg 51600
cctcgggtaa cagtttgcgg acctctgttc tcgatcaaac aaaatttgaa ggagtgtatg 51660
ctctgacaat atgaaacaag caggaagaat gttccccaga acttagaaat gtctgagtta 51720
aatcaattcc agaaaagaaa tgttttctcc tctgaaagga tatcctcaat caaattccaa 51780
gctaagattt tcaacttagt agcctgctct aatggacaca taaggctcat ttgaaattag 51840
cagcaatgtg aagtataaat agaaaaaaac agtttgttct tttaaatgat ttaaagtcag 51900
tttctactct ctagtttaca ggatccattt ccagagaaat actttccttc ctaaactatg 51960
caaagtccac ttaagctcag ccctgggcaa attatctccc ttaagtcagg ttgagaccag 52020
tgagttttta cggaatttcg tgcattcgcg agatgaatga acagggtgca ttcagtttta 52080
cttgagcttc ataagatctg cttctcccag gacctctttc ttttttctat taaggaacag 52140
tttttcaaag ccttatcacc ttccttagct cagccttaat aatactgtgt caatgtgtct 52200
ctgatcttta ctaatatgat ctatgtaaat gatttgaacg ctagaaactg atgaaatccc 52260
aatataaact atacaacaga cctcccatag gatattgagg cagcacaaaa aagaagaggc 52320
tttttttttt tttttttttt tttgagatgg agtctcgttc tgtcacccag gctagagtgc 52380
aatgactcaa tcttggctca ctgcaacctc aacctccagg gctcaagcga ttctcctaac 52440
tcagcctccc aagtagctgg gattacaggc tcacgccacc acacccagct aatttctgta 52500
tttttgtag acacggggtt tcgccatgtt gtccaggctg gtcttgaact gctgacctca 52560
tatgatctgc ccaccttggc ctctcaaaaa agaagggtat ctaagaagag ccaattgtct 52620
caaaatacag aaggtcggac agctcaaacc aagaccccca gaataactga gggccacccc 52680
attatgatgc aagacatgtt ccaaaatgaa aactgaagct cagagcatcg ctaacataga 52740
catatagttt tctaaatctg caggacactg atcaatagga aaataccatt tccaagagaa 52800
aaacccaaat atgcatgaag gcaaaagaaa ttttttgttt gtttgttttg agacagagtc 52860
tcactctatc ccccagactg gagtgcaatg gcgccatctc agctcactgc aacctccacc 52920
ttctgtgttc aagcgattct cctgcctcag cctcctgagt agctgggatt acaggtgccc 52980
accaacacgc ctggctaatt tttgtatttt tagtagagac agggtttcac catgttggcc 53040
aggctggtct cgaacccctg acctcaaatg atccacccac cttggcctcc caaagcgctg 53100
ggattacagg ctcgagccac catgcccggc cacaaaagaa atatgacatt gttgtcctgc 53160
tggtgtttta atagggacag caccagtagg ggatgcagtg aacaaagcct ggcacttgga 53220
ctgcaatggc ctatagatgt ggcattgggt ttgccattcc caggttctca gaagcaaaac 53280
tttctgagga atagtttaac tgaccccatc tctgtcatta ttcagaacaa gcccctgagc 53340
tggaaaacct caccgtgact gaggttggct gggatggcct cagactcaac tggaccgcag 53400
ctgaccaggc ctatgagcac tttatcattc aggtgcagga ggccaacaag gtggaggcag 53460

ctcggaacct caccgtgcct ggcagccttc gggctgtgga cataccgggc ctcaaggctg 53520
ctacgcctta tacagtctcc atctatgggg tgatccaggg ctatagaaca ccagtgctct 53580
ctgctgaggc ctccacaggt atctctttcc tttctattta tagcttctgt ttttcccagc 53640
aggaaactgc ccatgaataa tcagacacga ctagctttgg aatggacttg ttctgccact 53700
acttatgtct atggggtcta attgtgacac gaggccatca cgctacagaa ccccaaggca 53760
acttttaata cttcactcca catgaatgac acccttggga gttgtacaga gctcaacctg 53820
caggactaaa cacacagctc ccatgcagag ggcctttagc tccataaaca gcctccctcc 53880
tgttaaagga cgttaaggca ctatatatga aagggaaaga aaatcacaaa attctctcat 53940
ctactgaggg ttaaggcaat tctttaagat ttaaaaaaca aacaaaaaca aatattttcc 54000
tgcactttcc tctctttgct ccatttctaa aatttaaaga gttgcatgat gcatgtttat 54060
ctaaagaaag tcacattttt tcagtaatat tattattcag cttattcttg tgttaaagat 54120
tccctggatc ttgttgtgtg atttgatcac taaactagtt ctttgtcctt ctccagggga 54180
aactcccaat ttgggagagg tcgtggtggc cgaggtgggc tgggatgccc tcaaactcaa 54240
ctggactgct ccagaagggg cctatgagta ctttttcatt caggtgcagg aggctgacac 54300
agtagaggca gcccagaacc tcaccgtccc aggaggactg aggtccacag acctgcctgg 54360
gctcaaagca gccactcatt ataccatcac catccgcggg gtcactcagg acttcagcac 54420
aacccctctc tctgttgaag tcttgacagg tattctagac gaattcacta attcacttcc 54480
tcccctttgt ctctgctcgg gaggaatcaa agcactttct tgctttaaac tgggatcagc 54540
tcctaccaca ttggggaaat accagtgaat catctctgct tcttcctaaa tgtcacatcc 54600
cttatttggc taagtcctag ttgtagccca gttccatgga aaattccacc tactcctcct 54660
ttaaaaaagt gagaggtggc ttttctttca aagtaagttt cagtcacaat atctaaaaag 54720
ttggaaagcc caacctgtaa atgaagaaaa aatagctctg agctcatgag ctcattttaa 54780
gaacaccaaa ggactcccca tagccctatt tgtatgtcaa gaatagaaat tgtgaacaat 54840
tcctaaaact acttttcatt cccaaaagct gaatagaaaa accctggctt tagtgccaac 54900
agtttgagtg tgctcaacac ctgctgtaaa tcatgcatct ataatcaaca ctaaccagac 54960
aactcatccc aaccttagcc aaaatgccat ctcacctgaa tcccagcgtg caattgcctt 55020
tttaagtaac tcagcaactc tgtgtcctta tgcagaggag gttccagata tgggaaacct 55080
cacagtgacc gaggttagct gggatgctct cagactgaac tggaccacgc cagatggaac 55140
ctatgaccag tttactattc aggtccagga ggctgaccag gtggaagagg ctcacaatct 55200
cacggttcct ggcagcctgc gttccatgga aatcccaggc ctcagggctg gcactcctta 55260
cacagtcacc ctgcacggcg aggtcagggg ccacagcact cgaccccttg ctgtagaggt 55320

cgtcacaggt atatgaccct cctcccagac tggagacatg ataggagcca gcatgtcatt 55380
gaaatagaga aaattcaccc gtgtggcaga atcctacccc aatgttgttg acagacagcg 55440
tgaatctcta actcaggaag gtctgtgttg gaattccacc tcactagcta aatattcttg 55500
ggactatatg tgtatgtata tatatagata cacatatata cacacacaca aatatatact 55560
tatatatata gtgtgtgtgt gtatatatat atatgtgtgt gtgtatatat agatatatac 55620
tttttttttt ttttttgagt cagggtctca ttcttttgcc caggctagaa tgcaatggtg 55680
tgaccatggc tcactgcagc ctcgaactcc tagactcaag tgattctcct gcctcagcct 55740
ctacagtagc tggaaccaca ggtgtgagcc accacactca gctaagtttt ttgtattttt 55800
tgtagagatg gagtttcacc attttgccca gcctggtctc aaactcccgg gctcaagcca 55860
tcttctcacc ttggcctccc aaagtgctgg gattacaggc atgagccagc gtgccctacc 55920
tctattcttg ggtatatttc tttacatccc tgagcctcct ttttgtatct ttaaatcagc 55980
aatgaaccct acttcaggaa actgtcgtga gcaataaata agttaatgaa tgtcagtgct 56040
tagtaaagcg cccatcacgt agtaaatact gaatagcaaa tagctaaatt tatcatacag 56100
caacagagtc atgattttta aaatagcaca aacgttaggg ctgtggcaca aaaataagat 56160
cagaactttc tagaaaaacc ttattactag cctcagagcc accaactaac acccacctga 56220
taggcagatc tctacataat aacctttaga aatgatgcag ttagcttctg ctgaaactat 56280
ctaggactct ctcctcaaca gatacaaaaa gcagggacat ttccaagaaa ggacagccac 56340
ctttgacttc aatgcttttc attcaatttg ggaaggaaaa acaattcctc tcaattttcc 56400
accaaaaatg agctgctcca tctgttataa aaaactgtgc tgtacatgag atttctagaa 56460
atagcttcaa aaagggctat gggaaaaagg gccaactttt tggtgatagg gtttctgtgc 56520
tgagccctaa gcccatgccc cactctacat atgctgcctt ttgataatgc aatccaaatc 56580
actcaattca aaacaattgt tttccttgct cttatataac agataaactt ggtggttgct 56640
agaaactaca taaaattcca aaaataaggg ggccaggttt tattcctgtg ttgcctggaa 56700
tatcttcaga tcaaacaaga gcaaaaaggg cctttgagac aggtgtggtc tgtgtgcaag 56760
atgctcatat cttcctctgt agtccgcaaa cttttgtttt tagcagtaca actccttttg 56820
caaatgaaat cttccaagga accctactat ttgacagata agtgaacagc tgttcttggt 56880
gtgcctggaa gtaagagggg gatacctcca cccatgttct caacacagtt cttctagaaa 56940
agccccagat ctatgtggaa cacagtttga aaataacccc tcttaaaagc aagaggttgc 57000
tcagaggttg ctcagactct cacagggcat tgaatccttg gatacccaag agcaagtatc 57060
tatatctttc aactattttt ggttgaaatc cctgcagata ttctcctccc taacttctaa 57120
cccatggcca cttcagcctt caaaacagtg gctgagagac tgcatttaac cctcagaagc 57180
atcccacaaa acaatgggtt tttccatatc ctaaatccaa actttctaaa tcaccaataa 57240

caactacgtt aaatttcctt tctacattgt agaagcagta atagtgggtt ctgttctgca 57300
ccatttactg agaaaaatct cctttctttt aattaaaagt aggcacactc atttcacaca 57360
aacagctttg ctctgtgata cagccattct tcctttagac ataaagtacg agcagaaaaa 57420
ggtggaagaa gaaacaagct gagaacccag tacacaactt ttgatccctg aaagctgata 57480
gaagaccaaa gaaaaaacat catacttgtt cacacctgag acagcataca ctttatcaac 57540
ccaggcaggt agccagtggc tagtgatgag ataatctttc taattataaa taatgaaata 57600
aagcagggat ttctccctga catgagtaag aaattccaag ggaaaacact tgaacaatga 57660
tttctttatt aagctgacac ttattgggca ctcgccatgt atcaggtatg atactaagaa 57720
gttgataaat attaccattt aatcatcacg caatcccatg aaacagtacc atcaactcca 57780
tttaaaggta aagagagggt ggctcagaga gaagctgagt gacatgccca agattatgca 57840
gctagcaaat gctagtattg ggattctaat caaggactac ttgcctccaa attctgcact 57900
tgtaaccatt gctgtatctc ttaccctaac ccacaatcct acttttccat gtgaatttca 57960
cctgctagca atgcatgctc ccggtgggtt gtgcattggt gcagatgtct ttgccatctc 58020
aaggataaac catgtagata tttccctttc tggccatttt caactcctga agacaactgt 58080
ggccagggaa ccacagttca aattgcaaat gctcagagac caagaattaa aaaaaaaaat 58140
tatctttccc ctttctctct ttcctagagt tgggctgtgg gatgtttggt ttcctcattt 58200
ttggtttata gaactgacaa atacttttct gtccttcttc agaggatctc ccacagctgg 58260
gagatttagc cgtgtctgag gttggctggg atggcctcag actcaactgg accgcagctg 58320
acaatgccta tgagcacttt gtcattcagg tgcaggaggt caacaaagtg gaggcagccc 58380
agaacctcac gttgcctggc agcctcaggg ctgtggacat cccgggcctc gaggctgcca 58440
cgccttatag agtctccatc tatggggtga tccggggcta tagaacacca gtactctctg 58500
ctgaggcctc cacaggtact tcctctccct gtcaatgctt tctccccaag gttctttgct 58560
tccttttcaa gagatcatgc gcggagtcag catctcgcca taactgaatc cttttcatgt 58620
gcctttcagt ggccggctgt gcatgctcag agacctccct gtgggctatg actttgtcct 58680
catgcctttg ccatgtggat ggtgaatgat gcaggtgttg ccctttctga tctccagccc 58740
cccagccttt catactttac ctggctgatt ctacatcttt tttgaatgat gaatttttgt 58800
ttcaggaatc agcctgatgg ttttaattca cttctccagt gcttggagat aactaaaagc 58860
ttccctccct atatatgagt tctggtggaa cacagattat ttacttctga gagtcttttg 58920
ctagttggaa atggtgcttt tctatctcta aatccctcaa ggatatgggt agtgtggttc 58980
ttgcatgtaa gaaatgtttc tttaaaatgc acccagtata ataagagaac tgaattaccc 59040
tcctccgggc agttctctca gatactgagt cattgttaga acatcaaatt agcaaatagc 59100

tagaccagaa ggaaaaaaaa agaaattaca caattatgac tggagactgt accaaagaga 59160
aagccattca ttagcacaag tacacacagc agcttttgga acaaggaatg tcctgggcta 59220
tgaatgtcaa agaactgata cccttttgc tcttttaatc aagagtttct ctctggagtg 59280
ttggcctttg gatggcaaac tcccagcccc agacccagga gggtgcatta tagtttactc 59340
gtcactctac ccctgttaca agacttcagt gtttcccggg ggcctgccaa agtcagcagc 59400
cttcctctga cacagttttc cgttgctcat ctttattatg tgcctccatg ggtttgaagc 59460
tcggtagaga ctatcgcttc attttgcata tgactgctcc cctttttaaa taattattgc 59520
aactaaactt taatgcatac ctgtacatcc cagttgaatc tagcaagagc atagctctgc 59580
attttaacct cagtcatttc actaattttc actgagataa agaaacataa agcacaggca 59640
accactgcca ggacggtgac cctgaaatgc ctagaatttg cagccgaggc tactctgaag 59700
gcacccggac ctcctttttc tgagacccat aaaccatccc ttcccagaag gcaaagtaat 59760
cacccaccac ttctgatctt ttctgcagat cttgtggctt gggtgtcctc agtattttta 59820
tgtgtgtgtg tttttcttct taacaatagc aacattcttg ccactgatga attggcctat 59880
gggcaggaag ttccataatc ccatctgatc tgtatctcaa caaatctatt agaaggatct 59940
cagagaccag gagccttctt tcttgctaaa gagacagtac tagcttttca aaccctgctc 60000
ctacctgccc tctgcatgtt ttattcttat ggttggggaa agcactggca ggaccatctt 60060
caagacaaaa tgttatttct tcgctgaaag ggatggccta aactgccttg ccttggctta 60120
gccctcttgg ttggcaatgc aatactagcc tttttggccc acatagcagc agttaagaga 60180
ggacaagcta ttttaacaga atatgagaat ggctggctgg tccttctcta aagtatgaaa 60240
aaaataccaa gaattacact gagaaattgg taattaccat taagtcacaa aatcagaagc 60300
ctcaagttac caccttggga aggtaatggc atcagaatta tttagccctc atttgaatag 60360
atgggagctc cttcaaagta aagaatgtct gtggaaggca tccaagtaat cttggtttcc 60420
ttaatgcaat tcaagagact ggaagttgcc aggactcata tcttttgacc agtggccagc 60480
aaccctgagg tagggctccc agaggcaagt gtggttaaga tgcaacagtt cctgataaga 60540
tcattcagca catcttcttg gccagggcct gattatatgt ggcaactgca tgtagagaaa 60600
tctaattgcc ataccttcct ttttctaatt tcttttaaca aaactgcttt ttctctccct 60660
ttggcactaa ttggtaacat tgcttccttc catcatagcc ctgaattatc agatctctct 60720
acccagcaat ggttctatta acaacctcaa tcttaaaaca cagccaaata attaactcaa 60780
atttattttt ttcttctccc ctaccccttc cttttacagc caaagaacct gaaattggaa 60840
acttaaatgt ttctgacata actcccgaga gcttcaatct ctcctggatg gctaccgatg 60900
ggatcttcga gacctttacc attgaaatta ttgattccaa taggttgctg gagactgtgg 60960
aatataatat ctctggtgct gaacgaactg cccatatctc agggctaccc cctagtactg 61020

attttattgt ctacctctct ggacttgctc ccagcatccg gaccaaaacc atcagtgcca 61080

cagccacgac aggtacatgt gcattctccc atttctaacg cactctctcc acagtcttct 61140

ttgcagggtg aagcctctcc ttctcctctc ccattgatgt acaagccaca gggcactaac 61200

tcttccatgg tgctaggctg gggtgctgtt taacataact cttttccaac agcagcccaa 61260

ataatgcatt gccataaaga catgtgcctg atttatcact gaccaggatg tttacagcat 61320

aaaatgtagt tcagtctaat ttgccatatg gaatctagag aaaagaattc taacctctca 61380

taactaaaaa gctatataac tagacctgtt cttgactttg aaagatgtct gcctcagtca 61440

aagaggtctt gtaaatttta ttttgtatta tcttagtgtc tttgtcttcc tagtctcttg 61500

gacctcgcaa tacccaaata tcccagtgat ttaataatca tgttgatggt gttgggtatt 61560

tttttccaac tgggaaagta aagggaaaca gcaaagatta cttatcagat gagtgcagga 61620

ggcagccatg ccattcagac caccccagtt ccaacaccac ctctagaaga tcagaacatg 61680

ctgtcttgcc ctcagaatag tgaatgataa atggcaaaaa taaaagtaga atagttgtgt 61740

tcaaacaagg gacttagttt aaaaaacaaa agaataaact gctctcactt taaatttgtg 61800

gtgtctgtga gatgtgcact aagtcagatg ctctttgcca tgcagtaaaa gacaacccta 61860

tgtgaattta gaaaggggag cccccctttc taatgctaca ttcaaagtag cattcttagc 61920

acaatttaga cagtgccttc tctggagagg taaatgaaga aatcacaaac tagaaaaaaa 61980

gaaacaatat ctacagaaca aacacaggag aaatgaagaa atcacaaact agaaaaaaaa 62040

atattgtcta cagaacaaac acaggataat ttttccttat agtttacaaa aaggggactc 62100

atggtctggg aaatccaaga aaagggttga catcgtagca tcaaaattgc ctgtcattta 62160

ggtcacccat ggttgagaga gggtctgtcc tcattctaca tgaggctaca aattgtcgtc 62220

agggtgatcc atggataagg cagagagagc caacattatc cagttgctat ctcagccagg 62280

ttcaagtata aaaaaactta gaggagaaaa ttaggcaaac atatatgttt gtttctcatg 62340

gctcaaaccg attttcaaca agggacaaat tattttacca ttaacaaagg gtaaagtcta 62400

aaactttatg gatttagtct tatgtgtttt gattttgact ctacaatggt gttttaaatt 62460

gacacacttt ctttttttctt ttttttcttg ctttagtagc caaacctctt cttagccacc 62520

tcactgtctc aaacatgacc tggggcagtg tgtccatctc gtgggaagct caggagtctg 62580

cctttgatag ctttcttata gaagttagta attccgatca cccccatgag accatggtgc 62640

tgtctgtgcc tggggtgtct cgcagctctg tcatcaccaa cctcaaagct tcttctaatt 62700

acactgccca ccttcatggg ctgattggcg ggcagcgtgc tcagaccctg atggtccaag 62760

caaccacagg tatttcctat tatggcttct tcactcttca ttgaatttcc tctgaatagc 62820

tttccaaaaa tcacccgcta cccactttc accctcctag ttgctcccaa tccttctcaa 62880

atttgataga gccatccaaa tcaaagcttt aaagctttaa aataactctc accccaaatt 62940
aaagaatttc agtgtcaaac ctctttttcc taaatatcca ttcacatctg gagttgttct 63000
gtttgtttgt ataaagttaa cctgacttgc tttggttgaa tttctaaatc atcttttcat 63060
ctgatcttga ataatctttt tcaatcccac ccattatcat ctgcctgtgg tcgttctaat 63120
caaagtttgc ctatgtcatg ttcaaagagg tatgtgcatg ccatttctgg tgacttggtc 63180
agaaagatct acactctttt tctctctagc aggattttaa aaaataaagg tgccctgagc 63240
atgctgagaa tagactctga atcacaaccc aagaattgca tgagaggacc cagtagtctt 63300
atcttgttct cgtgattttt gtctgaagac tactcttttt tgacatgtac tttgtcaagc 63360
acattttgtc acccactgat agctgtcaca aagtctcagg ctcttctttc aagccatcat 63420
cttgtccaaa tcttggtgca tgtggtgaat attcagagca ttaaattttg aaaatgtttc 63480
tttttcctgg aagcagctag catgtccatg agtcatttca gaaggtttaa agtggtggga 63540
gaaaaatgcc cctcattttg catggaacat tgaccatacc ctaactacca ctatctttct 63600
ctgtttttc ttgcatctca tttctcagct tggttgcacc ctttgagagg cagctggagg 63660
taaagctgac ctcaagaacc tgagtcttat tctgcatggt gaatctgttg gcattgatag 63720
tctgtctggt agcatgagaa gaatcccagc atgcatcctt aatttgggag attgtttcta 63780
cagcaaaata tagggaaata acagagttcc acttggaaca ttttaatctt ttgtgtggtt 63840
gagggaattt gtcaaagcta tagctgcaaa tgattgcatt gttaccttct gaaactaatt 63900
gataaggagg atatcccatg tcaatgctaa tggctatatt ctgcttctga accaaatcct 63960
ttttgttctt agagatttta tgacatcgaa ttatgctgta taattgtgcc attaaagttt 64020
atccaaatga gccccttctt tcctccaaac tttggagctg aagcaaaata actcccaagt 64080
ccttggaaga aatattgtgc ctcttcccaa ccctaaatcc tgatcttaat atctgaatcc 64140
tccagtactg ttgcctctct ctaaaataca gtctgggccg cacttacttt aaaattagtt 64200
ttatttgata aatgagcatt taggaggcca gccccataaa catctagcca tatagacccc 64260
ttaacattaa atgagttgct ctcaaaactg aatgtctaga ctttaacaga aaagagatac 64320
ttaacctcag ggaaaccatc tatctgtcca aattatccaa taattgagtt tcctaagcag 64380
aaaattcaat gcctaacttc aagtttttct ttcacctttg cattcactgg gaattttgaa 64440
tggaagccat cttcaccttt acggtattaa atggtaaaat atagattttc ctacaatgca 64500
tattaagtac tgtaaaaatt tctcttgtat gggtattagc ctttaatttg tgcaaacagt 64560
agatactaaa aaagaatact aaatgaggaa actccaaaga agcattttct aaaacatgtt 64620
caatggaaca ccagctcctt gagatgttca aaggatattc ctcagatagg gtatacattg 64680
atcaataagt ttatgaaatt tgggccaaac aaaattaaac aggtttactg acagaaccag 64740
cctgtcagag cctttaaaat gcaaacccac cttgtgaatc tctatggtaa gacataatat 64800

tcggtgttta ccatgcatac ttaatcataa agctgtcttt tcttggaaca tcttacaggg 64860
cttgtgtccc cagtacaaac tttagaaaat gtagccttaa tacccttcac gttactccaa 64920
tctaccactc taatggcttg tctactcttc ttcatacatg actagcaaat ggtggtgttt 64980
atgctttaaa agaacacaga tctaaaaaat aaaataaaaa taaagaacac aaatatacac 65040
ctgcagagaa agcaatatat tttctatcat catattcaat gcaggtgtag acacagccta 65100
acatactgtc tcttctgtat gtatttggag atattgatgc tttcatgttc tatggctaca 65160
ttctggaaaa atccactgct tcctctaata atgtctctct ctttctctct gcccatattc 65220
cttctttaga accaaagcca cagttgggca cgctaatctt tagcaatatt actccaaaaa 65280
gcttcaacat gtcatggacc actcaagctg ggctttttgc aaagattgtt atcaatgtga 65340
gtgacgctca ctcactgcat gagtcccagc aattcacagt ctcaggagat gcaaagcaag 65400
ctcacatcac aggcttggtg gagaacactg gctatgacgt cagtgtggca ggaaccacct 65460
tggctgggga tcccaccaga cccctcactg cctttgtcat tacaggtact caatcagagg 65520
ttctcacttg tctaacacag agagaaaagg agataagtca tcttaaagga aaattcaaca 65580
aaaatacaat cttcactgca aatgtgtact ctcttatttt taattgacaa atagtaattg 65640
tatatattta tggagaatag tgtgttgttt tgatatacat atacattgtg gattgattga 65700
atcaacctaa ttaacatatc aatcacctca ccttatttat ttatttattt atttatttat 65760
ttatttattg tgctgtgaga acatttaaaa tctactcttt taggaatttt gaagtattca 65820
atatgttatt attaactatg gccagtcacc atgctgtgca gtagatcact aaaacttatt 65880
ccttctgtct aactgaactt tgtactcctt gaccaacatc tccccttct tcatctcccc 65940
aaccccagtc accggtaacc accagtcttc tctgtgcttc tatgtgttca gctttcttag 66000
attacacaaa tgagatcacg cagtatttgt ctttctgtgc ctcgattatt tcactctgta 66060
caatgtcttc tgggttcatc cgtgttgttg aaaatgacag aattttcctc ttttttaagg 66120
ctataaagta ttccattata tatatatgtg tatatatata tatatgtata tatacacaca 66180
tatggaatgt atatatttat atataaatat gatctatcta tacaccacat tttctttatc 66240
cattcatcca ttgacagaca caggttacaa tacattactc atgatagcca agataatata 66300
tgtaaacttc taagtaaaga gaaagcagat actattataa aggagaggac tttcacagct 66360
gctcttagga agtggagaga acgttctcaa gagttgtata aagcttttag ggttttttgt 66420
gcccagtttt attggctgtt ttatccctag tttagagatt ctgcctaaaa agtgactcct 66480
aagttagtaa aggcctaaga tcgaaatcct gttcataaat catgctaggt aacgcagttc 66540
atatatgtga caaaagtata accatctttt tctttaaaaa ataattattc ttaaaactga 66600
caacacagat gcagctagat gcagctaatt tgaaattcct gtctcctttt tttcttttga 66660

ttaaaaaaaa aatgcattta ccagtactgt cagaaaaaaa aaaaaaagaa aaaaaagaaa 66720

aaagaaaaat cacctagaaa agagacgctg aaccttctcc actaatcctc cttatccctc 66780

aggattgcca tcactgattg gggttggctt tgggtatgga aagaacgtag aacttggagt 66840

cggaagacct ggttgtatat ctcagcttag gcacctagca gatgtatgac ctaggataag 66900

tcattaactt ctctggtttt tttagatgtg atcggggttg gaagattgat gaaggttcaa 66960

cctcagatgg cagataaatt tcaagtttaa ttaagcccta cccattcact caacttgact 67020

tcttgaacat ttgccaatgt tcaatttggc aaatgctgcc aaatggtggc agcaaaaact 67080

tcaatggcaa aggctgcaca tggtgtcagt ttactaaatt ctagcccaaa aagcctcaga 67140

aaatatttag aagtctcttc cttttcatta gtgataattt tcagaagacg gactttgcca 67200

tctgattttc acattaaacc aggtctctcc cagggtctaa attgccatca ctaagacatg 67260

tcaattagta caattaaata tttaattata ggcagcatgg tgtcctggaa gagtgaggat 67320

tttgagatca agcttttgtg gtttctaaac ctctcccaca gctttttagc tacataattt 67380

tagcaagttc ttaacttgtt tgaaccctaa tttccttatg catggagaga ggataccaca 67440

ggcacttctg ggagccttgt ctcatagatt aaatagagtt ggtgaaattt aactgcctag 67500

catccagtag gaattttata catgctaggt tctttctgct gtctcccaac attattagga 67560

aagtggcttt cttaaaaata accaataagg ctagatagaa caatagacct atgcaatcat 67620

gaactccctc tcgtttttaa gagaactttg gatgtgtttt aatgtaagat taaaaaatga 67680

gtatttttc ctatccagtt acctaatgtg aattaaatca ttcagatgag tcttcaaatg 67740

cagaacatgg taatttctta agggactgag acaaggagga acgagatgag attctcactc 67800

ctcaggaggg gagaaatggc cacagatccc atcaggccat tgcttgtagc ggcttgcagg 67860

acctcccaca taacagaaga aaaggaagct gagagaccca gtcttgggaa tcctagatcc 67920

agttacagca ttgtctcagc taatccatga tgtgaaaaga ttgcgtttta aaagaagcat 67980

attaaaaaag aaaaaaacaa aacaggaggg actcttaaat aacaagccct tgattgccta 68040

attagcctga atgccctcac tttcattgga attcactgct ttgatggtgt ggaggtgcaa 68100

agaggccagc tatcaaagag agatgccact tatctaaaca aaatccagga acagaattcg 68160

agacccttgt ctctcagatg aagattgttt gcccagtata agagatttca tcctcgcttt 68220

tgatagaatt atgattcttg ggaaatcttt acgtggaaaa aactgcctca gaataaacac 68280

caagtgtttc aaaataacta caggaagtcc aagccacagc aaaatttaaa tctctattta 68340

caaaatgaag gtattcattc ccttattcat tcagtcacca aacacttctt tttaaaacat 68400

ttttctttat gtctcatgga ttagacccta ggcattagtg aattaatgca agagcattta 68460

tctctttgta agtttccatt tagctgtaat ggaaaagaag tgtctaaact agatgcagct 68520

aatttgaaat tcctgtctcc tttttttctt ctgattaaaa aaaaaaatgc atttaccagt 68580

actgtcagaa aaaaaaaaag aaaaaagaaa aaacacctag aaaagagacg ctgaaccttc 68640

tccactaatc ctccttatcc ctcaggattg ccatcactga ttggggttgg ctttgggtat 68700

ggaaagaaca tagaacttgg agtcggaaga cctgattgta tctcagctta ggcacctagc 68760

agatgtatga cctaggataa gtcattaacc tctctggttt ttttagatgt gatcggggtt 68820

ggaaggcagg tagcaatatc taccttactg tatcattttg aagactaact aggataacaa 68880

tgtgaagcct tgtcagttgg caagtatcaa tggaaactta gccaacataa gctcactcct 68940

tttggatatc ccaatgtgtg aaaaggccct ttagcacctg agaaatcaga atgcctggga 69000

catacatgtt tggtgaacga atgaaggaga gatggtgaca gaatttacta agacccagat 69060

gtgttcttgt tttctaactt tatccaggaa aataaacagc aaaagattta gcaaatctta 69120

tcatttctta gtgggttata accatgagct attaattttg acacaaaaaa aacttacctc 69180

ctccctttat ttcagcttgt gaggatatct cactaattga cttcattaat ttgattcaaa 69240

ctgtgcacta gaagtgggat agaaacacat gaagtatacc ggaaagaaat tatactcgaa 69300

aaaaaaaaaa agagctgaag gcagtgggaa gtagacaacg ggatccaaac tcaacaaaat 69360

gcaacctatt tgtataaaga actacatggc ctttggagtc aaacctgact aaaatcttcg 69420

ctctatctct tcttacaagc ttgtaacttt agctaagtca atcaacttct ttgaaactca 69480

gtttgctcac gtgtgaaatt agaataatac ctacctcatg ggctattaag agaattgcac 69540

aagataacac tatctggcac ccaataagtt cttactgaat tttcattttc taagcatata 69600

acacaaattg aatgatggac ttgggtatct attggtgttc ttggaaacgt gtgctatcct 69660

gacagagtca gtgctgctaa cctgagttat tttcactcct cccttctttc agaggccctg 69720

ccccttctgg aaaacctaac catttccgac attaatccct acgggttcac agtttcctgg 69780

atggcatcgg agaatgcctt tgacagcttt ctagtaacgg tggtggattc tgggaagctg 69840

ctggaccccc aggaattcac actttcagga acccagagga agctggagct tagaggcctc 69900

ataactggca ttggctatga ggttatggtc tctggcttca cccaagggca tcaaaccaag 69960

cccttgaggg ctgagattgt tacaggtatt tcaaatcaag tgagccattt gttcctcttc 70020

ctggtcccct tctgtgtaat ctgtctaccg gatcgccatg actttaacat ctttgttcac 70080

attccctact taatccataa atgtagtcta ctgtttcacc ttctccccac actccctctt 70140

gtcatatgta cttaatgaac actccccact cccaccccag aaatgagaag gtcgaaatat 70200

ttctgctttg tattcaaaat cctttagacc ttgtcttgtc catagctctt gtgtgcttac 70260

ctcatatcat cgtcccttgt gatcaacctg gctggtcccc agctacattt cagccttctc 70320

aaaagaaata taccaatgag tatatttcca aaacgtattt aaaactttg ccatctcaaa 70380

atctcaacca tgatcttaac aaacttaccc agtgggctcg tcattggaaa accaaatgtg 70440

aacttatttt atcggtaatc actaatatca gagagactct gcaacacgga ctaaatccat 70500
aattttctca agactaatga ttcctacaga aattaacaac ggaatagtca aacatctgtg 70560
tttcccaaag tctttctaga gattactagc tccacagaat gttcaaaggt cctactcggt 70620
gaggaaattc cacgttcaaa taagtttgga aagactgagt taaacaaagt taaagaggat 70680
ctttaactgc acggcttttc agaacaccct aacgtgcatc atctccaaga agtacttatc 70740
caggcagtat tttccaaact tatttgacaa aggatacttt ttaaggaaga ggatcaaaca 70800
ggaatagttc actgtgagca cactttggga aactcagctc taaatctaca ttgacattga 70860
gttttgtcta gttaaggcaa tgcaagatga aaccctgggt ccacagtatg tagcacaaaa 70920
ggttccaacc tcagttctac catctgaaca tttatctaat agttaataaa ataacaagaa 70980
agaatgcaaa aaaaaaaaaa aaaagtcaag ggtaggagcc ttctttagtg aaacagcact 71040
aagaacaaag caaaaagacc ataacatttt aaaagccatt ttgggcaggc tcaaggagaa 71100
tgtaatcagc agtatcatca tgagcagctc tacagatcat taatgctggt ttatttgtct 71160
tttcaaaaag aaaagaaaaa ggtcttgcag gggatgggca ctcagccact ggaagacaga 71220
gagcaaatgt aaacaattta ctggctttga acttatttaa taatatgtgg gacgggccaa 71280
tgctgagtcc agatgtggat ttactgaatt gcttttagac aaaactcaca tatgcaaaca 71340
tatgcttgac ttgtattgaa tgtgtctact ccatctttcc cctctcctga ctctactgga 71400
cttgttaatg ttgaatgatt ttatttttgg tatctcagga cacttctatc tctgaaagcc 71460
ggggcatcac tacatattga tacccctact ggagagaaga ttgcacggac tggtaaatga 71520
tgttgtcttt cctccttctc ccaccatttt ctctccctct agaagccgaa ccggaagttg 71580
acaaccttct ggtttcagat gccaccccag acggtttccg tctgtcctgg acagctgatg 71640
aaggggtctt cgacaatttt gttctcaaaa tcagagatac caaaaagcag tctgagccac 71700
tggaaataac cctacttgcc cccgaacgta ccagggacat aacaggtctc agagaggcta 71760
ctgaatacga aattgaactc tatggaataa gcaaaggaag gcgatcccag acagtcagtg 71820
ctatagcaac aacaggtact gtaaataaac aggagagaac agagaaaagt catgactcag 71880
gggtcttctt ctcacagggc tgagtaatca caggagcttt ggatgcaggc aggctggcgc 71940
tctgatcctg actctttcaa ttacagactg tgttaccttg gacaatttgg ctaagtcctt 72000
atgcaaaaaa agtatgatgc catttactta atagggttgt gaggattaaa tgagataatt 72060
catataaagc ccatagcgga aggcctggca ggtagcaagg gcccagcaaa tgagagctat 72120
tcacgttgcc attaatgcat catcatcgtt acaattatca tctttttttt tttacgttcc 72180
aaagattctt ccttctatct attatctcat catatctcat ctcatcatat catatctcat 72240
cattttatcc agagatacac ttccttgttc tttggccaaa tgttcgagct ataccaagta 72300
gggagataat ggagaagcag cacgtctatc tatacacatt aatatttata aaatgctaca 72360

tgtgaataat cagtacggtg atcagcaagg ataaaggaga gagctggcaa gattaacatg 72420
ctactgttta tgaagctctc taactttgat actcataaca actctgaaag gcacctagag 72480
tgtccttgga gtttaattac ctttttccat aggagctgtt tcaaggagaa aaaaaaaaaa 72540
gatggttaat aatatgactc ttaggcccag agtgatggct cacacctgta atcccagcat 72600
ttcaggaggc tgagacagga ggatcacttg agtccaggag tttgagacca gcctaggcaa 72660
catagtaaga ccctacctgt gcaaaaaaaa aaaaaaaaaa aaatcaaaaa cttagccagg 72720
tgtggtgcct catgcccgta gtaccagcta ctctgcaggc tgaggcagga agatctcttg 72780
agcccaggag gttaaggctg tggtgagctg tgttcgtgcc actgcattcc cacctggacg 72840
acagagcaag actctgtctc aaataatgat aataataaca gtagtaataa tatgactctt 72900
tgtaataata ataatactaa gatgaacatg actttttaaa gagaaaagga aaaacctcca 72960
acaagtcaag taaattattt catgaaactg agggagatgc agagacatag cattcgctgg 73020
agatggctgc ttcaaaggga accaggggac agaaccacaa aatcaccagg agagataaaa 73080
gggacctgag agatgatgta gctcaacctc ttctgtttgt gcatgaagac tctgaggacc 73140
agaatggtga aatgattttt ctatgatcat agagctagat ttggagtaaa gcccaatgac 73200
cagcttattc taatactatt taaaatgagc agtctggatg agagaaaacc aatgcatata 73260
gctacttgcc atctctgaca ggcataggca tgacatttgc caaaacctct ccttttttgtc 73320
taaagataga tattttatat tgttaagttt tcaggaccta ataagtggca agaaatcacc 73380
catgtaaaaa taataatcag tatttgtagg cttggagaaa atgctagaat ccaggcagca 73440
acctagtaag agggaaaaac cctgtttctt attaacaggg acaccctatc ctgggatgca 73500
ggtctcagtc tcctcctccc tgtggctgcc cataaaacga ctccagatca gaggcagagc 73560
cttgattttg atcttggttt ataaacagtg ttcttcgaag tttacttctt ctgatcattg 73620
atttgttttg ctgatctttt ttttctagaa gccctgcagt tctaaagcaa aaaagagaat 73680
gaataatcat gaattgaatt ttttttaaga acaacaaaaa aaaaaggcaa ggcagaccct 73740
gctcttttgg aaagccctct ctttgatttt cttctctaga gcctagtagc ttatagccaa 73800
agagaggtgc ccatgcgcgt gcgtgcttgt gtgtgtgtgt gtgtgtgtgt gtgtgtgtgt 73860
ctacatgcct gtgctccagg accaggagaa gacaggaaag gggctcttg agcacagaag 73920
atgcctaatt tctatcttca cctcatcaag tttataaatt tccccttttg tttttttttt 73980
ttaatccttt ctcctctcct ctcttcctcc ctcataccat tgtaaactct tctctgtaat 74040
gtcctatccc tcttcctcct gagaaggccc tgctgtagct cctcctctca ctcacaaggt 74100
acctctccat agcacaagct gccagccctg ggtgcccagg gcagtagaaa gggcagtggg 74160
agacagtgtt gcctggtggt taagccaaca accatgggac agtgtccttg gagtttcatc 74220

atggctctac ctcaattcct tcatctggaa aatagaattt aactcaaagg tcagccacga 74280
ggtttacata agatcatgaa tgtcaagtat ttagcagaat ttctttaccc acagtaacca 74340
tattagctat taatgtgttt ggccataaga tacacctact aaaatgtacc tacaccaatg 74400
aggttttcag tcaaagcaat agtgcgagta caccaggttg gcagagggaa gtcctcagtg 74460
cctgagttta tacacctcac tgttttttgt ttgtttgttt gtttgttttt tcccagttag 74520
actcatctcg atctttccta ggggacacct taaagtctag tatggcccat ggaggaaaga 74580
gggacattgg aaagaaaata aagagagggc aatcatattt actgaattct cactgcatgc 74640
cagatgctgt gctgggagtt tcacatttgt tatctctgct aatcctcaaa acatccctgt 74700
gggtgggtac cacttttccc tctggacaga tggagaaact gaggttcaca gaagtgaagg 74760
gacctgccca aggtcaccca gcctctcagt ggtatttgga caatgaagct ctttcaggct 74820
ccagagaccc atgtggtttc tgaaactttc catgggcagg gcaaaaaccc aggcagccac 74880
agagtctggg ggtggctacc aggccacaga agaaagctgt aggaggagat gtcttagatg 74940
agagaaaaat gcagaaagag gaagaaaaaa aaacccttgc tgaatttctg tgggtttcag 75000
catgtaataa tccctgtggg ttggagaggg gaaaagatga ggaagtgagt tccctagagc 75060
ctgaaaattg tttcctgaat tctctgggta ggcatggtta gctgaagagg gcaaaaatcg 75120
cagccaaaaa atacacagag aaaagccaaa ggaaactggg ttttgagggg aggtattttg 75180
ggaggcagaa actccccctg ggcatggctt ttctcacagg tagacccagc gtccactggg 75240
tgtttgcttt ggtgatgagc ctgaggttgt tctcttggct ttggacctcc ttccagttct 75300
aggaatgggt ttgcccaggt cagtgtttgt gtgaacgtta tttccaggat tccaggctgt 75360
gcttggctct gctgcctact cctgggaccc agaaagatca catcacaaga atgttcatgg 75420
aagccttggt ttggttctct gtaaaatggc tataatcaca tctttttggg ggcttctaca 75480
ggaggattaa gccttataaa ttgcaaagaa ctgtacagtt gtcaagtctt agtattattt 75540
aataaaagga actagaaagc aaagatttaa gaaaatgtta aaaatcaaaa atgaacgggg 75600
gtcacattcc aatctgggaa accattctcc cacgattcct tggaagtcag ctatggtcct 75660
atcactcccc ttgaattaga ccaaatgact tttctacaac cctgggccct gtacctgaaa 75720
tgaaacaatt ggtcctagca agtcacaact taaccacaga tcaccacatt ctccatccta 75780
aaggcttggc tcagacacaa attctaggca ctgagttttc ctaaataagg aattcaggaa 75840
gaaccattga tgaacaagac agttaactgt ttctgggctt ctcttgtgct ttcttcttga 75900
cagccatggg ctccccaaag gaagtcattt tctcagacat cactgaaaat tcggctactg 75960
tcagctggag ggcacccaca gcccaagtgg agagcttccg gattacctat gtgcccatta 76020
caggaggtag gagactcgga aaggggagag agaggaggag tgttgtgggt ttggatggat 76080
tcatgaaaaa gaccctgata attttcatcc tctaacatca tgatcaaaga aaatgaattc 76140

tttatttgtg agaccatctg gagagaagag aactcatttg taaagtgcta tgaggttctt 76200
agaagaaact ttcttgtgaa gggtcacttg aacaagacct tttttaacct tgaaattata 76260
aaattctatg agaaatagat ctctagtatt gtttgcacac aatggcaaaa gattctaaat 76320
atattggcat ttctctccct gcgcacactc atctgaaaag ctagtcttgg atatatgtga 76380
agttttacct ttgggtacca aatctgtctg tctcagcaaa tgatggcaag ctgtgcctta 76440
gaaaatttgt gagttctaac tcaaagcgca tgggctataa tagagaccac aaggaggaag 76500
cccagtcaaa tctgcctaaa gacactctgg caggtagtaa gctacctgtt gctgaaagtg 76560
tataaagaga ggatggttaa taacctcttc acaggaatgc tgttttgtct ctataggaag 76620
tctgaccagc tgatgtgcaa gttcttcact agccccccaaa ttctaagaaa tacaatttct 76680
gtgtttatta ataaacactg cagcacaaac ttgactataa tcaactttct tttcttaagt 76740
agaatttcct ctagtaaggg agtctataat cctaagccaa gacaatgggt gcaatagatc 76800
tgacatacat tacatagtaa gcttcccata ttgtttgtgg ggaaaaataa taagataaaa 76860
tttgcacaac gtgccagctc tctactgaac ggaaacttgc catttttagt acgttgtgac 76920
ttcatgtttt cccccgcccc tcctggcttt gtttttggca tctgcaccaa tagaaaaagc 76980
atacaaaaag taagccttct tcccttatgc cagagggtag gagcaaatga gaagtcaaga 77040
aagtggtgtt tgatgaagat atagtttcat ttctaaatca gatacactct ttttgggggc 77100
aacagtgaat aagtgtcagt gaggttcatt attcctcatc ttgcttttac tgtgttccag 77160
gtacaccctc catggtaact gtggacggaa ccaagactca gaccaggctg gtgaaactca 77220
tacctggcgt ggagtacctt gtcagcatca tcgccatgaa gggctttgag gaaagtgaac 77280
ctgtctcagg gtcattcacc acaggtgagt tggtggggtt gtgtgtgtgt gtgtgtgtta 77340
ctatttttca gaggtcactt gtgcatccta tgccatttct caccttcagc cttgtagctc 77400
atgactcagc agctggaaaa aaagggggggt tttatttatg agatcctagt ggaaagtgaa 77460
ctggatttag agtgccagag gcctctcttc tggcccatcc cagcctctgg tgagctgggt 77520
gaccttaacc ttgttgggcc tccatttcct ggactattgt gaaagaaaaa gtccaagttt 77580
gggagtcata cttacctgaa tttaaatcca aattctacca actacatgag ctatcagtcc 77640
attttactag ggtccacttt ccttctctgt aaaacagaca caattctgct tccctcacag 77700
ggtatggtaa ggatcacatg aagctgtgtt tgtaaagcat cctgaaatac aattgttcat 77760
gttagcccac catcaccttc acctccccgc ctattgcctg gaacccttgt gagtgtccct 77820
caaagcaggt gtttcacatg agcatgtgat ggtgacagct acaaaacagc tcctctctat 77880
ttggtggctc ttgcattcca ggcactgtta ctaaaccctt cacatttaat ccccacagct 77940
attatcatcc ccattttaca gaggagacaa ctgagacaaa tgaaatacct tggccaagcc 78000

cacataatta gtaaatgctg gaaccaaatt tgaacatagg tcggtctgac tccaaactct 78060

ctatccttct aaaataaaga gttcctttcc tagtgtaacc tgatttgtac agtacactgt 78120

aaagagggga aaataatgaa ttgcattgaa ggagaatgaa gtggaaaatt tctcttcaga 78180

cagccatgtc tcaaaacaat ttatcctttg atatgtgagc aattatccag tccaactcat 78240

taagggatac aaagaataga cctcatagga aattttaaaa atatcctcat agcctgaaag 78300

agttgacaac acacaattaa aatatcccat ctgaacccag agtcccgtcc accctgcctc 78360

acaggccctt atttccccaa agaaatcttt catcagtgga ttctccacgg caacagatgg 78420

caaaagctga acgtagttcc tcccaatgcc ccattgtcat caccagttaa aggagattct 78480

gtccatgcac agtgaagcag gaaggaactc agagtggaca atgtgggctc cccagttgcc 78540

ctgccacatc ctggctctca gaccagtcat ttcatccctc aaggctcagg tgcctctctg 78600

tgaaatgagc tgaggatagc ttccctccac cctcgccagg gctgctggca tgggcacaat 78660

tgagtagtaa gtgcaaaagc aagaggaaat ggtcagattc atgctggctt ctgagaaagt 78720

gatgttgggt agtaggagag gtcagttaaa agaggccagg gataaatatc ctttgaatat 78780

ccatcctttc tcagaacaag tcatttgaaa accatactta agacttcaaa tacctttaat 78840

agagatctta ataacaactg caagtattga gagctttccc tagtacatct tacacatcac 78900

acattgctta gctagtcttt attaactaaa cagtcaccct ttggcagctg aagaaagtga 78960

agtttggaga agggaaatga attttcccag ggcacagagg caccaggcag agaagccatg 79020

gtagaacccg ggaccaccgg cctccagagc ctggctttat gttctctgca agccatctaa 79080

ggagaaatac cactcaaagc tcagaaaagg ctcggggcca ggtgcggtga ctcacgcctg 79140

taatcccagc actttgggag gccaaggaag gtggatcacc tgaggtcagg tgttcaagac 79200

cagcctagcc aacatggtga aaaaacccat ctctactgaa aatacaaaag ttagctgggc 79260

ttggtggtgc acacctgcaa tcccagctac ttgggaggct gaggcaggat cattgcttga 79320

acccgggagg cagaggttgc agtgagccga gatcgtgcca ctgcactcca gcatgggcga 79380

cagagtgaca gtctgtctca aaaaaaagaa aagaaaaaag caaagaaaag aaaagagaaa 79440

agaaaagaaa aggctcgggc agtcttaact atgcaagtcc cctctgtgta agttctttct 79500

ctggggaaag taatataaac aataataatt cttgtcatgt gttcagcctt gttcagttta 79560

cacagctcct ttccacccat aatataatgg aaaaatcaaa ataacactga agatgaaacc 79620

agccagaatt actatcccta ttttacacat gaggatatag aggcccaaag taactgagcc 79680

agcaagggag ggagtcatta ctcaaagcca ggtcaggctg gctccaggct taggtctctg 79740

aaccattcca tgcagctgac actgtcctaa catcaggact ccataagcag tgtggacagc 79800

agagcagcta gagtctgatg tcccacccaa tgacttccct cctgtccacc atctttcttt 79860

gcagctctgg atggcccatc tggcctggtg acagccaaca tcactgactc agaagccttg 79920

gccaggtggc agccagccat tgccactgtg gacagttatg tcatctccta cacaggcgag 79980
aaaggtaagg ttttgtccaa gtctctcctc actctaagga gtgtctttcc tgctgtaaac 80040
tcttcccatt cgctcttctc cctgccacat ttggtacagg atgataaaaa tgcatgggta 80100
ggttgaaaag gccagtaatg ttcatgtggc tcccaactga aacctcaagg gagtctggta 80160
ggtcatttaa aataaaaata aaaatacatc tccagcctct acatgttttc ctggcccatt 80220
cctcctctcc tgttagtatc tctgcctacc agtgtgagac accccgttta ggaaacagtt 80280
gtcatctttt acaaagaaga aaaccagaat ctaaaatctt aaccagaccg cttcaggcca 80340
tactgagcag tctcaacttt tcagagacca caaaagaagc tagttttagc agatttaatt 80400
aaaaagtctg aaattttaag ccaggtacag tggcttacat ctgtaatcca gcactttggg 80460
aggccagggc aggtggatca cttgagacca ggagtttgag accagcctgg ccaacatggt 80520
gaaacctcgt ctcttctaaa aatacaaaaa ttagctgggc atagtggtgc atgcctgtag 80580
tcccagctac tcggcaggct gaggcaggag agctgcttga acctgggagg cggaggctgc 80640
agtgagccga gatcgcacca ctgcactcca gcctgggtga gagagggagg ctctgtctca 80700
aaaaaataaa aataaaaaat aaataaataa ataaaacaga aatgttttct tgtttggcaa 80760
ccatcagtgc taatgtaccc agttgacaga ttaaacactt gtttagagca ccagcaaact 80820
acagctgcca gaaagtaaaa tcaaatataa gtaagagact ggagaaagag agagtgcttt 80880
tcaatgaacc cctccatttt gtaatgagct actctagaaa taattgtgta atttcaattt 80940
ccaggtatag gtaagtttca tattttatgg tttggtattg ttttgctctc agtgacaata 81000
tgagcaggca ccagcgtctg tgcctggagc ctctgaaaat tttcattcag ttatggtaac 81060
caactaagag aggtcttttt gaggacagag cacaggtagg ggtggggaac tgatggggag 81120
gagggatgtt gggtttattc tttttactct ttaattctta aatagtccct gtgagcaaac 81180
tcttctgagt caggaaagat ggtgttgcac ctaaaatgaa tcaggtatag ttcatgggaa 81240
ccatggagat tgtagattca gggcaaacaa ggggcttggt tcagaagagg gggtggtgct 81300
cacaaatgat gaggtaagtt gaccaacttg tccccgcttg cctggtactg tctcagtttt 81360
agcaccagaa gttccacacc taggaaatcc cgcagtccct ggcaaaccag gatggttggt 81420
cacctgtgaa taggaaataa acacatgccc ccaagcaatg tgatcagtat tgctttccac 81480
cactggacat gacccagcag gcctgagaga tagtggccag gctctggtgc agctactgct 81540
gtccagtttt tgtgacccct cagcctaatg acttgatgac acttagtaaa ttataaaagg 81600
gcatgtgaaa aagtgacaaa gagaggagag catgcatcag gacaaattaa gcatgggcag 81660
caggcttggg gatgtcccct ccgaccgtcc tctacctcgg tctcagtggc aaagcgattc 81720
cagccagtac catgtgggaa aaaaatctcc tgacccccag ttccccttcc ttctgatttg 81780

gggcctggaa aaattaagtc tccatatgag aaatccttta cgtatgaggt gagtgctcaa 81840
gagagagaaa gagagcgtga gtcatttcag aaatcagacc tgcttttccg cagggtcact 81900
gaaagtttcg atgttcgaag gaggtgtgta tgtggtttgc caagcccact catggctcag 81960
acagtgagag gtgactgcct tggtatgtgt atttcatgtt tagtgccaga aattacacgc 82020
acggtgtccg ggaacacagt ggagtatgct ctgaccgacc tcgagcctgc cacggaatac 82080
acactgagaa tctttgcaga gaaagggccc cagaagagct caaccatcac tgccaagttc 82140
acaacaggta caaagacact gagagctgga agctctccct tctggggtgc ccacccacag 82200
cctgcatctt aaacaggaac cgctagccag ttcttctttc ttaagtcagg gccttgcaat 82260
gtgattacat cagcatcact taaattcacc agtgcccact cagtcaaggc ctgaagaaac 82320
acactatttc cttttgaact gaaatgacct tggtctcccc acggggaagg aaaagatttt 82380
aagaggaggg ctttgtaagt ggaaaaacag ttcccactgg ccctaaacct agtatttaat 82440
gatccacgtt ccaggcagga aaatattttt gaagaggcat attttttagg caatttttcc 82500
tagatgtttg ttaggtttct atacattgag agctttttac aaactatagc aagcagggaa 82560
acagaggaac catcctctct gtcagggtga gtattgatat tagcattcat ccatcctctt 82620
acttagaaac aaggggcttg ataatgatgt tcacagctgt atacttctct ctaaatccaa 82680
ccaaggccag ttagaaacat ccagccctta ggatgagata aaccatagag aatttgtctt 82740
tggagatcat ctggctcaac caccttattt atacagagga agttcttgcc aaaggtcacg 82800
tagttcagtg gtagggaaag gacttgaacc cagtcttctt gatacccagc ccgaatttct 82860
gccctacatt tgctgatctt atccgatatt aatcatcctg tcttctccag acctcgattc 82920
tccaagagac ttgactgcta ctgaggttca gtcggaaact gccctcctta cctggcgacc 82980
cccccgggca tcagtcaccg gttacctgct ggtctatgaa tcagtggatg gcacagtcaa 83040
ggtattttaa gtatataaca actcctcaat gctagttgaa gcatttgcat gatcttcaaa 83100
gtatggaaga attacctgat gctgtttttc taccccagtg catctgggga gctgaaaaaa 83160
aaaattagta gagactggat ggcatgccag accttctgaa tcatactctc cagcctcttt 83220
cattttaacc agactccagt tctgataggc agccaagatc aagaaccact gcttacaaga 83280
cttgaggaca ttttaggagc tggaatcttg atcctttaat attttgttga acaaaggcca 83340
aaaggtttct ctgcatgttt gccatatgta ttatctcaca gatggtatct ttatattata 83400
tattgtaata tgtttcaccg tgttcttatg atatcaactg ttaataccat ttttatccag 83460
tgtttatatt atttatattc gctacccaag aattttcttt cttttacctt ttaatttgaa 83520
ataactttag atcttcaaac aagcgaagga gatttcctaa actttacctt ccaaacttgt 83580
tattgaattt ttcattttgg ttatctcact ttatatttcc aagatctttt gcttgttctc 83640
cacacattga tctttttctg gcattttgtt tgtgcttcct atatcatcat ctctttatct 83700

ctaagaatat gttgctggta ttttattcta tatcctgcac agtgtttgtt acaagtttct 83760

ttgttttgct gctgttgttg agtttggttt ggtttgggtt tctgtctttc atgttggaga 83820

catccaccca atgtctgttg atcatggtct gtctttttgt atttgaacta ggaatgaaag 83880

agctaatagg gagttagctc aggcagggct tgtcaacttt gtggctccct gcagaatgat 83940

tgggcaagta cccacagttt tcttgggga cccccaaata acagtgccat aaaacctggc 84000

ctctcactgt gtacaaactt tcatcttatt atgtttccag tgtagcacct cggcctcctt 84060

gaaggctgag cctggtggat ttgaatccgg acttcctctc tggctcaacc tttccagaga 84120

gtaaacctca gcctaccatc tgaatgggaa agggcagtca tttagactgt tggagggaat 84180

gaggagatct tagagtctgt ttcttacaaa aaagcttcta aacaaaccaa acctttgttc 84240

aaccacactc cacattggcc tttaaagaca tctctgcttt tgtacacatt cctttactga 84300

acttgctgga ctctgtgact tgcaccaagc agcctctgac tagctctgcc catgctcctt 84360

agttaacctc agccttcttc attcattccc ctccatggtc tctctaccac tcactttcta 84420

tctaccaagt ttctggcaca cctgttgctg tctcctctcc cattcccctc atcttcatgg 84480

atttatacct tacattctct tgctctcttt ttagtgggtt ttagaagcaa gtagagatga 84540

gtggctgtat ccaatctgtc atatttaacc agaactggac acattgctaa taactatcat 84600

gccccttcct ggaattgata taacaatgaa ctatctcact gtgtgagctg aagcaaatcc 84660

tttatcttct ctgggtgtca gtgttcccat ctgtaaaatg aatgccttgg acttatccat 84720

ctttaaggtc ttctccttgt caggcattct aggattctgt gtttacctga atcatgagtc 84780

tcattttgat ctagagtacc ctaattgcct agaaaatggc tttgcacaga agcctagaga 84840

actgacaggt aattgtattc ctaagtaatt caatgaaccc agctttatcc ctgacagttg 84900

atctttcccc cagggactag aactgggggg ctgcaaaaag gcctctgggt attaaatgag 84960

cactcatccc agtttacttt tctgcatgtc cattcaccag ctttacatgt cttagaaaaa 85020

aaatcaacct ggtataggag aaaagagtcc tggacagggt gttgggaaac ccgcatgcaa 85080

gatcatcctc tgcctccaac aatttatgga atgagagcaa gtcttttagc ttctctgaaa 85140

ctcaattctc ccattgaaga aaacaggata aggtaccttc ccagttccca ggatgcagga 85200

gaaccttcca ggggcttctg cactgatgca tgactgccca aactaagctg gctgtttgca 85260

ggttgtagag aagatcgttg tgcttggttt actaggagtg taggttgaat tccaatccca 85320

gacactaagg tcatttgctg aaaagaattg gaagctagca agagggtgtg gaaagaggat 85380

ttcctgcaaa ctgaaaccaa tgacctcctc tgtacagcag cacagagacc agccacagac 85440

tacaagtgta gttagagttg gtcaacatct gtctgtcatg gatatctctg ttctagaaga 85500

gagacatgtg aggtcagctt aagttttaat cctaccatgc ctatggggct attataatca 85560

tctcctaact caagagatcc ccaaactgac tgcacattca aatcacttgg gaagtttctt 85620
aaatatagcc aggcccaact tctggctctg atgattccat cagtctgaga tgaggcccag 85680
gcatcatatt gttttaaagc ttctgagagt gataaagagc tatatttagg gaccactgac 85740
ctaactgacc tcaaagtccc ttaccctgtg tgattcatct tgctaccaga ttaagcttct 85800
taaagcacag cttggtttat gtcattggca tgtccaaaca tctttcaagg ctccccattt 85860
cttgtgatga taaacttcaa gttccaaagc ttagcattta taccttacag ggctcctcac 85920
tccgtttaat gataaacttt aagtttcaaa gcttgtcatt taaaggccct tcataatcta 85980
gtaacatttt ccactgttct aacacctgaa gctaagtatg gttgaggtcg cttattgcct 86040
ccttatatta tttcatttta tgttttaaat ctgctttatt caaagtttac tgatttaaat 86100
attaatctca tccaaaaaaa aacccccttc acagaaacat agaataatat ttgaccaaat 86160
atctgggccc catggctcag ccaatttaac acataaaatt aaccattaca atcccattct 86220
cccaatatag tttggcaggg ccttgggcag gtggtagcct tagccctgga gtctacagca 86280
gtgctctcat ccttaccaga tgcggtctct ccctccttat attattttaa cttgctctgc 86340
accttctggc ccaaggacct ttgcttgtgc tgtactctcc ccatctctga tatctcagca 86400
gtgcacatgg agcctggtag agagtgggta ctcaatgaat tcttgtccag taagtaagtg 86460
aatggatggg taaatgaact tataaataga atgacttctt atctataccc tgagaatttc 86520
tgtgaccaaa agccattata ctttttcttc tgctcttagg aagtcattgt gggtccagat 86580
accacctcct acagcctggc agacctgagc ccatccaccc actacacagc caagatccag 86640
gcactcaatg ggcccctgag gagcaatatg atccagacca tcttcaccac aagtaaggag 86700
gcccagggtg ccatccagcc ttgagatcta actttgactg aatcaaccct tgacttcccc 86760
tttgacttct actttaaaat tttcaaacaa acctctgaaa agaatcgatt cactgctact 86820
acttggaagc ctgaggaaag caaatgtggg agattatgag catcccggtg aaaggaaagg 86880
aggaagccca tctggattca gtccccattt ctatcacctg ctaggtgtgc cttaagaatg 86940
ctacttccat cctataatct ttagcttctt cgtctataca gggagattaa taataaatcc 87000
tctgtcacca ggaccttgca agtgttcatg aggtattgca tttgaactcc tagcccagta 87060
tgtggcccat ataattgtgt ggtcctttta ttgtcagagt tcaacatgag ctgaaagtct 87120
ctttctttct ccttcatcat gccagtgttt acaaaacctg tccacattca aaatcacaaa 87180
tgatcttccc accaagctgg tatggtgagc cctatgccca aagttctagt ggggaagcag 87240
acactttcag agactaaatg acctgttcaa gactatcttg tgagttacag tcagagttgg 87300
gctcagatat tccatgccca gggagcctcc ccctgcacct gtcacaaagt atcttctgca 87360
gatagcaaac ctgcaagttg ttccattgat aagtaagagt ggcatccatt cctgaggacc 87420
agaggagctg gcctttggag agccctgcct ttgaaaattc acgggacagt cattctgcac 87480

acatctgctg aagaaagtgc ttcccctttg ccagatctta tgccagatgc taactatgaa 87540

tgtatcctaa agatgctctt atactagggc tgtgaaataa ataagaaaac agctcatgat 87600

acaacctcag atgaatgtcc tcagatcaga aaaaagaact gctatgatca tcagctctaa 87660

gacccatcta ctaggcatag ttccaagatt gcccatttct atgagttttt aattgtttgc 87720

atcacattat tgcacctgga aattccccag gcattctagt ccatagagtt aagctaagaa 87780

ttaagctcca gctcagtcct cctgcactga gagcatcacc atttctcctt attccacagt 87840

tggactcctg taccccttcc ccaaggactg ctcccaagca atgctgaatg gagacacgac 87900

ctctggcctc tacaccattt atctgaatgg tgataaggct gaggcgctgg aagtcttctg 87960

tgacatgacc tctgatgggg gtggatggat tgtgagtacc accagggact gcagagccct 88020

caggcctagg ggaagaggga gagggcaaag caggggagga gaagagggtg atctcctcct 88080

gatgcactct gacaccccca tgtgtgaggc cttgcaagat agtgcctgtc acacagaggc 88140

actcaggaat tctctcttaa acaaaagaat gggaaacacg ctggctactt tttgaaacgg 88200

ggagataagt ttcttgtagt ttatctcatc caatcctcat caaacaactc tgtgatgtat 88260

aaatgacagt tgatcattga acccattgct tacattaatt agcactgact aagcatttat 88320

tatctaccag gcactgtgct aagagtttaa gatgaattat tccgacccca ttaccatagg 88380

agctgggtag tgtatttttg aattaataaa ctctattttt ttagaacagt tttaggttca 88440

tggcacaatt aagtgaacag aagttttcat atacacccca cacccaccca cacaacctcc 88500

ccattatcaa tatcctgcac cagaatggtg catttgttac aatcaatgaa cctacattga 88560

cacatcatta tcacccaaaa ctgggtggta tttttaatcc ctgtttttca ggtgagaaca 88620

ccaagactta aggaggggca ggtgaccacc caaagttaca tagcaaatta gggatggaca 88680

gaatggagcc tgatgctctc tccacagtgc cacactcctt cctctcacaa cgatgccctc 88740

tttcctgcaa tagcttaagt acacccagat tcatcgatat gtttataggt gttcctgaga 88800

cgcaaaaacg gacgcgagaa cttctaccaa aactggaagg catatgctgc tggatttggg 88860

gaccgcagag aagaattctg gcttggtaat gcagccctgc atgtttatat ctaaaatgcc 88920

tgattctgcc ctccttgtca cttttcctaa cccacaggga cctatgggag agaaagagat 88980

gaggaagaag aactacttct ggtggcagac acagaatgtc aaattcttaa tgaaaaagac 89040

tatcatttaa atgctctgaa tcatcaaaat gtaaaccaaa aagtatctga gacagatctc 89100

aatcaattta gaggtttact tagccaacgt taaggacacg cccagaaaaa aagaacacaa 89160

agccacagga acagcctgtg gtccatgcct tttttttttt tttttttttc cagagataat 89220

attaagagtt ttagtgttta aagggggaaa gccacaggaa caacctgtgg tccatgactt 89280

tctttttttt tttttttttt tttttttttt tttttttttt cagagataat gttgagagct 89340

ttagtattta aaggggaaaa gtgggctgga gggatatggt cacattactg aatccacatg 89400
ttgcaagata aaaaaaagaa aaaagcagaa tgacaaaaaa gagaatggtc aattttgtat 89460
tcatctcaca ctcagtaaat cggcacttta cataagataa agtgaagata gaataactat 89520
ctgtggagat atctggcctt ttatctgtag catctgctta gggacaaaag gagaggcagt 89580
ttctcccatg actcggcttt cagtttattt tttttcctat tggcagagtt aaattggggt 89640
cccaagattt tattttcctt tcacaaaaag taaaatctga ttaaggaaga accaaaactc 89700
agcccttatg tgttaaccta gccttaagtc attctctgga ccagtcatag aatcctcctc 89760
actgatatca ttgatatctt ccctcctgtg ccccaggttg tgctccaggt gctaagatgg 89820
gtttcttaga gccataatat ttaacccccca tagcacccct acaaagtact caattgccac 89880
cgggtgggca aggtgtcagg tgtcagaagt ggttgactag tgggttggta agacgaattt 89940
actaacaaca gtataggttt gaaaaaggaa cgtttattag aaagaaagaa tgctgcaaaa 90000
ggacttacag cccctgcagt ggatttttcc ttaggggtat ttatggacct taaggcagga 90060
gcttaaggat aatttggacc ctattagcca tgtagaacat gatacatgat tacatttgta 90120
gacattttgg tgccttaatg tcagcaaggg ttgcaaaatg agttttgata tgcatgtatt 90180
ccagagatgt atagaagttc cagttaccta taaatttttg ggaaataaac ctggaaccag 90240
atgcttgctt gagatatacg gaagcctaat tacctttaaa tccctcagtt aaggggtttt 90300
tgtctccgag tcctacttga tggtcaccag gtgatttttg ctctcctcac cacctcgcat 90360
ggataaaatc acagcctcaa aaagactgag tggtttgccc ataggagtct aaccaatgaa 90420
cagttgagct gaaatttgga atctcctaag tcaacactac aatcctatca cccatccatt 90480
catccatgca atccacactt tccaaccacc taactgcaga gaacattgtt ctatgcacca 90540
ctgcctaccc cagaaaatgt ttatcatatt ataataaaca tttatctata ttattcattg 90600
cccttcattt gtaaggcact ttacaaaatt ttatttgttt gtttgtttgt ttgtttagac 90660
agagtttcac tctgtcgccg aggctggaat gcagtggtgt gatctcagct cactgcaacc 90720
tctgcctcct gggttcaagc aattcgcctg cctcagcccc ccaggtagct gagattacag 90780
gcaccctcca ccatgccctc ctaatttttg tatattcagt agagacaggg tttcaccatg 90840
ttggccaggc tggtctcgaa ctcctgacct cagatgatcc gcccaccttg gcctcccaaa 90900
gtgtggggat tatgggcgtg agccaccgtg cctggcctgt aaggcacttt agagtttaaa 90960
aagtactttc accaccttat ctccttggat cttcactaat gcactgggag gttggaagtg 91020
gggcaaaaat gtcaacccga ttttacctga gcctagtatt actccagcaa gcacaagact 91080
gagcttcaag cacaggcctg tgtgatatgg ccctttttttt ttttttttttg ctttctgaga 91140
tggagtcttg ctctgtcacc caggctggaa tgcagtggca caatctcagc tctctgcaac 91200
ttctgcctct cgggttcaag cgattctcct gcctcagcct cccaagtagc tgggactaca 91260

ggcacccacc accacaccca gctaatttt gtatttttag tagagacagg gtttcgccat 91320
gttggccagg ctgataacga actcctgacc tcaagtgatt cccaccaccc ccaccccccac 91380
cccctcccac cccatctctg cctcccaaag tgctgagatt acaggcatga gccactgggc 91440
ccagttggcc ctgtcttaag tgtacccagt tgccgctttt acagagtgaa atagaaatga 91500
gggcctcagt cacctgtgct tcttagcaac ccttgcctgt catccacagg gctggacaac 91560
ctgaacaaaa tcacagccca ggggcagtac gagctccggg tggacctgcg ggaccatggg 91620
gagacagcct ttgctgtcta tgacaagttc agcgtgggag atgccaagac tcgctacaag 91680
ctgaaggtgg aggggtacag tgggacagca ggtacagtag tggctgcacg tagagcctac 91740
aaagccatgg agaccttctt gacatttaca gtttatttct ctaactttca ttccttcctc 91800
attaatccag caatttatta aatcaacaaa tatttaagta cctactgagt gccaatccag 91860
aaaaactaca attcaaaagt gttgggtaga tatttgggtc ctgaaacatt gagtatgttg 91920
ggaatcaggg caatcaggat tgatttctaa tgaccatctt tcaaaaaaag tgtgtctttc 91980
ttacccagtt ttagatatca gttgtagact gattaaaaaa aaaattcccc aaaggcctta 92040
atattcatcc aatccagcac ttctcaattt actctgggtg tatggaccca ctggcgatct 92100
tgtgaatctc cagatgctga cacaggttgt ctgggatggg gctgagattc tgcatcttct 92160
ccaagttccc tggtgatgcc aatatagctg gtgttgctgg tctgtggacc acagcttgag 92220
tagcaaacca tccactccat cacctagctg cttgaatctc ttccaagaca tccctgccat 92280
gtggtcatct agtctcagcc tgcatcctca taaatagaag gtctcacctc tcaagtggcc 92340
cctgcattcc ttgtctggct cataaccttc attccagtct tgaagatttc ttggacttga 92400
agtggttttg tgtggtttcc tggctccaca taagaggaga aataaataag gaaacaacat 92460
gtattaaggc cctactaggt accatcctct tttgtagata ttccacctgc attcatctta 92520
tttattcctc ataaaaccat tttatgaaga cgttgttttt atacagagga ggaaagaagt 92580
tcagaagagc tcagtgattt gtccaaggta aaacagcttg tagaaggtgg atgcaatcct 92640
ggctgcagaa aactgcatcc gacaatgttc tcagtgacaa agaagggtca ggactgagag 92700
tgtggaccac ccccatgcaa cccatgccca ctttgggtgg ctttctgtta tgtttgtgag 92760
agcagaagag atagaagctt ggcacgtttc cagcctccct gagccctgaa agaaaacatc 92820
cataaggtga aaagtctcca gataaaaggt tgccaaagcc aggctgagcc taaggaggca 92880
caaagctctt ttgagatggc agtttagtga taataaagag agacacccag gggcaaacat 92940
tgctttagtt cttgaatccc tcaaagaatg ttggcatttt attacctgga aatgaatacc 93000
tctttggttg agatggcttc aggcaagaag aagaatgtta cagggcaagc aaattcatta 93060
agcagcagga atgtggcctc tgttcctctc tctttgtgct tccattgagg aatgagccat 93120

aatatttccc tttactgaga taacgtttgt aaggctgcat tttcttgtgt gatttggctg 93180
cacccaggcc ttctgcagga gagcatcctg tttcattaat atctacgtgc cgtcctcatg 93240
ctctgggtag catcatcagt taaaaaggta tctgggttcc tttgacaact tgctccaaat 93300
tatgacggta gaatgcctca gagtcttgaa atgtttgtgc acagcacttg tctggattgg 93360
gagtgaggag acccacaaag caatggtact gaaatgcttc aaaaattcaa tatattaaat 93420
tacaaattag atttccaaat agtagtacat gttaatgggc ttcttgtcta agaatcacgg 93480
aacatggtct gatgaatact ttaaaaagta actatgtgat acagagacta tttagggaca 93540
tctaatgtct gaagggctta attatgacta ataaatattt gtatgtggga gaaaagcatg 93600
ccattattaa atgcatactt ctcaccttac tctctgaggt ctttatagaa agggaatgca 93660
gaaaagtcat aaaaataaca atcatagtaa gtatttgtat gccatgccac ctgtttagca 93720
tacaccttta ttgtcacaat ggcactactc agtatataga atcaaagtgc tggttccatt 93780
gtatagatga gaaaactgag gttcaacaag atgcttgact tgtgcaaggt cacagctaaa 93840
aagtgaaaag atgggattcg aagctagact ctccaaagcc agccacagcc ctcttcccac 93900
cacattatgc tgcttctgtc ggaaataagt actggggtga cactcatgtc ccattcagtt 93960
ccgcattcaa tgctgactct gggatcaatg ttcaaggcca gtcccacacc cagaaccttt 94020
caattgtcac ctctggttct gccctttact gtccaggagg gaaatttcag ctgagatttc 94080
tcaggtccat attttctgtc tctcccaggt gactccatgg cctaccacaa tggcagatcc 94140
ttctccacct ttgacaagga cacagattca gccatcacca actgtgctct gtcctacaaa 94200
ggggctttct ggtacaggaa ctgtcaccgt gtcaacctga tggggagata tggggacaat 94260
aaccacagtc aggtgagtgg accgtgaggg cccagagcag gccaagcttg ggaagctgga 94320
gggggctaat ggaagtacaa tttaactccc tatcctagca gagactagga ggcaggaacc 94380
ttacttgtat gacaacatgt ttttcccaag gcagtgagca atctcaagga cactgcaggc 94440
caggtaaaat agaaacagca caggttaacc cttgacagtc ccaaggacaa acactggtca 94500
aatggattcc aaaaacagaa cgctttactc agacaaaatc tctgtctgaa ccccaaaagt 94560
aaagcagata aaagtggagg ccatttaggt gaagcagggt tgatgacaca gaacccctgg 94620
cctggggcat ctttggaatc ccaagaacac cttaaaatga agtccacaga ctttaaatct 94680
cctccttttg tgaagaaacc aaggcccaga aaaagctgac tttcctgagg tcacagagtg 94740
aagttggcag cagagccagc tctccccact tccagcccct ggcattttcc ctgcaccaca 94800
cacacctcta gcacttctac aatcacagaa acagattttt ttcgatccct tttagaaata 94860
ggcgaacgga tcaaattcag taatctccct tactctgagc cttgcctaca ctggcacatt 94920
cctgaaaagt gtaaagctgt gtgggactag ccacttaatc ataaccaaac ccctgcccag 94980
gtccctccac agaagttagt caccatccct gggaggaggg ctggatcttc ccttggggcc 95040

acaccacgct gatccctgga ccacaaggat gcttatgagg tctgctcact ggaatcccaa 95100
ggactttata aaaatgcaaa gggccaaatt ccactttatg ataaccccag ggattctgat 95160
tcaatgatct caggcaagac acagcatctc attttttttt tttttttgca tatttgcatt 95220
tttgttgttg tggggtcagg ggggaggcag ttgatagact ccacaagcga ttcctgacat 95280
gcagcaggtg ttgggacctg ttgcttttca atattagtca ctttgataca catgtttatg 95340
ccctaattaa ctgctagcaa atgacacctg cgaccttaag tgtagacctt accttacata 95400
atttctggca cttttcaaag cactgttacc ttctgtatct caagggttta tcatttttac 95460
cctgtgcagg aattaggatg tggtttttca tgcctgtttt acagaggagg caagagtcac 95520
aggacatttc ttgacagcag tcctgtgttg tttccatctt attgtgttat ttcatcaata 95580
tttccattct gagcattatt ttatcatctt tattgccttg tctcaaaaat actcgtataa 95640
tacaaagttc ttctgggaaa agaaaaaaaa aaccaacaag ctacatgtta gaaagcaagc 95700
ctctctttca tccttccatt caattatgga agaagaaaga gatgacatgg aaaggagcca 95760
caggagggga gccccagctc cagctctgct cctcctgctg tgtggcctct gcaaagtcac 95820
tctccctctc tggctttagt gcccaccaca gtacaagaag gcgtggcttc caaccctgaa 95880
ctatcagagg aaagaaccag ggcaaaggca ggcagcatgt gcaccaagct gttggactac 95940
catactcact gtaactgagt aattacaagc ggatttgccc atgaaatgaa tgagccattc 96000
ttaaacctca catccctgta agactcagaa actgcccatg tcagtgaatg ccaaacaatt 96060
agaagcaaat aaagtgtctc tatttttttc ccatctcact agccattaac ttccccacag 96120
tggggcaagg ggccatgtgg ggtttcatgg attgactcta ccatctgcag acttcgctga 96180
agagtcaaag ttgaaacctg gaagccaaga cacagataca gtttttttctt gtagaaagct 96240
ttggccaaaa tttcaaggat atttccccct acttgagact agggaagtaa tatttaccaa 96300
gcacctacga cgtgcccagc tctgtgatag ctcctttaca aactcattta atcttcatct 96360
catctttcaa ggctaacatt atcagatctt aagaaagaaa aaaaaatagt aaaaccgagg 96420
ctcagaagat attgagaact taagactatt tatctctcaa gtaagaacac tgatatttaa 96480
ccctatatgt caaattgtaa aactgtgttt tgttgagctg ttcgttctgc tattccatac 96540
caaacccccta tcaaaatact ccataggaat ctcgtgaatt acagacacac acacacacac 96600
catgtatgta gctattcaat tctagaactt ctaataatga cctgaactcc ttcagaacct 96660
attcctaagg acggcagatt tcatcagcca ccattttgcc tcaagaggaa agaccaaagc 96720
tccatgcaaa ctcaggcgac tcttgactca ggtgaaaaac tgggtcagat tcagccagtt 96780
cctttgaaag tttccacaac ccagaccaac agagagatag gcaagaggca gctgtgatat 96840
accaaatggt caaattgaaa cagaactcat ttagtttgaa tgactaaata gcaatgatca 96900

gtgaacacct cgcctaacag taaccaacct ggaggccttc tctctctctc tctctctctc 96960

tttttttttt ttaacagggc gttaactggt tccactggaa gggccacgaa cactcaatcc 97020

agtttgctga gatgaagctg agaccaagca acttcagaaa tcttgaaggc aggcgcaaac 97080

gggcataaat tccagggacc actgggtgag agaggaataa ggcccagagc gaggaaagga 97140

ttttaccaaa gcatcaatac aaccagccca accatcggtc cacacctggg catttggtga 97200

gagtcaaagc tgaccatgga tccctggggc caacggcaac agcatgggcc tcacctcctc 97260

tgtgatttct ttctttgcac caaagacatc agtctccaac atgtttctgt tttgttgttt 97320

gattcagcaa aaatctccca gtgacaacat cgcaatagtt ttttacttct cttaggtggc 97380

tctgggaatg ggagaggggt aggatgtaca ggggtagttt gttttagaac cagccgtatt 97440

ttacatgaag ctgtataatt aattgtcatt atttttgtta gcaaagatta aatgtgtcat 97500

tggaagccat ccctttttt acatttcata caacagaaac cagaaaagca atactgtttc 97560

cattttaagg atatgattaa tattattaat ataataatga tgatgatgat gatgaaaact 97620

aaggattttt caagagatct ttctttccaa aacatttctg gacagtacct gattgtattt 97680

tttttttaaa taaaagcaca agtacttttg agtttgttat tttgctttga attgttgagt 97740

ctgaatttca ccaaagccaa tcatttgaac aaagcgggga atgttgggat aggaaaggta 97800

agtagggata gtggtcaagt gggaggggtg gaaaggagac taaagactgg gagagaggga 97860

agcacttttt ttaaataaag ttgaacacac ttgggaaaag cttacaggcc aggcctgtaa 97920

tcccaacact ttgggaggcc aaggtgggag gatagcttaa ccccaggagt ttgagaccag 97980

cctgagcaac atagtgagaa cttgtctcta cagaaaaaaa aaaaaaaaaa aatttaatta 98040

ggcaagcgtg gtagtgcgca cctgtcgtcc cagctactca ggaggctgag gtaggaaaat 98100

cactggagcc caggagttag aggttacagt gagctatgat cacactactg cactccagcc 98160

tgggcaacag agggagaccc tgtctctaaa taaaaaaaga aaagaaaaaa aaagcttaca 98220

acttgagatt cagcatcttg ctcagtattt ccaagactaa tagattatgg tttaaaagat 98280

gcttttatac tcattttcta atgcaactcc tagaaactct atgatatagt tgaggtaagt 98340

attgttacca cacatgggct aagatcccca gaggcagact gcctgagttc aattcttggc 98400

tccaccattc ccaagttccc taacctctct atgcctcagt ttcctcttct gtaaagtagg 98460

gacactcata cttctcattt cagaacattt ttgtgaagaa taaattatgt tatccatttg 98520

aggcccttag aatggtaccc ggtgtatatt aagtgctagt acatgttagc tatcatcatt 98580

atcactttat atgagatgga ctggggttca tagaaaccca atgacttgat tgtggctact 98640

actcaataaa taatagaatt tggatttaaa 98670

<210> 20
<211> 87533

<212> DNA
<213> Artificial Sequence

<220>
<223> recombinant murine TNC gene

<400> 20
agcttgctct agtccaggag cagcgccagg atccagttcc ctcatatccc agttcccaag 60
gcttctgttt cacaattaga atactcattt gtggagcaat gcaaaaatca cacttcacta 120
acaactactc tgcgggggcg gagggagctg aaattcctac tttttttttt cttcactata 180
aattgtatgc aaatggtccg ttagctggcg gcgcgcctgg agtgagtgcg tctttcatga 240
gctaaccctg ttccgctggg agggctgtgc ctttaactca ccaactgaaa aaaaaaaaag 300
tgggaaagga tgtctggagg cgaggcgtcc cattacagag gaaggagctc cctatataag 360
cctgccaaag ctggctgctc cggccacagc ctgcctactg tcacgcgtct ctcccgcgcg 420
cagacacaca ccctagcctc tggtgggcac agacagtggc tgggaagacg ctagggaccg 480
cgcgcttggg aactcgttgt ggcttaaccc aggtacttct tcacggagct ggtgcaccca 540
gagactttgc ttttcccgac ctggaaaagg tgagtctgtt gatcccatca cccaggatct 600
ttctggtgtg ctcctggcat tcccggtacc cagtaagatg tgggtttgta actataaaac 660
ttttgtttaa tttccaagcc cggtgtgatc agatgcggcc cgattattct gtgcttgccc 720
tcctgcggtc atgtatttgg tatggatctg tcaatagtca gaggaactca gggaactttt 780
ggtaaagttt tgtctctgcc tttctttgga ggaaatgtct tactttaact cttacccttt 840
tacctcgttg attctcacct gtggattttc cccttttgga gcgttagaga agttgcattt 900
taaagtggca ttcactcaca gcacttgggt gggatgagta ccacgtaccc gtagtaggga 960
gaacacaacc gtgcatctac acagtctcag cacttgacag ttttcgttcc cctcttgtcc 1020
catttgatcc tcagtgtcca ctctggggaa agataagagc tggataccag agctaggaga 1080
tgagttctgg gcacggatga aaagcccaca ggctgtaaga tgaaactggt gggcacgtct 1140
gtgggttctt gaagcagggt gccatcctcc tgattcagag acctcaggtg gggcatgtgt 1200
agaaggtttg gaggcatctg aaccccatat tgctcccaaa tcggcctccc tcctgacact 1260
ttatcccaga accccagtaa catattccct tccctggtga actttggaaa gagcacagag 1320
attcctctat gccatcgttg tgacaggggg aataagaggt tggggtttga ccctagaagt 1380
ggcttgggca ttcccactag tgccactaca acataaatgt gtcaggaaaa tatgtaactt 1440
ctatggacct tagtttccac ttctctaaag tggggacagc agctcccctt ataatgagga 1500
gcgaatgagg aacaaaaaag atcaaatcag caactgggca atctctgtca tggcccaaag 1560
ctgccaacac cctggacgtg aaaagcgggg cctcttaacc cagtttttca gtgtgtctgc 1620
ttaccccaa agcctctgcc ttcttatctt gcccaatctg agacagcctg ctagcctggc 1680

acgtagtgtt ttacaagcaa ggggtgtgtt ggcaccatta ggagaaaagg tggcagcctc 1740
gggcttgatg tgaatctgga gttctgactc cagaatgtcc tcaggcattt acaggctgaa 1800
gtgcttggct atggagtgct gtgtggctgg tggtctttgg aatcagattc tttgcctcag 1860
aggatgagcc agactgggag ctctctttaa gagtgaatgc ctctttttcg tgtgttttcg 1920
tggaatcttt ttttttcttt gttttataga gctttcccta tatgcacctt agaaatgaaa 1980
gctgctcccc accccactgc ctttcctgag atgcaacaaa gctcaccagg gctggtgaga 2040
ggcatgctct gtctaaataa gctgatggaa agttctcctg tcctatgaac caaggctttc 2100
gaagggcatg gggatggggg gtagtgtgcc ttggcctggt acattccaag aatgtgacca 2160
catctaggga aagaaggggg tgcagtgtgt gaatattctc tgggttactc aaatggggtg 2220
agcacgagag agagagagag agagagagag agagagacag agacagagac agagacagag 2280
acagagacag agagaatggg tgaagccaag cttggtaatc acccactccc tcatttgttc 2340
aaaacaccta gcttcgcgct cttggcctct ctttcccctg tctcaagagg tgacaaggct 2400
tccatccatc agttttgtca ctcatacaaa gagggtgagg agttacctct cctggggctc 2460
tgtgacatac acaccagaag agttcaatag acagaggtgt ttctgtttac cttgttacta 2520
gatcacagat actttcaaga ttaatgtcct aaacaaatgt ttgtcaagtt taacaataga 2580
agcagttatt aataaattat gaacaaccct cctccctgag tttctgacag ataaaaatga 2640
ggttgcatgg gctaacatgg ggttaagggg tgcaaacaca ctattcatac ccctggaaca 2700
tttttaccac attgcaaaca atgggacaca cagctccaga tctcacagct aggcagtgct 2760
tactcagatc agtgttctga aatccagcag ctatgggagt aaggacagtt ccccaaatga 2820
aacaggagac aggaatttta gaggcttaag aaaggttggt tacttcaatc aatgtcagta 2880
atcactatag ccagcataga attaaccagt gtatgagcta taggctcttg atgcttccct 2940
gggcatcatg gtatcaaatg gccacagcag gtctctggag catgggtgta cttataagac 3000
agagagactc acagagcaga gagattgggt cttggaactc aagtgatagg gccagaatct 3060
aagctcatat ctttctttgg aggctgtgct tcttttcaac gtgtcagctt cttgtatagc 3120
cctcagaggg aaagcacttc ccttctgaca aaatatgccc caccaggcag actgtggcca 3180
cccataaata cagcaatagc ctgatcatat aaccagaata gaatttggaa agggaactca 3240
ggctgacaga atgaaaaaca tactccctga atcctatccg ttctcatgtt cgctgtgaac 3300
taaaaccacc tctcatctct cttcccgcag tttcatgaac ttacaaactg gctttcacaa 3360
agcataaagg ctcctccata ctgaggctgt atgggtttga aaagtagaac ttgagaaaca 3420
aatatttagt tggttctcca tatacagttc ctctatggta gtcagacact gacataaaat 3480
agctaatagt cagggatgaa gtgtgacaca gtgatgggga agactgtcta cttagttttc 3540
taatactgca aattatgaat caacagctcc atttaataga tggagaaact gaggctcaag 3600

gaagctgagt agctcagccc aggatcttct cacaagagtc agaactggct gcaaacccaa 3660
actttctaag taaaacgcca ccgttttgtc tcttgtgtca cgctgcctct tgatgtcaga 3720
cttggctacc caagtgcaga actgtgcacg gtgtcctttg gggaatggcc agaagagcac 3780
acaagactcc agctggcatt ggtgcctctt gatttcacag aggagccctg cagctatcgt 3840
ttttcatggt gcacaagaaa ggttagctgg tggtagtgca acccaatgct tagaaacgga 3900
aacattccaa tttcagctga gggacagggt gagtgaggga gaaaggagag agagagagag 3960
agacagagac agagacacag agacagagac ggagggacag acagagacag acagagacag 4020
acagacagac atagacagac agagacagca gagagaaaca gagacaggaa gatagagaca 4080
gaaacacaaa gagaaagaca gagaggaaga cagagtgagc agaaagagag aaaaatcaca 4140
cacacacaca cgagagagag agagagagag agagagagag agagagagag agagagagag 4200
agaaaggggg tggcaaggtt gcagtcatgt ccttcctagt gattttcatg gcatcagccc 4260
acacttccca tggctgtgca ggtgggagct acaaaaggga gaaaagggag acagccatac 4320
ctgcatcttc taaggtgtag gagagctaaa tgtgtggttt taagggggct gctggggaga 4380
taaaaatggg gctctcccag gtctctaaca gaagtcaggg aacagctata gcctccatgc 4440
cactgattct gccgaagcct caaagaggaa gattgtgatt caaacacctc tcaccacaca 4500
aagatagctg ggaatcccct tgaatcccgt cccagaccag tccaaacacc ttcaacatgt 4560
ggaaaacatt acatgaaggc ccctttccaa gaaggaaaac cccttaatgt atagtgagac 4620
gagcctccta aaaatcccaa tcctttacaa cacaaagcac actggtgact ttgccttcta 4680
tgaaatctgc acacacccta tgacttcttg tggaggagaa accactgcat agttctcttg 4740
attccgagcc tggcgctcag acttttgttc agttataccc ttaaatctaa tcatctttgt 4800
tctttctcct tttctgccat atatacttcc tagagcagtg gttctcgact atcctcatgc 4860
tgtgatcctt taatacagtt tctcatgttg tgatgacgtc cccccccccg aaataaaatt 4920
atttttgttc cttcataact ttaattctct acgattatga atagcaatgt aaatttgctg 4980
ttttctgatg gtcttagaca actcctgtga aagagtcctt caactcccaa agaggtcaca 5040
agccacatat taaaaaacac tctcctagac agtaagagcc acctggagtt cagtgctctc 5100
tgccattctt gattctccac catggaacaa aatcagaatg cccacactca aagcaggttg 5160
tgaacggaaa atcctttcca tgtgtccttt cactctattt gtgagtggaa atttttaaaa 5220
gattatttca ttccaagtca tctcagatgt ttgaaatttc ctgaagttca caggtcctga 5280
aatgtgggca cttaaactca gatggctttg gctggtcctt taaccttcct caaacagact 5340
gccaagagac aaaggtgttt cataagccct gaaagcagat agcttgcttc agaatcagcc 5400
aaaggtcacc tcttacccta aaagaggaaa ggtggtttct gttaacatct ctaaagaatc 5460

gtccaaacta tccttgatgc ggggagatga agggtaactg tgaaagtaac tgcatttgtt 5520
actttgtcca ttgtcaaggc tttcatttgg gttacagttt gcagggacat aatctgtcct 5580
ggcagaggag gcaaaatggc aggagctggg ggtggctggt catcccgcat ccatgctcag 5640
ggaacagtga ttgagagctg gtactcagtc ggctttctcc tttgtatgga gtccagaacc 5700
cccactgcat ggattgatgc cacccacact tagggtgagg cttctcacgt caattaatgc 5760
aatctagaaa atgactaaca gacacgtcca gaggtttgtt tctagacccg tatttttca 5820
aatcttatca aaaaaacaat caacattaac cagcccacta aatgttgtca cctactcgat 5880
caatactgtg ccattttacc attgttatta tttgcaatat cattgccatt ctaatgttgg 5940
ggacctgggg tgaattatgt atgtagcctg ttgaatatca tgcaggtaga tgatagggga 6000
aaccttaaca taaacccggg ccaagtccct agaaggaggt aatgaccatc aggctacctg 6060
agccatgcta ggcaccatgc ttctgctcct cctgtcgtgg ggaggtcatt gtccccagag 6120
tctgcctgcc ttctgcaggc atcagaccct cagaatgtaa tgtctgtgaa gttctctgct 6180
gttcccaata caacagggtt tgacttctgt aggtgtgctc tgcagctctt tcaggtttca 6240
ggactgagca agtttctcat gtgcaggacg gatcttccca acgtggtatg ctcatgaacc 6300
agtgcattaa tgaaacactc ctatgtacat caccttgaca tgctcccaga atatatacat 6360
aaaggctttg caggaaggta aaatgaggcc tttgttactt gcttaaagta gaaggtaaag 6420
gcattcagag ctccttcata ggatcaacac catcgagttt gtttccctcg atatgccaca 6480
ggattgtaaa gaattgactc aagtgtctac ataacaaaac agcttagaga tgacaggcta 6540
tttgctcgat aaacatttat attttaagat ttgttttgag ttatgcatat ggatgtgcgt 6600
ctctgttgag gtaggtgcac agaaggacag tttctctagg gggtcagagg agagtgtcag 6660
atctctggag ctggagttcc aggcggttgt gagcattcct acttagatcc tcagacctgg 6720
actcatcctc gctgaaagga cagcacaagc catctccaca gcctcacatt tgcattttgg 6780
aaaggtagtt aggtaaagca ggcaatgctt aaatgggtga atgaaatatc aatgaggggc 6840
tttagacaag tctgtggatt ctgtgcactg gaagagtcat ataagacagt taaggtaacc 6900
gttgctgagt taaaaagtgg aagagaatca agactgttgg gaaaagcatt agttttattc 6960
aaaatgttaa agctatagta ctaggtctgg gaatgttgga taaaagtaca gtgttcaggg 7020
atcccgagca gccatacaac aggacgccac tgcatgtcac ctgaaggagc aacagactga 7080
gatcaaaaga agatagtcca gaactccatt agaccatctg ccagcccttg caacacactg 7140
agtgtaagtg ccagtatatg aaatggtgta tctttttaaa aaaagattta tttattttat 7200
gtgtgagtgc actgtagctg tcttcagaca caccagaaga gggcatcaga tcccattaca 7260
gatggttgtg agccactatg tggttgctgg gaattgaact caggacctct ggaagagcag 7320
tcagtgctct taaccactga gccatctctc cagccctgaa ataataatat cttaaaaatt 7380

taaaaaaaaa tgtatacatc taatactaaa gccacctatg gtgatagaag tctggtggag 7440
gtaaccacag aaggagctcc ggcacagcag gggagcctgc ttgctgggga aagacaggaa 7500
gccagacagc tccctcccgc agtgggcagt ggcatcatgg atagcctttg tcattgacaa 7560
tcatacagaa gtagctggaa gcagggatgc tatagaagaa cgttcttaca gaggttgctc 7620
tcactagaac ttagaatgtt aatgctgagg aggctgattg atgggtaccc tcaaagccag 7680
gagcttacag atcagattca aaaagaaatc cagaggtaat tcaggagatg ccatggagag 7740
aagggtgagg taaaagtcac tttcaacctc atttcatcta aagcccctct ctgtttatgt 7800
tttgtgcacg gaactttcac atgagataga aaggaaggtt tctgtggcac aaaaagaaga 7860
agaaaggagg agagggagga ggaggaggag gaggaggagg aggaggagga ggagaaggag 7920
aagaaaaatg tttgtacaaa tcactggccg actttaacat cttcatttta aagatgctga 7980
aactgagaca tggggaagag agatattttc ctaagatgtc atgccagctg gcagcgggtc 8040
agggcagaat cttgggtatc ctgactctgc tcttagaatc cagggacaac ataaaagtct 8100
gcagttaata tatcctctta caaaagaaaa atgaactgtc ttattctcta aatagtatag 8160
cacatgtgga ttgagctaat aattctatta gttacttacc caccacaaaa acatagtcat 8220
taaaaacatc acaggtgctg ggcatggtgg cgcacacctt taatcccagc acttgggagg 8280
cagaggcagg tgaatttctg agttcgaggc cagcctggtc cacagagtga gttccattac 8340
agccagggct acacagagaa accctgtctc aaaaacaaca acaacaacaa acaaaaaata 8400
aaacatcaca ggttgtgtct tatggcttct ataggtctag aatcaaagca tagctgagca 8460
gagttaggct ggaatttcta ggatcagttg tcattggcct gggtttagat catatctgaa 8520
gacttgagtg ggatcagcc ttcttccagg ctgtacctgt tgactggacg ccatttctca 8580
ggaggttcta gactgaagct tgtcagttcc atgctgcctt aataactgct cacatggggc 8640
atttcaacaa agccatttat ctcatccaag ccaatcacac acacacacac acacacacac 8700
acacacacac acagagagag agagagagag agagagagag agagagacag agacagagac 8760
agagacagag acagagacag agacagagac agagacagac agacaaagac attcgataaa 8820
tggaatttgc ttgacaataa tcgcccaaag taggatttta gtttttttta aactcttcta 8880
actttctaat tttttaactt tctaattttt tttttcattt ttgcagcact ggccttgatg 8940
cccggccagc aagcactctg ccagtttagc tacactttct gtattctgag atcaggtcct 9000
aggtaattga tgttagtctt gaactttctc tgtggccaag ttttcttggc caactcaatg 9060
accttgagtt ttctccagcc tcctgcttcc tagggctggg atgacttgca cacaccacca 9120
cacctggttt cggaagtaat ccattgaact aatttgatca tgatgatacg tgtgttattc 9180
atttgggggt ctagctgtgg ttctgtgata cagcacacaa tctacaggat ggagcagagt 9240

ttgaaatgac tagggttctg caggatccca ggctggctgg ttgactctct ctctctctct 9300
ctctctctct ctctctctgt gtgtgtgtgt gtgtgtgtgt gtgtgtgtgt gtgtgtgtgt 9360
gtgtagtggg ctggtcttaa tgttacagat cttttagacg aagaccttct ccaatggtct 9420
tcaatgaaac agctgagagg actttagctt atgcacgtat ctttatttgg gctgggtttg 9480
tatgcattta ttcagggtga accagagcta tatatgggcc ttggagagcg ggaaggaatt 9540
ttcagggtaa ataaaatcat tggctagagt ttaaagttct gtgaatgcct catttgtgtg 9600
gagaggcatt ccaggaatct caggtactta tggtgaggct tcttattggg ggtggggaga 9660
ggaagttgaa cttgtaggtc tgccaaggac tactcacctt tcccaagata gaaggtgtga 9720
gaagttgggt tccccaaaca aagtcagaga aggggaagcc ctgctgctaa tgggagtcct 9780
ccattttctg ctgagttctg ttgatttaac cagtcatagt agacatttac cctaaattag 9840
caggatttcc tcacagacaa ggagccaaag aaacaagttt ccttgtttta cgatctttca 9900
tgtgagctgc catcagaagg tgctatccag atttaagatt gaccatcctg cttcaaataa 9960
tctgatcgag aaaaccctca caggagtgcc cagtagctta gattccagat tcagtcaaac 10020
tgaccatcaa gattagccat cacacaaaat atattttact gaacgttcaa gcagtgaaaa 10080
tgaaaagtac ctgcttctta tcacccatct aggtatgata attaatttat aggcttccca 10140
gcatataaat taagactaaa ctttagaatt tcattttctt tctttcttct ataccatatg 10200
cagtatggtg tgtcctctac tgctgttatg gtgtgttagg atatccactg ctatgaagag 10260
acaccatgac caaggcaact cttataaagg acaatattta attgggtctg gcttacaggt 10320
tcagaggttc agtccattat tattgagatg ggagaacggc agtgtctagg cagacatggc 10380
attggagaag cacctgagag ttctacatct tgttccaaag ggaaccaagg gaagactggt 10440
caatcccacc ccttacagtg acacatttcc tccaccaagg ccacgtctac tccaacaggg 10500
ccacactttc tagtagtgcc actccctggg ccaagcatat tcaaaccacc acatttggtt 10560
taaatataaa atgctgctga aggtttgttc tttcagtgct taattctcaa ctggagatgt 10620
tgttccaggg actatagatt ctatagtcaa agaattttca agggcaaggt ctgggtggca 10680
gaatgttaca tccatccctg ctccctgttt ttatctctgg ctttgtgctt cctggcttgc 10740
aatatgtgag tggcttctga cacatgcttc agtcaccaca tcttctatat tagtcatggc 10800
tctcaagaaa caagacgaat aggatgaata tatattaaag aagatttact aggttggctt 10860
accatattaa gatattgtct gtagtataac actgactgtt gtcataatgg agagccagag 10920
aacaggtggc tactctgtct acaaggctag atgccccaga agatccaatg tgatgtttaa 10980
tgtctggaag aatcctgaag cacccctaat ctttagtcca tattgggaag ctaaagaatc 11040
tgagtcctca tgtcagtgaa agatggcagc aacagaggta atagcggcaa cagagtagat 11100
acactcacca gtgggagaat aatgcaaaca gacagaagag aaatcgcttt ccccttggat 11160

cttctcatat actgtcatca aaggaagttc ccacacactc tgagggagat tgcttccctc 11220
cttaattttc ctagtaaata accttacaga cctgccttga acttctgagc taattccata 11280
tccattcaag ctgacaacaa aaagcaatca tcacaactcc atcccttgtt aaactgatac 11340
ccaaccacat ctccttatgt tatgcttcaa ttccaaaatg gtactgccaa gcatgctgta 11400
attatctgta aatgtgctat accaccccac ccactactcc ccagaatagg tgacaaagtc 11460
ttttgaggag atcttagtat tttactgtct cacaaattta aaatattctc tgtcatgatg 11520
gactacaatt ctctgaaaca gtgagccaaa attaaacttt tccttcccta agttggttct 11580
ttttagggga ttttgatctg atcaatgaca aagtaagtat cacaagtcaa gcacttctct 11640
gcctccccat gccctctgtt agcaacctcc atactcgatt ctcagtgtct tataaacgag 11700
ccaatacaag agattcctac tggggcccca tcacaccttt gatctaaatg acaagttaga 11760
attatagagg ccagaatgtc tatgcccaaa tatttccaag tagttatttt ctacaaagaa 11820
aattcgcatc acctagttgt tatttatggt ttcctatcgt ccagacccct ggcccccaag 11880
cagacgctgg gcctcagcta gcatcatgct ttccacacac agctttcatg cttatttttt 11940
tcttgtaaat gtcattttct attacataaa atggcattct ctgttttata tctaaatgat 12000
cttctttctt tagttgtctg ttctgatgcc acctcctcca ggtacaacag atactcccat 12060
agtgtgccct tttgcaccat cccttatatc aaactgtaat tattatacaa tagacaaacc 12120
cttctgacac agcagttctc aagaggatat ttgtacctgg ttcacctgaa aatcagcatg 12180
attcaaaatt aatcttaaat acctgataaa gtctttattt ccaagactca aaaatccatt 12240
tctcatgagt cttgcttaga aaaactagag ttagaagtta tttatgtgtt tccagcactg 12300
gacaggaaac catgtaggaa gcagacttca aggattatct ttgctttcaa ggccagtcct 12360
aaaaataact gccatcatta cttgggattt gggaaaagcc agtaccaagg acagaggaca 12420
tctgtcaggg gatttgagaa tgagtcagag atggctgttg taacaagatt gatcaatcac 12480
caactagtgg gaggaatgaa tggtctcagg gaagagatga agacaaggat ctctttgtgt 12540
ttggatatgt ggaaaatcac aacccataga aagaaatgaa tcgtgattaa gagtgtggct 12600
tcggtatctg agggctctgg gttcaaaata tttttgtttg ctatgtaagc ttgactaaat 12660
aactgctcta actttaatat gtaatatata acgtataatt tataatgtat aatgtatgat 12720
atatgatata tatgacacac ttagcaatgc ctatcttaga agatcatgat ggttattaaa 12780
tagtattgtg tattacattg cttccaaaaa actggtatgt gacacaaata gtgatggtca 12840
ttcatgttga tgctgctttt gagacagttc ttggtaataa gaccccactt ggtcagctgt 12900
tctcaaattg tatcctataa aacagatcct tactgaaagt cacttaaaga gggttctgtg 12960
gctgctaagt ctggaaacac ttgacaatat cttggaaatt cagggttcat atagtctttt 13020

cagctctagt tccaaaggct gacaacacag tagcagaaac aataaggtaa tatcctagaa 13080
ttcattataa aactattttt cttctatgtg ggcttctatg gcaaaatttt gtgtgctggg 13140
tagtatggaa agaacagaaa tccacttgcc tggagaattt gatcttaaga ataaagcacc 13200
aatagattcc atgtcttgga agtgtccaca tactggttca tagattaaga ttcacagctg 13260
tgcctacaca ggttggaaga gacaaggagg ggagggtcca tggggcatca ctctgaagga 13320
cagtgatccc attatagaaa gccctatcca catgaacagt tactttctaa caagtaatac 13380
tacaatattg ttaggttcca atacatgagt gttgggagga tccacaaata tccagatcat 13440
gccatgaggg aagagaagtt atagaaagga gccagattaa ctcctaaaac cttcagaatg 13500
gaatatggga ctgaggtctt tagacattgg ttaggaatct gaatatccct gtttgattag 13560
aaactaatgg aatgtttgat gcgatcaaat tcagaatctg agaaattctg tccagctctg 13620
gtgaatccat atccccaatc tgatgagacc actgacaccc ttatccatat gaaagctgcc 13680
ttaaaatgtg ttggaccatg atcttggaaa cgataccaga gcaccaggct ccccagatgc 13740
ttaagggaac agtaaatttg ccctcacaca tttaagctga cagtaaaaag ggcaaacatc 13800
ctacttgagc caaaacaggc atgcccaggt caggaaacct agctggagag gcagagacat 13860
gtagagtgtc atcccaatta gcaactcttt ctggctaggg aacccagtga gcaaagccag 13920
agtctggcag attctggaac cctgtctgag ccaaagagaa cttgccaaca tgtacccagg 13980
agctaataac cacttgcaaa cagtgcacct tggcttcagt gctctccaga gaaaaagtgc 14040
acgaagtagg agagccagcc agagctcaat gaggaaggca gaggtcgaat acttccagag 14100
ttacatgtgg agactactta gacagctgct aggggtctgg gaagcagaag atgaaggaac 14160
tgtttctgca ctccttatct tgtcccttac tgagcagttc aacttgtaag gtggcacttt 14220
ttttcagaac tgaagaaagt gtttgaggag ccatcccagt gtggcacaat atgattgaaa 14280
tgtcccacat gagagtcaca gtgcaagctc caaaggctgc ttctgtctca tgttctgtct 14340
catgacttag tccttatgct tccctgtgtc ttagtttaac ttccaaacag gtagagtaat 14400
ttgctcctca ctagcgtttt atgtcagtaa taaaatcaat gaaaaacctg aagcataaca 14460
ttaggcacaa gacaagtttg ttttaatgtg tgtgtgtgtg tgtgtgtgtg cgtgcgcgcg 14520
tgcgcgcaca cacacacaca cacacaagca ggcaaacata tttgtggaag tcagaggaca 14580
atttgacaac ttaggggagt tagttctctc tttatttcac attggtctca gggattgatc 14640
tcaatcacgt ggcttgacaa cgggtgcctt tatccactga actattccac aggctcacaa 14700
aacagattta taaacagtaa ataagaaaat gttggtttta ttgccaatcc agctctattt 14760
gaatatataa caaaaactgt aataatcact atattcctcc aggggtcagc aaaatgtgat 14820
gtagaagcct atttttgtac attttttttgt attagaaaaa agccatgttt gttttcatat 14880
tttctataac agtgcctact acaatagtaa atttagatac ttatgaagtt gtgtctatgc 14940

gaccaaaata cctcacagaa gcaacataga ggaaaaaggg tttgtttggg ctcggaattt 15000

cagagtctta ctccaggatg gcaggggagg gcagctcagg actcagcctg aggtgtcaga 15060

agcatgcagc cctggcttgc ttacctggat ggcagggagc atagtaaatt agactggaaa 15120

gagattggct ctaaacccac aatgcccacc acccaccttt ctggcctcct tctacccacc 15180

tggcccacat tcccaaagac tccacagcat ccaaattatt cccacagctg aagaccaagt 15240

gttcaaactg ggaactcaga ttcaaaccct aggagaaaga ggtgatctga cagcacatgt 15300

attctcaggt catttaagaa aatgttaact gacccttcca atgagtatgc cagattttca 15360

tgcagtttcc agttactgtg accttggcaa gccatatgtt attatacaca taataagaca 15420

aagacagaaa tgaaagtttg tttaataagt cttcccagca caggtagagg cacaatgcca 15480

aactctatac aagaaggaag tgtagaagac tgatagtcac gactgtgccc catcctccct 15540

ttatttattt tgcactttct cactctctta ctctttcctc tttcctcccc ctccatccct 15600

ccctccatta tcagctccct ttccctcccc accttcttct tctccctttc cttctcctat 15660

ggtctttcct aaccctgttc cctcaggtgg aatattctga ttatcccacc atatgctgaa 15720

ataaataact ctgctgagaa ggaatttaat tctctttcct tctttgaaga tttgcttgtt 15780

tagacataaa attctgcctc cttctgatca cggccactgt gataaaaaat actcggagaa 15840

aaacatgaaa gggtttattt tgttcacaac ttgggatcca ttattttggg acagtatagc 15900

acaatgacgt gaagcagcta gtaactacat tatattcaac atcaaaagaa ttgggtgttg 15960

aattaaggga tgcatatctt gtgttcaaat tactttcttt gttcttatat agtccaggat 16020

accatgccta gggaatggta ccacccatga tgagcaggtc tttctgcctc aattaacgta 16080

atcaaaacaa gcaaacccac aaaccacctc atctagacaa ttcattattg aggtttttct 16140

cccccaggtg actcagttgt gtcaagttaa caatcccaat gtaaaattcc aaaagaaagt 16200

ttgaagtgtc agggtctctg aaattttctt atgttctgac tgatttcttg gctatacata 16260

gtatgaggcc caattttcca ggacccattg atgtttaaac tcccctgtct gctacatctt 16320

gctttatcat cctctcattt attaacattc tctcttggcc aagctggagg ggagcagggt 16380

gttctcttcc agctctagag acctgctgga gatacagaca ttagttcact tggcttcagg 16440

ccctctcctc aagcccaaaa tggtaaataa aatcgccgtg cgcaccccag ttttccaatt 16500

tcattctccc tctatgactc caacaacttc attcacatta atgaaactga gtgttcccct 16560

ggcgtttaac tattacataa atgttaattt ctgagaccaa gcaataaatc ctgtttcaaa 16620

aagaaaaaaa aaacactcaa taggacaaaa ggaacctaaa cttccacaga ataagacaga 16680

gacagtatcc ttttttttcag attcaagttg tactgactag ataacttgct gaactgtctc 16740

aagcctgtgc ttttgatggc aggaaaatat gaacagtgtt ctctgattgc ctaagcttgg 16800

atcgtgatga aaaggcattt gtaatcgcca agaaccaaat aaatatactg atttgtctca 16860
ccctaggctc tagttgcttg agctcttcca tcctggggct tcaccctttt atgaaagaca 16920
ggtcctggcc aggtatagca tcatttctat tgtgtaagga ctgaaaagag agaggatcaa 16980
gggtttgtcc ggtgccagta cttactgccc cttgtgtgcc atcaaaccca acctgtgacg 17040
tacttcaaac acaaaagaat caagtaagtt tgggaaacac acataaacga tcttcttgga 17100
ggtttccaca aacacataaa agaccctgga aaatcttgaa ataaagtaag atacgtaatt 17160
tgtttggctt aaaataacat tggctttaaa aatagccttc tccaaacatg attaactagg 17220
aaataactct ttgctcagca ttaatacctc tcaacatgcc acagactaca cattaggaaa 17280
tagcttcctg catttgtatg gctaaattaa gcctaattct gagtttaggc ttatctttat 17340
ctctattttc tggctcttgt gtaacttcat aaagttgttt tacactgtta ataggggttt 17400
ttttgggggg gaggtggcaa actcaagctt attttgaagt taatcctttg aacttagtca 17460
tgcaaaccag cctttggctt cactaaattg ctggaagaaa gctgagggtt aagaatgctg 17520
cctctaactg atgagtcaga ccacctgaat taaactctcc tctcctcttc tagctatgtg 17580
accccagttt ccccatcctc agggagagga aaataaagga agaaatgtca gggatcttcg 17640
agaagaagca atgaaataat ccatacaagg cacagacagt acagatgttt tcaatcagga 17700
ctggttatta ttgtagcttt taggtgatcc ctttttgaga aaggaaaagt agacaccatg 17760
tgtctgcatg ccttttctag gcacactagt taattactgg agtggactca gttttccata 17820
gtgtctggtt tcttgctacc aatcttagag atccattttt atttcagagg tcaggtccta 17880
tcaaagtgta tcaaggctgg ctgatcttca tgcctcctgg gtttggtaac tgccagtgtc 17940
tctatctggc tgcatctaag agcaaacctc aggccagcag aattccactc atttgaatgc 18000
caagactgtt atttcagaaa aggtttccga acactctagt ccccaaatgc caaatattta 18060
gcacatgcat tcacagtgtc ctgctcctgt gttgctctct atctataacc tgtccctcct 18120
gaataaatag ctctaattct ttaaccttgg ctcaatcgtt tgagacctag gaaagagcca 18180
actgcatttc ttaaaattct gatggaaatt caaaatagag gaggctattg ttccaggaga 18240
aatgaattca aatttgtttt tgaaatgatt ttcaagaatt tcataggaat ctatagtgtt 18300
gccatttggt caaacccact ttttattttt tcttttatgt ctaggcaagg agcagaagag 18360
ggaaaactac tgctctgcag aaattttatt tcaaaaattg aaaccagacc atcaggaggt 18420
agcttgtggt ctgaatgtat caatgggcct agtcattttt catcagagct gctcttaggt 18480
atactaaacc acaaatatga atctcattat tgtgtctaga tggaaatatt ttatggatta 18540
aaatttgggg acttaatttg gttagcaatg aaacttctgt tcaagaagag ggaggagggg 18600
agccacaaaa taccattcat ccaaacacct gaaaggaaaa aaaatcaata taatatctgg 18660
atccctttgt atttaatatt agccattttg cttgtgttta agtggaaggc taaatttggt 18720

ttgacagaac tattgccaaa ttgcaacagg tcgttacaac caagaacatt ctaaaacttt 18780
ggttggttgt atcttaggta cagtttttag tgttgggtat acacctcgat accacacata 18840
acttgccttc cacatataac atgaaaccca aatccctata tagtctatag tctaactatg 18900
cataactaaa tcaacctagc ataagtataa aatagaactg aaacatttct agacatcaaa 18960
aggcaattca aaagacagaa aggaaaataa ctaacttcca ataatggatg gcattttagt 19020
ttcactggga ggacagatta agcctgtcca ttcaagtgga gcaaatgggc tttcaagaca 19080
ccaagacctc tctccaataa taagtcagat tctgcagcat ctcagaggta cccaacagct 19140
tgcttcactc cacagcagca ccatcaggtt ctgatcaaac agaacagata gactcccctt 19200
cagcctgctg tttgaaggtc agataggcag agcagcaact gctctcaaat gctaactcct 19260
ttcattgact tccagtgcaa agggcagtga atgatcatgg actcagaggg acttgggttc 19320
acatccttac tccttagtga cacttcaatt tgcacatatg acataataat tatatgtctc 19380
agtttcttta tcttcaaaat gaagataaaa aactttatct cgcaacttgg aaagaatttg 19440
aaatcatgtg tttaaaattc ttagcacaag ctctagaaaa atacaagcaa tgaatcactc 19500
agtatcttct tcacttctat tatcattgtt ggtatccttc tccttgaact ctcaagaact 19560
cttcaaccag tatttggttc ccctgggcct ttcctccttc agtaatacct cggtgagctc 19620
cttggccaag agtactactt ccattcctta actctttcac tgaggctggc actgctacta 19680
ctcttgtgca tgtataatct ccacatgcat ttccataatg acctctggga aaaggacaca 19740
gctcttcact cagtcgctcc tccccagtgt ccttcacact gtgaagccca cagtgagtaa 19800
gaagcaatta tctctggaaa gaatttcagc attcctcccc ctttctgttg ggatagaacc 19860
atgtaacatg ctctagtttg tagtggcact tgagagtagg ttgaaaaaca gttacgtttt 19920
ggactgtcta ggaggtagtt ttaagtttta ttactgtaga gttcacattt gttttggcta 19980
ctgttgtatc attcatactt aactagaggc ttgacacacg gtaggtattt aataaggatg 20040
gatggatgga tggatggatg gatggatgga taaaagaaag gaggatgaat ggaggccagg 20100
tagatgaatg gatggatgga tagatggatg gataaatgga tggaggtcag gtagatgaat 20160
ggatggattg atggatggat gaatggaaca aagacataca gaatcaagaa ttgtagaaag 20220
ctcttagcac aagagtcctc tcagttcctt tctggagtac acttctctct ggagaccatt 20280
ataatggact caagttaagc attaatggaa acttttcata tgtacttcct tctattattt 20340
ttattttact tttgagatta taatataatt atgacacttc tttcttcccc ttcttttctc 20400
caggccttcc cacatacccc acctccctgc tatccttcaa atccaaggcc tctttttca 20460
ctaattggta ttgcaagcat agatataaca tgtccagtac ttagcattat aaacttacat 20520
gtatgtttca gggctgacat tttagcactg aacaaccatt tctctggaga ggaccacctc 20580

tttcattgcc agtgcattgc ttgattacct agagttcttt gtgtagggtt taggcctcgt 20640
gggctttcca tattaacttt ctcatgtctg ttgttggcat ccttagttag ctcagatttg 20700
gttagccatt gttggtgaga ctttgtgagg atagccgtgg atattactag gaaacacaga 20760
tttatatcag actcctcaat tctctgattc ttacactctt ttatcctatc ttctataaca 20820
tcccctgaaa tttatgtgtg gttgcatttt acagatatat ccattagggc tgagctccat 20880
aactctacat cttgattggt tatggttttc tgtagtgctc tccatctgtt gcagagacat 20940
attcttggcc ttatttttat tattttttct agctttactg aggtagaatt taaaagcatt 21000
ggtgtagtag tagacagaca gattgattct tagtagacag acagatagaa atgttaccac 21060
tttcaagatc ataactaaca tatctaacac cacacaaagt tactttgttt gtataggatg 21120
agactaacaa atatcttcag tcttggtaaa ttccaaatat acaatgtaat attatagatc 21180
cattttacca taatggtttc catatataaa tatatgttat gatgaatgca tatggctgtt 21240
tggggtaatc tatgcagcat aagtgtacag taggactttc tcagaactgt gtcattattt 21300
gtctgcatgt acaaagtatg tgggtagcac taatttacca cttctggact cagtgatcct 21360
ctcctgttgg ttgtattccc tgctcagtta tgattttgat tttataatct gatttctgac 21420
tgcatacgga aagcactgcg aggctgggcc aagtgttttc cattaagaga acagttttga 21480
aagactaaac cacagacagt ctgttctgaa gaaatcgctg gccgggtatc atggacaacc 21540
atcccagtag cagctctcgg cctcagaaaa gtcttggcca aagttctgac ctcccagaaa 21600
agagctccac ccaactcatt cactcccttg ttcctgctac tgaattagat gaaggctaag 21660
ggaatcctgg gtctgcagcc acagcctttt cgatctccta aggatttcag aatgccctga 21720
tatcagacta actgaaacac tgaggactgt aggctcccac agagaaagcc acagtgggaa 21780
ctagaaagat ggttcagtag gtgatggtgc ttacagagca agcctgatga cccgagttca 21840
gatccctagg atccatgtaa ataaccagat ggaatcccaa cagtgtgtaa tcccgacatt 21900
cctacagcag gaagagaatc agggacagaa tcacctagaa gttcatggac ctgctagcaa 21960
actgggctca gtgcaacaga gaagcaaaag agaccttgct tccaacaagg tggaaggtga 22020
caattggctc ccagaagctg gcctctgccc actccctgtg tacacatgca tgcacacaga 22080
cccacgtgca caattaaata aaacctaatg ggaaaaaagg aaggagggag aagagaaaga 22140
ggaggaaaga gaaagaaaca atgagcttaa ggaaaactga atacgtttgg agaaaaggaa 22200
ggacttctcg acagtacctt aagaatttat ttttggtcac aaagtagcct tctgctaacc 22260
gccttctcca aacaagacaa agcaatcatg cctactagac ttaaggtcta tcttctttta 22320
agtgttagct aagaaagtgt tttgcataaa ttatctcatt tcttctcaca tgcagctggt 22380
attgatgttg tcattataca gatgttagca cagaggccta gacaagcaat attttatatc 22440
cacttccata gcaacaaggt ggaattgtgt ggaattgctg ccacccatcg tttttacct 22500

aaagataatt tgcaacttgc tgtggttcat actaataaga agaacagagt caagcatatt 22560
tgtctgcctc tagagcaggt ggtcccaaac atcacatagt ctcttctagg atatttattc 22620
actttagcaa ttatgatttg tcccagtaaa tatctgtttc tatgactgcc tccttgactg 22680
acctgtgacc tcagtacaaa cacagagctg tccatcactg gtctctgaaa ctctctacca 22740
gcctaagtct atcccaagac ctatagcaca tcattcaact catctctagt gggaaaaaaa 22800
agaacaaatc gtttttaagt gacaagtctc tgaggcaatc agcagtccca ttgatatgct 22860
actggacacc aatcaagggt ctatgatcct atctcaaata gataatgact tgccgatact 22920
aaaattcaga ccctcatggc tcaggagggt ctttggcttg tcagtttaag tttttttctgt 22980
ctatttttttc acccagagtg tcctggattc actcaactga ggggatcaga tgtccttgga 23040
aaacacatct acacttaagg ctatttcctg gctcctggct cttcctgaca cattccaaga 23100
aggaatttat ggctgttggg agggacgagg agtgaggaat ttgtagacca ttttagaagc 23160
ctgtgttagc agccccatgc ttgactgacc tagcacaata aaattttctc ctctgttagc 23220
cagaagaaaa atttgccgta ataccttaga gttgccaaaa tgatgtggaa acaagttcat 23280
ccgcatttga aaaagtagag tcattgttgt ctatccgagt ccaggatatt ccatgtatgt 23340
acctaagtca aaaagataaa cacattccat gagctactaa tcacgatgtc acaagtaact 23400
aacacaaagt gtgtctccag ccacataagg ctatccagat aaagttgata cttgtctaaa 23460
actgaaatca tgtattgctt attctccaac tatgaccagg gaaaatattc aaagaatgac 23520
tcctttatct cataatggtc tccagcctgt gaagagataa ctaaagataa tctttagtat 23580
atctcatgct tcttataggc gttgaagtgt ccaaactagt caccaaccca gtcatccata 23640
aagagcatct acagcagcac ttgttctttt gggtacatga agacaacccc acatctcctt 23700
ctctgggatt gatatatggt aatactgata cactccatgt tggcttcctc agctatgaat 23760
gaaggagcac aagtcatcag tttcctataa cacattctct ggactcttca tttctagtca 23820
cttcccatag atataagagt ccagggcact ggagagatga atctatggtt aagagcatct 23880
gctgcacttc cagaggacct gggtgtgatt cctagcacct acatggtagc tcacatccat 23940
ctataacttg agttctagat taggggttat tattcactct tctggcctcc acaggcagca 24000
ggcatgcatg tggtacatag atatacacac aggcagcagg catgcatatg atatccataa 24060
agatatacat gtaggcaaaa acattcacac acaaaatatg tttttttaat gataatcatt 24120
tatctgtact ttttatcatc tttgtaaatt aacttgttca tcctgaaatt ataaaagcac 24180
aagctatctg aacttgactc cccatgtcta acctctatct ctcaaactgc aatctcaact 24240
aatttcccca aagtctaccc tagacataaa ccagccagtt atctgcgtgt agcagaatat 24300
ttttgcattg ctaatgtcat tatttctctc cagttctaca ctctctagag tacacatttc 24360

tatttctagc aaattgatcg tcacaaggtt tagtttctca tacaccagtg atttctacag 24420
cttctctgat ccagatccaa gttgccactg aatttgggac tggattcaac taaggccaag 24480
ttctgactta aacaaaaagt gctattggca tagactcttg ctagtgtgtg atcccaagag 24540
tgcaccacaa tgtcatgacc tgttgattga ctacctataa agcaccaaag ttctgaagct 24600
ccagtacaga cctctaatcc ataattcccc ctggtgtgtg tgtgtgtgtg tgtgtgtgtg 24660
tgtgtgtgtg tgtgtgtgtg tggtgtttgt gtgggtggtg gtggtgccac agaagccaga 24720
agtcaacctt aagtgtcttt cctcaatttc ctccagagcc atcaaccttg tttggggggt 24780
ttgggtttct tgggggtaag gaggtgagac tgggcctatc ccttggtctg gagcttgcag 24840
attccacctt gaagcctcgg agatctacaa gcatgtactc ctatgcccac ccttttacat 24900
agattcaaag aaccaaactc aagtcctcat gctttcatga caaacacttt actaactgag 24960
ccatctttga aattttttct gagccttatt ttataaacaa ggccagggtt gtggattgaa 25020
tgacaagtat cttgcccaag ttatggaacc agaaagggct tagatgttcc acaacacaga 25080
agatgtggat gatctttccc aaagcaccac ccccaatcct cagacaccat gagctgatgt 25140
gaaagaaaac tctcaaaaat gtcattcctc ccctcttaac tacactatga aatagtgtct 25200
ctcttcccac attatcctta agtcttcctt tggggtctca accaacagct caggtgtttg 25260
tcataatgag agaagacctt aggtaacttt ctgtcaagta tttatttctc acatataagc 25320
cattgttttg taggtcttaa aatgagccag aatttagcta ggctctcatg ttttcactca 25380
gtaatttctc aagcatcttt gacagctcct ctaccctacc tgaatgtctg tgcatatgaa 25440
tggcatcgtg actgatgaat agttagctca gcagtcaaaa gaacttcaag gtacatcttc 25500
ctccacattc tgtctcattt aatcctcctc actatgtcac atcctcaatt gcttccttcc 25560
cactaagcca tactttgcta ttcgttagac aattcctgtg attttcaaag ccataagcag 25620
ttgactctat tctcagaaaa caaatcatct acattcttgt ttttctttgt tctctaaata 25680
ctgaaaggat tgttctgttt tctttttagt tttgttgtta gaagagtcca tgcctgatct 25740
tatatttatt tctaccaccc acagaacttt ggttgctata gaacattcgg tataaatttt 25800
ctcggtacca gtcaatttaa gaagtcacta gaaactagtg gacaactctt aatttgggta 25860
cctgattccg aagtgcattg tcacgtcctt ctaaagctta gattgcgcaa tgttctggat 25920
agataaatca aggaaaaggg aatctgaggt acaaagaaag gatgctacgc aagagcctca 25980
tgactagaaa gaagcaatgt aggacctaaa acctcatttt cccctgagta ctgtgcttcc 26040
cccctctgtc tccagtgaca gagaaacaga agcacttatt tcagcttgaa gaatttataa 26100
ttacagtcag gagtcattga acatctcctg cagctacact tcacaggacc agaaaaattc 26160
cctcagaagg cgtggcttgt aaatactgag ggacaaggaa tgatctcatc gggtcagcct 26220
tctaaccctg ccattggcca ggattttcac tgtaaaaagt gaaacaaggg acaaagttaa 26280

gtaacttccc taagtgctca gatccgaagc tgcgatacaa gcccctgcct accttttcct 26340
aatgttctgc caggtctttc ttttctctat tgtctcttta cattttcaaa ggttgagtga 26400
gtaatgtcaa tctttcaaaa ttgcaagatt tttctagaga ccattttatg tcctcattac 26460
catatatagt aaagggatat caagaaagtt tcagacccag gggcaagaa ggcaaaaatc 26520
actcggaatt gtggccactc cacagtgtat tagggacagt gcctaataac tattgttttc 26580
ctttatttat agatccttct tccaagaaga ttggggacat ccaactgtat tcctttgagt 26640
acatctaaat accaaactct actgggagtt caggcaccct acagatctag agacacctag 26700
ccagtccaac ctccaccatg ggggccgtga cctggctact gccaggcatc tttctagctt 26760
tgtttgccct cactcccgaa ggtggggtcc tcaagaaaat catcaggcac aagcgagaga 26820
gtgggctaaa catgaccctg ccagaagaga atcagccagt ggtgttcaac catatctaca 26880
acatcaagtt gcccatgggt tcccaatgct cagtggatct ggagtcagcg agtggggaga 26940
aagacctgac ccccacgcca gagtccagtg ggagcttcca ggaacataca gtggatgggg 27000
aaaaccaaat tgtattcaca caccgcatca acatccccg ccgggcctgt ggctgtgctg 27060
cagctccaga tgtgaaggag ctcctgagca ggctggagga gctggagttg ctggtatcgt 27120
ctctaaggga gcagtgcacc atgggtacag gctgttgcct ccaacctgca gaaggtaggc 27180
ttcccagaag gcaacagggt tcgttgaccc tgtctctgca ccaatgagaa gatggtgagc 27240
ttaaaggact ccatgagtga ttttttaaag ctggtttcct gggaaactag acagacaagg 27300
tagtgatttc aaagagagac agagggagag attttaaggt caaggaaagg tagagattgg 27360
agccatttct cttctgttct tggaagagtt gtgctaataa tagctctcac actaatttcc 27420
tagcatttga aaaatctgtt tcataaagct ctgtttcact taattatatc tgttggattt 27480
attggtctga agagtgagct cagcagttaa gagcacttgc tctcccagag gatacagatt 27540
ccatcaccta catgggcact caaaacatct gtaattccac tctcagggat caacaccctt 27600
ttagatcctc agcgtacacc agccatacat gcaaagaact cagacctatg aaatgataaa 27660
ataaataaat ctaaatgttc taagatttat ttatttgatt acttttgtgt atatatgttt 27720
gtgtgtgagt gtgtacacat gtgtgggtat tcagtcaaac actcttataa acgtgcacta 27780
agggcagaag aaggcctgga catcctctat cactgcctct caattcctct gaggcagcat 27840
cctccttgaa cctggggctc acattttctc ggctaaacta gaaaccagca agccccagtg 27900
atgcccctgt ctctactcac atgcaagatg aagatacagg catacagtca taggagctgg 27960
gattggaact ctgcttctca tgattgcaca gcaagcaccc tcatctctct agtccctact 28020
ttaattttat cttggtcaaa catgtttggc tctgaaatat aatttgtagt taccctaaaa 28080
catctgagaa taacacagtg tccctcctcc caaaggcatc tctcaaagag actgctgtgt 28140

agccttatca actcactgta caaataggga aacaaaggcc cagaggggag tgaggttctc 28200
caggtcctgt tgagttaggt gttaacacag tcttccacag tcctgtgttc ttacaatggc 28260
ccatttctgc ctgggtggta ctgagcctgt gagatcagga ggagagtatt ctaacgtgat 28320
ggaacaaaca cagcgtgata atgggaaggt ggtcaggtca gagctgccca cacagccacc 28380
tgtatgtacc agacacagta actttcttct gtgatcctcc caacaaccct ttaaggtaaa 28440
gtttaacatc tctatgtgaa tctaaaaact gaggttttaa aagtcgacca aatttcacag 28500
gatgattcag tgataaagga gaacacgcat agactttgga tgtgcccggg aacttcactc 28560
tctctggagg acactcggga cccttgcaca tctattccag taatcctgct gcttaggagt 28620
tcattatatc cccccgcccc acctcccaga attccagagg aatacaaaag cattaacttc 28680
aagtcagaaa tgagtatctg tttccaccac ttagaaaata ctatcagaaa gggcattgat 28740
aatgatgttg agagtggacc tgactaccat atgcagggag tactgtggga ctctaggctt 28800
ctaaggcaat tttcaaaagt ttatctttat taataaatca attcattttc tctctgtcaa 28860
ccccccttgag gaaacatgtg tctgagaaga cctggtgggt ttaatgtcag tgtggcttaa 28920
atgtgactga attccatctc attttttcca ctaagcctct gctctccacc cagttgatgc 28980
ctaggcatgc agctgaattt gagtgagggc tttttcatgt gagtaccaaa agccagctct 29040
gatgtaccca gctaaatacc tattatcttc aatgcaggtc gtctggacac caggcccttc 29100
tgcagcggac ggggcaactt cagtgcagaa ggctgcggct gtgtctgtga accaggctgg 29160
aaaggcccca actgctctga gcctgactgc cctgggaact gtaatctcag aggccagtgc 29220
cttgacggcc agtgtatctg tgacgagggt ttcactgggg aagactgcag ccagctggcc 29280
tgtccgaacg actgcaatga ccagggcagg tgtgtgaacg ggtctgtgt gtgcttcgaa 29340
ggctatgccg gccctgactg tggcctggaa gtctgcccag tgccctgcag cgaggaacac 29400
gggatgtgtg tggatggcag gtgtgtgtgc aaagatggct ttgcgggtga ggactgcaac 29460
gagcccctttt gcctcaacaa ctgctacaat cgtgggcggt gtgtggagaa cgagtgtgtc 29520
tgcgacgagg gcttcactgg cgaggactgc agtgagctca tttgccccaa cgactgcttc 29580
gaccgcggtc gctgcatcaa tggcacctgc tactgtgaag aaggtttcac gggcgaagac 29640
tgcggtgagc tcacctgccc caacgactgt cagggccgtg gccagtgtga ggagggacag 29700
tgtgtttgca acgagggctt tgcaggagca gactgcagtg aaaagcggtg tcccgccgat 29760
tgtcaccacc gtggccgctg cctcaacggg cagtgtgagt gtgacgatgg gttcacaggg 29820
gctgactgtg ggatctcca gtgtcccaat ggctgcagtg ggcatggccg ttgtgtcaac 29880
gggcagtgcg tgtgtgacga gggctatacc ggagaagact gtagccagcg gcgatgcccc 29940
aatgactgtc acaaccgggg tctctgcgta cagggcaagt gcatatgtga acaaggcttc 30000
aagggctttg actgtagtga gatgagctgt cccaatgact gccaccaaca tggccgctgt 30060

gtgaatggca tgtgtatctg tgatgacgac tacactgggg aagactgcag agaccggcgc 30120
tgtccccggg actgtagcca gcgggggcgc tgtgtagacg gacagtgcat ctgtgaggat 30180
ggcttcactg gccctgactg tgctgagctt tcctgcccca gtgactgcca cggccatggc 30240
cgctgtgtga atggccaatg catctgccac gagggcttca ctggcaaaga ctgcaaagag 30300
caaaggtgcc ccagtgattg ccatggccaa ggccgctgtg aggacggcca gtgtatctgc 30360
catgagggct ttacgggcct ggactgtggg cagcgctcct gtcccaatga ctgcagcaac 30420
caaggacaat gtgtgtcagg ccgctgcatc tgcaatgaag gttacacagg gatagactgc 30480
tctgagggtg agtatcagca agctggcctg caagtggtca tgatggtgta tatacgcaat 30540
gcctatcaga gacagatagt gggatttatc aacataccaa ttcttttaat gtgccctgtt 30600
agtatattcc tgttaaacac acactctttt tctgacccta tgaggggttt atactcagtc 30660
tcttggcact gggcaactta gatcaaaggc tcagagaagt aatactgttg tccaaaagac 30720
tcaaatgata cttaaatatt atctacctca actgtattga atcagccatt cattgtcctt 30780
tacttcgtat cagtttcctt tctttctcta ctctggctat gaaccaacag caaacaaagt 30840
tcttccattt gaatggaaat gataattccc tgggctcaac agacatttct attaaataag 30900
attaacttat taatgcagtc aactaaagaa tggtctctgg ttacctggga accagacctg 30960
atgcgtttgg tgctatcctt atccaaggac tgtgagcatc tcccagaata ctgtttccag 31020
gagttatcat aagcataggc atatagtatg tgttcaagaa acattgacta aatgagtaca 31080
cagcaaagaa caaaaacaaa caaaaaaaaa aaccactcat gctcaatgac cgttttctct 31140
tcatttttct cgccctcata tcatgataaa gaacatctta caaaggtagg caggaacaaa 31200
taaatagccc cactgtttag tatgtctgaa ctgtgcaagc ttagtaaaga catgtgggtt 31260
ctaaacctga tgtaggcaac cacagggaaa cctcttgccc ttagtcacag ctaaggaaca 31320
agagagtctg gttagacaaa ccctatagga ctaagtcaca ggggccaggc tgaggtgata 31380
gttttctata ccctggtata actctgagct aagacccaga aagtggaaaa gtgtcgaaca 31440
cagtgttgaa agcaacacta ttggccaggg acttcaacat aaaggcaatg ggctaataga 31500
ttacaaagct acttctggtg gtaaaggtct gtaaacccag ctcctacaga ggctgaggca 31560
agaagatcaa gttcaatgcc tgcctgggtc acattcaaga taagcctagg ccaggtggtc 31620
aaaatagatt ggatggcatt ctcagagaac taatgaaaat gtttttaaag taaaaagaaa 31680
tagaggagga gaattttctt atagggaaag tgacagacct cttttaaata ctttttagag 31740
tattttattt tattttattt tattttattt tatgtgtgtt agagtttacc ttgaatgtat 31800
gtgtatgaac cacctgtacc tgcagatgtt aaaaaagggc atcagagtcc ctggaactgg 31860
agttacaggt ggttgtgagt cattacttgg atactgggaa tggaaccctg gtcctctgca 31920

agaacaaaag tgctcttagc cactgcgtaa tctctctagc cccttgactg acagaacttg 31980
tatctagtac actcagtaga gctctggctg agtccagaag gttgggaaca atgtgtttta 32040
agaacagaca acttcagatc tctattgtaa atagcagaag agacttatca agggagttaa 32100
gcctataggg tactacacga atactcttga cctccaatat cctttcttat tcctctcagt 32160
gtcccctccc aaagacctta ttgtgacaga agtaacagag gagactgtaa atctggcatg 32220
ggacaatgag atgcgggtca ctgagtacct cattatgtac acacccaccc atgctgatgg 32280
cctagagatg cagttccgtg tgcctgggga ccagacatcc accaccattc gggagctgga 32340
accaggggtg gagtacttca ttcgtgtgtt cgccatcttg gaaaacaaga ggagcatccc 32400
tgtcagtgcc agggttgcca cctgtgagta ggcagaggat tctgggtaga ctcacctagc 32460
cttggccaag ctgtgtagca ctatgcatac tttaagtgcc caatatatac taacacactt 32520
cacccaatga gacagaagca attgttaacc ccatgtgcag atgaggaaac tgagaccaga 32580
cagttcatca tctcctctca atccccacag ctaacaaaga tggagttggg ctttaccctg 32640
gaaagtctta ggagattaga tcttaatctt cttatcacca ttgtgtggag ttctcctcta 32700
ttgacctcac tgtctcaggt tattccttta agagtcagta caattacctt catgcctgag 32760
aagcaaactg tggctagaaa ctggccctta cttgctaagc aataacagca ttggcctttc 32820
cttactgagt gtgcactgca tgccaagagc taggccagca gacttcacgg aatgtattac 32880
tcagtccttg gtgcatacct gagggaagac acagttacca ccttctccca ggaaaagcaa 32940
tgatctcaga gctttgtata aatcttgtcc aaagtcacag tgttatcggt ggtgggttag 33000
atgctgatcc aggtccatct gattccaaat cctatgtatg ctttgctaat tcaacaatat 33060
gaaatcataa ataagcagtc atatgattgg tcataatagg ggcattcaca ttttctcaca 33120
gtagactgca tgccagaaaa gcctttaagt agaccccaga tttggttact agcctcaaag 33180
acagctgtct gtcacaggtt tagtgtatcc ctagctgtgt ggctacctag gatcttgcct 33240
ttccttatat cctatttgtg ctgttactta cagatttgcc tgcacctgaa ggtctaaaat 33300
tcaagtctat caaggagaca tctgtggaag tagagtggga tcctctagac attgctttcg 33360
aaacatggga gatcattttc cgaaatatgg taaggggctc cctggaggaa gtctttgata 33420
taggagattc acaaggtggt caaaggcccc tgctgcttct tatgttactt ggtttcctta 33480
gctttgggtg gaagactggg cactatcact ttgtgtgatc attatgtatt taagtgatgt 33540
taatttcttc cctgctgtta ataaatagaa aagtatagca tttagagatg agagtccgga 33600
caaacacaag ttaaatcatc gataggtacc aacttgcaaa tctctatttg ggataacaga 33660
aacagaacca gtctgcccta tacagtattt cctggcacat ctcaagtatg agtagatctt 33720
cctaggacac aaagattagg taaatcggtt ttctggttat aacccaggcc ctgtccccaa 33780
ttatctggga aagcattggg caagccacca acttaaaact gctgacttac agaaggaagg 33840

gctgagttac ggatctcaca gggtgccgtt tgacaggaac aggataagtg caggtgtcta 33900

caggaggaat aaacaaactc gagaagcagc gttctcatct ctgttttctc agcatctaac 33960

ctcccaatct gctgggctac ataggagggt acctaatgaa taaattaaac aagaagcaga 34020

aacaagtgaa gcataaagat gcatggctgt ccctgcctct gaatcttagc cctttgttgt 34080

ttccatcctg cagaacaaag aagatgaggg agagatcaca aaaagtctga ggagaccaga 34140

gacctcctac cgccaaactg gccttgctcc tggccaagag tatgaaatat ctctgcacat 34200

tgtgaaaaac aacacccgag gccccggctt gaagaaagta accacaaccc gtgagtacca 34260

gccaagacct gtgatagagc tgattgtgtc tctttgcaac ttctggctta tagcataccc 34320

tctgacacag catctgcatc agtgagaatt tgcttaccaa tacaaagtcc atatggagtg 34380

ccttaagtga taattctgca ggtttgcgat ttggacttgg gctcagctgt gtgatgcttg 34440

tccttgactg ggatcacttg tgcatctttg tgaactgaga ctgtttggcc tagaatggtt 34500

ccagttaaga cagtcacctg taatccttgc actctagaag ctgagacagg gtattagcat 34560

gagtcatggc catcctggac tccatggtga cttctaggat gatataagct atggaaggag 34620

acactgtcac aataaaacaa aagcaaagca taatgagatg gcaatttttt tttttatcc 34680

ttggagcctc cttgtcattg ggttagccgg ggcatggtgg catggctgtt gagtagactc 34740

aaaattgaca tttctgtcac tgctgtcaga ggaaattctc aggccaggac acatgggaga 34800

gacagagaaa cagatgcatc ccctctctga agctcctcaa aggtatattc tcaggactgt 34860

agatgcagaa aaggtaacca tggagacgat cacaagtcat ctaccacacg agctcattta 34920

atcctaacac caagtctccc atgacagagg tgaggtgtat acggatggta ctgtgtatga 34980

aaacgagtga atacgccttg ggaagttga gtcactttaa catgatgtag gccagacagt 35040

catgactcaa gcacagctga gaaatggaga aaagaggatc cccaaggctt gctgatcagg 35100

tcaaggctag ccagggaccc tggctcaagg agtaagttgg acagtccaag gaaagatgcc 35160

taaggttgat ctctggccta cagatacatt cacacataca tatacactca catacaatct 35220

ttcacacata cacacacaca cgtacatcaa acacatactc tcatacatgc acatataaac 35280

atgtatactc acatataaac acatgctcac atatacactc acacacactg tcacagtcac 35340

acagatactc acaaacacac acaactttca aaaatacaca tatactcaaa caaagtctca 35400

tagataattt caaatacaca cacacactca cactcataca tctttcactt gacactaatg 35460

tccttagaaa ttcgtggggt caagtaggat ttaataaact agagacttct atgggatata 35520

actgaattta gaattgttct aacagttttg ttctttttaa aaaacaagat acaggagaaa 35580

tggagaaaga gatcctgcat tggcatttag aaggaaaatg tctcattttc ttcaagagaa 35640

cagaggtgcc agctctttaa gaaatcaact ctggttggca gggggtgctg ggaaagttaa 35700

atctgttgac acatgacaaa aaggaacatc ttgcacttag atgcttgcac gttttgcata 35760
gctcagggcc agtacgcagg actgtctcct tcataagcaa atgttttcca tcatcctgac 35820
aacagctctc tgtggtaggt aggaggcagg tactgacagg aaaaaaatca ttagatcacc 35880
agcaaggcag agaaaagatc aaggaaatgc ttatccactg tagggaaagt acactgaaga 35940
ggcaggtgac ttttacctgc cctttcctct ttaatccaaa ttatttaagg cttggctgct 36000
ttgtattctg cttattaagg gacaaacccc cagcctttga agttccagaa tatgtaaata 36060
ccaacgttga tggacgtgca cttctctgtt ccttttgtga ccatttttga gcatttttgc 36120
agagttaaag gaccttaatt ttttggtgca aatcatgttt gatcccttct ccctggcacc 36180
tcattgaggt ttactgtgat cattctagac acaaaaaaat gctgacaaga tccatcatcc 36240
tgtgatgatg aagggatgtg gtggtcctga gtttaaatcc cttccttgcc attctctttc 36300
actttggaac atcctacttc tttaagcctt agatccaacg tttggaaaac tgggttcata 36360
gcatctttct catcaagttg tcttaagctc aaggcacaga tggcacaaca ccatgggatg 36420
tggtcagcag agagttatgt cagtcttcac tattccgaac acagagttgg ggttatcagt 36480
atccctagcc atacccttag cagtgtaagt gggtttgtgg ctgacagctc tgagaatgga 36540
agacagtgtc actgtctagg taatagcatc attttcatga tgtgttttgt ggattcatta 36600
aatactagct gacacataac cacttagact tcaaaaatga acaacacagc atcctcacaa 36660
tctatcaaaa acttggtcct tttcccatac tctgcatgaa tcctggctac ttccatttaa 36720
aaacacttag tggatggatg ctaaaagcat agactaaact gcttttaaag ataaaaataa 36780
acttatacat taaatgccca ggatgacctg aggatcgtga aggccaaaca tccattttct 36840
gaatgaatga atgaatgaat gaatgaccca gtctaaatgt aacctgtaag ggtccatgat 36900
ttgctgaaga atgacacccc agactcaaat agtatgtaaa gcaaagagtg ttttattctg 36960
cagaagtaca gcatgatggg gtctcccatt accaaattag agagacaacc aagtgagctt 37020
gcaggctcaa tttaaaacac attggagttc cagggcagat gagctttgtc ttacgctgtc 37080
tttaggcatt ctggagacat tatcagggag tgaagcttgg ccatttttct tccctcttag 37140
taattgactg gcttgggctt gcctggactt gcccagttct taggcctagt caactcatct 37200
gccaatttgg agtctgtcat gcaatcagcc tagccttctc aaccaaactg taaaaacatc 37260
tgtgacaaga caatgagcag cagtagctac aaggaagagg atgtatgtac cctacaggga 37320
agaggatctg tgtactgtac agggaagagg atgtgtgtac cctacaggga agaggatgta 37380
tgtaccctac agggaagagg atgtgtgtac cctacagaga agaggatgtg tgtaccctac 37440
agggaagagg atgtgtgtac cctacaggga agaggatgtg tgtaccctac agggaagagg 37500
atgtgtgtac cctacaggga agaggatgtg tgtaccctac agggaagagg atgtgtgtac 37560
cctacaggga agaggatgtg tgcaccctac agggaagagg atctgtgtac cctacaggga 37620

agaggatata tgtacctggt tgacatgaaa ggcaaagagg aaaaggaaat ctcaatagat 37680
ggaacaaaag aatagccttg tcgagaccag gaggtgagga agcatgaaat ccaggtgtat 37740
ggggcaggaa gtgagtttaa caggatgaca caggaatccc cagaatgagg acaaaacagg 37800
ccatgcttga aacagaaatt actcttgccc tggttccttt catcaaaaac attttttgag 37860
gctggctcgg tggctcagtt agggtaaggg agcatgtcaa gcctgataat ctgaatccaa 37920
tcatcaggtc cctcctgcta ggaaagaacc aaccccagaa gacgttttcc tctaaattcc 37980
acacgtgggc tatacaaaga catgcagcac tcacatacag acacactcag aaatacatgc 38040
acaaagagag acatgtatgc ataagcaaca catatataga cacactcaca cacacacaca 38100
cacacacaca cacacacaca cacacacaca cacagacata cacaacacca gcaaaggaaa 38160
ggctttcatc acccatcacc tgtatgccag gttccgttct cgatgcttta taagctttct 38220
ctgttttact attggaaaaa tgagggcaaa agccctccat gtagatctta cagtccctcc 38280
tttcagcttg ccagacagaa actgcagctc ccaagggcaa aagaaagtaa ggggtcatta 38340
tgagtcaaat gtctactgtg gtctgctgca aagtccccac tactctcaac cccttctgcc 38400
ggcattgacc ctttggttcc tttgttggtc tcaggcctgg atgcccccag ccatattgag 38460
gtgaaagatg tcacagacac cacagcactg atcacctggt tcaagccctt ggctgaaatt 38520
gatagcattg agctctccta tggcatcaag gatgtacctg gagaccgtac caccatcgac 38580
ctcacacacg aagacaacca gtactccatc gggaacctga gacctgacac ggagtatgag 38640
gtgtccctca tctcccgcag agtggacatg gcaagcaacc ctgccaaaga gaccttcatc 38700
acaggtgagg aaagggcaga gtaccaattc tgccccactc catccatgag tcctccaggc 38760
ccatatagca ctaaagatgg tcgagggcct tttcatctct gtgcattcat tttagataat 38820
aattagtgac tttttcaaag aaagggggat taaataacct tccaaacata cccaggcaca 38880
ggcaaacatg tgtgcatatg tgtgcatgta tgtgtgcatg catgatagag agagaaatgg 38940
gggaaagagg gagagaaaag agaaaaagga agggctggtc tgatattctt agacaacatt 39000
tctggttaaa gtttaatggc tcagtgggta aaggcatttg cctgacatct tagcaaatgg 39060
cattacttca aaggtcaatt atatatccca tacacatttt gcaggagttt ggtagcacca 39120
ggtgatcaac actaagttat taaaaataca ttgattcaac agaacagttt tcatatccac 39180
agtcttatgt tggggggcttt gtattattcc tcagacacag aaaatgaaat caatgtagcc 39240
aattgaaatt accacctacc aatcctcaag aaaggaactg ggactgtgct ctttaacttt 39300
gtgaatcagt taccaagaca ggggcttcgg gggaattctg acatttctgt ccattggcgg 39360
gtgccttttg aatctctgat tgaaatgtca agttctcctg cactcatcaa acctggcata 39420
atgcccatct tccctttccc tcctaaacag gcctggatgc tcccaggaat ctccgccgtg 39480

tctcacagac agacaacagc atcaccttgg agtggaggaa cgtcaaggca gacatcgaca 39540

gttacagaat taagtatgcc cctatctctg gaggtgacca tgctgagata gatgttccaa 39600

agagccagca agccacaacc aaaaccacac tcacaggtga gccgtgaccc agcggtgctg 39660

tgccacaggg gccctcgaaa ggacaggagc atatgtccct attttgcaac cctgtagaga 39720

ttatgaaata ggcccagctt ctgtccattc ctctcactgt cactgcatca tgtgatgagc 39780

atatacaatt cacagtggcc agaagccagt ctggggtcac ttttaaagtc ttctgggagc 39840

ctgccttcta ctagtgccca accttcacat tctcttccag gtctaaggcc cggaactgaa 39900

tatgggattg gtgtttctgc tgtcaaggga gacaaggaga gtgatccagc aaccatcaat 39960

gcggccacag gtgaggtgca tggcaatgct accgttgctg aagtagactg aacaggaaag 40020

gcaggactgg tgagaaaagt ctatctacaa attcaaagaa tgagattaga ggagaaatgg 40080

gcctctagtg ccatggagcc cagatgtcta catttaaaag aacgaaagca gaactcataa 40140

gctcactcct aagggaacta ccctagcaca actctctaca gaggaaagag aagaaaggaa 40200

aattggaagc aaatattata gtatgcctgt ttttagttgt caacttattc tcttaaaata 40260

agcctgttta attggtacat atgtgaagtt tttttcccct tagcttctga gaaaatacct 40320

ccctagggca gatagttaag taaccatata gattaactac agcatctact acagcatcct 40380

ggttcccgta gcatcctgta gtgccagcct tgggtgaaga ttacctaggg ctggattgga 40440

gactgttgtt tccatcctga agtgtttttc tgaaaaaaat ctactactag atacatcaca 40500

gctttagatt tcagtagtgt tgcacagtgt gtatgtgtgt gtttctgtgt gtgtgtctgt 40560

gtgtaaatgt gtctgtgtat atgtctctct gtgtgtatgt ttgtgtgtct gtgtgtgtgt 40620

ctataaacca ctgcacatta tttttaattt tctccaaatc ccaaaaggta gtgtgtaaca 40680

tctcctgtct ataaaaggtg aggtcagtca ggtgactgcc ctgcagttac aggtctcctc 40740

agggcacact cagctccaga agtccctgtg cctggcagcc taaagatgtc tcatcattct 40800

ctctccagaa attgatgcac ccaaggactt acgggtgtct gaaaccacac aagacagtct 40860

gacgtttttc tggacgacac ccctggccaa gtttgatcgt taccgcctca actacagcct 40920

ccccacaggc cagtcgatgg aagtccagct gccaaaggat gccacctccc atgtcctgac 40980

agacctggag ccagggcaag aatacactgt tctcctcatt gctgagaagg gcagacacaa 41040

gagcaagcct gcacgtgtga aggcatccac gggtaagtca ccctgagctc taagccctct 41100

gctaactctg ggtggcagct ttgagtctgc caggcttgtc aggaatgtga tgcccagagg 41160

agaaataagc tcctttctct cctcctggag ctctctgcca cactgaaatg gcagcttcct 41220

tgtctttcgt gaagattgac gtttttctcc tgtcagtcaa ctgattgaac caaattgcct 41280

catggagaaa cgctgacttc taagcaacac taactaggag aatgtaaata gggagctgtg 41340

attgtctgtc ttgtctcttg ttgctgtttc atttgtgctc catggtattc tagaaatttc 41400

ccatttctcc cctttaatct aatgacagct ttacttttca ttgttttaaa gatgcccagg 41460

atgttggacg tcattatttg aacatcttcc taggagtgtt tttattacag ctctcttgcc 41520

ctcctcagga tagcccagga gaacggcact tggtctgaaa cctgccacgc tgccttctca 41580

ctttatttct agtcatgaag cgtcttctgg ctctccttgg ttgatcccag gtccctttta 41640

gtcctattag ccagctaacc taccgacttt ctctcctgtc actttaattc acggactgaa 41700

ctgcagagag aatctggaga taaaaatggt tcttcctgta tgaaaaatga cacatccaat 41760

ttcagccatg aagttttcaa gatcgtttga gttttcacaa gtgacagact tagcttccca 41820

tttcattagc atgcatgcag gcatgcagct acctcatcta ctgcagtgtg ctgctgtttg 41880

aagaacgctg tgtcacatga ccatcatagc ctgattgact cccacactcc gtagagtctt 41940

tggagaaaac tagaagctgg gactatcaga gatcaggatg ttcaggggtc actagcgctg 42000

gtcatgtaac acagaagtca cccagctgac cctcagccat ctgaaagaga atgacaatga 42060

ggcaatttag aaatgtcatg cagggcccaa gttatctatt aatatcttca actgaataca 42120

acagaatttt caaatgtcag tctcctccat taaattgatc tgccaccagg cagtacaaga 42180

gaaagtaaaa ttgtttcaaa ttgttcagga tccagactcc ttcactcttt ctcttccacc 42240

atttttttag ggtttagctc cgattctcaa aatggggtca gtatcactac atggctgctg 42300

caatcctcca tcatccaggt tctaggcagt aggacggaga tagagtcaaa gctccaaccc 42360

atggctcttt ctagagattg tatcaataat ttccctgtat atctcattgg ctgttcttac 42420

ataacacgag aaggctagtc tcctatcaga atagccactg taaagtccac aggcattttg 42480

aaggaaaaca gagaaatttg tgagacaaaa ggacagtcta agcaagtaag aaatgagtgc 42540

agtggttaat tctgggaaca gtgcagactg ctccctcctg agctgtaggg tgctgaggct 42600

gtccgagaag cttagaagga aaagttcatc aaagataggg aaatagaatt gaaacgagtt 42660

agcttgaata acttgtataa tctacccttg catgagttta ttattgtgtg ccaaaggcat 42720

taaaaaaagg gagaaccaga aatgtaagaa gaaaaagaac tctctccttg ttcctcctct 42780

ccctcttcta ctctttcctc tttcacaaag ctccagaagg agggacccca cccccatctc 42840

cccgcatctc cagaatcaaa ggcagatgtt gcaagattaa catttcttat gctggtcctc 42900

ctgcattttc tccaaggaag ccccatgctc cagccacagc agtggcccaa gtccagacct 42960

cctccatgag gtggcaccat attgaaagca gagttaatct tcttaccctt cattcctgtg 43020

ctccataaaa aaaaccaaac ttgtacataa cagcaccttt ccaagggctg ggtggcctcc 43080

agagtctggc ttctctggct tagcttccac tattcatgtc cttggccaac acatgactgt 43140

ccccttgtat gtttgctgag cctgcctcct ccaccaggcc tgtgctccct ttgttatatt 43200

cctattctgt cccctgtttt ccacttatca tttgctctag tctcgtccat ttcaagtgct 43260

attcaaacca agggcattct ctgagcaagc tgtgaaacat acaccccccc ccagcccacc 43320
cctgatgctg ctccaaggct tcgtggctac ttaccaactt gagaatgggc agtaaccacc 43380
tcattgtccc tgacctccac ccacccgtga gcagattgta gggacaatct cctttgtcta 43440
ctgctgcctg cagtgcctgg aacacatcca aagccagagt gagggtcagg aacatctgca 43500
gagatccagc tgctctgggg acagcattgc cacattttgt tttctccttt ttgtccttct 43560
ctgctgtagc agctgtctag cacccttcag gagcaagggg caaggtagtt attccctagg 43620
aattctttaa gttagtgcaa tatggatgac aaaaggagag agcatgaaag ggggagggag 43680
agagggagag acataaaagg ggggggtag aggcaaggcc tgctcccatc cagcacagaa 43740
ggcctgtgtg tttcagcctt gttctcttct gctccctcct cctaactctc ccatactctt 43800
ctctcctcct tcagtttttc cttttctctt tctgctttta cattcctgtt agctaaactc 43860
tgcctaaagc caaggctaca aattcactgg aagcaataca agacataaag gaggacaata 43920
ggcaagagcc aagggccatg aggcatattt tcacagcaac ctaagaaccc aattgtgtct 43980
cgcatgaatt tgggaggcgg aaaaatgaaa ttttgtttct aaccacagct tcaagcgtta 44040
aacaacactt cgctctgatt taccatttta cttttctatg gagtaaactg aagtggattc 44100
aaatagttat cctttcaatg tacatttatt tatataatat atgatgacca catgctaaga 44160
atcagacaga agaggggac attcgggaca aagagaatga gaaagagtgc actgcagtgg 44220
gaaagataga accaacatgg gaagaacgta gttctgctgt tacagaggca tggggagtca 44280
caggaggaca gacctgatcc tcatcacaca cctgaaaaag agaacaaaag gctctgagga 44340
gtctcaaaag aagagctacc tagatgagtc cttgaggagc aagaggaggc aaggttcggg 44400
gagagtgcag tgtccttccc agcaggggga atagcatctg agaggcttag agtcaaggga 44460
tgttctgtag tcatcaaaca agcttagccc tgctgtgtgt ctaagtagaa ggacagtatt 44520
tctaaggctt caggccttct ggaacaggtg caaagcacag atgttcaggg attttagctt 44580
tattcttcat agctactaag aaccaccaga aagctccaag gcagccaaga cttcatattt 44640
caggtatttc attctctaga aaatgagttt ggtgccattt gaagtattct tgtagtcttc 44700
cagcttgcat tgaattctct gcttgctgtg ggagaatcac tctctcacat acaaagcaca 44760
aaaagagctt cgggctctgc atcctgccag cctatggtaa ccaccatcta tacaatggtg 44820
ggaatctgaa gggatgacga catggccaga aattccttcc tgcagctgct tggcccacca 44880
tgagttcaga tgacaggaga tttggaaatt ctgacttcct gggcccactg gcattactca 44940
gagggtaaat tccaaagccc agctggaaga gaatgacaat agccttttca ctgtggcctg 45000
taggaaagct agggtgcaca cagacaatgc agtgagccca caaggatgtc attcaccctg 45060
aaaatgagga ctcagcactt atacacagct ttgcagtcca caactgcctg ctctgtcttt 45120
attcagagca cccctccatc cctctaagtt catcggtgga agtttcatct ttcatatatg 45180

tgagcatgtg tttagggtca gcttagtgtg agtggtgtgc caacagcatc cagtgtagat 45240
atgagtacac accacttaca taaaggaaag ccctatctgt atccttcttg atctgcctgt 45300
gaaaagaaag ggggaaaggt ggaggactag agacccaaac aggatggaat ctgagtccca 45360
atggagacag cattgtggga tagttctctc aactgaggga agaggtgtgg ggggtcatga 45420
agcagaagca ccagagacac aggcaaaaac tgaagtgcag agatgaaatg agtctcagaa 45480
gagctcagtc taatgtgctg tccctccact ctttgtacct gtgagcattt taacagcttt 45540
ccctgtgatg atctatagta ccctcctccc tcctccgcat taggcaagtc tccttacttt 45600
atgcagcctc aaatctaagc tcttttcatg gggaacaata tgattaatgt caccattaga 45660
gataaggctt aagataaatt cctggaaaga aaagatagtt acttcctctt caccatggta 45720
taaattctca atgtcagtga agtttctgat tagtggtttt agacagcagg agaaggaaat 45780
tttttctgt tttttttttt tttgtttgtt tgtttgtttt attttcggcc tgaccagtac 45840
tttcccctta tccctactct actgaagtgg tttgttaaaa cctgacctga aactcaggaa 45900
ctgaaaagtc caaattaaac tataatgacc ttctaggaaa ggccaaggca gcacaaagag 45960
aaaagaaacc taagcagatc taggaggctg caaaatgcag agcgctggaa aaagacccaa 46020
gaccaagctg aggttattcc gtgtagtgaa aaaccactct gccaactgga aactgaggca 46080
tggagcctcg ttaacagatg agaatagtct tctctaggat acttgtaaac aagtaaacat 46140
atttccaaaa ggaaattcaa ctgtataaag gcaagattaa tgctacacta accagtttat 46200
tgcattttgt gggacagcat cgctgggcat acagtgacag catcctggta ctggactcta 46260
tgggccttgg ccaatggatt tgctgggttc ttaaaaggca agcatttgat gaatagtgta 46320
actgaccttg tctctgttat tgtttagaag aagtgccttc cctggaaaat ctcactgtga 46380
ctgaggccgg ctgggatggc ctcagactca actggactgc agatgacctg gcctatgagt 46440
actttgtcat tcaggtacag gaagccaaca atgtggagac tgctcacaac ttcacagtac 46500
ctggtaacct ccgggctgca gacatcccag gcctcaaggt tgccacttct tatagagtct 46560
ccatctatgg ggtagcccgg ggctatagaa caccagtgct ctctgccgag acctccacag 46620
gtactttctt ccttcatatc taaagcttct gtttttctca acaggaaact gaccaaggat 46680
aatcacgcat cattgatttt agaatggcct tgttcagcca caagctgaag tgacatgggg 46740
gtctgatggt ggatagccag gccatcgcgt gacatagttc taaggccatg ttaacatttc 46800
acataaacaa cacattgctg ctgtatggaa tccatcatct gagacagtcc aaataaacca 46860
aggatggtga ccccacatta aggactgatt atgtgctaga gtccaaagga aattagccac 46920
agaaggaatt cctgaattct ctgaaattga taaactaaga aagcaaatat ttccccgccc 46980
ccttcttctg ctctttccaa ggtttaagat gttacatgaa gcatgtttat ctaacaaatg 47040

tcgtgttttc aggggatgta aagcctgccc ttgggttcag gatttcctat atcttacaat 47100
gacttttaaa cgcattcttt gtccttctct agggacaact cccaatctgg gagaggtcac 47160
tgtggccgag gtgggctggg atgccctcac gctcaactgg actgctccag aaggagccta 47220
taagaacttt ttcattcagg tgctagaggc tgacacgacc cagactgtcc agaacctcac 47280
agtcccagga ggactgaggt cagtggacct gcctgggctc aaagcagcca cccgctacta 47340
catcaccctt cgaggggtca cccaggactt cggcacggcc cctctctctg ttgaggtctt 47400
gacaggtatt ttagaccggt tcactccttt tctcctctcc ctgtctctgc ttaagaggaa 47460
tcccatctct aaatgagact tagacccttc catgttgggg aaatcctagt gaatcacttc 47520
tacttcaata tcttatccct tatttgggca ggtcctgctc ttagtctagt agacagctcc 47580
agacaaaatt ccacctactc ttctttttca aaagtgagag gtgacttctc tcgaaaagta 47640
tgtttcaaat tataatatct aagaaacttg aaagcaaaat ttgtaagtga aaaataaaat 47700
cactctgagc tcattttaca aatactcaag agcttcccat aaacctagtg gtatatcaag 47760
aatggaagtt ttgagctcct ccaaaaacta ccttctattc ccaaaggctg aaagaaagcc 47820
ttggctttaa ttctaacaat tggagtgtgc tcagtactgc agtaaatcac acatctgtag 47880
tcaatacaaa ccaaagagct catctcaact ttagtcaaac cccatctcac ctgaacccca 47940
gagagcagtt ccctttatag taacgtcatc cctgtgtatt ctcatgcaga gaaggttcca 48000
agtctgggaa atttcacagt gactggactg agatgggatg ttctcaaact ggactggaac 48060
ataccagaca gaacttatga ccagtttatc acttgaatcc agtagaatag ccaggcagaa 48120
tgggctggcc ttattattcc tggtcgcctg tgttcctttg aagtcccagg cctcagggct 48180
ggtactccct acacagtcac cctgcactgc aaagtaaggg gcctccatac taatgcacct 48240
gctgtagagt tcatcacagg tacatgacca ttctggactg gatttttata tatgccagtc 48300
tggctgttcc cagaatagca aaactccctc ataggtaggt cttgcctgaa tatggtttgc 48360
aggcaacatg aaactgtgac ttagcaggtc tggattagaa ttctgtagta ctggctggat 48420
gttttccttt gcatctctga gccttttttt cttgccattg aatcaaaaat gataccctgc 48480
tttagtatcc ttccatgagc aatggatgga ctaagaaatg aaagggcttg acaaaatgtt 48540
catcatctag ccagtgttga acagctaagt gagtagttgt attattcagg gtcttctaga 48600
gaaacaaatt atagaatgaa tatatatgag atttattacg gaatacatat gggatttatt 48660
atgaatataa atattgaatt tattaaaatg gcttacaaac tgtagtctag ctaatccaaa 48720
aatggctgtc taccaacaga aagtccaaga atacaacaat tattcagtca tgagactggg 48780
tgtctcacct gttctttagt atgcactgca atcctgaata cacaggccct catgccagaa 48840
aaagaatgga ctcaccagca agagtgagag tagacagaca gagagggagc ctcattcttc 48900
tgtgtgtttt gtaaaggctg ccagtttcat agaggttacc agcagaaggt gtggcccagt 48960

taaaaagtgc ttcttcctac ctcaaaaaaa aaaaaaaaaa aaaaaaaaaa aaaaaaaaaa 49020
aagctggatt aaagatatat cttcctgtct caagagagct ggattacaag tgggtcttcc 49080
cacttcaaat gatttcatga agaaaaaaat cccgacaggt gtgtccagct ggttaggttt 49140
tagttcattc caaatgtagt caagttgaca atcaagaatc gccttcacag ttgtcaaccc 49200
aaccatggga agacaaagca caggaagtgg gtattttttt ggtgcaggag agcagaactt 49260
ttcagagaaa tcttttcaat actgccctca gagacattaa gtaataccca attccgagtc 49320
ggatcccttc agatctggaa actgatctag tcatcttatg ccgaagtcat ctaagcccat 49380
ttctgaagtg cacttaaagt aagaaccttt ctaagaaaag atggtatgtt taagttccat 49440
gttttccatt taagctgaag agaaaaacaa attcatctca gtccttggaa tgccccttga 49500
aatatgctgt tccatctatc acacagagct ttatgctatt ccaaggagga tggaatgaat 49560
ggtaaaattt gtggtgataa agttttgatg ctgtacccct ctccatttgc cttgtatcct 49620
tgtattttga taatgccatc aaattaccta attcaaacca tcaattttcc tacttgttta 49680
tatatatata aagtaaactt gtccattgct gtgatgtaca cacataccag atataaggaa 49740
gatgattatg ttcatttacc taagtcagcc aggttatcat gttgccttat tcctggactg 49800
ggttgagata aacaaaggtc gtctgggatg tctgctctag gtcagggatg ctgatgagct 49860
acgtgtacag tctgcaagcc ctgctgcagc tgcctagctc ctttagtaaa tggaatgttc 49920
tggtgaagtc tttcgtgcag tagataaaca gttctcagca agtgcacttc tgtccccaga 49980
cctctcagaa aaccctgggt cagtgttggg acatcttccc caaaggatcc tgggacactt 50040
aggaacaaga attatacact tcttaaaaaa aaaggctaag atctctaact tctaattcat 50100
ggccacttct gccttaggaa gatggataag ggtatacttc agtttgtaaa gggcttgcct 50160
ctcatccttg aggaccaggg tttgatcctc agatcctcat aagggtatag aagtataaat 50220
acttagaatc tcaactgaga aaatgggggc aagaggatta tattgatgat ttctggagag 50280
ccaatctagt tgaaatggtg tgttccagat tcagtcaaag accctgcctg aaactaaagt 50340
taggagtgat tgaggaagac atttgatgcc aacctctggc ctcctgcaca caactgcata 50400
catataaaca ctctctctct ctctctctct ctctctctct ctccctctct ctctctgtct 50460
ctctctctct ctcatacaca cacacacaca cacacacaca cacacacaca caaattagag 50520
ctgagactgt actgaatccc aaacagtgaa tttttccata ccccaaagtt agactttctt 50580
aatcagcaat aactgaagat cgttcttaca cagttaaaaa gaacaactaa cagtgggccc 50640
tgctttgcac atgtactaag caatacagac tcactcttaa ttaaaagtaa gcaagctgac 50700
ttcacacagc cttgctttgt gatgcagcca tgcttcatgc caagggttaa gacagtggga 50760
gagtgcaagc caagccagga atctattacg taacatcatg tccttgaaag ttgcagaaaa 50820

agctcagaga taaaccagac cacctgtcaa aacttgggaa cacgagttct tcagaagccc 50880

aaatacagag caggtagcca gccacaagat gtcccttcac ctcaagtaat aaaatgaggc 50940

atggcttcgc atatgactaa gatattccaa aacacatcaa caggctctat gttttatgtt 51000

gccatttatc agacagtttc tgtgccttgt gtatgacatg aagatgctaa taaatatcag 51060

gtaatcacag aacccaatga gatggtactg tcacttctac ttaaaactga ggctcagaga 51120

gaaagccaga ctcccaagat gatacagctt gccaggtctg tggacctcca actcctgtgc 51180

tttgactact cctacttcct tacccctcta caatcccaca ttgtcctggt gcctctagcc 51240

actgcctgtg cattcccaga gatttccatg cttcggcctt tcaaggagga atgatgcaaa 51300

gtttcctttt ctgcctgttt caactcttct ccaaaaccat aaccagagta gcaaaaatct 51360

gaatacagaa atacccggag atcaggagct acagaaacaa aacactcttt ctctcccctt 51420

ctgcttttct ggagtagggt tgtggggcat tcggctgcct tgcttggttt gcagcactaa 51480

cagcaaattc tctgccttct tcagaggatc tccctcagct gggaggcttg tctgtgactg 51540

aggtcagctg ggacggcctc acactcaact ggaccacaga tgacctggcc tataagcact 51600

ttgtcgttca ggtgcaggag gccaacaatg tggaggccgc tcagaacctc acggtacccg 51660

gcagcctcag agctgtggac atcccaggcc tcaaggccga caccccttat agagtctcca 51720

tctacggggt catccagggc tatagaacac cgatgctctc tactgacgtc tccacaggta 51780

tcatctccac tgcccatgct tagggttctc tcttgcgctc gctcttgcgc tctctctctc 51840

tctctctctc tctctttctc tctctctctc tcttcactcc cccttctctc tccccactcc 51900

tctctctctc tctctgagat ggcaagggaa ctagcttttc ttcctggcta attcctttct 51960

acgcatgacg catgctcaga gccctttcgg tgagctgttc catcattcca atgccttcgc 52020

cacagcagtg acgagccatg taagatactc acacttctgg cctccgacat ccactttctt 52080

gattcaccta gtcagtacca caatggcacc tttgcacggc ggcctctgtt tggagaacca 52140

gcctgtttgg ttttaacttc catctgtagt cctggcagag aatcaaaagc ccaccctccc 52200

cacatgtgag tttgatggag cacagaccat ttattttaga gactcttttg ctgtttgagc 52260

attatttcaa aatttctcgt ggagatgtgt cctacagtgc ttacacttac acagtgattc 52320

tttgaaatga agtgagcaca acaggagaac tgaattaccc tccagtaggc aaaattctct 52380

cagctgcaga gtcattgcta gaacatcaaa ctatcaagct atccgtcaaa gagagaaaac 52440

atacaattct gaatggataa tataccaaag ttactgttgt tctgtagcag gacactcagt 52500

agctctcaga gcaagaagta tttcaggcta taaacggaac agagttgcta cccccttttt 52560

gctctgtgaa tcaagagtta tttcccgaag caagctccca attcctacac cagggatgcg 52620

ttctggttta ctcgttacac tgttacagtt ataaagcttt caatggact cttcccaagt 52680

gaacggcctt tcctaaccct gttttctgtg gctcgtcttt atggtgtgcc ctgatgagtt 52740

ctttctcatg aaaatgcctc ccttttttta tacagtcact gtgtctcata gcaaaccatt 52800

acatctttgt tgattcaaaa ggataactct gtgattttgc ctcagttgtt acagaaactg 52860

tcactgatgc atacataaaa cgtaagcaac cccagggtgg acagtgacct caggctgacc 52920

agagattaca gttgaggatc tgctgacttt tctccagggt tcttgtctga catccacaaa 52980

ctattctgtc ccagaaggca gagcaatcaa tcacccgcca cttctgatct ttcctgcaga 53040

tctcgtggtt tggatgccat ctgtattctt ttttatggta gcaacattct tgtctctgag 53100

gagttggccc acggagggga agggctataa ttaatctgtg aatctgggat taagggtctt 53160

tctcctcgct acatagattg tactggcttc acaaatgcct cccatgtgca cacactgctt 53220

gctatatcac cacttctcag gaaagtaaaa gctctggcag gacatctccc cagaaaaata 53280

ttatttctta gctaaaaagg gttgtctgaa cggtttggct gagctgtctt ggctgccaat 53340

acaggaccaa ccttcttggc ctacatagac ataagtaaaa gagaacatgc tattttaaca 53400

gatgatgaga acagctggct ctcccttatc taaagtatgg aaaaaaataa tcaagagtta 53460

ccctgagagg tgggaggatc ttttcaagtg acaaaatcag aagcctccag gtcccactca 53520

gggaggccca tgctgtcagc attatttagc actcatttag atagataggc attctctcaa 53580

agctaatgca ggaatccaca taatcttggt ccctgagcct gaaagctgtc agccctcata 53640

tcttttgact gattgccaat ggccctgagg cagggctctc agagacagat ccagctaaga 53700

tgaagccaat tccactgaga ctagccagcc cgtcttctgt gctgaggtat cttgccaggc 53760

ctgatcatca gtggcaaatg caggggagaa aagtctaaca gctatcccct cctttgctca 53820

gcttcctctt aacaaaacca gtttctcctt ccctggcact gattagtagt ggcagcatca 53880

ctttgccttg ctataaccct gaactatcag accttactcc ccgggcatga ttctattaac 53940

cacctccacc ttgaaacaca gccaaataac taaatcattt gatttttctc ctctctctcc 54000

ttccctctca tctttcagcc agagaacctg aaattggaaa cttaaatgtt tctgatgtaa 54060

ctcctaagag cttcaatctc tcctggacag ctaccgacgg gatcttcgac atgtttacta 54120

ttgaaattat tgattctaat aggttgctgc aaacagcaga acataatata tctggtgctg 54180

aacggactgc ccacatctca gggcttccac ctagtactga tttcattgtc tacctctctg 54240

gaattgctcc cagcatccgt accaaaacca tcagtaccac ggctaccaca ggtacatgag 54300

cgttctctag tttctgacgc tttctctcca cagtctctct cagcaggatg aagccacttg 54360

atttcattgg ccaatgaggt ataaaccatt gaatatcaac tttcctaggt tgctgggctg 54420

catcattgtc taacaaaatt ttcttctgac agccacctaa atggtacatt tgcatgaatc 54480

catatctctg attatcataa gcagaccatt ctaagtatca tatgaaattt tgtccaggtg 54540

gccacaaata aggacctccc tcctttggta actttaaaat catcatgata attgtcctag 54600

gcttgcaaag atgtctcttc taatcaaatt tacgtttctt ttttttgat tatcctaata 54660

tctgtctctc tgtaaagtct tgaatatcct catacccaaa tatcctagtg atttaagata 54720

attgtcaacg gtgccctgtt ttgcttttt tttttttct ccagctggga aaatggcaag 54780

aaagagcaat gatagttaac tcatcagcta agcaagaggt acccacgtca tccatattac 54840

ctcaacctga aagccagctc aggaaaagat gtcccctgtc tagaatgctg agagacacat 54900

agcaaaaaga tgataatagt ccttctcaga taagaaacct gggtccaaag ggagcaaact 54960

gttcttgctc tgcatatgca gtcctgtggc atgtgttctg tgccagatga tttctttagt 55020

gccttgagga agactccaga gaggagagca ttgcccactc aagcaacatt cttagcacat 55080

ttggggcaat atcttcactt tacagataaa ttaacatggt ccagactaga aaaaaaatgt 55140

aaggaatgca gtttttcctt atagttcaca aaagaaggtc catattctag ggaacacaag 55200

aaaaggattg atattatagc atcaaaatta ccttctgctt agatcaccaa tggttgatgg 55260

agacctaatt tatatgctgt tttagactgt tatcagggta gtcctggaaa aaaaggagct 55320

agggatactt ggttcctagt ctattcatgt taaagtatag taacacagag gggacaaaag 55380

taagttgacc atgtgtactt gggttgaatt ggttttcaac aaagaataat ttcttgcctg 55440

aaaggcagag tctaaaacct tatggactta gtcttctgtg ttttaaattt tttctctaat 55500

ggtgaatttg atgattatac ttttatatgt aatcttattg ctgtcataga cagctctcct 55560

cttagccaca tctctgcctc aaatatgacc tcaggagctt gccttaaata gctttcttat 55620

agaagttagt tggtttgatt gtcaccaaac agtggtatac tctatcatca gtcaacctga 55680

aactctgcca tctccagcct caaacaaggc atcttctaat tacagcactt agctgcccag 55740

actgataggc agatagcgtc ctcagaccct gatggtccat gcagtcacaa atactaccca 55800

ttagaggttc ttcggtctcc attgagctcc ctttgagcaa cattttaaaa atcagccact 55860

gctccttttt catcctctta cttctaccag atcctttta atttaatgta ggcatctaat 55920

caaagcttta aagctttaaa gtacactcca tgccaaataa gggaccttca tagtctgttt 55980

ctcccatata cccccttgcaa tctggagttg ctctgtgatg agttaatatg tcttgctttt 56040

gtttgaagtc ctaaatcatc ttttcctctt ctctttaaca atatcccctc acagaaccca 56100

ttctcacctg tctgtagtcc ttctaatcaa agcttgcctg tgtcccattc aaaggaatgt 56160

ctacatgctg ttcaaagtca aacatgatct atagtctctt ttcatttgcc tggattttat 56220

atattgattg ctacccttgg gatgctaaga aagaacccta aattgcaagc aataagtctc 56280

atgagaaaac ccagtggtgc ccatcttgtt cttgagattt tcgttttctg gattcctgac 56340

agataggcat cctgatagat gtctcttttg acatctactc tgttgagcat gcactgtcat 56400

ctgctgttgt ctgttccaaa gcactagaac tctcttccca tccatcatct tgtcaaagtt 56460

gcaatgcatg tggtgaatac tcagagcatt aaatctttaa atatgtctat ctttcttgga 56520

agtagacagc atgtcaagga agtgttcgta agggaagtga taggagaaaa atgtcccttg 56580
cctgcatgga acactgacca aatcctaact agcattctat ttctgggttt tgtcttatat 56640
ctcacaattc accatggttg catcctctga gagttcactg aaggtaatgc taacctctgg 56700
aaccaaggcc tccctccctc catctgcatg atgaatgtgt tagcactgcc agactcccta 56760
ctgtagcatg agaagaatta tagactgtat ctttaattca agagatagct tctaaagcag 56820
aagacagtga agagctgagt tccatctgga ggactttatc ctttgtgtgg ttgtaaaaac 56880
actgccaaag tggtagcaaa actacagctt tatcagcacc tcagaatgac aagacagaaa 56940
aggcatgcca gcgctatgac tgtaagctgt ttctgagcca gattggtggg atccttagag 57000
actttagagc atccatgcca tggagttttg tagttaaata ttgctcaaat gagcctattc 57060
ttatctctaa atattggagc tgagacaaaa caactcccag gttggaaaaa tactttgcct 57120
ctttccaact ctaaatccta tcaatagctc agtcctctag ggctactgat tctctttaga 57180
atcaacctgg tttgacatac ttcacatttg tatcctttac taaatgacta ggagtatgga 57240
cctacgtaca tctcagttga tactagctat gaaagcctat tatgttaaat gagtttctca 57300
tattactatt taagaaacaa gactattttt tttttgattt tccgagatag ggtttctctg 57360
tatagccctg gctgtcctgg aactcatttt gtagaccagg ctggcctcga actcagaaat 57420
ccacctgccc ctgcctcctg agtgctgaac aaaacatttt tttacctcaa cagaattttc 57480
tctttacaaa ggttctatta agcaaactcc ctaatcaagg aatttgatgt ttaacttcaa 57540
gttttcctac atcttacata tctactagac atttttaata taatccatct tcacttgtat 57600
agcatttggg gtgaggtata gattttccta ttatgcaggc atttaagttt ccaatgtcct 57660
tgaagaaatt tcacatagat gcagatgcta gcctttaatt tgtgcatcca tatagatcaa 57720
atgaacacta agaaaaccct ccagaagcac tttctaaaga ctactcagtg ggataccatc 57780
tcactgaggc atttagagac attcctaaga tatgggatcc atagcctagc aatgttggag 57840
tcaacaagac tgcatagatt tagttataga acaagctttt caaagccttt gaagggatat 57900
gtgctctgtg aatcttattg gtaaaagaaa atggttgctg tttatgatgg atgcttcaac 57960
atgaagctgt ctttacctgg gacatcttga cctcagagat tttggcaaat gtagtcacaa 58020
tatattttat tcgtcctatc tgtacttctg atggctggct aacttacctt tacaaatggc 58080
tccaaatgtg tttacacttt aaaagaacac aaataaatga ctgcagaaaa atcagtaact 58140
tctttgtcat atagtaaatg taggcataga tactgcctga cattcaattt tttctgcaag 58200
tatttggtta aatacagaca ttcctctttt tctctctttc tctctttctg tttctgccca 58260
tgttctttcc cttcagagcc agaaccatag ttgggcatgc taatctttag caatattact 58320
cctgacagct tcaatatgtc atggaccaca caagctgggc tttttgcaaa gattgttatc 58380

actgtgagcg atactcattc acttcatgag tctcagcaac tcacagtccc aggagatgcg 58440
cagcaagctc acatcacagg cttggtagag aacactgcct atgatgtcag tgtgacaggg 58500
accacctggg ctggtgaccc cagaggaccc ctcccctcac acccctcact gtctttgtca 58560
ttacaggtat tcaaaggttc tctctgtcag tttaggaatc tggaaaaagg agataggcac 58620
tctaaagaaa actccaatcc caaacatagt cttgactcca aatataaagg cctttggttt 58680
taacctatag gaaatgaata tatatgtatt catgtcatat atatgtcata tatacatata 58740
tattcattag gatacatatg gtacaaagtg atattttaac atatgtattt attgaagaat 58800
caccaaatca gatcaaatta atttatcaat catgtcacct accttttgtg ataatactta 58860
aaatctaatc tttcggctgt ttggaacatc ggctactatt agttattatt aactgaggca 58920
actattctgc gccttggtta gcttattctg cacctcactg aagcgtattc ctactggcca 58980
gttgaacttt gtgcctcttg accaatggca agcggccaac cccacttctg gtgaccagta 59040
ttcttctttc tactcctatc agttcaattt gtcaagattc tccataaaag ggagatggtg 59100
aaatggtttc tcttcctctt cttggcttat ttcacttaat gttctccagg cttacttgtg 59160
acatcaagaa tgatagaatt cccttctttt atggctatat agtattcttc tacatgtact 59220
cagcaaatgt tgttactaat gtagccattg agggaaacgg attgctatgc aatgttattt 59280
ctaatagcag agaggccaaa tgtaaatttc tagccactaa aagcaatggc actcctgaaa 59340
ggaggaggac ccttacagac cctcttggaa gctctttaga catgtataga gcttcgagta 59400
tcttttggcc tgttttctag aatgcttaat ccatgcagag agtctgaaga aagtgaaatt 59460
taaattagcg atgatctaga gcaggaatta tgtccataaa ttgaggtatg tacggtgtaa 59520
agtgcctagt gtcctagagg gtccccaaat cctataattt gcaatttaaa aaaaaaaaaa 59580
acaacccaaa gccaaacaaa aaaacaaaac aaaaacctta ctcttaaaac agtcaagcac 59640
agaaacggac atgttcaacc taggaagata gatacatgct aaactttata cacttattaa 59700
atttgagctg tgatgtttct atggatcata taaagagata ctgcctattt tctttcctaa 59760
tcttacctta agtcgatggt aaaggctgtg catggtttca taaaagtaaa tctctagctt 59820
gacaaacctc taaaaaatgc ttagaaattc tcttctacta aactctcagg agatagacgt 59880
agtcatgacc cttaaactaa gctagtccct cccagagtct aaattactat aaatgagctg 59940
tgttaataag tacagctaac taattgtggt agctgagatc aaatctggtt gatttctaaa 60000
cctatcccac atcatcttac ctacacatag ccctggcaaa tttgtttgga gcctgatgtc 60060
cttatgttag ggaagagggc actcactggt gctgctggcc atctacatta ggaatctagc 60120
agacacagtg aaaagtaacc gcctagcctc cactggatcc tctactagtg ttatgtcctc 60180
ctgttgcttc ccaacagtat aaggagagtg ggcttcttaa aagtcaattg gggtgtaggt 60240
caaagttaag tccacatgtc tggcatgagt aagaccttag atttgttccc catccacaag 60300

cccaagggaa acaatggaaa catgcctcac aacttactct tagaaccctg ggtatgtttt 60360

agtctaagat tttaaatatt tttttccttt caattatcaa atgtaaatat aagctgggtt 60420

tttttttaa gtacagcaga aagaatgggg tgagaggaag actctttcta ccctgagcac 60480

ccagagctca tcaggccctt acatgtcacc ttctgaagga cctcccacaa gtcagagaga 60540

agccagggaa gctgaaagag cagccctcag gagggaaaca gagatccagt tacagaattg 60600

tttctgctaa tccctggtgt gaaaagactc tacattttga aagaggcata ccaaagaaga 60660

aatgaaaatg aataggaaag actgtgggag gaaaaaaaca agcccttgat tgactaatta 60720

gcctgaaagt acctgttttc tctggaattc actgctttaa tagcatggag ttaacagagg 60780

ccagctatca aagaagggca ccacttctct aaacaaaacc aacatttgag aaccttgttt 60840

tacaaatcaa gactgtttgc atggtaaaaa aaaaatttca tatttgcttt gatagagtta 60900

cagctcttgg gaaatcttat gagaaaaata aactctaaaa ataaacatta actgttacaa 60960

aatcagcaca ggaagtccaa gccacaacaa agtctaattc tctacttatg gatgcattca 61020

cttagccatc caggtgccaa gaacttcttt ttaaacattg tctttatatc ctagggaaat 61080

ttgaccataa gcattagtac aaggtcattt atcactttga aaattctcag ttagatgcaa 61140

caacatgagc gagtgagcaa gggaagggga gaagagaaga gaagagaaga gaagagaaga 61200

gaagagaaga gaagagaaga gaagagaaga gaagagaaga gaagagagag aagagaagag 61260

aggctctaaa ttacatgttg ctaaagcaga cttgggcatg ggattttcac ccttctcccc 61320

taagtgctga atgagtatgc atcggtcttc tctctctttt ctattagcgc tgtgtcccca 61380

ctctgagtgt gtcagagcac ttgatagcat ttactaattt tagcattgtg ttaggatgtg 61440

caaagaatac caagcttgca gcaagaaggc cttggaaaag tcagagactt tctctcccag 61500

ttgatgtcct ggaggccagc agacagatag caacacctac cttacagcat cgttgtgtta 61560

gactggatca acattctgta gcctcctcta ctgggacttg ttttggccac cataaggtca 61620

ctttttggaa agctcaagta tgtgaaaaga tccctccaca actagggaat aagtgggctg 61680

ggacacagat agacaaaaca taaaaccaag ataacaaaat atgttcttgc tttctaactt 61740

aacctagaaa aaccaaacaa cttaagaatt gccaaactta gaattccttc ctggtttaag 61800

aacataaatc gttaatttgg ataagtaaca acacaacaaa tcttattact cctgttatcc 61860

aagtttatga actcaatagt tggcccaaat aattgtattc aaaatatgaa ccagattaag 61920

atatgaaata taccagaaaa gtatgcttgc aacaaccaat ttttttaaa aatctgattt 61980

gtttcaaaac aagaaacagc aagaagtaga ttaaggttcc aaagtgacac aaggtgtcct 62040

atttgtatag tgagatatgg atggcccaag ttaggctcag ctagaggcct tgctctatca 62100

taccttgcta aggtgagact tgggctaaat cagttaatca tttagaaact tttcttttga 62160

cctatgattg ggaatgatag ttactgtaca gtcttgttaa gaaatttact ctatgtaaca 62220
atatcaggca cccaagacat tattactaaa tattcctttt ctgagcattt aactcatggg 62280
atgcttgact cagaatctat cagtgttctt ggtatttgag catttactca gaatctatca 62340
gtgttcttgg tatttgagca ttttaactca tgggatgctt gactcagaat ctatcagtgt 62400
tcttagtatt tgagcattta acttatgggg tgattgtcag ggaatctatc agtgttcttg 62460
gagtcaggag cattgaactc acaggatggt tgactgggaa tctatctgtg ttcttggggc 62520
tgcacgctgc cttagtcaaa tcagtgctgc taacctgagt cattttcact cctcccttct 62580
ttcagaggcc ttgccccttc tggaaaattt aaccatttcc gacactaatc cctacgggtt 62640
cacagtttcc tggacggcat cggagaatgc ctttgacagc tttctagtaa cggtggtgga 62700
ttctgggaag ctgctggacc cccaggaatt cacactttca ggaacccaga ggaagctgga 62760
gcttagaggc ctcataactg gcattggcta tgaggtcctg gtctctggct tcactcaagg 62820
gcaccaaaca aagcccttga gggctgagac cattacaggt atttcaattc aagagagcca 62880
tctgctcctc ttcctggtcc cttttcctgt gtagtctgtc ttcggatccc tgtggtttct 62940
aacattgtct gtattctcca cttaatccac acacgtacct tgttctctca tgtcctccac 63000
attttccctt ttttcagaat ctattgagtg ctcacacaca ccccatccta gaaaggagaa 63060
gatggaaacg tgtctcttta atgtcccaaa ccctttagtc cacgtcttgc ctgtagctct 63120
cacgtgctca ctacagtcac cttcgtttgt gttcatcctg gctggttgcc agctacattt 63180
cagctttctc aaaagaaata tgccaatgag tatattttga aaaagtattt taaaatcttt 63240
actatttcag agtctcaacc tgtgatctta accaacttac ccagtggtct tatgattggg 63300
aactgaatga aacttactat acaggccttt aaaaaaaaaa aaaaacaact aataccagag 63360
agattcttca acccagacta aatccataat gttctcaaga ctaatacttc ctatagaaat 63420
atcacattaa taattttaac agtcagaaaa ctgtgtttcc caaagtattt ctagagaaaa 63480
caagcgctga tggctgttta aagatctcat ttgataacat ttcatgttca gttaaatttg 63540
gaactaggat ctttgaccac acaggctttt cagaatctct tattatgcag agggcagtag 63600
ttcccaaact tgtttgacaa ggaatgcttt gtaaaggatg gagccataca acaggaagag 63660
tttgctttga gcacacacac tttgggaaac tcagcttcaa atttattttg acactgagtt 63720
ttgtctagtt accaaaatga aactctggag tacaaagtat atataatgat atctcaagag 63780
gttctaagcc tcatcctacc atctaaatgg taaactagca aggaaataaa attaaacagg 63840
atatactagg agccctcttc tggatagcag ctccggagaa caaagccaaa agaccataac 63900
attctaaatc attttgctca ggctcgaggg aaatataatc atcagaatcg tcatggacag 63960
ctccacagat cattaaagtc ggtttatttg tcttttcaaa aggaaaagaa aaggagctcc 64020
cagcagacca gcactcagcc actggaagac aaagagcaaa tgtaaacaag ttatggcttt 64080

gaacttgttt aataatgcac gggaggggcc aaggccgagt ccagatgtgg actgatacag 64140
ttacttttag acaaaactca cagatgcaag cgaatgcttg gcttgtattg aatgtgtcag 64200
ctccgtgttt tccttctcct ccccctacta gacttttta atgtggaatg attttctttc 64260
ctggtatctc aggacactct gtctctgaaa accgaggcag cactgcattc tgatactcca 64320
ccagaaggaa gactgcatgg atggctaatt aatgctctgt ctcctccttc ttccaccatc 64380
ttctctccct ctagaagctg aaccggaagt tgacaacctt ctagtttcag atgccactcc 64440
agacggtttc cgtctgtcct ggactgctga tgaagggata ttcgacagtt ttgttatcag 64500
gatcagagat accaaaaagc aatctgagcc acaagaaata tccctccctt cccccgaacg 64560
taccagggac ataacaggtc tcagagaggc cactgagtac gaaattgaac tctatggaat 64620
aagccgcgga aggcgatccc agccagtcag tgccatagca acaacaggta ctgtaaacaa 64680
gcaggtgcca tagcaacagt gggatctata cacaaacagg agaggaaaac ggaaatccaa 64740
gactggaatt cttcttccta tagggttgca taatcacaag agctctggag cacagagagg 64800
cttctgttta gatcctggtt cctttaagca tagactgtgt tctcctggac tgtgtggaca 64860
aatctctgca ccataaaaag agtatgattt ccttagcagg gtggtgagga tttagtgatc 64920
tgattcatgt aaaagtaaca acataagacc tgggatgtag taagggcgtc atagtgtctc 64980
tcctagatca tcaactatta gacatttaaa ttccaaaaac ttccttctat tgaatagtat 65040
gtatcggaga atatgatccc ttgttctttg gtcagatgtt acagtcacac tagatgagtg 65100
agggacagtg gagacatgga accgagacct gaaaggatta tgacatgttt ataaaatgtc 65160
ccacgtgagc catcagtact gtggttagca aactgaagga tggagctggt aaggctagca 65220
ggacggtctt cttgggctca caaatacaag ccgacactgc gtgtaagagc cctccacggg 65280
ggaaataaga cccttttcc tatcagctat gctaaggaga aaggcaaaga tacccagaca 65340
catcattact atggtcataa aacttatgtg actctttaga gggagaaaca aagacctgaa 65400
aatgcagtac tttaaaaaaa aaaaaaaaac ctggaggaat agtcaagatg gcattgcata 65460
gagacaaccc cctgaaagga atctgaggaa gaatcagaaa atcacaagga aaggagaagc 65520
actgagaagt gatgagccac acaccaaaga tcttggcctt gtctattgat acatcaagaa 65580
tccgaggccc agaaagctga agtactgtgc ctaaggtcag gctgctataa tgggcttatt 65640
gtgacagtgt gactgtaatt attttaatag tcaaaccaga tgacaggtaa ccagtgaaca 65700
gatatactag ctatgtctga cagatacagg gaatattagt ccaagctccc taagacatgt 65760
taagacatgt tttattgtat gggtatgtag gagctggtgt gtgtgtctgt gtgtgtttgt 65820
gtgcctgggc ttctctatgt ctgtgtatct gtgtgtgtgt gtgtgtgtgt tcacttgcat 65880
gtagaagcct gaagtcaact tcaggtatca ttctcagaag ccatcgacta ctctgtcatc 65940

tgtctgtctg tttttctgtg tgtgtgtgtg tgtgtgtgtg tgtgtgtgtg tgtttgtgtg 66000

tgtgtgtgtg tgtgacagac aaagagacat acagagacat tgtgtatgtc catttatata 66060

tggtacatat gcatgtgcaa aagtctgagt ctacaggtac tcatgcctac atgtgcacag 66120

aggccagggg agaaagtagg gtgtcctgct ctatcattat ctatcttgtt ctattgagac 66180

gaagtatctc actgaccctg gagctgggct ttcagccagc aagctccagg gatttcccct 66240

cacatacaca cacataacta gcactggagt tatacacaca aggccactgt gcctagcttc 66300

ttatgtaggt gcaagagact tccacccagc acctcatact tcccactgag ccatctcccc 66360

agcccacctt actttttat actgtacctc tgactgacac ccagggctta ccagtccagg 66420

tagactgaca ggccagcaac acccagggat ccagtcactg tgtccccaat gattagaaag 66480

tgtgacacca tgtccagcat tttgcatggc tgctggggtt gtggtcagtc aggccatcat 66540

gcttatgcag taagcatgct gcatacagaa ctattgtctg agcagcatgg caagataata 66600

gtattgtgac aagaggcagg atgctacaac ctgcaagaat ctacccatag aaactatagc 66660

cagtgcttgt agacatggag acagtgcatt agtgtgtcga gaggaaaggc actgctcttt 66720

ctgcttacag taccccccag ttcacacact ggttgcacag tgcttagcat agctgtgctc 66780

cagagtatcc caccccaaca ttgatctcca tccccatgg ctgccaaaga atggcttcag 66840

atcagaagca gagactgatc ttggtttata aacactgctc ttcaaagctc tgatcactga 66900

ttttgttttg ctgatttttt tccttcttct ataatccctg tagttctaaa gcaaaaacaa 66960

aaagaataat catgagttga tttgcctttt taagaacaaa agaaaaacca gagcctgttt 67020

tgttaggaag aaccccctct tgggcttctc tctgtggggg tcttaaattc acagccaaag 67080

agaaaagcac atatgtgtct gtgcagcgtg tgtaagcaat gtgtctatgt gtgcctgcac 67140

acccatgctc caggaccagg agaggccaga agacatgttg taagcagcac agcaaggaca 67200

tgcctacctc atcttcagtc cccagcttca agtgtccctt ttatctgttt ccaccccac 67260

atacacttct ctctcttccc ccaaagcacc ttgagctctc ctcaacaaca gcaacctctc 67320

cctcttacct cctggtaatg gtctgctgtg attcctcctt gtaacatatt tctgtctggc 67380

tataagctgc ccacccttga gaatccagag cagcaaggaa ggcagtgcaa gcaagtggct 67440

agccaatgat ctgggcaaac tcctggagcg tagccatgtt tttcccattt agcagctgtg 67500

tgaccttgac cttgagcaaa tcccaagacc ctcatctgaa aaacaaacta actcaaaggt 67560

caacataaga tttaaacagg acacgaatag tgagggttta actctttcct ctgcctgttg 67620

caacaatgct agctattata tgcttggtga gttgtccatt ttatttttaa tgaacaacaa 67680

aaaccaattc ttaagaatat aacagtggac tctgaccatg tagctgcata ttgattttat 67740

ctagaagtaa taaataatat cactctcttg tctctctaga aacatacaaa tagagaaaac 67800

acaacacgta ttgaattctt tgagcagtaa cttcttcctc ctctaggaag ataaacactt 67860

ccacgtccct gtgcacaacc ataaaggttt ctgcaaaaca ggcaagcagt cccattaaca 67920

gagaatcgtg actgctattc tcttccagag acccttgcaa cccaccaaaa atgtttaggg 67980

taaggacctg aaagtatttc cttggtctgt cccagtatct gtccacacac tcatgttcct 68040

aggtctctct tccaatcatg aggtctaaag tatagttgtc acctcttttc tcaaacttca 68100

gaacagtctc tactctaaac tgcccatatt tcccaatcag gcaccctcta aactcctcag 68160

ccccatttca atcaagatag acttgaagcc acacttacca aggttatttc tattccaaag 68220

ttcttgcccc caaacccacc aactacctcc taattgagca taagcctaat gcatattaaa 68280

taaccaatgc aagtttgtag aatgaggact aagtgttaga tggtagacaa ttgccaataa 68340

cagaaggcat tcattataga atttgagaag caaggtttta tatggatagc aagcacttta 68400

agggttttta tcaagcccat gcatttagct actggaagtt ctttttttatt attattgagt 68460

ctaattggaa ggaaagcatt gttgcatagg tatctctaac tcttgcatgt gtgtgtgtgt 68520

gtgtgtgtgt gtgtgtgtgt gtgtgtgtgt gttcacacct gtggtacatg tgtgaaagat 68580

gtctgaaaga tgcaggtgca tgtgtgtttg tgcttataga ggctgtatgt cttcctctat 68640

catattttat cttatatatt gaggcaagat ctcacactaa aacccagagc ttgccaattc 68700

agtgggtcta gctacctgat gctcatggaa ggggaagaga tctctttcct cagcttcctg 68760

agaacttgag ttacagacag gtcattgtgt ctaaccagct tgtatgtgga tactggacat 68820

ctaaactcag accttcatat ttgtgcagtt agtcttttag tgttatgcca tatctccagc 68880

ccctctccag ctttttaata ggtatgactc tggtgttttg tgcttagcta tctgttgtag 68940

ggctcaggac ttgaggaccc catagcagaa agctacacac tgaagtaaca aacacctagg 69000

aagctcttta gtagaaaaat atcatgccca ctgctcctgg aattccatca cgattgtgac 69060

attgctacag atgatgcctg gtggctttcc tactatgtgt taccaaacac atggccctga 69120

gtaagtcatg gatgaggcca cgggttctgc agcatataag acagtgtaga tattactgtc 69180

attgtctaga tgagaaaact gagatctgaa gaagaaagct ttgtttataa gacacagatt 69240

gtgaaatgta gcataaccta catcagccag caaggtttcc agttagaaca agtcagtgaa 69300

ttgaataagc agagaaaaat attccaaggc agagttcatg tacatcagta ggatctactc 69360

tcagctagac tcatctagat ctctcctgag gagtgtctta aggttcaggg tagactgtgt 69420

aggaaaaggt cgcataagga agaaactaga gaggacaatt gcacctagtg aattctctgc 69480

atgccagagt ctatactaag ggttttacat ccattatctc tactaatcct caaaacattc 69540

ctgtgggtac cactttcacc attggaaggg tgtagcaact gaagctcact gaagggaaga 69600

gacttgtccc aggctgccca acctctcagt ggtgtttgaa caattaagat cttgtaggtt 69660

cctcagactc tgtggcttga aaaagaaaag atttgccacg tacagggaga ataaaaagaa 69720

aaagaaaaaa aaaggagtgg tagccgcaga gatctgggta gtttctgggt cacaggaaaa 69780
aaaaaactgt aggagcagat gtgttagatg agagaaaaag acagaaatgg gtatggaaaa 69840
aattctctcc gaaaacctct acgtgtttcc aagcttaatg accaaaacgg gctgaagcag 69900
ggaagtagtg aggaagcagg ctcccttgag actggaaatt gtttcctgag ttagcatggt 69960
cagctgaagc aggcagaatc ctaagccaaa aaacacacag agagaagcca aaggaatcca 70020
gatttgggga ggagaaggga agacagcagc cgggcccctc tagtcatggg ctgcttcttg 70080
ccagtggacc cagtgtccct ggtgtaggtt tagttatgac ctgaccttgc ttcttggctt 70140
tggatctcct tgaagataca tgagtgggca gtcaaaatta gcttccaatg agcttaattt 70200
ccggatgcca ggctgcccag tgcagtgatc cagaaaggtc ccatcagaat aatgttagtt 70260
ggagaaggga ggcttacttt ggtcgtttgt ataatgacta caaccgaatt ttcatctcat 70320
gcgtgtttat atgagaaatg agctctataa gaaagaacca tgcactttta tatcttacca 70380
ttaaaaaaga aacgtaaaac aaacatctgg aaaaaatgcc aaaaattaaa tcaaaataag 70440
ttattcagat ctgtgaagtc actctcctct cctctgtgga aactggccaa tgtgctaaca 70500
tccatcttgc gtgaagccaa ttgaccatga agcccacagt cacctggtct acgtggtcac 70560
tgtatctgct gtgagttttc tttcatgtca cagtgtaacc atgtatcatg cattctctat 70620
tcctgaggct ccgttcagtc atcaactcta gatactaaag gctcctgggc aaggaatcca 70680
ggggaaaaag ctattgatgg acaacatagg taatgtttca gggtttctgt tttgttttct 70740
ccttaacagc catgggttct ccgaaggaaa tcatgttctc agacatcact gaaaatgcag 70800
ccacagtcag ctggagggca cctactgctc aggtggagag tttccggatc acttatgtac 70860
ctatgacagg aggtagggaa cctggaggtg gaagacggtg gggagaacat gcaacattag 70920
ggttaaatgg caaatgagac cctgggtgtt cttatcagac tttcttcaag cattatggct 70980
aatgaaaatc acttccctcc ttctgagatc atctagaaag atgccctttg taaagacatt 71040
ttggaaactt ggaaaaagtc accttattaa cactctctgt gaccttgata ttgtaaaata 71100
ttttcaagga aatggttccc tagtactttt gaacacaaac acaagggaat ttatataaac 71160
aatctttttt tttccccttt gatcacagtc atctggaaag ctgagatatg tgtgaggttt 71220
taccccttagg gaccatatcc tatctcacta caggatagag aactgagtct tagcaatgtt 71280
agaacttgga acacatagac tttaatgaag acctcaggga agtatagttc gaacacagcc 71340
tagcaggtag tgagctgcct cttgttggag gtttgaaaag acaggctggt tggtaacggc 71400
aacactgtag catctccatt tgggcgtttg gccagctgga ttgcaaatcc ttcactcttc 71460
ctgaaattct aagaaatata aattccagac ttcctaataa atttaagagc acacatttaa 71520
ctgggaccat ctttttttatc atgactaaaa tctctccaag taagggggtc tatagcatct 71580
taagtcaacc caatgagtta catagcttgg atctatgcta cattgtgagc ttcccatata 71640

gtgcatggag ggacaagaat aaggaaaata aaatttgtgc aacatgccaa ctccttcccg 71700
aaaggaaact tgccattttt agtatactgt gacttcatga ttttttcctt tcctgactgg 71760
gtttttggca tctgcaccaa tggaaaaaaa cattatacaa aaagtaaggt cttgtccttc 71820
atgccagagg gtgggatgag aggagaggaa ctccagagaa tgaaattgaa taaagatata 71880
atttaatttc caagttgcat ccacccggtt tggaggctat gacaaggaag tgtggaagag 71940
gctcccctga tcttgctttc gctaatctcc aggtgccccg tccatggtga ctgtggatgg 72000
aacagatact gagaccaggc tggtgaagct taccccgggt gtagagtacc gcgtcagtgt 72060
gattgccatg aagggattcg aagaaagcga tccagtctcg gggactctaa tcacaggtgt 72120
gtgttccccc atcatgttcc atccttgctc ttggcatgtt gactcgtgac tgagaacagc 72180
acaaatgctt gctcctggag tcccagtggg aagtgagctg gaattagagc atccagcacc 72240
tccctctggc tcatccttag cctctaataa actgggttgc cccagagaca tcctgaatgc 72300
cctctggcct ccatttcctc tgctactgtg gaaaggagtg aagtatagga accatactga 72360
gctgagaccc taaatgtgtt agccacgtga gttgtcagtt aatttctctg gggtctgatg 72420
aacttctctg taaagtgggt atgactctgc ttcccttgtc agtgttgcaa ggggtcagta 72480
agcataaaga caactttccc tgtcacctca ctccttcccc ctgttgcctg gaatcattgt 72540
gatcacctcc cgggacatgt gcttctgcag atatgggatg gggaggactg tggccacgct 72600
gtcccctgtt tagcaagggt tgacctgatg cactgtgtcc tcactgccgt agtcattcca 72660
agcttgtagg agaggcacct gagacatgag gagattacaa aacaacagga ctggaaatgt 72720
ggaagaaaac aagggtcatt cagactaacc ctctcaacca tttaaaataa aggttgtttt 72780
cctgttaaaa tctaatttaa gaataatggc tgctattgtg ggagaatgaa gtggaaattt 72840
tcccttcaga agtctctgtc tctagacaat ttatcctttg agatatgaca aattgtccaa 72900
cccagtccat tcagtgatcc gaagaatagg cctcccagga aatttcaaaa atatactact 72960
tccatacaag aattggtaat gtacaattaa aacagcctat ctggacgcaa agtcctgtct 73020
cctctcctct tgggcttcag ttttccctaa gaaatcttgc attgatgatt ctccacagcc 73080
acagaaacaa aagctgggtg tgtctcctcc caaggccaca ttgtcactag cagttaaaga 73140
aaggtctgta cacacatgag agaatcaggg ggacactgtg aggtgaatta tattgactct 73200
gacactggtt tgtcacttcc tgactcttca gccagttccc taaggctcgg gtaccagtct 73260
gtgagctggg ctgctgtctc ctaggctgtt gagggagtaa ttaaattatc gttagaaaaa 73320
ataagagaga gatcccgcct tcccatcaac tttcaaggaa gaaactctga acaaggaaca 73380
tactcctttg aaagaagcca gagattaaga cccctggaat atcctctgac aatataagtc 73440
atttgaaagt aacgcccaag ggccagagca gtggcttatc gtttttactt tccttgtttc 73500

tttctctttt ttgtttttgt aagatttatt tatttattta tttatttatt tatttattta 73560
tttaatatat gtgagtacac tgtcagtgtc ttcagacaca ccagaagagg gcatcggatc 73620
ccaatacaga tggttatgag ccaccatgtg gttgctggga attgaactca ggtcctctgg 73680
aagagcagtc agtgctctta accgctgagc catctctcca gcccatggct tatcttttaa 73740
gagcatactc tgctcttatt tgagtcctcg aatttaattc ctagtatcca taaccgaagc 73800
cttgaacagt tacacttaca tgcacatacc ccatacagat aaatacatac ctgttgtctt 73860
atccatattt gtcattgttg tctgatataa tcttactatg tggctctggg tggacacaaa 73920
ctcatgatcc tcctgcctcg ggaccccaag gattgaaata aaaaagacta aaaagtttta 73980
caaagtatag cattaataac aaatgctgag agcttccatg ttatataggc acacttcagt 74040
gaagtggcct ttccaaagac atggagacac aggaaaataa gccacaatag aatctgaccc 74100
tagagacctc caggttatta cctataagat cccctaaata aagatatcac ccaaagccca 74160
gaaaagcctc aggcagtagt gaccatacag gtctcctcta tgtatgttta tttatgttcc 74220
tttcctggaa aaaaggaaca tgaatcctga caataacctt tgtcacacat taaacattgt 74280
tcaggaactg cctacctgta acatcacata taagtcaaaa agaaaaagcc agccaggatt 74340
atcatgccca ttttacatat gaggaaatag agaccaaaac taatacaaca gtaaggaaaa 74400
gagtcatgat ataagtcagg ttagcctgtt caaaggggcc tgactctgag cccatctaag 74460
cacatgtcct ctgatgttat cctctaacat tttgaagtct tctcctttat ttctttccag 74520
ctctggatgg tccatctggt cttctgatag ccaacatcac agactcagaa gccttggcca 74580
tgtggcagcc agccattgcc actgtggaca gttatgtcat ctcctacaca ggggagagag 74640
gtaaggtcct gtcccacctt ctctacactc tgatgagggt tcatcttacc gcagactcct 74700
cccattcccc ttccaatgta ccatgggatg agaaaactca gtggtacgct gtaagagcca 74760
gtcatgtctt tgcctacctc cactgtatcc tcgaaagact tgggcaagcc attcaagtga 74820
aaagagaaag aaaaagaagg cgagtctgga gctttgcacg ttttgctgac ccatcccacc 74880
ttgctagact cttgagttac ttttctcatt gctatgacca aatacctgac aactagttta 74940
aggggtagca ggtttactct ggattctaat tcaagaaggt aactcccaaa tgtcaagaaa 75000
gtgctcgcag cagtaacagg aagctgctgg tcccaaggta tccaaagtcc ggaagcagga 75060
aatggggccc tctataaagc cttagagcct acctcaggtg gctcacttcc ttaagcaggc 75120
tccacctcct cactaaacca aaaccttctc aagcaaggct accaactagg aacaaagttt 75180
tcaaacccat gagcctgtgg ggacatttca tattcaaagc acctcaggga gggacagact 75240
ctcaaggaag cagctcttat tctttacaaa gaaggtttcc agagtctcag gtcttaacca 75300
aatggcatca tgatgtcctg gggtgcctag ccttctcaga ggccatggga gggcctaact 75360
gaagccaatt tgactgaaag aatctgcttt tcccatctgc caccatctgt accaactcac 75420

ccagcaggca aattaaactc ttgctgagac catcaaccaa acacaaccac caggaagtaa 75480

atcaaatgca atcaaatgca aatcaaataa ggaaaagagc cgctttcttc tgattccatt 75540

catatgttta tgatgatcta gtctagaaag acctctgtta tttcattttc taattttcat 75600

attttatggc tcaagactgc ttggctttca ctggcagtat tacaggacac cataacccat 75660

gggggggggg cctttttttt ttaaatcttc attcagctct ggcaaaaccc tgagtttcct 75720

gggaaaagag aaaagatgag gcaggaggag aaaattcagt tcatccattc tcctctttgt 75780

tattccctgt aaggaaattc tcctgagaca gggagagaat ggtgtcccct ccccctgaac 75840

ttatggctgg agggaatggt gaaaatttta gatcaggact aatcaagacc tagccaaaga 75900

ggtccagaac tctgaccttc tttgctgtgg gggtcactga aaggttgtgt cttagaagga 75960

ggtatatgtg ctgtttgcca aggggtaact gccacacgtg tgtgtttcat acttagtgcc 76020

agaagttaca cgcacagtgt ctggaaatac agtggagtac gagctgcatg acctggagcc 76080

tgccacagag tacatcctga gtatctttgc agagaaagga cagcagaaga gctctaccat 76140

cgccaccaag tttaccacag gtaccaagac agccagagtg caagttctcc tttctgagtt 76200

gcccacccac agcctgcatc ttaaaccaca tcccctcccc tgctcttcct ccctaagtca 76260

ggcccttgca gtataatgac atcagtatga gtaaaattta ccagtgccca ctcaatcaag 76320

acccaaagaa gcataccatt tccccttgaa ctgaaatgac ccaacagatg agaaaaggat 76380

atttaaggga agcttttgta agtggaaaaa cactacctac tggctctaaa cccagcattt 76440

aatgatccat gctccaggct gggaaatagt ttttaagaag ttttcagggt agtttcctta 76500

gatgttcatt ggggttttat gtattgagat ctttttacaa agctcaacat ttggagaagt 76560

aaaggactgt cccttctgtt ggggtgaggg ccaatattag cactcctcag ccctgtcggt 76620

gagaaataat ggatctgtaa tgattttcaa cccaaatcca acccaaacca cccaggaata 76680

tgttgggctt ttagaccctt gaggtgatag gactcatata acctttgact ttggttcaag 76740

ttcctcatcg atagtcaggt gggattcttg tccaaggcca tggttaatgt tattgaacaa 76800

acttgaggcc agttctcttc ttgctgtctg ttctacacct tctagctcat ttgatacata 76860

ttctcttctc cagacctcga ttccccaaga gaatttacag ctacagaggt tcagtcagaa 76920

actgccctcc ttacctggag acctccccga gcatcggtca ctggatacct cctggtctat 76980

gaatctgtgg atggtacagt caaggtatct ataaatcaca cattgctggg taaaacattt 77040

gatgtgcata atcatcaaag tagcaaagga ttcgctgata ctgtttcttc actccaatac 77100

aactaaacac ctaaaacaaa caaaaaacta gtagagactg gatatagaac cagaccttgt 77160

gagtcacaaa ccacatgctc actaatcttt cttcctttaa ccaggctcaa gctatctgag 77220

caacagccaa gatcaacggc agtgctttat aagacttaac attttaagag ttgggctcat 77280

ggccatctaa tgtttcattg agaaagtacc aacagccttt tctgcatctt tgccacgtag 77340
tgtcaccacc tgactgactg tgatctatct caacacatcc ttgcaatgtc acttgttaag 77400
accagctact tctattgcta atattacctg tgttctcccc tccatgaact ttactgtatt 77460
tctaagctag gttacttatt ctcttcatac ttagctatat gcagcatctc ccctatattt 77520
tttattagat attttcttca tttacatttc aaatgctatc ccaaaagtcc cctataccct 77580
ccccccacca tgctccccta cccactcact cccacttctt ggccctggcg ttcccctgta 77640
ctggggcata tagacaaggg atctcgccta tattctgtgt agctacttca cttttgctca 77700
gaaatacatg tcactgctat tgtagtctat agctcgggaa ttatgtttct tctagatcac 77760
attagttttg ttgctgtggt ttgggttagt ttccactgtc catgctagaa acttcttccc 77820
agtgtctttt agtcctggct tatcctttca tacttatgcc aagaacaaag aatctgatta 77880
gaagctcagc tgaacataag ggtttgttga cactggggtt caatgtagag atcagctgct 77940
gtgtacctgg aaatttgtgg gggaacccca tagtgcttag gctcttattc tatgcaagct 78000
ttgcttgctc ttacacttcc agtgtagtat cttgacctcc tcaccagctg atcctggtgg 78060
atttgaattc agagcctcca ctgtaatgca gattgctctg ttccttgagg ggacaaggag 78120
aatggggaat caagctattc ctcgtgcaaa gcttctaaac tgactgaacc tctcttgagt 78180
cacacactac attgcctgca aaggcagcta tgctgttata cacatgcatc agaaactcgc 78240
tgagtgcttc cttgtatgtc agccagcctg gggtagttct gcccactcca ggtggttcct 78300
agcactttct gctctggcac tgcaggaatt gttccctcta ctatacacct tctgcccagc 78360
aaggtacata ggagctacct gtttctatct cttcctccct cccattcact tttgtagttt 78420
catttcttct ggtccactcc tgttttataa gatatgaaaa acacatagag aaatattggt 78480
atttctgatc tgccatgttt aaccacaagt agacacattt ctagcttttc tggagttgag 78540
acagaactca tagtatgaac tgaagctggt cctttattgt catggtgtca gtgcccatac 78600
ctttaagata cgagcattgc acttctagac cccaaaagtc ttctgcaggt ttgaggttct 78660
aggattgtgc ttccctggtt cataaacctc atttcagact atccagactt agacaaatga 78720
acacagaagc agagagctga ttgccagtct aggctcaggc aattcaataa accttgcttt 78780
gttccctgag agttgatcct tccccagaaa ccagagctga tggcaccaca aaggtcacta 78840
ggtcactggc attgagtgaa cacacataaa tcatggataa ggtggtggga gactaggagg 78900
acgtgacact gtcctctgcc atcaacaaat tatggattgg gagcaagtat cttagcttct 78960
gggaaattca attcccacac tgtaaaaaca tacataacat accctcactc caggaagtcc 79020
ctaggaaggg aaataacctt ccagtggcct ccactctgac ctgttattga ctaaacccaa 79080
tgaacttggt gagttctaga gatcagaatt gtgtttgccg tgatggagga ggaattccag 79140
tctcaaacac caaggccatc tattaataag aactggagcc cagtatggat ctgaggcaat 79200

gtggatcttg tagaaactga gcccaaacac tagtctagaa cccagggacg agccataagc 79260
atagtagggc tcacacatct gttcctcccg ggtcagctca agtctttatc ctgccccacg 79320
cactaggcta ttaacgtcat cttgcaactc aagagttcct cgacctgagt tttagtttcc 79380
ctagagatac gtctgcctcc ccaccttctg aaaattcagt gagtctacac tgaggcccag 79440
gttatcagac tgtttaaatg ttgtgataaa ggctaagccc cctttcagaa ctgctgacac 79500
aaatggtctc agtgtctctt gccccttgtg caattcacct taccatcagc ttaagcttct 79560
taagcctcaa cagcttaatc ctgttcaaaa cctctcagac ctcccaactc cttgttgttg 79620
ataaacgcca agccagtcaa gccaaagtgc gtaacatccc attaatggtg tcctgtaatc 79680
cctctttgcc catttctatt acccaccccc acctaaaatt aagtagaact aatgcaaagc 79740
agtgcttctg tgttatttaa cccccctgcc tttctcatcc cagagctttt acttatgcag 79800
cactcttgct atctcagaca ttccagaggt acacttggaa tctggcatgc agtaggtgct 79860
gggtggggtt ttatgttaag tgaataaatg attgcattag ccagtgagag gaagcatctt 79920
tgccgtgaga ctatctggga ccaaactgat tcttttgcct gcccacagga agtcattgtg 79980
gggcctgaca ccacctccta cagcctggca gacctgagcc catccaccca ctactcagca 80040
aggatccagg cattgagtgg gtccctgagg agcaagctga tccaaaccat cttcacaaca 80100
agtaaggggt tcagagagga acacagtctt aagatctgac cttgtgacag ctaatcaacc 80160
ttccacttcc ccttcaactt ctagtttaca ttttcaaaca aaaatctgaa acgtatcaat 80220
tccctattag tgcttagaag cctggggaat gtagatgtag gcatcctagt gaaatgagta 80280
gaggaccaat gagagctcat ctacatccaa tcctcacccc catgtcctac tggatacaga 80340
ttagaagaca cctatattcc atgagcctta ctttcttcat ctttgcaaag tgatataaaa 80400
aaaaacaaaa caaaccaaaa gactcccctc cttcacaagt atctcatggg agaagttgag 80460
gtatcatatt tgaattccta gcttatctgt ggctcttata ctgttaagtt taatataagc 80520
tctgtcatga ggaacacaga gaagcagggg ctgcctgctt gtaggaacct agtcactgca 80580
tagatgaact gaagtgtgtt tggacagtag ccacctcctc cctccactct cacactcttt 80640
ctctttatcc tgcagttgga ctcctgtacc cattccccag ggactgctct caagcaatgt 80700
tgaatggtga tactacctct ggcctctaca ccatctatat aaatggtgac aagactcaag 80760
cactggaagt ctactgtgat atgacctctg atggaggtgg atggattgtg agtacaacag 80820
tgggttttag agttctggga catgggcttg gatgaaggga agtgtgtact ctcaaataca 80880
tgtgcataag actttgcaag gtaatccttg tcacatggaa gcccttcaca attatctatt 80940
taaataaaaa atagaaggca aggactggat atgtagttca gttagtagag cgcttgctta 81000
gaatgaagaa ggttttgagt tcaaacccca gcactgcaga aaaacaagaa caaagttgct 81060

aactgtctac ttgcactcag aaggtagagg cagaaggatc aggaattcta ggtcatccta 81120
aaatatcagt tttgagccat tctggactaa cagaatgagg tggcaggagg aactctgcta 81180
tgaatggcag tgacaatcaa aactactagt tactataacc agtgttggct aagtagttac 81240
tctttaccag acttctgata agaatgtcag ggcttatttc aatcccatga ccatattgac 81300
tgtataggat tttcttttat caataaaacg tgtttgtagg tcacaccccc aacctctcct 81360
ccactttcag cttcagtgtg atgcttcatg aagactgaga ggctaagatc caacgatgtc 81420
accagcacta agaagcgttg tccattccta gttttctgaa cacctagacg tagggagggg 81480
agggacatta tgtgcccaaa gtcacaaggc aaataactaa gcaatggaga aagctggata 81540
tggatgactg ttcttccctc agtctggcat gcctccttcc aaaaagacat cttctcttct 81600
cttcctttct tttacttaag tatccatgtt gttgtaggtt ttcctgagac gcaaaaatgg 81660
acgtgaggac ttctatcgca actggaaggc ctatgctgct gggtttggag accgcagaga 81720
agaattttgg cttggtaatg cagctctgaa agtttgtgac agatatgcct atttctgtcc 81780
tcttggtcac ttaccctcct gtcgtgagca acagggagag gaaaggatca aaaggaaaga 81840
agtttggtag gagccacata gtgttgaatt aattaaatat tccaggtcac aaaaataaag 81900
cattatcaag ccagacctgt ggacaaacct tgcattaagt cagacactaa aaccaattat 81960
ggagccaacc tcagtgctgt cgtgaactgc tttcctctta agctcaaaca ctgagatggg 82020
tctcctggtt gtaatctttc acctagaaca tccctgcaaa gagcagaatt cccatcttac 82080
tcatgagaaa acagaccctc aaggacgttg tatggtttgt ctatggcaat ctagccaatt 82140
gacaaataca ctgtccttca aattttccca aactagtata acagggccat cattcatcat 82200
ggacactttg cagcaaacca catggacaaa gcattgttat ctgctccact gtccgagcct 82260
aagacacccc atcccaaagt tatcctttgc ttcacattgg ttataaattt tagagtgtac 82320
aagtcacgtc cctggattat cacctttgac cttcacaata caataggaag ctgataagca 82380
aaggtgttga actgttttct tctgaggctc agaaacatga gatttgtttt gtctgccttc 82440
ataccagcaa acagagaatg tttcaagccc aagttttcag gatatgtccc actgttgagt 82500
ggtagggacc tcctcagtca cctgtgattg cttaccagac attgcccgtc atccacagga 82560
ctggataacc tgagcaaaat cacagcccaa gggcagtatg agctccgggt ggacctacaa 82620
gaccatgggg agtctgccta tgctgtgtac gacagattca gtgttggaga tgccaagagt 82680
cgctacaagc tgaaggtaga aggatacagt ggaacagcag gtatggggta gctacactgt 82740
gagtctggag tcatgaaatt cctatggatt cacgcattca ccactcctgg ttctgaatca 82800
acagatattt gaatgtcttc tggatgctga tctagaagaa ctatcattaa aaaggtctag 82860
gccaacatct gcaacatctg gtctctgaga ggtagagtag gtcaagaatc ttgtcaacta 82920
tggttgaatt ataatgactc tcattaaaag aatatgtttc ttattctctt gtagttagtg 82980

gttgtagagt ggtgatagga aaatggtctt tcaaaggcct ccatcttcac cagaagcttc 83040
cttgtaatgg gtatctgatg tctctgagaa tctgctgatg tgtgaattct gatatattgg 83100
gatgtcatgg ggagacgtta agatgagttt ctcctaagtt ccctggtgat gcctacattc 83160
ctagcattgt tagtcttttg actgccaagc aaccttttgt tcttcaccta aattataacc 83220
ctaatgggct aggttatgat tacctgatac ccaggctatc tgacaatatt tagtggtctg 83280
aggtcttact tatgactgta gccagatacc tttgtatata agagacatgc caatttatac 83340
tatttggggg agaatagagg tttttgtctt tgacttaaag tgacccttgt acagctttct 83400
ggctccacat taaaggagaa actaataaca gagctatgag atggcatcct tctctgtagg 83460
tattttcaac tactgaaccc tcacaaacgt cttttaggaa aatgatataa agaggagaaa 83520
agtggcttgg aaaaattcag gaaatttcag ctcacagaag gagtatgtga gtgtgtgtgt 83580
gtgtgtgtgt gtgtgtgtgt gtgtgtgtgt gtgtgtgtgt acacatgcgc acccagccat 83640
gtgtgcctag agaacagtgt aaacaaagag gggctgggtc aagaatgcag accatcttgt 83700
gtaagccagg gcctctttgg gtggccttat tttatgatta tttaaacagg agacagattt 83760
gtgacccctc tgggagcccc tccagaaagg aaaaaatctt cacctttgac taataggtgc 83820
cagagccagg ttgcagtaag aaggacacag agcccctttg agaggcagtt tggtgtgatg 83880
gaagagagaa agccagggct ggaaattatt ttaacactta catgcctcaa aaaaaaaaaa 83940
aatctgacac tttatcacct aacaataaat acctctttgg ctgagatcat gtcaagaagg 84000
atcttgttac ttggaaagaa aatccaccaa ccaacaagaa tatgacttac gttctctctg 84060
tatctctggt ggaggagagc acttggtttt tcccttcact cagagccatg ttttcaggtt 84120
gtagcttcca gcatgctttg gtgatagcgc ctcacaggaa agcttcatca atatctgctt 84180
gctctcctgc aactcatggg tatcatcatt agttaaaaag actctgaaag agctttgaca 84240
gcttgctcca agatctcagc atcaaatacc tggatgtctt agaatgcttg cacacagccc 84300
tatggcttgg gagtaagaag gactcagata atattctcaa gtagcactct atgaaaaagc 84360
aggttcttag gtaagaatca gacaacacca gcctggtaca cactctttta aaaatgatta 84420
caagaaaccc aggctacctg acaacattta gagagtgaag gtcttaatta tgattgtagc 84480
cagacacctt tgtatatgag ggacctgccc tctgttatta tttttgcttt gtttgtaagt 84540
ttatggggtc ccattatgaa gcctacgttg gcctcaaaca tatgcacttc ctgccttagc 84600
ccatcccacc gcatgctggg attacagatg tccaccacca tgcctgacac acacatcatt 84660
attaaatgca aatttctcac cctgtcctca taggtctttt atgcaaagca aatatagaca 84720
aatcataaac aacaaccctg gctctttgta tgccacttct ccctgtccag aaaaacgttc 84780
tggacagtat attagaacag cgctgtgagc tagacagaat ccactggatg tctgcatttt 84840

tttagatgag aaaaagaaag cttagaggaa gctcagcctg tgacaggtca caattagaaa 84900
gacagagaga tggacttcaa tggaactttc cagaaccacc tgcagccttc tttcttaact 84960
tataacagtg cttctgtcag agataagtct tgggggtgac attcatgtct catgctttga 85020
tcctcagtcc ggtttgaggt tcagtgccag tgtacccaag gatctttcag tccccgcctt 85080
tgattctgcc cttgacaacc ttcggagact ttgatttggg gggagtcttt attctctctt 85140
atctttctcc cttttattct caataaggtg actccatgaa ctatcacaat ggtagatcct 85200
tctccaccta tgacaaggac acagactcag ccatcaccaa ctgtgccctg tcctacaaag 85260
gagctttctg gtataagaac tgtcatcgtg tcaacctgat gggcagatat ggggacaata 85320
accacagtca ggtgagtagt ctgacaaggt tggagaagca agatgtgggt gagggaagga 85380
cacttcaatt agaaaatgtg ttatcttcca acacagtgag aaatctcaat ggcatggcag 85440
ctgagtgagc gaagaggaaa aacagtacaa attaactctt agatcccaag gagaaacact 85500
gtccaaatag ctcacaaatt tagaactttc cacacagata aaattactga tagaactctg 85560
tgaagctgat caaagtggat gctattcagg ggagacaggg atgatataaa ccctggcaga 85620
gcacaggccc acaagcatct tcatagtccc aagaaggacc tgggcagaag gcatgaactg 85680
caaatcccct tgactcaaac cctgtgagag ctgaccttcc tgaggttgca gaatgatttt 85740
ggaagccagc tatgaagttt gcttcttctt ctcaccttta tgtgggctct caggtctcct 85800
ggcttgagag gtgaactcct ttccctgttg agctgtccac tcagctctca catggaagca 85860
ctgatatgca atcctaaccc tgcatgtcaa gttcaaagct tatatcctac ctgcactctt 85920
ttttgctgtt ctatgccaaa cccttaataa aatgccccca tgggaatctt gtgaattcac 85980
acacacacac acacacacac acacacacac acacacacac acacacacag tacccagctt 86040
ctcattatgt tgtgctcata aaaagataat aaaaaggtat taccatatgc tctatgaggg 86100
ggggggggta tgggggaagg ggatgccttg atgggcccat gccaaggcat cccttcccct 86160
gagggaccag ccacacagtg gtatagtata gaatagagtt tattcagggt atggggaggg 86220
gagttaaaag ggtattagag gcagagagag ggagagaata agaaagtaga ggcccgacat 86280
gaccatgtgg agagagggga gaagggaatg gggagagaag gagcaagagg caagggagag 86340
aagcaggagt aagagagaaa ggaggggggca agcagctcct tttatagtgg gccaggccta 86400
cctggctgtt gccaggtaac tgtgggagga gcatgcctgg acatttccag gtagctataa 86460
aagtggagtt tagacaaaat accaacaaat gtcgggcact attttatttc aagatggctg 86520
cccaaggctc tatgcaaata gcaatgattc ctaacgatgg gtaaagagtt tagattcagc 86580
tggcccattt gaaagttttc acagcactgg ccatcaagag acattcaaga cacagctgcc 86640
tgcaaaatgc caagtggtta taagctaaaa aaaaaaaaac cctttgtgtt ttgatgacta 86700
aatggtgatg gcccatgaac atgtgtccaa atggaactaa cccaggggtc tctgtttttt 86760

```
ttttttttct gcagggcgtt aactggttcc attggaaggg ccatgagtac tcaatccagt    86820
ttgctgagat gaaactgaga cccagcaact tccgaaatct ggaaggcagg cgtaagcggg    86880
cataaattcc agggataatt tggtgagaga ggaatggggc ccagagaaaa gaaaggattt    86940
ttaccaaagc atcaacacaa ccagtctaac agtgaagctc tccttgggca tttgatgggc    87000
gtccaagcag accacacgga tcactgaggc actgatgtag gcccctctgc aacgacttcc    87060
tttgcaccaa agacaacagt ctacaagttt ctactttctt gtttgattga gcaaagtctc    87120
ctagtgacag caacaccatg aattttactt ctcttgggtg gttctgggaa tgggagaggg    87180
gcaagctggc catacagggg acatggtcat tctccgagcc agctgtgttt tacacaaagc    87240
tgtacaattg ttattggttg aggtcacaaa gatgaaactt gtgtcattgg aagttacccc    87300
ttgccccacc cccaccctcg atgttttgta catttcctac aactgaagcc agaaaagtga    87360
tattgtttaa atttcaagga cattaatatt attaatataa taatggtgat ggtgatgatg    87420
aaaactgagg actttaaaag agattttccc ttcccaaacg tgtctggaca gtacctgatt    87480
gtattttttt tgttttgttt tgttttttaa taaaagcaca gtacttttca gtt           87533
```

## Description

## FIELD

The invention relates to a single-domain antibody directed against Tenascin-C (TNC), to an isolated nucleic acid that encodes a single-domain antibody directed against Tenascin-C, an expression vector comprising said isolated nucleic acid encoding a single-domain antibody directed against Tenascin-C. The invention also relates to a host cell comprising said nucleic acid and/or vector. The invention further relates to a said single-domain antibody directed against Tenascin-C (TNC) for use as medicament and to a pharmaceutical composition comprising said single-domain antibody directed against Tenascin-C (TNC).

The invention also relates to an immunoconjugate comprising a single-domain antibody directed against Tenascin-C (TNC) and a conjugating part.

The invention further relates to the use of a single-domain antibody directed against Tenascin-C (TNC) in an *in vitro* or *in vivo* diagnostic or imaging method and/or for detecting a tumor.

The present invention finds application in the therapeutic and diagnostic medical technical fields.

In the description below, references in square brackets ([ ]) refer to the list of references at the end of the text.

## BACKGROUND

It is well known that the extracellular matrix (ECM), is a highly abundant compartment of tumors and is a good tumor biomarker ([59], [60]). ECM is often more stable than antigens located on the surface of tumor cells. Furthermore, ECM is accessible to antibodies and their derivatives entering through the bloodstream [26].

Tenascin-C (TNC) was discovered over three decades ago is one of the extracellular matrix (ECM) molecules that is highly expressed in tumors such as breast, colorectal and/or gastric cancers ([1], [2], [3], [4]). High TNC expression levels is known to be correlated with shortened lung metastasis-free survival in breast cancer and overall survival in glioma patients ([5], [6]). At a structural level TNC is a large modular hexameric glycoprotein [7]. Each TNC subunit displays a central oligomerization domain, followed by 14.5 EGF-like repeats (three disulfide bridges per EGF-repeat), 17 fibronectin type 3 (FNIII) repeats (8 constant and 9 additional domains that are subject to alternative splicing) and a globular fibrinogen domain [7]. At physiological level, TNC is transiently expressed during organogenesis [8] and its expression is largely restricted to a few sites in the adult organism such as in some stem cell niches and in lymphoid organs [9]. At pathological level, TNC was shown to act at multiple levels to promote tumor progression into cancer by enhancing survival, proliferation and invasion of tumor cells. In particular, TNC can drive the formation of new but poorly functional blood vessels and to corrupt anti-tumor immunity altogether enhancing metastasis. Yet, in addition to tumors, TNC is also known to be highly up-regulated in wound healing and in chronic inflammation ([10], [11]). Using stochastic tumorigenesis models with engineered high and low levels of TNC it was formally proven that TNC indeed is a promoter of tumor progression. TNC is known to act at different tumor stages by inducing and activating a wide range of cellular signaling pathways such as Wnt, Notch, JNK and TGF$\beta$ ([12], [13], [14], [15]). TNC is also known to act on stromal and immune cells thereby promoting tumor angiogenesis and immune escape ([16], [17], [18], [19], [20]).

There is therefore a real need to find a method and/or a product that could inhibit or alleviate the pathological effects of TNC. In particular, there is a real need to find a method and/or a compound that could inhibit the tumor progression and/or cancer, for example induced and/or due to TNC. In addition, there is a real need to find a method and/or a compound that could inhibit or alleviate inflammation, for example by inhibiting the promoting inflammation effect of TNC.

Since TNC is known to be highly expressed in cancer tissues as well as its role in inflammation, research has been carried out to specifically detect TNC in situ as well as to inhibit its main pathological effects. These approaches included down regulation of TNC expression with siRNA or aptamers ([21], [22], [23], [24], [25]) and/or the use of TNC-specific antibodies for the delivery of radiotherapy or drugs ([26], [27], [28]). Monoclonal antibodies recognizing TNC have been also developed. For example, several TNC-specific monoclonal antibodies have been developed as well as aptamers and antibody fragments (scFv) that are currently undergoing clinical evaluation ([30], [63], [64], [65], [66] [67]). The anti-human TNC G11 antibody ([68]) was used to target TNC in glioma xenografts upon coupling with 18F-fluorodeoxyglucose ([30]). Phase I and II clinical trials have been performed with the F16 anti-TNC antibody in glioma patients ([69], [70]), breast cancer ([71], [72]) and Hodgkin's lymphoma ([28]). Coupling the F16 antibody to IL-2 (Teleukin (registered trademark)) was used to deliver IL-2 into the cancer tissue ([71], [73]). Since 2013, a phase II clinical trial using Teleukin (registered trademark) is in progress on melanomas (EudraCT 2012-004018-33). For radiotherapy F16 was labeled with 131 Iodine (Tenarad (registered trademark)) ([28]).

However, all these means and/or approaches demonstrate intrinsic limitations and caveats. In particular, the efficiency of the corresponding treatment does not appear to be enough for clinical efficiency. For example, due to formalin fixation, usually used in routine pathology service, often epitope recognition is lost which reduces the antibody recognition efficiency ([29], [30]). In addition, it is not clear whether targeting TNC with the known antibodies can reduce tumor progression ([67]).

There is therefore a real need to find a method and/or a product that could inhibit or alleviate the pathological effects of TNC. There is also a real need to find a method and/or a product, for example a more specific and/or sensitive product, that could efficiently allow to detect TNC in biological tissues, for example to identify tumorous tissue and/or inflammated tissue.

Moreover, it is known that TNC impairs cell adhesion to fibronectin and regulates the immune-suppressive tumor microenvironment, for example by chemoretention immobilizing dendritic cells in the stroma.

There is therefore a real need to find a method and/or a product that could inhibit/alleviate the immune-suppressive tumor microenvironment effects of TNC.

There is therefore a real need for a compound and/or method overcoming the shortcomings, disadvantages and obstacles of prior art, particularly for a compound and or method for treating and/or preventing pathological effect of TNC, for example treating and/or preventing tumor progression, cancer, inflammation.

## Description

The present invention allows to overcome the drawback and inconvenient of the prior art by providing an isolated single-domain antibody directed against Tenascin-C (TNC) wherein said single-domain antibody comprises the amino acid sequences:

i) GYTNSIYT (SEQ ID NO: 1) as variable heavy (VHH) chain complementarity determining region (CDR)1,
ii) IXaSRNGNT (SEQ ID NO: 2) as variable heavy (VHH) chain complementarity determining region (CDR)2, wherein $X_a$ is G or A
iii) AAGSSWDLILQAYAYDY (SEQ ID NO: 3) as variable heavy (VHH) chain complementarity determining region (CDR)3.

The inventors have surprisingly and unexpectedly demonstrated that single-domain antibodies directed against Tenascin-C (TNC) according to the invention bind Tenascin-C (TNC) with a high affinity. In addition, the inventors have demonstrated that single-domain antibodies directed against Tenascin-C (TNC) according to the invention is highly specific for human TNC (hTNC).

The inventors have further surprisingly demonstrated that single-domain antibodies directed against Tenascin-C (TNC) according to the invention allow to target TNC and/or identify TNC expression in non pathological or pathological biological tissue. Advantageously, the inventors have demonstrated that single-domain antibodies directed against Tenascin-C (TNC) according to the invention allow to detect tumors, in particular extracellular matrix of tumorous tissues, and also advantageously, cancerous microenvironments.

The inventors have surprisingly demonstrated that single-domain antibodies directed against Tenascin-C (TNC) according to the invention inhibit and/or reduce tumor and/or metastasis progression, for example by restoring the adhesion of cells in tumorous and/or cancerous tissue, advantageously tumorous and/or cancerous and/or mesangial cells to fibronectin and TNC decreasing and/or avoiding therefor the formation of metastasis.

The inventors have also surprisingly demonstrated that single-domain antibodies directed against Tenascin-C (TNC) according to the invention allow to increase the immune response in cancerous tissue. In particular, the inventors have surprisingly and advantageously demonstrated single-domain antibodies directed against Tenascin-C (TNC) according to the invention allow to inhibit and/or reduce the immobilization of immune cells, in particular dendritic cells, in cancerous/tumorous tissue. In other words, the inventors demonstrated that single-domain antibodies directed against Tenascin-C (TNC) according to the invention allow advantageously to inhibit the immune-suppressive functions of TNC, for example in tumorous and/or cancerous tissues.

In the present invention, the term "isolated" and its grammatical equivalents as used herein refer to the removal of a nucleic acid or a peptide or a polypeptide from its natural environment.

In the present invention, "polypeptide", "peptide" and their grammatical equivalents as used herein refer to a polymer of amino acid residues.

In the present invention, the terms "single domain antibody", "single domain antibody (VHH)", "monobody" and "nanobody" have the same meaning referring to a variable region of a heavy chain of an antibody, and construct a single domain antibody (VHH) consisting of only one heavy chain variable region. It is the smallest antigen-binding fragment with complete function. Generally, the antibodies with a natural deficiency of the light chain and the heavy chain constant region 1 (CH1) are first obtained. The variable regions of the heavy chain of the antibody are therefore cloned to construct a single domain antibody (VHH) consisting of only one heavy chain variable region.

In the present invention, the term "heavy chain variable region" and "VHH" can be used interchangeably.

In the present invention, the terms "variable region" and "complementary determining region (CDR)" can be used interchangeably.

In the present invention, the term "CDR" is intended to mean the three hypervariable regions of the variable regions of the heavy chains of a single domain antibody body which constitute the elements of the paratope and make it possible to determine the complementarity of the antibody with the epitope of the antigen. These three hypervariable regions are framed by four constant regions which constitute the "framework" regions (FRs) and give the variable domain a stable configuration.

In the present invention, Tenascin-C (TNC) refers to a glycoprotein that in humans is encoded by the TNC gene located on chromosome 9 with location of the cytogenic band at the 9q33. Preferably Tenascin-C (TNC) refers to a glycoprotein having amino acid sequence having at least 80%, for example at least 90% of homology with sequence :
MGAMTQLLAGVFLAFLALATEGGVLKKVIRHKRQSGVNATLPEENQPVV FNHVYNIKLPVGSQCSVDLESASGEKD-
LAPPSEPSESFQEHTVDGENQIV FTHRINIPRRACGCAAAPDVKELLSRLEELENLVSSLREQCTAGAGCCLQ
PATGRLDTRPFCSGRGNFSTEGCGCVCEPGWKGPNCSEPECPGNCHL RGRCIDGQCICDDGFTGEDC-
SQLACPSDCNDQGKCVNGVCICFEGYAG ADCSREICPVPCSEEHGTCVDGLCVCHDGFAGDDCNKPLCLNNCYN-
RG RCVENECVCDEGFTGEDCSELICPNDCFDRGRCINGTCYCEEGFTGEDC GKPTCPHACHTQGRCEEGQCVC-
DEGFAGVDCSEKRCPADCHNRGRCV DGRCECDDGFTGADCGELKCPNGCSGHGRCVNGQCVCDEGYTGEDC
SQLRCPNDCHSRGRCVEGKCVCEQGFKGYDCSDMSCPNDCHQHGRC VNGMCVCDDGYTGEDCRDRQCPRDCS-
NRGLCVDGQCVCEDGFTGPD CAELSCPNDCHGQGRCVNGQCVCHEGFMGKDCKEQRCPSDCHGQGR
CVDGQCICHEGFTGLDCGQHSCPSDCNNLGQCVSGRCICNEGYSGEDC SEVSPPKDLVVTEVTEETVNLAWDNEM-
RVTEYLVVYTPTHEGGLEMQFR VPGDQTSTIIQELEPGVEYFIRVFAILENKKSIPVSARVATYLPAPEGLKFK
SIKETSVEVEWDPLDIAFETWEIIFRNMNKEDEGEITKSLRRPETSYRQTG LAPGQEYEISLHIVKNNTRGPGLKRVTT-
TRLDAPSQIEVKDVTDTTALITW FKPLAEIDGIELTYGIKDVPGDRTTIDLTEDENQYSIGNLKPDTEYEVSLISR
RGDMSSNPAKETFTTGLDAPRNLRRVSQTDNSITLEWRNGKAAIDSYRIK YAPISGGDHAEVDVPKSQQATTKTTLT-
GLRPGTEYGIGVSAVKEDKESNP ATINAATELDTPKDLQVSETAETSLTLLWKTPLAKFDRYRLNYSLPTGQW
VGVQLPRNTTSYVLRGLEPGQEYNVLLTAEKGRHKSKPARVKASTEQAP
ELENLTVTEVGWDGLRLNWTAADQAYEHFIIQVQEANKVEAARNLTVPG SLRAVDIPGLKAATPYTVSIYGVIQGYRT-
PVLSAEASTGETPNLGEVVVAE VGWDALKLNWTAPEGAYEYFFIQVQEADTVEAAQNLTVPGGLRSTDLPG
LKAATHYTITIRGVTQDFSTTPLSVEVLTEEVPDMGNLTVTEVSWDALRLN WTTPDGTYDQFTIQVQEADQVEE-
AHNLTVPGSLRSMEIPGLRAGTPYTV TLHGEVRGHSTRPLAVEVVTEDLPQLGDLAVSEVGWDGLRLNWTAADN
AYEHFVIQVQEVNKVEAAQNLTLPGSLRAVDIPGLEAATPYRVSIYGVIRG YRTPVLSAEASTAKEPEIGNLNVSDIT-
PESFNLSWMATDGIFETFTIEIIDSN RLLETVEYNISGAERTAHISGLPPSTDFIVYLSGLAPSIRTKTISATATTEAL
PLLENLTISDINPYGFTVSWMASENAFDSFLVTVVDSGKLLDPQEFTLSGT QRKLELRGLITGIGYEVMVS-
GFTQGHQTKPLRAEIVTEAEPEVDNLLVSD ATPDGFRLSWTADEGVFDNFVLKIRDTKKQSEPLEITLLAPERTRDIT-
GLR EATEYEIELYGISKGRRSQTVSAIATTAMGSPKEVIFSDITENSATVSWRA PTAQVESFRITYVPITGGTPSM-
VTVDGTKTQTRLVKLIPGVEYLVSIIAMKG FEESEPVSGSFTTALDGPSGLVTANITDSEALARWQPAIATVDSY-
VISYTG EKVPEITRTVSGNTVEYALTDLEPATEYTLRIFAEKGPQKSSTITAKFTTDL DSPRDLTATEVQSETALLTWRP-
PRASVTGYLLVYESVDGTVKEVIVGPDT TSYSLADLSPSTHYTAKIQALNGPLRSNMIQTIFTTIGLLYPFPKDCSQAML
NGDTTSGLYTIYLNGDKAEALEVFCDMTSDGGGWIVFLRRKNGRENFYQ NWKAYAAGFGDRREEFWLGLDNLNKI-
TAQGQYELRVDLRDHGETAFAV YDKFSVGDAKTRYKLKVEGYSGTAGDSMAYHNGRSFSTFDKDTDSAITN
CALSYKGAFWYRNCHRVNLMGRYGDNNHSQGVNWFHWKGHEHSIQFA EMKLRPSNFRNLEGRRKRA (SEQ ID NO: 18). Preferably, Tenascin-C (TNC) refers to a glycoprotein having amino acid sequence of sequence SEQ ID NO: 18.

In the present invention, in the peptide sequences disclosed herein, the amino acids are represented by their one letter code according to the following nomenclature: A: alanine; C: cysteine; D: aspartic acid; E: glutamic acid; F: phenylalanine; G: glycine; H: histidine; I: isoleucine; K: lysine; L: leucine, M: methionine ; N: asparagine ; P: proline ; Q: glutamine ; R: arginine ; S: serine ; T: threonine ; V: valine ; W: tryptophan and Y: tyrosine.

Unless the context requires otherwise, throughout the present specification and claims, the word "comprise" and variations thereof, such as, "comprises" and "comprising" are to be construed in an open, inclusive sense, that is as "including, but not limited to".

In the present invention, by "consisting of" is meant including, and limited to, whatever follows the phrase "consisting of." Thus, the phrase "consisting of" indicates that the listed elements are required or mandatory, and that no other elements may be present.

By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

In the present invention, the variable heavy (VHH) chain complementarity determining region (CDR)2 amino acid sequence of the isolated single-domain antibody directed against Tenascin-C (TNC) may be selected from the group comprising IGSRNGNT (SEQ ID NO: 12) and IASRNGNT (SEQ ID NO: 13).

In the present invention, the isolated single-domain antibody directed against directed against Tenascin-C (TNC) may comprise the amino acid sequences i) GYTNSIYT (SEQ ID NO: 1) as variable heavy (VHH) chain complementarity determining region (CDR)1, ii) IGSRNGNT (SEQ ID NO: 12) as variable heavy (VHH) chain complementarity determining region (CDR)2 and iii) AAGSSWDLILQAYAYDY (SEQ ID NO: 3) as variable heavy (VHH) chain complementarity determining region (CDR)3, or the amino acid sequences i) GYTNSIYT (SEQ ID NO: 1) as variable heavy (VHH) chain complementarity determining region (CDR)1, ii) IASRNGNT (SEQ ID NO: 13) as variable heavy (VHH) chain complementarity determining region (CDR)2 and iii) AAGSSWDLILQAYAYDY (SEQ ID NO: 3) as variable heavy (VHH) chain complementarity determining region (CDR)3.

In the present invention, the isolated single-domain antibody directed against Tenascin-C (TNC) may consist of the amino acid sequences selected from the group consisting of WLQLVESGGGSVQTGGSLRLSCVVSGYTNSIYT-LAWFRQAPGKEREGV AAIGSRNGNTYYDASVKDRFTISLDKAKNTVYLQMNDLKPEDTASYYCAA GSSWD-LILQAYAYDYWGQGTQVTVSS (SEQ ID NO: 4) or WVQLVESGGGSVQTGGSLRLSCVVSGYTNSIYTLAWFRQAPG-KEREGV AAIASRNGNTYYDASVKDRFTISLDKAKNTVYLQMNGLKPEDTASYYCAA GSSWDLILQAYAYDY-WGQGTQVTVSS (SEQ ID NO: 5) or a functionally conservative variant thereof comprising a conservative substitution of one or two amino acids in one, two or three of the CDRs included respectively in the amino acid sequence SEQ ID NO: 4 or SEQ ID NO: 5. The functionally conservative variant may also comprise one or more substitutions, in particular one or more conservative substitutions in the regions respectively of the amino acid sequences SEQ ID NO: 4 or SEQ ID NO: 5 which are not CDRs, such as the "framework" regions.

In the present invention, the isolated single-domain antibody directed against Tenascin-C (TNC) may comprise the amino acid sequences selected from the group consisting of WLQLVESGGGSVQTGGSLRLSCVVSGYTNSIYTLA WFRQAPGKEREGV AAIGSRNGNTYYDASVKDRFTISLDKAKNTVYLQMNDLKPEDTASYYCAA GSSWDLILQAYAYDYWGQGTQVTVSS (SEQ ID NO: 4) or WVQLVESGGGSVQTGGSLRLSCVVSGYTNSIYTLAWFRQAPGKEREGV AAIASRNGNTYYDASVKDRFTISLDKAKNTVYLQMNGLKPEDTASYYCAA GSSWDLILQAYAYDYWGQGTQVTVSS (SEQ ID NO: 5).

In the present invention, the isolated single-domain antibody directed against Tenascin-C (TNC) may consist of the amino acid sequences selected from the group consisting of WLQLVESGGGSVQTGGSLRLSCVVSGYTNSIYT-LAWFRQAPGKEREGV AAIGSRNGNTYYDASVKDRFTISLDKAKNTVYLQMNDLKPEDTASYYCAA GSSWD-LILQAYAYDYWGQGTQVTVSS (SEQ ID NO: 4) or WVQLVESGGGSVQTGGSLRLSCVVSGYTNSIYTLAWFRQAPG-KEREGV AAIASRNGNTYYDASVKDRFTISLDKAKNTVYLQMNGLKPEDTASYYCAA GSSWDLILQAYAYDY-WGQGTQVTVSS (SEQ ID NO: 5).

In the present invention, the expression "functionally conservative variant" refers to variants in which a given amino acid in a single-domain antibody according to the invention is replaced without impairing the overall conformation and the function of the single-domain antibody, including replacement of one amino acid with another having similar properties, for example polarity, hydrogen bonding potential, acidity, basicity, hydrophobicity, presence of an aromatic group etc. The amino acids having similar properties are well known to those skilled in the art. For example, arginine, histidine and lysine are hydrophilic-basic amino acids and can be interchangeable. Similarly, isoleucine, a hydrophobic amino acid, can be replaced by leucine, methionine or valine. Such changes should have little or no effect on the apparent molecular weight or the isoelectric point of the nanobody. A natural amino acid can be replaced by an unnatural amino acid, such as an amino acid in D configuration, or a beta or gamma amino acid. For example, table 1 below discloses examples of conservative substitutions.

Table 1: Examples of conservative Amino acid substitution

| Original residue | Substitution example |
|---|---|
| A | V, L, I |
| R | E, Q, N |
| N | Q, H, K, R |
| D | E |
| C | S |
| G | N |
| E | D |
| H | N, Q, K, R |
| I | L, V, M, A, F |
| L | I, V, M, A, F |

(continued)

| Original residue | Substitution example |
|---|---|
| K | R, Q, N |
| M | L, F, I |
| F | L, V, I, A |
| P | G |
| S | T |
| T | S |
| W | Y |
| Y | W, F, T, S |
| V | I, L, M, F, a |

In the present invention, isolated single-domain antibody directed against Tenascin-C (TNC) of the invention may have a suitable binding affinity for Tenascin-C. For example, the single-domain antibody directed against Tenascin-C (TNC) of the invention may have an affinity for the Tenascin-C (TNC) from 0.5 nM to 800 nM for TNC. For example, for human TNC the single-domain antibody directed against Tenascin-C (TNC) of the invention may have an affinity for the Tenascin-C (TNC) from 0.5 nM to 800 nM for TNC of human origin. For example, for mouse TNC the single-domain antibody directed against Tenascin-C (TNC) of the invention may have an affinity for the Tenascin-C (TNC) from 0.5 nM to 800 nM, for example from 400 nM to 750nM, for example equal to 500 nM for the single-domain antibody directed against Tenascin-C (TNC) of SEQ ID NO: 5, or equal to 711 nm for the single-domain antibody directed against Tenascin-C (TNC) of (SEQ ID NO: 4).

The term "affinity" is intended to mean the binding capacity between a macromolecule and the antigen that it binds, in particular the binding capacity between a single-domain antibody (nanobody) and the antigen that it binds, for example an isolated single-domain antibody directed against Tenascin-C (TNC) of the invention and the Tenascin-C (TNC) of different origins.

The affinity and thus the capacity of the isolated single-domain antibody directed against Tenascin-C (TNC) of the invention that can bind to the Tenascin-C (TNC) can be measured in vitro by several methods, including surface plasmon resonance (SPR), in particular using a BIAcore 2000 instrument-Pharmacia Biosensor, Uppsala, Sweden) or for example by means of an ELISA assay or for example by Immunofluorescence Titration. For example, the affinity may be measured by ELISA, in, for example, using any saline Phosphate buffer thereof and the effective concentration for 50% of specific binding being determined from the standard curve as disclosed in Hmila et al., 2010 [32].

In the present invention, isolated single-domain antibody directed against Tenascin-C (TNC) according to the invention may be obtained from a nucleotide sequence.

An object of the present invention is thus an isolated nucleotide sequence encoding isolated single-domain antibody directed against Tenascin-C (TNC) according to the invention.

In the present invention, the isolated nucleotide sequence may code for an isolated single-domain antibody directed against Tenascin-C (TNC) which may comprise the amino acid sequences:

i) GYTNSIYT (SEQ ID NO: 1) as variable heavy (VHH) chain complementarity determining region (CDR)1,
ii) I$X_a$SRNGNT (SEQ ID NO: 2) as variable heavy (VHH) chain complementarity determining region (CDR)2, wherein $X_a$ is G or A
iii) AAGSSWDLILQAYAYDY (SEQ ID NO: 3) as variable heavy (VHH) chain complementarity determining region (CDR)3.

One skilled in the art taking into consideration his technical knowledge and the amino acid sequence - nucleic acid translation code would be able to determine the corresponding nucleic acid coding sequence.

In the present invention, a nucleotide sequence coding the peptide sequence of the variable heavy (VHH) chain complementarity determining region (CDR)1 of the isolated single-domain antibody directed against Tenascin-C (TNC) may be of sequence 5'-GGATACACCAACAGTATCTAC-3' (SEQ ID NO: 14).

In the present invention, a nucleotide sequence coding the peptide sequence of the variable heavy (VHH) chain complementarity determining region (CDR)2 of the isolated single-domain antibody directed against Tenascin-C (TNC) may be of sequence 5'-ATTGGTAGTCGTAATGGTAAC-3' (SEQ ID NO: 15) or of sequence 5'-ATTGCTAGTCGTAATGGTAAC-3' (SEQ ID NO: 16).

In the present invention, a nucleotide sequence coding the peptide sequence of the variable heavy (VHH) chain complementarity determining region (CDR)3 of the isolated single-domain antibody directed against Tenascin-C (TNC) may be of sequence 5'-GCGGCAGGATCCTCTTGGGACCTCATCTTAC-3' (SEQ ID NO: 17).

In the present invention, a nucleotide sequence coding the peptide sequence of an isolated single-domain antibody directed against Tenascin-C (TNC) according to the invention may be selected from the group comprising

ATGGCTGCATGGTTGCAGCTGGTGGAGTCTGGGGGAGGCTCGGTGC
AGACTGGAGGGTCTCTGAGACTCTCCTGTGTAGTCTCTGGATACACC
AACAGTATCTACACGCTGGCCTGGTTCCGCCAGGCTCCAGGGAAGGA
GCGTGAGGGGGTAGCGGCTATTGGTAGTCGTAATGGTAACACATACT
ACGACGCCTCCGTGAAGGACCGATTCACCATCTCCCTAGACAAGGCC
AAGAACACGGTGTATCTGCAAATGAACGACCTGAAACCTGAGGACAC
GGCCAGCTATTACTGTGCGGCAGGATCCTCTTGGGACCTCATCTTAC
AGGCATATGCGTATGACTACTGGGGCCAGGGGACCCAGGTCACCGT
CTCCTCAGCGGCCGCATACCCGTACGACGTTCCGGACTACGGTTCCC
ACCACCATCACCATCACTAGACTGTTGAAAGTTGTTTAGCAAAACCTC
ATACAGAAAATTCATTTACTAACGTCTGGAAAGACGACAAAACTTTAGA
TCGTTACGCTAACTATGAGGGTTGTCTGTGGAATGC (SEQ ID NO: 6)

and

ATGGCTGCATGGGTGCAGCTGGTGGAGTCTGGGGGAGGCTCGGTGC

AGACTGGAGGGTCTCTGAGACTCTCCTGTGTAGTCTCTGGATACACC
AACAGTATCTACAWGCTGGCCTGGTTCCGCCAGGCTCCAGGGAAGG
AGCGTGAGGGGGTAGCGGCTATTGCTAGTCGTAATGGTAACACATAC
TACGACGCCTCCGTGAAGGACCGATTCACCATCTCCCTAGACAAGGC
CAAGAACACGGTGTATCTGCAAATGAACGGCCTGAAACCTGAGGACA
CGGCCAGCTATTACTGTGCGGCAGGATCCTCTTGGGACCTCATCTTA
CAGGCATATGCGTATGACTACTGGGGCCAGGGGACCCAGGTCACCG
TCTCCTCAGCGGCCGCATACCCGTACGACGTTCCGGACTACGGTTCC
CACCACCATCACCATCACTAGACTGTTGAAAGTTGTTTAGCAAAACCT
CATACAGAAAATTCATTTACTAACGTCTGGAAAGACGACAAAACTTTAG
ATCGTTACGCTAACTATGAGGGCTGGTCTGTGGAATGC (SEQ ID NO: 7).

By way of non limiting example, nucleotide sequence coding for peptide sequence SEQ ID NO: 4 of isolated single-domain antibody directed against Tenascin-C of the invention may be the nucleic acid of sequence SEQ ID NO: 6.

By way of non limiting example, nucleotide sequence coding for peptide sequence SEQ ID NO: 5 of isolated single-domain antibody directed against Tenascin-C of the invention may be the nucleic acid of sequence SEQ ID NO: 7.

An object of the present invention is also a recombinant vector, in particular an expression vector, comprising a nucleotide sequence according to the invention.

Another object of the present invention relates to an expression vector comprising the isolated nucleic acid according to the invention or a nucleotide sequence coding an isolated single-domain antibody directed against Tenascin-C.

The present invention relates also to an expression vector comprising an isolated nucleic acid according to the invention or the nucleotide sequence selected from the group comprising SEQ ID NO: 6 and SEQ ID NO: 7.

In the present invention, the vector may be any one of the vectors known to those skilled in the art to produce proteins. It is generally chosen as a function of the cellular host used. The vector may for example be chosen from the vectors listed in the catalog https://france.promega.com/products/vectors/protein-expression-vectors/ [83]. It may be, for example, the expression vector described in document WO 83/004261 [84]. The vector may for example be selected from group comprising pET-21, pcDNA3.1 expression vector, FJB IgG expression vectors and pHEN2 and derivatives phagemid vectors.

The vector may be, for example Adeno-associated Virus (AAV) vectors, a plasmid, a Yeast Artificial Chromosomes (YAC), a Bacterial Artificial Chromosome (BAC) or a phagemid vectors.

The vector may be any adapted adeno-associated virus (AAV) vector known to one skilled in the art and/or commercially available adapted. It may be, for example an adeno-associated virus (AAV) vector selected from the group comprising AAV1, AAV2, AAV3, AAV4, AAV5, AAV6, AAV7, AAV8, AAV9 or AAV10 (AAVrh.10).

The vector may be any adapted plasmid known to one skilled in the art and/or commercially available. It may be for example a plasmid selected from the group comprising pMX, pUC19, pHP45-CmR, pcDNA3.1(+), pcDNA3.3-TOPO, pcDNA3.4-TOPO, pFastBac1, pET100/D-TOPO, pET151/D-TOPO, pRSET A, pYes2.1V5-His TOPO, pDONR221, pGEX-3X, pMECS, pET45b(+),pET-28a, pCMV/ER/myc plasmid, pSecTag, pHENIX vector, pGEX6P1, pCDNA 3.1, pCANTAB-5E, pMB1, pUC8 to pUC19.

The vector may be any adapted Yeast Artificial Chromosome known to one skilled in the art. It may be for example a Yeast Artificial Chromosomes selected from the group comprising pYAC-RC, pYAC3.

The vector may be any adapted Bacterial Artificial Chromosome known to one skilled in the art. It may be for example a Bacterial Artificial Chromosome selected from the group comprising pUvBBAC, pCC1BAC, pBAC 108L.

The vector may be any adapted phagemid vectors known to one skilled in the art. It may be for example a phagemid vector selected from the group comprising pHEN2, pBluescript II, pMECS, pHEN4, pHEN6, Phage vector fd-tet Gill, pSEX, preferably pHEN2 phagemid vector.

The vector may be any adapted lentiviral plasmid known to one skilled in the art.

The vector may comprise a polynucleotide sequence, for example an expression cassette, comprising the following in 5' to 3'order:

a promoter sequence;
a nucleic acid sequence encoding a signal peptide,
a nucleic acid sequence encoding an isolated single-domain antibody directed against Tenascin-C (TNC), and, optionally
a nucleic acid sequence encoding a tag protein.

The promoter may be any adapted promoter known to one skilled in the art. It may be for example promoters of housekeeping gene, promoters of viral gene, tissue specific promoter, inducible promoter, a promoter of bacterial origin.

It may be for example any adapted promoters of housekeeping genes any promoter known to one skilled in the art. It may be for example promoters of housekeeping genes for example Sigma 70, Sigma S and Sigma 28.

It may be for example any adapted tissue specific promoter known from one skilled in the art.

It may be for example any adapted promoters of viral genes known from one skilled in the art. It may be for example promoters of viral genes selected from the group comprising CMV promoter, Rous Sarcoma Virus (RSV) promoter, Simian virus (SV) 40 promoter of adenoviral vectors.

It may be for example any adapted promoters of bacterial origin known from one skilled in the art. It may be for example a promoter selected from the group comprising Lac promoter, LacUV5 promoter, tac-promoter, trc-promoter, T7-promoter, pL-promoter, pR-promoter.

The nucleic acid sequence encoding signal peptide can be any adapted sequence encoding signal peptide known to one skilled in the art. It may be for example a nucleic acid sequence encoding pelB signal peptide for single-domain antibody secretion.

The nucleic acid sequence encoding for tag protein may be for example any nucleic acid sequence encoding for tag protein know to one skilled in the art. For example, it may be any tag disclosed in Parkinson J1, Blaxter M. "Expressed sequence tags: an overview."Methods Mol Biol. 2009;533:1-12. doi: 10.1007/978-1-60327-136-3_1. It may be for example any nucleic acid sequence encoding for a tag selected from the group comprising T7, FLAG, hemagglutinin (HA), vesicular stomatitis virus glycoprotein (VSV-G), V5 (the C-terminal sequence of the P and V proteins of simian virus 5), histidine, for example His6 or c-myc.

The vector may comprise an origin of replication sequence. It may be for example any adapted origin of replication sequence known from one skilled in the art. It may be for example an origin of replication sequence of pMB1, ColE1, p15A, pUC.

The vector may comprise a regulatory promoter sequence. It may be for example any adapted regulatory promoter sequence known from one skilled in the art. One skilled in the art taking into consideration its technical knowledge and taking into consideration the promoter would select/adapt regulatory promoter sequence.

The vector may comprise a Polylinker (Multiple Cloning Sites) sequence. It may be for example any adapted Polylinker known from one skilled in the art. One skilled in the art taking into consideration its technical knowledge would select/adapt the Polylinker (Multiple Cloning Sites) sequence.

The vector may comprise a Ribosome Binding Site sequence (RBS). It may be for example any adapted Ribosome Binding Site sequence (RBS) known from one skilled in the art. One skilled in the art taking into consideration its technical knowledge would select/adapt the Ribosome Binding Site sequence.

Another object of the present invention relates to a host cell comprising a nucleic acid coding an isolated single-domain antibody directed against Tenascin-C (TNC) of the invention or an expression vector comprising a nucleic acid coding an isolated single-domain antibody directed against Tenascin-C (TNC) of the present invention.

The nucleic acid or expression vector are as defined above.

The host cell may be any suitable host cell for the production of a single-domain antibody directed against Tenascin-C (TNC) of the present invention from the aforementioned nucleic acid or expression vector comprising a nucleic acid encoding a single-domain antibody directed against Tenascin-C (TNC) according to the invention.

In the present invention, "host cell" is understood to mean a prokaryotic or eukaryotic cell. Host cells commonly used for expression of recombinant proteins include cells of bacteria such as Escherichia coli or Bacillus sp., Yeast cells such as Saccharomyces cerevisiae, fungal cells such as Aspergillus Niger, insect cells, and/or mammalian cells such as Hamster and CHO cells. It may be for example *E. coli, Pischia pastoris, Saccharomyces cerevisiae,* or insect cells, for example an insect cell-baculovirus system, for example SF9 insect cells used in a baculovirus expression system. The mammalian cells may be for example selected from the group comprising murine cells, human cells. It may be for example cells selected from the group comprising HEK 293, PER-C6, CHO cells, CAR - T cells, CAR-NK cells. In the present invention, the host cell may be a CHO cell.

The transformation of prokaryotic and eukaryotic cells is a process/technique well known to a person skilled in the art. The transformation may be carried out, for example by lipofection, Electroporation, heat shock, or chemical methods. Depending on the cell to be transformed, a person skilled in the art can easily determine the means necessary for the transformation of the selected host cell. Thus, the expression vector and the method of introducing the expression vector into the host cell will be selected in accordance with the selected host cell. The host cell transformed by an expression vector will produce a corresponding protein, for example in recombinant form. A person skilled in the art can readily verify that the host cell produces the protein, for example recombinant, for example using immunoprecipitation followed by the Western blotting technique and/or ELISA technique.

For example a method for producing a host cell expressing a single-domain antibody directed against Tenascin-C (TNC) of the present invention can comprise the steps of :

(i) introducing, *in vitro* or *ex vivo,* a nucleic acid and/or an expression vector of the invention into a host cell,
(ii) culturing, in vitro or ex vivo, the recombinant host cell obtained, and
(iii) optionally selecting the cells which express and/or secrete said single-domain antibody directed against Tenascin-C (TNC)

The introduction of nucleic acid and/or an expression vector of the invention into a host cell may be carried out by any method known to one skilled in the art and adapted to the host cell. It may be for example a transformation process/technique as disclosed above.

The culture of prokaryotic and eukaryotic cells is a technique well known to those skilled in the art. Depending on the cell, a person skilled in the art may easily determine the necessary means, culture medium, induction, time and temperature conditions required for the culture of the selected host cell.

The selection of cells which express and/or secrete protein, for example single-domain antibody, directed against Tenascin-C (TNC) can be carried out by any method known to one skilled in the art and adapted to the host cell. It may be for example using an ELISA of periplasm extracts or on selective medium for example through selection marker of resistance.

An object of the present invention is also a method of producing a single-domain antibody directed against Tenascin-C (TNC) of the present invention.

In the present invention, the method of producing an isolated single-domain antibody directed against Tenascin-C (TNC) of the present invention may be carried out by any method known to one skilled in the art to produce an isolated single-domain antibody and/or nanobody. It may be, for example procedure by chemical synthesis according to solid phase synthesis method (Merrifield (19962) Proc. Soc. e.g. Boil.21:412; Merrifield (1963) J. Am. Chem. Soc. 85:2149; Tarn et al (1983) J. Am. Chem.Soc. 105:6442 [85]).

A method of producing a single-domain antibody directed against Tenascin-C (TNC) of the present invention may comprise culturing a host cell according to the invention and recovering a single-domain antibody directed against Tenascin-C (TNC) of the present invention from the cell culture.

The culture of a host cell may be carried out by any method known to one skilled in the art and adapted to the cell. Culture of prokaryotic and eukaryotic cells is a technique well known to those skilled in the art. Depending on the cell, a person skilled in the art may easily determine the necessary means, culture medium, induction, time and temperature conditions required for the culture of the selected host cell.

The recovery of a single-domain antibody, an antibody or antigen-binding portion thereof from the cell culture may be carried out by any method known to one skilled in the art. It may, for example, be a method selected among electrophoresis, ultracentrifugation, differential precipitation, ultrafiltration, membrane or gel filtration, affinity chromatography.

In the present invention, the isolated single-domain antibody directed against Tenascin-C (TNC) of the invention may be also produced by recombinant DNA methods, for example as disclosed in U.S. Pat. No. 4,816,567.

The isolated single-domain antibody directed against Tenascin-C (TNC) of the invention may also be produced and isolated from phage nanobody libraries using the techniques disclosed in Hmila I et al. FASEB J (2010) 24:3479-3489 [32], Vincke C et al. Antibody Engineering, ed. P. Chames (Totowa, NJ: Humana Press), 145-176 [42], Abderrazek RB, et al.. Biochem J (2009) 424:263-272 [43].

For example, of making single-domain antibody (nanobody) libraries generally by immunization of a camelid (e.g. camel, dromedary, alpaca, vicuna, llama, etc.) with the material to be targeted for selection of specific single-domain antibody (nanobody). For example, a dromedary may be immunized against, for example Tenascin-C. For example, a dromedary may be immunized with human Tenascin-C to generate dromedary lymphocyte-producing immunoglobulins specific for Tenascin-C. Lymphocytes can then be isolated from blood samples, and extracted lymphocyte RNA can then be used to construct phage display - based nanobody libraries, for example using a pHEN4 or pMECS phagemid vector. The nucleic acid from which the VHH domain sequences derive may be total RNA or more specifically mRNA isolated from the cells within the sample taken from the host. The expression vectors may be any suitable vectors used for library construction, and are preferably phage or phagemid vectors allowing selection of target - specific antibody fragments using phage - display - based selection methods. The recovery of a single-domain antibody, may be carried out by any method known to one skilled in the art. It may, for example, be a method selected among electrophoresis, ultracentrifugation, differential precipitation, ultrafiltration, membrane or gel filtration, affinity chromatography.

Another object of the present invention is an immunoconjugate comprising:

(a) an isolated single-domain antibody directed against Tenascin-C (TNC), and
(b) a conjugating part selected from the group consisting of a detectable marker, drug, toxin, cytokine, radionuclide, enzyme, gold nanoparticle/nanorod, albumin nanoparticle magnetic nanoparticle, transductor, PolyEthylenGlycol (PEG)-Liposome and a combination thereof.

The isolated single-domain antibody directed against Tenascin-C (TNC) is as defined above.

In the present invention, "conjugated" means two entities stably bound to one another by any physiochemical means. It is important that the nature of the attachment is such that it does not impair substantially the effectiveness of either entity. Keeping these parameters in mind, any covalent or non - covalent linkage known to those of ordinary skill in the art may be employed. In some embodiments, covalent linkage is preferred. Noncovalent conjugation includes hydrophobic interactions, ionic interactions, high affinity interactions such as biotin - avidin and biotin - streptavidin complexation and other affinity interactions. Such means and methods of attachment are well known to those of ordinary skill in the art.

In the present invention, the conjugating part may be conjugated to the N-terminus or the C-terminus or internal amino acids of the single-domain antibody directed against Tenascin-C (TNC) (nanobody). For example, the single-domain antibody directed against Tenascin-C (TNC) (nanobody) may be directly conjugated to the conjugating part. For example it may be directly conjugated to conjugating part if the conjugating part is linked directly, for example via a peptide bond to an amino acid, for example the N - terminal amino acid, C - terminal amino acid, or internal amino acid of the single-domain antibody directed against Tenascin-C (TNC). For example, alternatively, the single-domain antibody directed against Tenascin-C (TNC) may be indirectly conjugated to a conjugating part for example when a linker is used to connect the conjugating part to the single-domain antibody directed against Tenascin-C (TNC) components may be linked indirectly to one another by linkage to a common carrier molecule.

In the present invention, linker molecules also designed as "linkers" may optionally be used to link the single-domain antibody directed against Tenascin-C (TNC) to another molecule, for example to conjugating part. In the present invention, the linker may be any linker known to one skilled in the art adapted to link a single-domain antibody (nanobody) to a conjugating part selected from the group comprising a detectable marker, drug, toxin, cytokine, radionuclide, enzyme, gold nanoparticle/nanorod, albumin nanoparticle magnetic nanoparticle, transductor, PolyEthylenGlycol (PEG)-Liposome. For example, the linker may be for example a multiple amino acids, or non - peptide molecules. For example, it may be a peptide linker, for example a glycine - rich peptide linkers as disclosed in US patent N° US 5,908,626, for example it may be a glycine - rich peptide with an amino-acid length about 20 or less. For example, the linker may be a non - peptide or partial peptide linker. It may be for example any non - peptide or partial peptide linker known from one skilled in the art. It may be for example a cross - linking molecules, for example glutaraldehyde or EDC (Pierce,Rockford,111). It may be for example a bifunctional cross - linking linker comprising two distinct reactive sites, one of the reactive sites for example may be reacted with a functional group on a peptide, for example the single-domain antibody directed against Tenascin-C (TNC) of the invention, to form a covalent linkage and the other reactive site may be reacted with a functional group on another molecule, for example the conjugating part, to form a covalent linkage. It may be for example a linker selected from the group comprising glutaraldehyde ; N,N-bis(3-maleimido-propionyl-2-hydroxy-1,3-propanediol, discuccinimyidyl suberate , dithiobis (succinimidyl propionate), soluble bis-sulfonic acid, m-maleimidobenzoyl-N-hydroxysuccinimide ester ; m-maleimido-benzoylsulfosuccinimide ester ; y-maleimidobutyric acid N-hydroxysuccinimide ester ; and N-succinimidyl3-(2-pyridyl-dithio) propionate and any salt thereof.

In the present invention, the detectable marker may be any detectable marker known to one skilled in the art adapted to be directly or indirectly conjugated to the single-domain antibody directed against Tenascin-C of the invention. It may be for example fluorescent or luminescent markers; for example, fluorochromes, radioactive markers, MRI (magnetic resonance imaging) or CT (computed tomography) contrast agents, or enzymes capable of producing detectable products.

In the present invention, the drug that may be conjugated directly or indirectly to the single-domain antibody directed against Tenascin-C of the invention may be any adapted drug known from one skilled in the art. It may be for example an anticancer drug, anti-inflammatory drug, anti-fibrotic drug, an anti-infection drug, anti-oxydative drug.

In the present invention, the anticancer drug that may be conjugated directly or indirectly to the single-domain antibody directed against Tenascin-C of the invention may be preferably a cytotoxic drug. As used herein, the term "cytotoxic drug" refers to a molecule that when entering in contact with a cell, optionally upon internalization into the cell, alters a cell function (e.g. cell growth and/or proliferation and/or differentiation and/or metabolism such as protein and/or DNA synthesis) in a detrimental way or leads to cell death. As used herein, the term "cytotoxic drug" encompasses toxins, in particular cytotoxins. It may be for example cytotoxic drug selected from the group consisting of an antitubulin drug, DNA sulcus binding reagent, DNA replication inhibitor, alkylation reagent, antibiotic, folic acid antagonist, antimetabolic drug, chemosensitizer, topoisomerase inhibitor, Catharanthus roseus alkaloid and a combination thereof. It may be, for example, a compound selected from the group comprising calicheamycin, dolastin 10, dolastin 15, auristatin E, auristatin EB (AEB), auristatin EFP (AEFP), monomethyl auristatin F (MMAF), monomethylauristatin-D (MMAD), monomethyl auristatin E (MMAE), and 5-benzoylvaleric acid-AE ester (AEVB) and duocarmycin; nitrogen mustard analogues for example cyclophosphamide, melphalan, ifosfamide or trofosfamide; ethylenimines such as thiotepa; nitrosoureas for example carmustine; alkylating agents for example temozolomide or dacarbazine; folate-like metabolic antagonists such as methotrexate or raltitrexed; purine analogues for example thioguanine, cladribine or fludarabine; pyrimidine analogues for example fluorouracil, tegafur or gemcitabine; vinca alkaloids for example vinblastine, vincristine or vinorelbine and analogues thereof; podophyllotoxin derivatives for example etoposide, taxans, docetaxel or paclitaxel; anthracyclines for example doxorubicin, epirubicin, idarubicin and mitoxantrone, and analogues thereof; other cytotoxic antibiotics for example bleomycin and mitomycin; platinum compounds for example cisplatin, carboplatin and oxaliplatin; pentostatin, miltefosine, estramustine, topotecan, irinotecan and bicalutamide, and toxins for example ricin toxin, liatoxin and Vero toxin.

Advantageously, when the single-domain antibody directed against Tenascin-C is conjugated with an anticancer drug, it may advantageously have an increase therapeutic effect when use for the treatment of cancer.

In the present invention, the anti-inflammatory drug that may be conjugated directly or indirectly to the single-domain antibody directed against Tenascin-C of the invention may be for example any an anti-inflammatory drug known from one skilled in the art. It may be for example nonsteroidal anti-inflammatory drug. It may be for example anti-inflammatory drug selected from the group comprising aspirin, ibuprofen, naproxen ibuprofen, diclofenac, celecoxib, mefenamic acid, etoricoxib, indomethacin.

Advantageously, when the single-domain antibody directed against Tenascin-C is conjugated with an anti-inflammatory drug, it may advantageously have an increase therapeutic effect when use for the treatment of inflammation.

In the present invention, the anti-fibrotic drug that may be conjugated directly or indirectly to the single-domain antibody directed against Tenascin-C of the invention may be any anti-fibrotic drug known from one skilled in the art. It may be for example an anti-fibrotic drug selected from the group comprising pirfenidone, Nintedanib, prednisolone, bethametasone and dexamethasone.

In the present, the anti-infection drug that may be conjugated directly or indirectly to the single-domain antibody directed against Tenascin-C of the invention may be any anti-fibrotic drug known from one skilled in the art. It may be for example any adapted antibiotic know from one skilled in the art. It may be for example any adapted anti-viral drug know from one skilled in the art. It may be for example an anti-viral nanobody. It may be for example an anti-viral selected from the group comprising nanobody anti-Hepatitis-C virus (HCV), for example as disclosed in Surasak Jittavisutthikul et al, 2015, "Humanized-VHH transbodies that inhibit HCV protease and replication". 2015 Apr 20;7(4):2030-56, hepatitis B virus (HBV) nanobodies, foot-and mouth disease (FMD) virus-specific nanobodies [86], for example as disclosed in Harmsen, 2007 [87]), influenza (H5N1) virus Nanobodies, for example as disclosed in Hultberg et al. ,2011 [88]), respiratory Syncytial virus (RSV) Nanobodies for example as disclosed in Schepens et al.,2011. "Nanobodies specific for respiratory Syncytial virus fusion protein protect against infection by inhibition of fusion"2011 J Infect Dis. 2011 Dec 1;204 (11):1692-701. doi: 10.1093/infdis/jir622. PMID: 21998474 [89], Rabies virus Nanobodies, for example as disclosed in Hultberg et al, 2011, Poliovirus Nanobodies, for example as disclosed in Thys et al, 2010 [90], Rotavirus Nanobodies, for example as disclosed in Van der Vaart et al, 2006 [91], human Immunodeficiency virus (HIV) Nanobodies: HIV gp120 Nanobodies, for example as disclosed in Sebastian et al., 2012. Rotavirus A-specific single-domain antibodies produced in baculovirus-infected insect larvae are protective in vivo. BMC Biotech 2012;12:59. doi: 10.1186/1472-6750-12-59. PMCID: PMC3444942 PMID: 22953695 [92], HIV Rev Nanobodies, for example as disclosed in Verccruysse et al., 2010 [93], HIV Nef Nanobodies, for example as disclosed in Bouchet et al, 2011 [94], CXCR4 Nanobodies, for example as disclosed in Jahnichen et al, 2010 "CXCR4 nanobodies (VHH-based single variable domains) potently inhibit chemotaxis and HIV-1 replication and mobilize stem cells" Proceedings of the National Academy of Sciences Nov 2010, 107 (47) 20565-20570; DOI: 10.1073/pnas.1012865107 [95], Newcastle disease Virus Nanobody, for example, as disclosed in Sheng et al.,2019. "Nanobody-horseradish peroxidase fusion protein as an ultrasensitive probe to detect antibodies against Newcastle disease virus in the immunoassay" NanoBiotechnol 2019 17:35. doi.org/10.1186/s12951-019-0468-0 [97].

Advantageously, when the single-domain antibody directed against Tenascin-C is conjugated with an anti-infection drug, it may advantageously have an increase therapeutic effect when use for the treatment of infection.

In the present invention, the anti-oxydative drug that may be conjugated directly or indirectly to the single-domain antibody directed against Tenascin-C of the invention may be any anti-oxydative drug known from one skilled in the art. It may be for example an anti-oxydative drug selected from the group comprising ascorbic acid, tocopherols, tocotrienols; β-carotene, Ebselen, Edaravone, Superoxide dismutase, Glutathione, N-acetylcysteine and Ascorbic acid..

In the present invention, the toxin that may be conjugated directly or indirectly to the single-domain antibody directed against Tenascin-C of the invention may be for example any toxin known from one skilled in the art. It may be for example a toxin is selected from the group consisting of Auristatins (for example, Auristatin A, Auristatin F, MMAE and MMAF), chlortetracycline, metotanol, ricin, ricin A chain, cobustatin, docamicin, Dora statin, adriamycin, daunorubicin, paclitaxel, cisplatin, cc1065, ethidium bromide, mitomycin, etoposide, tenoposide, vincristine, vinblastine, colchicine, dihydroxyanthracnose diketone, actinomycin, diphtheria toxin, Pseudomonas exotoxin (PE) A, PE40, abrin, abrin A chain, modeccin A chain, α-Sarcina, gelonin, mitogellin, retstrictocin, phenomycin, enomycin, curicin, crotin, calicheamicins, Sapaonaria officinalis inhibitor, glucocorticoid and a combination thereof.

In the present invention, the toxin cytokine that may be conjugated directly or indirectly to the single-domain antibody directed against Tenascin-C of the invention may be any cytokine which have a therapeutic effect, for example in inflammation and/or that could enhance an immune response. It may be for example a cytokine selected from the group comprising IL-2 , IL-6 , IL-8 , IL-10 , IL-12 , IL-18 , TNF ,IFN-γ , IFN-β , chemokines , and IFN-α.

In the present invention, the radionuclide that may be conjugated directly or indirectly to the single-domain antibody directed against Tenascin-C of the invention may be any radionuclide known from one skilled in the art. It may be for example a diagnostics radioisotope selected from the group consisting of Tc-99m, Ga-68, F-18, I-123, I-125, I-131, In-111, Ga-67, Cu-64, Zr-89, C-11, Lu-177, Re-188, and a combination thereof; and / or a therapeutic radioisotope selected from the group consisting of Lu-177, Y-90, Ac-225, As-211, Bi-212, Bi-213, Cs-137, Cr-51, Co-60, Dy-165, Er-169, Fm-255, Au-198, Ho-166, 1-125,1-131, Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133 Yb-169, Yb-177, and a combination thereof.

Advantageously, when the single-domain antibody directed against Tenascin-C is conjugated with a radionuclide, it may be used in diagnostic method and/or as a marker in diagnostic method.

In the present invention, enzyme that may be conjugated directly or indirectly to the single-domain antibody directed against Tenascin-C of the invention may be any enzyme known from one skilled in the art. It may be for example an enzyme selected from the group comprising beta-lactamase, Alkaline Phosphatase (AP) and/or Horse Radish Peroxydase.

Advantageously, when the single-domain antibody directed against Tenascin-C is conjugated with an enzyme, it may be used in diagnostic method and/or as a marker in diagnostic method. For example when the single-domain antibody directed against Tenascin-C of the invention is conjugated to Alkaline Phosphatase (AP) or to Horse Radish Peroxydase, it may be used for example for Enzyme-Linked Immunosorbent Assay one step Detection or sandwich ELISA or immunostaining approaches for example immunolabeling, immunohistochemistry.

In the present invention, gold nanoparticle/nanorod that may be conjugated directly or indirectly to the single-domain antibody directed against Tenascin-C of the invention may be any adapted gold nanoparticle/nanorod known from one skilled in the art. It may be for example commercially available gold nanoparticle/nanorod, for example commercialized under the reference 741949 by Sigma Aldrich. It may be for example colloidal goldparticles having a diameter from 4 to 6nm, for example 5 nm diameter colloidal gold particles or palladium nanoparticle

Advantageously, when the single-domain antibody directed against Tenascin-C is conjugated with a gold nanoparticle/nanorod, it may be used in diagnostic method and/or as a marker in diagnostic method and/or for drug delivery.

In the present invention albumin nanoparticle that may be conjugated directly or indirectly to the single-domain antibody directed against Tenascin-C of the invention may be any adapted albumin-based nanoparticle known from one skilled in the art. It may be for example albumin-based nanoparticle as disclosed in Fei Fei et al. 2017 [97].

In the present invention, magnetic nanoparticle that may be conjugated directly or indirectly to the single-domain antibody directed against Tenascin-C of the invention may be any adapted magnetic nanoparticle known from one skilled in the art. It may be for example commercially available magnetic nanoparticle.

Advantageously, when the single-domain antibody directed against Tenascin-C is conjugated with a magnetic nanoparticle, it may be used in diagnostic method and/or as a marker in diagnostic method and/or for drug-delivery.

In the present invention, the transductor may be any detectable transductor known to one skilled in the art adapted to be directly or indirectly conjugated to the single-domain antibody directed against Tenascin-C of the invention. It may be for example any transductor known to one skilled in the art adapted used in biosensor and adapted to be directly or indirectly conjugated to the single-domain antibody directed against Tenascin-C of the invention. It may be for example an electrode, a SpyTag/SpyCatcher isopeptide ligation system, self assembled monolayer (SAM)-modified transducer surfaces, quartz cristal sensor surface, QCM-based immunosensor, 11-mercaptoundecanoic acid (MUA) sensor surface

In the present invention PolyEthylenGlycol (PEG)-Liposome that may be conjugated directly or indirectly to the single-domain antibody directed against Tenascin-C of the invention may be any PolyEthylenGlycol (PEG)-Liposome known from one skilled in the art. It may be for example PolyEthylenGlycol (PEG)-Liposome as disclosed in Oliveira et al, 2010. [98]

Advantageously, when the single-domain antibody directed against Tenascin-C is conjugated with PolyEthylenGlycol (PEG)-Liposome, it may be used in diagnostic method and/or as a marker in diagnostic method and/or for drug-delivery.

Another object of the present invention is a pharmaceutical composition comprising an isolated single-domain antibody directed against Tenascin-C of the invention and/or immunoconjugate of the invention and a pharmaceutically acceptable carrier.

The isolated single-domain antibody directed against Tenascin-C is as defined above.

The immunoconjugate is as defined above.

The pharmaceutical composition may be in any form that can be administered to a human or an animal. The person skilled in the art clearly understands that the term "form" as used herein refers to the pharmaceutical formulation of the medicament for its practical use. For example, the medicament may be in a form selected from the group comprising an injectable form, an oral suspension, a pellet, a powder, granules or topical form (e.g. cream, lotion, collyrium). For example, pharmaceutical composition may be a pharmaceutical composition for oral administration selected from the group comprising a liquid formulation, an oral effervescent dosage form, an oral powder, a multiparticule system, an orodispersible dosage form. For example, when the pharmaceutical composition is for oral administration, it may be in the form of a liquid formulation selected from the group comprising a solution, a syrup, a suspension, an emulsion and oral drops. When the pharmaceutical composition is in the form of an oral effervescent dosage form, it may be in a form selected from the group comprising tablets, granules, and powders. When the pharmaceutical composition is the form of an oral powder or a multiparticulate system, it may be in a form selected from the group comprising beads, granules, mini tablets and micro granules. When the pharmaceutical composition is the form of an orodispersible dosage form, it may be in a form selected from the group comprising orodispersible tablets, lyophilized wafers, thin films, a chewable tablet, a tablet and a capsule, a medical chewing gum. According to the present invention, the pharmaceutical composition may be for buccal and sublingual routes, for example selected from the group comprising buccal or sublingual tablets, muco adhesive preparation, lozenges, oro-mucosal drops and sprays. According to the present invention, the pharmaceutical composition may be for topical-transdermal administration, for example selected from the group comprising ointments, cream, gel, lotion, patch and foam.

The pharmaceutically acceptable carrier may be any known pharmaceutical support used for the administration of an antibody, nanobody and/or single-domain antibody to a human or animal, depending on the subject to be treated. For example, pharmaceutically acceptable carrier, diluent or excipient includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent or emulsifier

The pharmaceutical form or method of administering a pharmaceutical composition may be selected with regard to the human or animal subject to be treated. For example, for a child, for example from 1 to 17 years old, or a baby, for example under one year old, a syrup or an injection is preferred. Administration may for example be carried out with a weight graduated pipette, a syringe. For example, for an adult over 17 years old, an injection may be preferred. Administration may be carried out with an intravenous weight graduated syringe.

According to the present invention, the pharmaceutical composition may comprise any pharmaceutically acceptable and effective amount of isolated single-domain antibody directed against Tenascin-C of the invention and/or of immunoconjugate of the invention.

For example, in the case of cancer, the therapeutically effective amount of the single-domain antibody directed against Tenascin-C may reduce the number of cancer cells; reduce the tumor size; inhibit (i.e. slow to some extent and preferably stop) cancer cell infiltration into peripheral organs; inhibit (i.e. slow to some extent and preferably stop) tumor metastasis; inhibit, to some extent, tumor growth; and/or relieve to some extent one or more of the symptoms associated with the cancer.

For example, in the case of fibrosis, the therapeutically effective amount of the single-domain antibody directed against Tenascin-C may reduce inflammation and/or allow the restoration of the tissue function. For example, in case of lung fibrosis the therapeutically effective amount of the single-domain antibody directed against Tenascin-C may reduce inflammation and restoration of function of the lung, for example breathing. For example, in case of kidney fibrosis the therapeutically effective amount of the single-domain antibody directed against Tenascin-C may improve the secretion of urine and/or reduce proteinuria and/or reduce hematuria.

For example, in the case of inflammation, the therapeutically effective amount of the single-domain antibody directed against Tenascin-C may reduce the inflammation and/or pain and/or fever and/or rash due to inflammation.

For example, in the case of viral or bacterial infections, the therapeutically effective amount of the single-domain antibody directed against Tenascin-C may reduce the number of viral or bacterial infected cells. It is known in the art that Tenascin-C plasma levels are increased in critically ill patients suffering from infection and sepsis, Sepsis patients represent 10% of all admissions to the intensive care unit (ICU) with a 20-30% mortality rate, for example bacterial pneumonia e.g. by the gram-negative bacterium Klebsiella (K.) pneumoniae is a common cause of sepsis. For example in the case of infection and/or sepsis, the therapeutically effective amount of the single-domain antibody directed against Tenascin-C may reduce the percentage of mortality and/or reduce the inflammation and/or pain and/or fever due to the viral or bacterial infections.

As previously mentioned, the inventors have also surprisingly demonstrated and is the first to demonstrate that isolated single-domain antibodies directed against Tenascin-C (TNC) according to the invention inhibit and/or reduce metastasis progression, for example by restoring the adhesion of cells in tumorous and/or cancerous tissue, advantageously tumorous and/or cancerous and/or mesangial cells to fibronectin and TNC decreasing and/or avoiding therefor the formation of metastasis.

As previously mentioned, the inventors have demonstrated that single-domain antibodies directed against Tenascin-C (TNC) according to the invention allow advantageously to inhibit the immune-suppressive functions of TNC, for example in tumorous and/or cancerous tissues, and/or to increase the immune response.

The inventors also demonstrated that single-domain antibodies directed against Tenascin-C (TNC) according to the invention allow to reduce and/or inhibit and/or block the interaction of TNC with other molecules, for example molecule of the Extra Cellular Matrix, allowing therefor to reduce the matrix networks. In particular, the single-domain antibodies directed against Tenascin-C (TNC) according to the invention allow to reduce and/or inhibit and/or block the enhancement of matrix networks due to TNC which is a characteristic of fibrosis. The single-domain antibodies directed against Tenascin-C (TNC) according to the invention may also reduce and/or inhibit and/or block TNC-induced cellular signaling, in particular TGFb signaling pathway that would lead to matrix production such as laminins, collagens, fibronectin and TNC itself. Accordingly, single-domain antibodies directed against Tenascin-C (TNC) according to the invention allow to treat fibrosis.

The inventors have demonstrated that single-domain antibodies directed against Tenascin-C (TNC) according to the invention allow to reduce and/or inhibit and/or block chemoretention of dendritic cells to Tenascin-C (TNC), for example on a TNC/CCL21 substratum. Accordingly, single-domain antibodies directed against Tenascin-C (TNC) according to the invention allow the release of dendritic from Tenascin-C (TNC) and/or other antigen presenting cells allowing and thus regulate immunity, for example during an inflammation. Accordingly, single-domain antibodies directed against Tenascin-C (TNC) according to the invention allow to treat inflammation, for example by regulating the immunity response.

Accordingly, another object of the present invention is a single-domain antibody directed against Tenascin-C of the invention for use as medicament.

The single-domain antibody directed against Tenascin-C is as defined above.

An object of the present invention is also an immunoconjugate for use as a medicament.

The immunoconjugate is as defined above.

According to the present invention, the medicament may be a medicament for treating disease in which TNC may be involved. For example, the medicament of the present invention may be a medicament for treating cancer, fibrosis, inflammation, viral or bacterial infections.

Accordingly another object of the present invention is a single-domain antibody directed against Tenascin-C of the invention and/or an immunoconjugate of the invention for use in the treatment of cancer, fibrosis, inflammation, viral or bacterial infections

In the present invention, cancer may be for example any cancer of any organ known from one skilled in the art. It may be for example any disease involving abnormal cell growth with the potential to invade or spread to other parts of the body. It may be for example cancer of any organ or tissue of a human or of an animal. It may be for example a cancer selected from the group comprising lung, liver, eye, heart, lung, breast, bone, bone marrow, brain, head & neck, esophagus, trachea, stomach, colon, pancreas, cervical, uterine, bladder, prostate, testicules, skin, rectum, and lymphomas.

In the present invention, fibrosis may be for example any fibrosis of any organ known from one skilled in the art. It may be fibrosis occurring in lung, liver, kidney, skin, knee, shoulder, intestine, bone marrow, brain.

In the present invention, inflammation may be any inflammation and/or inflammatory disease known to one skilled in the art. It may be for example inflammation and/or inflammatory disease of any organ or tissue of a human or of an animal. It may be for example an inflammation and/or inflammatory disease of an organ selected from the group comprising lung, liver, eye, heart, lung, breast, bone, bone marrow, brain, head & neck, esophageal, tracheal, stomach, colon, pancreas, cervical, uterine, bladder, prostate, testicular, skin, rectal, and lymphomas. It may be an inflammatory disease selected from the group comprising chronic liver inflammation, chronic kidney inflammation, chronic lung inflammation, chronic heart inflammation, heart inflammation, liver inflammation, kidney inflammation, lung fibrosis, rheumatoid arthritis, osteoarthritis, lupus, skin inflammation.

In the present invention, infection may be any infection known to one skilled in the art. It may be for example a bacterial or a viral infection. It may be for example a bacterial infection of an organ selected from the group comprising skin, lung, liver, eye, heart, lung, breast, bone, bone marrow, brain, head & neck, esophageal, tracheal, stomach, colon, pancreas, cervical, uterine, bladder, prostate, testicular, skin, rectal, and lymphomas. It may be for example a gram-negative bacteria infection or gram-positive bacteria infection. It may be for example a bacterial pneumonia, for example a bacterial pneumonia by gram-negative bacteria, for example *Klebsiella pneumoniae.*

It may be for example a viral infection of an organ selected from the group comprising skin, lung, liver, eye, heart, lung, breast, bone, bone marrow, brain, head & neck, esophageal, tracheal, stomach, colon, pancreas, cervical, uterine, bladder, prostate, testicular, skin, rectal, and lymphomas.. It may be for example a viral infection selected from the group comprising respiratory tract, for example SARS-Cov-2 virus infection. It may be for example a viral infection selected from the group comprising bone marrow, for example HIV virus infection.

In the present invention, the terms "treating", "treat" and "treatment" include (i) preventing a disease, pathologic or medical condition from occurring (e.g., prophylaxis); (ii) inhibiting the disease, pathologic or medical condition or arresting its development; (iii) relieving the disease, pathologic or medical condition; and/or (iv) diminishing symptoms associated with the disease, pathologic or medical condition. Thus, the terms "treat", "treatment", and "treating" extend to prophylaxis and include prevent, prevention, preventing, lowering, stopping or reversing the progression or severity of the condition or symptoms being treated. As such, the term "treatment" includes medical, therapeutic, and/or prophylactic administration, as appropriate.

For example, in the case of cancer, the treatment may reduce the number of cancer cells; reduce the tumor size; inhibit (i.e. slow to some extent and preferably stop) cancer cell infiltration into peripheral organs; inhibit (i.e. slow to some extent and preferably stop) tumor metastasis; inhibit, to some extent, tumor growth; and/or relieve to some extent one or more of the symptoms associated with the cancer.

In the present invention, the medicament may be in any form that can be administered to a human or an animal. It may for example be a pharmaceutical composition as defined above.

The administration of the medicament may be carried out by any way known to one skilled in the art. It may be for example administered intravenously, intradermally, intraarterially, intralesionally, intratumorally, intracranially, intraarticularly, intraprostaticaly, intrapleurally, intratracheally, intranasally, intravitreally, intravaginally, intrarectally, topically, intratumorally, intramuscularly, intraperitoneally, subcutaneously, subconjunctival, intravesicularlly, mucosally, intrapericardially, intraumbilically, intraocularally, orally, topically, locally, inhalation (e.g., aerosol inhalation), injection. It may, for example, be carried out directly, i.e. pure or substantially pure, or after mixing of the single-domain antibody directed against Tenascin-C with a pharmaceutically acceptable carrier and/or medium. The medicament may be in any form as mentioned above. For example, the medicament may be an injectable solution, a medicament for oral administration, for example selected from the group comprising a liquid formulation, a multiparticle system, an orodispersible dosage form. The medicament may be a medicament for oral administration selected from the group comprising a liquid formulation, an oral effervescent dosage form, an oral powder, a multiparticle system, an orodispersible dosage form. The medicament may be a medicament for buccal administration selected from the group comprising tablets or lozenges formulated in conventional manner. The medicament may be a medicament for inhalation administration may be, for example, delivered in the form of an aerosol spray presentation from pressurized packs or a nebulizer, with for example the use of a suitable propellant, for example dichlorodifluoromethane, trichloro fluoromethane, dichlorotetrafluoroethane, carbon dioxide or other suitable gas. The medicament may be, for example, a medicament for parenteral administration. The medicament for parenteral administration may be in the form of a sterile aqueous or non - aqueous solutions suspensions, and/or emulsions.

The invention also provides methods for the treatment of disease in which TNC may be involved comprising the administration of a therapeutically effective amount of an isolated single-domain antibody directed against Tenascin-C (TNC) according to the invention and/or pharmaceutical composition comprising an isolated single-domain antibody directed against Tenascin-C (TNC) according to the invention to a subject in need thereof. For example, it may be a method of treatment of disease selected from the group comprising cancer, fibrosis, inflammation, viral or bacterial infections.

The effective amount of may be as mentioned above.

The administration of may be carried out by any adapted way known to one skilled in the art as mentioned above

In the present invention, an isolated single-domain antibody directed against Tenascin-C (TNC) of the invention may be used in a combination therapy, for example with a therapeutic agent.

"Combination therapy" (or "co-therapy") includes the administration of a compound binding Matrix Binding Motif (M-motif) on Tenascin-C (TNC) and/or pharmaceutical composition of the invention, and at least a second agent as part of a specific treatment regimen intended to provide the beneficial effect from the co-action of these therapeutic agents. The beneficial effect of the combination includes, but is not limited to, pharmacokinetic or pharmacodynamic co-action resulting from the combination of therapeutic agents. Administration of these therapeutic agents in combination typically is carried out over a defined time period (usually minutes, hours, days or weeks depending upon the combination selected).

"Combination therapy" may, but generally is not, intended to encompass the administration of two or more of these therapeutic agents as part of separate monotherapy regimens that incidentally and arbitrarily result in the combinations of the present invention. "Combination therapy" is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents.

Sequential or substantially simultaneous administration of each therapeutic agent can be effected by any appropriate route including, but not limited to, topical routes, oral routes, intravenous routes, intramuscular routes, and direct absorption through mucous membrane tissues. The therapeutic agents can be administered by the same route or by different routes. For example, a first therapeutic agent of the combination selected may be administered by injection while the other therapeutic agents of the combination may be administered topically.

A "therapeutic agent" is any compound known in the art that is used in the detection, diagnosis, or treatment of a condition or disease. Such compounds may be naturally-occurring, modified, or synthetic. Non-limiting examples of therapeutic agents may include drugs, therapeutic compounds, genetic materials, metals (such as radioactive isotopes), proteins, peptides, carbohydrates, lipids, steroids, nucleic acid based materials, or derivatives, analogues, or combinations thereof in their native form or derivatized with hydrophobic or charged moieties to enhance incorporation or adsorption into a cell. Non-limiting examples of therapeutic agents may include immune-related agents, thyroid agents, respiratory products, antineoplastic agents, anti-helmintics, anti-malarials, mitotic inhibitors, hormones, anti-protozoans, anti-tuberculars, cardiovascular products, blood products, biological response modifiers, anti-fungal agents, vitamins, peptides, anti-allergic agents, anti-coagulation agents, circulatory drugs, metabolic potentiators, anti-virals, anti-anginals, antibiotics, antiinflammatories, anti-rheumatics, narcotics, cardiac glycosides, neuromuscular blockers, sedatives, local anesthetics, general anesthetics, or radioactive atoms or ions. A therapeutic agent may be a toxin, a small therapeutic molecule, a therapeutic nucleic acid, or a chemotherapeutic agent. A chemotherapeutic agent refers to a chemical compound that is useful in the treatment of cancer. The compound may be a cytotoxic agent that affects rapidly dividing cells in general, or it may be a targeted therapeutic agent that affects the deregulated proteins of cancer cells.

In the present invention, the therapeutic agent may be preferably selected from group comprising anticancer drug, anti-inflammatory drug, anti-fibrotic drug, anti-infection drug, anti-oxydant drug.

The anticancer drug may be as mentioned above.

The anti-inflammatory drug may be as mentioned above.

The anti-fibrotic drug may be as mentioned above.

The anti-infection drug may be as mentioned above.

The anti-oxydant drug may be as mentioned above.

The inventor has also surprisingly demonstrated for the first time that isolated single-domain antibody directed against Tenascin-C (TNC) according to the invention target TNC and allow to identify TNC expression in non pathological or pathological biological tissue. For example, single-domain antibody directed against Tenascin-C (TNC) according to the invention target TNC, in particular expressed at the surface of tumorous cells, cancerous cells, and allow for example to detect tumorous cells and/or cancerous cells expressing Tenascin-C.

Thus single-domain antibody directed against Tenascin-C (TNC) according to the invention may be used in immunochemical studies, for example in immunoprecipitation, western blotting, ELISA, immunocytochemical studies, for example confocal microscopy, immunoelectronic microscopy, and/or immunohistochemical studies.

The present invention also provides single-domain antibody directed against Tenascin-C (TNC) of the invention and/or an immunoconjugate of the invention for use in an *in vitro* or *in vivo* diagnostic or imaging method.

In the present invention, the *in vitro* or *in vivo* diagnostic or imaging method may be any method known to one skilled in the art in which a single-domain antibody directed against Tenascin-C (TNC) and/or immunoconjugate could be used. For example *in vivo* diagnostic or imaging method may be selected from the group comprising Single Photon Emission Computed Tomography (SPECT), Positron Emission Tomography (PET), Contrast enhanced ultrasound imaging, and Magnetic Resonance Imaging (MRI) by using for example Mangradex nanoparticles. For example, the single-domain antibody directed against Tenascin-C (TNC) of the invention and/or an immunoconjugate of the invention may be used in method of diagnostic or imaging method, said method comprising a step of administering a single-domain antibody directed against Tenascin-C (TNC) of the invention and/or an immunoconjugate of the invention to said subject and a step of detecting said single-domain antibody directed against Tenascin-C (TNC) of the invention and/or an immunoconjugate of the invention.

Another object of the present invention is the *in vitro* use of a single-domain antibody directed against Tenascin-C (TNC) of the invention and/or an immunoconjugate of the invention, for detecting a tumor cell from a sample.

The single-domain antibody directed against Tenascin-C (TNC) is as defined above.

The immunoconjugate is as defined above.

In the present invention, the sample may be a biological sample. The biological sample may be any biological sample known to one skilled in the art. The biological sample may for example be a liquid or solid sample. According to the invention, the sample may be any biological fluid, for example it can be a sample of blood, plasma, serum, urine, tissue, for example muscle, or a sample from a tissue biopsy.

In the present invention, tumor cell may be any cell from a tumor, preferably any cell of a tumor expressing which express Tenascin-C known to one skilled in the art. It may be for example a tumorous cell, cancerous cells, activated fibroblast, reticular fibroblast, macrophages, dendritic cells, glioblastoma cells, melanoma cells.

In the present invention, the method for detecting tumorous and/or cancerous cells may be any detection method known to one skilled in the art. It may for example be Fluorescence-activated cell sorting (FACS) applied in flow cytometry. It may be any immuno-enzymatic method known to one skilled in the art, for example it may be an ELISA

In the present invention, when the conjugating part of the immunoconjugate is a detectable marker and the immunoconjugate is used in a method for detecting tumorous and/or cancerous cells it may be revealed with a conjugate molecule.

In the present invention, the conjugate molecule may be any molecule which binds to the immunoconjugate comprising a detectable marker known from one in the art. For example, when the detectable marker is biotin the conjugate molecule may be streptavidin.

In the present invention, when the conjugating part of immunoconjugate, for example comprises a detectable marker, a radionuclide, a radioisotopes, an enzyme, gold nanoparticle/nanorod, magnetic nanoparticle and/or fluorochromes, it may be useful in imaging process, for example for detecting/localizing primary tumors and/or metastasis and/or cancer and/or chronic inflammation, healing defects of chronic wounds, acute and chronic bacterial and viral infection, arteriosclerosis, aortic aneurism, maybe even auto immune disease e.g. lupus and tissue rejection in organ transplantation.

Advantageously, the inventors have demonstrated that the isolated single-domain antibody directed against Tenascin-C (TNC) or immunoconjugate of the invention, for example due to its better affinity and specificity to the Tenascin-C, allow to provide a better detection/results with regards to the presence or not of its protein, for example in a sample.

In other words, the isolated single-domain antibody directed against Tenascin-C (TNC) or immunoconjugate of the invention of the invention provide more reliable results and a better detection efficiency.

Thus the isolated single-domain antibody directed against Tenascin-C (TNC) or immunoconjugate of the invention enable increased sensitivity of the method and, for example, the diagnostic prognosis.

Other features and advantages will become further apparent to those skilled in the art upon reading the examples below, given by way of non-limiting illustration, with reference to the appended figures.

**Brief description of the drawings ,**

Figure 1 represents the specificity of purified/isolated single-domain antibody directed against Tenascin-C (TNC) (nanobodies for human Tenascin-C (hTNC)) Figure 1A represents a comparative alignment of amino acid sequences of two rhTNC- specific single-domain antibody (nanobodies) showing the four hallmark amino acid changes at positions F 42, E 49, R 50 and G 52 according to IMGT Scientific chart for the V-Domain numbering. Figure 1B is a photography of a SDS-PAGE analysis of the purified single-domain antibody directed against Tenascin-C (TNC) (rhTNC nanobodies). single-domain antibody directed against Tenascin-C (TNC) (Nb3) and single-domain antibody directed against Tenascin-C (TNC) (Nb4) (fusion of 6xHis and HA tags at the N-terminus) were expressed and separated on 15 % SDS-PAGE gels. The gel was stained with Coomassie blue. Lanes represent MW: Prestained Molecular Weight Marker, size indicated in kDa. 1, 2: Nb3 eluates 1 and 2. 3, 4: Nb4 eluates 1 and 2. 5: Purified periplasmic extract from Nb3 after induction. 6: Purified periplasmic extract from Nb4 after induction. The molecular weight of the Nbs was about 15 kDa in size. Figure 1C is a diagram representing the binding specificity assessment of both single-domain antibody directed against Tenascin-C (TNC) Nb3 and single-domain antibody directed against Tenascin-C (TNC) Nb4 towards rhTNC. One hundred microliters of rhTNC (50ng) were coated onto microtiter plates, and 100 μL single-domain antibody directed against Tenascin-C (TNC) (nanobodies) (500ng) were added. After incubation with a mouse monoclonal anti-HA tag antibody and then a monoclonal anti-mouse HRP, absorbance at 492 nm was measured by an ELISA reader. Anti-Botl nanobody (500ng) was used as negative control (NC). Values were the means of triplicates. Error bars represent the standard deviation of triplicates (ordinate refers to the absorbance at 492 nm and abscissa refers to single-domain antibody directed against Tenascin-C (TNC) Nb3 or Nb4). Figure 1D is a photography of a Western blot corresponding to immunolabeling of rhTNC with selected single-domain antibody directed against Tenascin-C (TNC) (Nb) candidates. A total amount of 40 μg of parental HEK293 (HEK) and modified HEK293/TNC (engineered to express hTNC) total cell lysates were analyzed for TNC expression by Western blot. Immuno-identification of rhTNC with Nb3 and Nb4 at (2 μg/ml) candidates and with B28.13 (monoclonal antibody anti-hTNC) as positive control. GAPDH was used as loading control (1:1000). Furthermore, 100 ng of purified rhTNC (hTNC) were loaded. (ordinate refers to the absorbance at 492 nm and abscissa refers to single-domain antibody directed against Tenascin-C (TNC) Nb3 or Nb4). Figure 1E is a diagram representing an estimate of the potency (EC50) of single-domain antibody directed against Tenascin-C (TNC) Nb3 (diamonds) as well as single-domain antibody directed against Tenascin-C (TNC) Nb4 (squares) ligands interacting with rhTNC. EC50 is the effective concentration at which 50% of rhTNC epitope sites are occupied by Nb ligand (ordinate refers to Optical Density at 450 nm and abscissa refers to the concentration of single-domain antibody directed against Tenascin-C (TNC) Nb3 or Nb4 in nM). Figure 1F is a diagram representing binding affinities of single-domain antibody directed against Tenascin-C (TNC) Nb3 and single-domain antibody directed against Tenascin-C (TNC) Nb4 towards recombinant mTNC as measured by isothermal fluorescence titration. (ordinate refers to ratio ΔF/F0 (ΔF over F0), ΔF indicates the variation of fluorescence intensity between initial fluorescence intensity at the resting state and after stimulation and abscissa refers to the concentration of single-domain antibody directed against Tenascin-C (TNC) Nb3 or Nb4 in nM)

Figure 2 represents the identification of interaction sites of single-domain antibody directed against Tenascin-C (TNC) Nb3 or Nb4 on rhTNC. Figure 2A are images representing the binding of gold-labeled Nb3 and Nb4 to hTNC determined by negative staining and transmission electron microscopy (Nb adsorption to hTNC surface). Representative of 500 micrographs. The hTNC molecule in the absence of single-domain antibody directed against Tenascin-C (TNC) Nb3 or Nb4 (Nb ligand) is depicted. Black dots designated with arrows represent identified binding sites of single-domain antibody directed against Tenascin-C (TNC) Nb3 and Nb4 on hTNC. Figure 1B represents the measurement of single-domain antibody directed against Tenascin-C (TNC) Nb3 and single-domain antibody directed against Tenascin-C (TNC) Nb4 binding according to the position (nm) on TNC (ordinate refers to the percentage of binding and abscissa refers to the position of binding in nm of TNC).

Figure 3 represents the detection of hTNC in human tissues by single-domain antibody directed against Tenascin-C (TNC) Nb3 and Nb4 by immunohistochemical analysis. Figure 3A are images of representative immunohistochemical (IHC) staining images for hTNC in FFPE embedded tissues from U87-MG xenografts with single-domain antibody directed against Tenascin-C (TNC) Nb3, single-domain antibody directed against Tenascin-C (TNC) Nb4 (2 μg/ml, respectively), Control, no monoclonal anti-HA secondary antibody (absence of unspecific immuno-reactivity towards hTNC) or stained with B28.13 (1 μg/ml) (positive control). Scale bar, 5 μm. This figure shows that single-domain antibody directed against Tenascin-C (TNC) Nb3 and single-domain antibody directed against Tenascin-C (TNC) Nb4 specifically recognize the native hTNC expressed in the ECM of U87-MG xenograft. Figure 3B are images of localized fibrillar staining of TNC by single-domain antibody directed against Tenascin-C (TNC) Nb3 and single-domain antibody directed against Tenascin-C (TNC) Nb4 in OSCC tissues. FFPE-embedded OSCC tissues were incubated with a polyclonal anti-TNC antibody (1 μg/ml) or with Nb3 or Nb4 (2 μg/ml, respectively). Figure 3C are images of Immuno-detection of hTNC by using single-domain antibody directed against Tenascin-C (TNC) Nb3 or single-domain antibody directed against Tenascin-C (TNC) Nb4 on FFPE embedded tissue from an hepatic metastasis from a patient with carcinoma of the gallbladder (CGB hepatic metastasis). Scale bar, 200 μm. Figures 3 D and 3 E are images of immunofluorescence (IF) staining corresponding to the detection of human TNC by imaging on fresh frozen U87MG (D) and U87MG-shTNC (E) cryosections. Staining by single-domain antibody directed against Tenascin-C (TNC) Nb3, single-domain antibody directed against Tenascin-C (TNC) Nb4 and/or by B28.13. the nucleus are stained with Dapi. Scale bar, 7μm.

Figure 4A are images of immunofluorescense (IF) staining of KRIB cells cultured on fibronectin (FN), a mixture of FN and hTNC (FN/TNC) or a mixture of FN and hTNC incubated with single-domain antibody directed against Tenascin-C (TNC) Nb4 at 1mM (FN/TNC-Nb4 (1mM)) or 2mM (FN/TNC-Nb4 (2mM)). After 2 h, the KRIB cells were fixed and stained with phalloidin to detect polymerized actin, anti-vinculin to detect focal adhesion complexes, and the nuclear marker DAPI. Figure 4B is a diagram representing the results of a cell adhesion assay on three different substrates coated with fibronectin (FN) (black), fibronectin and Tenascin-C (FN/TNC) (grey) and Tenascin-C (TNC) (light grey) (1 μg/cm2) when incubated with single-domain antibody directed against Tenascin-C (TNC) Nb4 at 1μM or 2μM. The ordinate represents the numbers of spread cells. Figure 4C are images of Mesangial cells cultured on FN, TNC or FN/TNC substratum without (Ctrl) or with single-domain antibody directed against Tenascin-C (TNC) Nb3 or with single-domain antibody directed against Tenascin-C (TNC) Nb4, respectively. Figure 4B is a diagram representing the results of cell adhesion assay on three different substrates coated with FN, TNC or FN/TNC without (ctrl) or with single-domain antibody directed against Tenascin-C (TNC) Nb3 or with single-domain antibody directed against Tenascin-C (TNC) Nb4. The ordinate represent the percentage of number of adherent cells. Figure 4E is a schematic representation of the Boyden chamber transwell chemoretention assay of DC2.4 toward a gradient of CCL21. The bottom surface of the insert was coated with FN, Col I, or TNC. Figure 4F is a diagram representing the quantification of DC2.4 cells on the coated surface upon migration toward CCL21 (5 h after plating) and pretreatment or not (Ctrl) with single-domain antibody directed against Tenascin-C (TNC) Nb3 or with single-domain antibody directed against Tenascin-C (TNC) Nb4, respectively. N = 2 experiments, n = 4 wells. The ordinate represents the number of cells per field.

Figure 5 represents three dimensional topology of the single-domain antibody directed against Tenascin-C (TNC) Nb3 /TNC interaction, in particular with 5th TNC fibronectin type III repeat (TN5), complex (figure 5A) and residue contact map (figure 5B). On figure 5A the zoomed part of the complexes represents a special view on residues generating important hydrophobic, electrostatic and H-bound interactions. TNC is represented as white surface with electrostatic surface coloring. Nb3 is presented in grey; crucial residues forming binding sites are presented in spheres. The most implicated residues in the interaction forming an H-bound are labeled in the figure, with interaction connections illustrated by a dashed line. Figure 5B is a diagram representing the contact interaction map of amino acid residue interactions between the single-domain antibody directed against Tenascin-C (TNC) Nb3 and TNC. Black square represents hydrophilic-hydrophilic interaction, grey square represents hydrophobic-hydrophobic interaction and characters in grey represent hydrophilic-hydrophobic interactions.

Figure 6 represents images of a 1% agarose gel of VHHs genes amplified by PCRs after being sequentially digested with Ncol and Notl and cloned into the digested phagemid vector pMECS. Figure 6A is an image representative of analysis of PCR products from the dromedary antibody fragments (using the primers CALL001 and CALL002) by electrophoresis in a 1% agarose. The upper band of 900 bp corresponds to DNA fragments (VH-CH1-hinge region-part of CH2) of conventional IgG1, whereas the lower band of 700 bp corresponds to DNA fragments (VHH-hinge-region-part of CH2) of non-conventional IgG2 and IgG3 isotypes. M: Molecular weight marker, Lane 1: PCR products with indicated sizes. Figure 6B is an image representative of nested PCR products obtained by reamplification of the 700 bp fragment, using sense primer SM017 and the antisense primer PMCF by electrophoresis in a 1% agarose gel. The band of 400 bp corresponds to VHH fragments (V-D-J-REGION of non conventional IgG2 and IgG3 antibodies). M: Molecular Weight Marker, Lane 1: Nested PCR Product. Figure 6C is an image representative of digested pMECS phagemid and VHH inserts by gel electrophoresis in a 1% agarose gel. M: Molecular Weight Marker Lane 1: pMECS phagemid double-digested with Ncol, Notl and Xbal restriction enzymes. Lane 2: Nested PCR Product (VHH) double-digested with Ncol and Notl. Figure 6D is a gel electrophoresis image to assess percentage of clones with proper insert of the cloned VHH, 19 single colonies of the transformants were randomly picked and their DNA was used for colony PCR library with MP57 and Gill primers. The amplicons were separated on 1% agarose gel. Clones without VHH DNA insert have a smaller amplicon size. Note, that 78.94% of clones have an insert of the correct size of 700 pb. M: Molecular Weight Marker, Lane 1 -19: PCR products issued from 19 tested clones Figure 6 E is a diagram representing the result of ELISA on periplasmic extracts. The periplasmic extract from 25 random clones derived from the third biopanning round were analysed with ELISA. rhTNC at 1 μg/ml was coated in each well. Periplasmic exctracts obtained from clones selected against Aahl (IC (1)) and Botl (IC (2)) toxins were used as irrelevant controls. A total of eight clones against rhTNC were selected on the basis of absorbance. C3, C4, C5, C7, C8, C9, C11, C29 correspond to selected positive clones expressing rhTNC-specific single-domain antibodies in their periplasmic extracts (showing specific signal towards hTNC in their periplasmic extract The x-axis shows the number of clones, and the y-axis the absorbance values at 492 nm.

Fiigure 6 F is an image of 1% gel electrophoresis agarose of positive hTNC transformant clones identified by ELISA (with higher absorbance) were the subject of colony PCR to confirm the presence of VHH DNA insert of the expected size of 700 bp within the pMECS construct. Lane M: Molecular Weight Marker.

Figure 7 are IHC stained image of detection of TNC in human tissues with single-domain antibody directed against Tenascin-C (TNC) Nb3 and single-domain antibody directed against Tenascin-C (TNC) Nb4. Figure 7A are images representing IHC staining images for TNC in FFPE embedded tissues from human ulcerative colitis (UC). Staining of TNC with single-domain antibody directed against Tenascin-C (TNC) Nb3 and single-domain antibody directed against Tenascin-C (TNC) Nb4. Control, no anti-HA secondary antibody. Scale bar, 700 nm. Figure 7B are images representing IHC staining images for TNC in FFPE embedded tissues from human tongue OSCC with monoclonal anti-TNC antibody (B), single-domain antibody directed against Tenascin-C (TNC) Nb3 (C) or single-domain antibody directed against Tenascin-C (TNC) Nb4 (D).

Figure 8 are images of immunofluorescense (IF) staining of OSCC cells cultured on fibronectin (FN) or a mixture of FN and humain TNC (FN/TNC) incubated with single-domain antibody directed against Tenascin-C (TNC) Nb4 at 1mM. After 2 h, the OSCC cells were fixed and stained with phalloidin to show polymerized actin, anti-vinculin to show focal adhesion complexes, and the nuclear marker DAPI. The OSCC cells spread and formed actin stress fibers and numerous focal adhesion complexes (identified with arrows on the figures) on FN as well as on FN/TNC substratum in the presence of Nb4. The OSCC cells cultured on mixture of FN and TNC with the presence of Nb4, maintain their adhesion function.

## Equivalents

The representative examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. It should further be appreciated that the contents of those cited references are incorporated herein by reference to help illustrate the state of the art.

The following examples contain important additional information, exemplification and guidance that can be adapted to the practice of this invention in its various embodiments and the equivalents thereof.

## EXEMPLIFICATION

The present invention and its applications can be understood further by the examples that illustrate some of the embodiments by which the inventive product and medical use may be reduced to practice. It will be appreciated, however, that these examples do not limit the invention. Variations of the invention, now known or further developed, are considered to fall within the scope of the present invention as described herein and as hereinafter claimed.

## Examples

### Example 1 : Production, purification and characterization of single-domain antibody directed against Tenascin-C (TNC)

#### I. Materials and methods

##### a) Purification of recombinantly expressed hTNC

HEK 293/hTNC cells, previously stably transfected with the human TNC (hTNC) coding sequence SEQ ID NO: 19 were used to produce hTNC as previously described (37, 38). Cells were cultured in Dulbecco's Minimal Essential Medium (DMEM, catalog number 11995040 Gibco Life Technologies, Inc., Paisley, Scotland) supplemented with 10 % (v/v) foetal calf serum (FCS, catalog number 2-01F90-I BioConcept, Allschwil, Switzerland), 10.25 μg/mL G418 and 1.5 μg/mL puromycin under a 5 % $CO_2$ atmosphere at 37°C. The recombinant hTNC (rhTNC) was purified from the conditioned medium lacking Foetal Calf Serum (FCS) as previously described ([37]). First, fibronectin (FN) was removed from the conditioned medium by affinity gelatin-agarose chromatography as described previously ([39], [40]), and the flow through was purified by an affinity chromatography column ([41]). The chromatography column used was Ni-column resin (ProBond (trademark) Nickel-Chelating Resin, Life Technologies, ref: R801-01). The purity of the protein was checked by Coomassie Blue stained 7% SDS-PAGE and by western-blot, under reducing and non-reducing conditions. SDS-PAGE was performed (7%) with 2.5μg of the purified recombinant hTNC. Sample was reduced with DTT reducing agent (2M) to a final concentration of 0.1M. After the running step, protein band was transferred on a Poly VinyliDene Fluoride (PVDF) membrane that had previously required a pretreatment with ethanol. Subsequently, the membrane was incubated for 1h30min with 5% skimmed milk to saturate its free binding sites. After a rinsing step with PBS-Tween 0.1% solution, the membrane was incubated for 1h under moderate agitation with a specific anti-hTNC monoclonal antibody (B28.13) produced in mice and diluted 1000 fold. After three successive washes with PBS-Tween 0.1%, the immunodetection step was carried out by incubating the membrane with a peroxidase-conjugated goat anti-mouse antibody. The revelation step was carried out by the addition of the chromogenic substrate 3,3'-Diaminobenzidine (DAB) of the peroxidase

The concentration of rhTNC was determined by Bradford assay (catalog number 500-0006 Bio-Rad Laboratories, Hercules, CA, USA).

**b) E.coli strains and vector**

The phage display vector pMECS of 4510 bp was utilized to construct the VHH library, hosted in E.coli strain TG1. This phagemid vector contains a sequence encoding a PelB leader signal to secrete the cloned VHH-encoded Nb in the periplasm with two C-terminal Hemagglutinin (HA) and 6X Histidine (6X His) tags for VHH-detection, when hosted in E.coli strain WK6 [42].

**c) Generation of phage-display VHH-library**

The anti-hTNC nanobody phage-display VHH-library was constructed as previously described with slight modifications ([32], [42], [43]). Briefly, three days after the last boost of antigen injection, 150 mL of anti-coagulated blood sample was collected from the jugular vein of the immunized dromedary as recently detailed [44]. Peripheral blood mononuclear cells (PBMCs) were extracted by density gradient centrifugation using Lymphoprep (catalog number 17-829 LONZA, Basel, Switzerland). Subsequently, total RNA was extracted and purified. An amount of 40 μg of total RNA was reverse transcribed into cDNA with oligo-dT primer and the SuperScript II First-Strand Synthesis System for RT-PCR (catalog number 18064-014 Invitrogen, Carsbad, CA, USA). Thereafter, cDNA fragments were used as template to amplify heavy-chain IgG encoding variable domains using specific primers (CALL001 (5'-GTCCTGGCTGCTCTTCTACAAGG-3' (SEQ ID NO: 8) and CALL002 (5'-GGTACGTGCTGTTGAACTGTTCC-3' (SEQ ID NO: 9)). The CALL001 primer at 20 μM anneals to the template strand of leader-signal sequences of camelid VH and VHH genes. The CALL002 primer at 20 μM in $H_2O$, anneals to the coding strand of CH2-sequence of camelid IgG. The reaction mixture is containing dNTP mix (each nucleotide at a final concentration of 10 mM), CALL001 primer (0.4 μM final concentration), CALL002 primer (0.4 μM final concentration), FastStart Taq DNA polymerase (1.25 U), 10×PCR buffer with 20 mM $MgCl_2$, 2, 3 or 4 μL first-strand cDNA material and $H_2O$ to bring the total volume in each tube to 50 μL. Tube control is without cDNA template and will serve to ensure that PCR components are not contaminated, i.e., a negative control). The cDNA is denaturized and the polymerase is activated by 7 min incubation at 95°C, followed by 30 PCR cycles, each cycle consisting of 60 seconds at 94°C, 60 seconds at 55°C, and 60 seconds at 72°C. A final DNA extension step is included for 10 min at 72°C after the last PCR cycle. The 700 bp PCR fragment (VHH-CH2 without CH1 exon, corresponding to heavy-chain antibodies) obtained was purified from a 1 % agarose gel using the Qiaquick gel extraction kit (catalog number 28704 Qiagen, Hilden, Germany). Subsequently, these sequences were used as template in a nested PCR to amplify VHH-only variable domains with nested-PCR primers (SM017 (5'-CCAGCCGGCCATGGCTGCATGGTGCAGCTGGTGGAGTCTGG-3' (SEQ ID NO: 10)) and PMCF (5'-CTAGTGCGGCCGCTGAGGAGACGGTGACCTGGGT-3' (SEQ ID NO: 11)), annealing at the Framework 1 and Framework 4 regions, including NcoI and NotI restriction sites, respectively (catalog numbers R0193T and R3189M New England Biolabs, UK, respectively). In the Nested PCR was carried out in order to amplify VHH-only sequences and introduce cutting sites of NcoI and NotI restriction enzyme. The reaction mixture is containing dNTP mix (each nucleotide at a final concentration of 10 mM), SM017 primer (0.4 μM final concentration), PMCF primer (0.4 μM final concentration), FastStart Taq DNA polymerase (1.25 U), 10×PCR buffer with 20 mM $MgCl_2$, 2 μL PCR product material (CALL001/CALL02) as template and $H_2O$ to bring the total volume in each tube to 50 μL. Tube control is without DNA template and will serve to ensure that PCR components are not contaminated, i.e., a negative control). The DNA is denaturized and the polymerase is activated by 5 min incubation at 94°C, followed by 40 PCR cycles, each cycle consisting of 45 seconds at 94°C, 45 seconds at 58°C, and 45 s at 72°C. A final DNA extension step is included for 10 min at 72°C after the last PCR cycle. The PCR product was ligated into the pMECS phagemid vector (T4 DNA Ligase, catalog number 15224-041 Invitrogen, Carsbad, CA, USA) using a molar ratio 1:3 in favor of the inserts. Freshly prepared electro-competent E.coli TG1 cells were transformed by the ligated product and plated overnight (O/N) on selective Luria-Bertani Miller (LB) media supplemented with (100 μg/mL) ampicillin (catalog number 271896 Sigma Aldrich, MO, USA) and glucose 2% (catalog number G8270 Sigma Aldrich, MO, USA).

Colonies were recovered from the overnight-incubated plates at 37ºC. Library size was estimated by plating serial dilutions.

**c) Selection of anti-rhTNC nanobodies (Nb)**

A representative repertoire of the VHH library was displayed on phage particles using M13KO7 helper phage infection (catalog number 170-3578 New England, BioLabs, UK). Three consecutive rounds of immuno-affinity selection were carried out on 96-well microtiter plates (catalog number M5785-1CS Sigma Aldrich, MO, USA) pre-coated with rhTNC (1 μg/panning, O/N at 4ºC). After each round of biopanning, bound phage particles were eluted (100 mM triethylamine, pH 10.0, catalog number T0886 Sigma Aldrich, MO, USA) and immediately neutralized with 1 M Tris-HCl, pH 7.4 (catalog number CE234 GeneON, Germany) and used to infect exponentially growing TG1 E.coli. Following the third round of biopanning, individual colonies were randomly picked, individually. VHH expression was induced with 1 mM isopropyl-D-thiogalactopyranoside (IPTG, catalog number 2900245 5PRIME, Germany) in the periplasmic bacterial compartment. Solid phase ELISA of each periplasmic extract was carried out on rhTNC (1 μg/mL), using a mouse anti-HA antibody (catalog number H9658 Sigma Aldrich, MO, USA) and revealed with a goat anti-mouse IgG-peroxidase antibody (catalog number A9044 Sigma Aldrich, MO, USA).

**d) VHH sequence analysis**

The VHH sequences of clones that scored positive in periplasmic extract-ELISA was determined using the Genomic platform of Institut Pasteur de Tunis facilities (ABI Prism 3100 genetic analyzer; Applied Biosystems, Foster City, CA, USA). The VHH nucleotide sequences were obtained using the ABI PRISMTM BigDye Terminator v3.1 Cycle Sequencing Reaction Kit (catalog number 4337454 Applied Biosystems, USA).

**e) Production, purification and characterization of single-domain antibody directed against Tenascin-C (TNC) (nanobody against TNC)**

Recombinant vectors of selected positive clones with highest binding capacity to rhTNC were used to transform WK6 electrocompetent cells. Single-domain antibody or nanobodies (Nb) production was performed in shake flasks by growing each recombinant bacteria in Terrific Broth (TB, catalog number 743-29175 BD Biosciences, FL, USA) supplemented with ampicillin (100 μg/mL) and 0.1 % glucose. The Nb periplasmic expression was subsequently induced with 1 mM of isopropyl β-D-1-thiogalactopyranoside (IPTG), overnight (O/N) at 28°C. The periplasmic extract obtained by osmotic shock was loaded on a His-Select column (NiNTA, catalog number 1018544 Qiagen, Hilden, Germany). The His-tagged rhTNC-specific Nbs were eluted with 500 mM imidazole (catalog number 1-0125 Sigma Aldrich, MO, USA) and an amount of 5 μg was checked on a 15% Sodium Dodecyl Sulfate (SDS) gel upon-PAGE (Bio Rad), following a dialysis with a 12 kDa cut-off membrane (catalog number D9527-100FT Sigma Aldrich, MO, USA). The final yield was determined using Bradford assay (catalog number 500-0006 Bio-Rad Laboratories, Hercules, CA, USA) and the molar concentration was estimated using the theoretical extinction coefficient of the VHH sequence. The specificity of the purified single-domain antibody directed against Tenascin-C (TNC) or anti-rhTNC nanobodies was assessed by ELISA. Briefly, 0.5 μg/mL of rhTNC was coated onto microtiter plates O/N at 4ºC and unspecific sites were blocked with 1% (w/v) gelatin (catalog number 48723 Fluka Analytical, USA) supplemented with 0.05 % Tween 20/PBS at 37ºC for 2h. Affinity-purified single-domain antibody directed against Tenascin-C (TNC) Nb was added (5 μg/mL, 1h). Following a washing step for purifying single-domain antibody anti-TNC specific (anti-TNC specific Nanobodies), periplasmic extract of each recombinant clone was extracted then incubated with the Ni+ - Nitrilotriacetic acid agarose (Ni-beads, Qiagen) for 2 hours in a cold-room at 4°C, with shaking. The periplasmic solution containing soluble single-domain antibody directed against Tenascin-C (TNC) Histidine-tagged (Histidine-tagged Nanobodies developed against TNC) was purified using immobilized metal ion affinity chromatography (IMAC). A filter was put in an empty PD-10 column, and the periplasmic extract with Ni-beads was poured into the column. The column was washed with PBS for 5 column volumes and drained. The bound proteins were eluted with gradient concentrations of imidazole (from 0.3M to 0.6M). The bound single-domain antibody directed against Tenascin-C (TNC) Nb was detected with a mouse anti-HA antibody (catalog number H9658 Sigma Aldrich, MO, USA) and revealed with a goat anti-mouse IgG-Peroxydase conjugate (catalog number A9044 Sigma Aldrich, MO, USA). The same amount of a Nanobody specific for BotI scorpion toxin (*Buthus occitanus tunetanus*) was used as irrelevant Nanobody (control). The NbBotI-01 was prepared with the same strategy used to develop anti-AahII Nanobodies (Ben Abderrazek et al., 2009 [96]). BotI like toxin was purified by FLPC and HPLC chromatography. The 40 N-terminal amino acid residues were sequenced and its LD50 was estimated to 50ng/mouse. BotI-like displays similarity to BotI toxin (UniProtKB/Swiss-Prot: P01488.2) BotI enriched fraction was injected in dromedary. The BotI-like toxin was used for the two last boots. VHH library was constructed and screened as previously described (Benabderrazek et al., 2009 [96]). Two Nanobodies with sub-nanomolar affinity were selected

(NbBotI-01 a NbBotI-17). The best-in-class NbBotI-01 is able to neutralize 50% lethally effect of 13LD50 of BotI-like toxin in mice injected by i.c.v route whereas NbBotI-17 is able to neutralize 50% lethally effect of 7LD50.

**f) Assessment of single-domain antibody directed against Tenascin-C (TNC) (Nanobody) binding affinity**

The assessment of Nb binding affinity was performed using two methods: (i) indirect ELISA was carried out using a serial Nb dilution ranging from $5.10^{-7}$ M to $5.10^{-12}$ M, (ii) Isothermal Fluorescence Titration (IFT) was performed using recombinant murine TNC (mTNC (SEQ ID NO 20), 700 nM, 0.01% Tween-20). Briefly, the single-domain antibody directed against Tenascin-C (TNC) (Nanobody) Nb concentration varied from 500 nM to 3500 nM. The fluorescence emission spectra for mTNC/Nb complexes were collected and subsequently subtracted from emission spectra for mTNC and the resulting curves were then integrated. The mean values resulting from 3 independent measurements were plotted against the concentration of the added Nb. The resulting binding isotherms were analyzed by nonlinear regression using the program Origin (Microcal Inc., Northampton, MA, USA). The following equation describes the bimolecular association reaction, where Fi is the initial and Fmax is the maximum fluorescence values. The $K_D$ is the dissociation constant, and [mTNC] and [Nb] are the total concentrations of the mTNC and the Nb ligand, respectively:

$$F = Fi + Fmax\ [Kd + [mTNC] + [Nb] - \sqrt{}((K_D+[mTNC]+ [Nb])\ [\![\hat{}2]\!]\ - 4[mTNC][Nb])/(2[mTNC])]$$

**g) Negative Electron Microscopy imaging**

The single-domain antibody directed against Tenascin-C (TNC) / rhTNC interaction complexes were visualized by negative staining and electron microscopy as previously described [46]. Each single-domain antibody directed against Tenascin-C (TNC) (20 nM) was conjugated with 5 nm colloidal gold particles (AuNPs) according to routinely used procedures [47]. AuNP-single-domain antibody directed against Tenascin-C (TNC) conjugates were incubated with rhTNC (20 nM) for 30 min at room temperature (RT) (i.e. 20°C) and subsequently negatively stained with 2% uranyl acetate. Specimens were assessed and electron micrographs were taken at 60 kV with a Phillips EM-410 electron microscope using imaging plates (Ditabis).

**h) Western blot analysis of single-domain antibody directed against Tenascin-C (TNC) (TNC specific Nb)**

Cell lysate (40μg) from HEK293, HEK293/hTNC in RIPA buffer (catalog number R0278 SIGMA Aldrich, MO, USA) or purified rhTNC (100 ng) were boiled at 100 ºC for 5 min, before loading on a 4-20% gradient SDS/PAGE gel (catalog number 456-8095 Mini-PROTEAN TGXTM, Bio-Rad Laboratories, Hercules, CA, USA), then, transferred onto polyvinylidene fluoride (PVDF) membrane (catalog number 1620174 Bio-Rad Laboratories, Hercules, CA, USA). After blocking with 5% milk, PBS/0.1% Tween-20 (catalog number 1706404 Bio-Rad Laboratories, Hercules, CA, USA) the membrane was incubated O/N at 4ºC with rhTNC-Nb (2 μg/mL). After three washing steps, with 0.1% Tween 20/PBS, the membrane was first incubated with a mouse anti-HA antibody (1h 30 min at RT (i.e. 20°C)), and then with the anti-mouse IgG horseradish peroxidase conjugate diluted at 1:1000 (catalog number AB_772209, NXA931, Amersham GE Helthcare, USA). Immunocomplexes were revealed with ECL (catalog number 28 980926 Amersham GE healthcare, USA). A prestained protein ladder (10-250 KDa, catalog number 06P-0211 Euromedex, France) was used. Mouse monoclonal antibody B28.13 (1 μg/mL) raised against hTNC was used as a positive control [48].

**i) Immunofluroescence assay**

Glioblastoma cell xenografts had previously been generated by subcutaneous injection of 2 x $10^6$ U87MG or U87MG-shTNC cells into the flank of a nude mouse [49]. Frozen (-80°C) sections were cut (7 μm thickness), fixed with 4% paraformaldehyde (catalog number 30525-89-4 Sigma Aldrich, MO, USA) for 15 min at RT (i.e. 20°C), and permeabilized with 0.5 % Triton X-100 in PBS and blocked with 10 % normal donkey serum (NDS) in PBS for 2 h at RT (i.e. 20°C) (catalog number 017-000-121 Jackson ImmunoResearch Inc, USA). Sections were co-stained with the single-domain antibody directed against Tenascin-C (TNC) Nb and B28.13 antibody diluted in PBS, 10% NDS O/N at 4°C, rabbit anti-HA antibody (ab236632 abcam, UK, 1:1000 dilution, 90 minutes at RT) and donkey anti-mouse labeled with Texas Red fluorophore (catalog number PA1-28626 Invitrogen, Carsbad, CA, USA), and donkey anti-rabbit labeled with Alexa Fluor 488 Green fluorophore (catalog number AB-2313584 Jackson Immuno Research Inc, USA) were used (1:1000 dilution for 90 minutes at RT). After each antibody incubation, sections were washed 5 times with PBS for 5 minutes. For staining of cell nuclei, sections were incubated with 4', 6-diamidino-2-phenylindole (DAPI, 0.2 μg/mL, catalog number 32670 Sigma Aldrich, MO, USA) for 10 min at RT (i.e. 20°C). Slides were sealed with a polymerization medium (FluorsaveTM Reagent, Calbiochem) under coverslips and stored at 4ºC until analysis. Pictures were taken with an AxioCam MRm (Zeiss) camera and Axiovision software.

**j) Human tumor samples and immunohistochemistry**

Surgically removed tongue tumors, embedded in paraffin blocks, were retrieved from the tumor bank of the Centre Paul Strauss (Strasbourg, France) or the Department of Pathology, University Basel (Switzerland). Informed consent was obtained for all subjects. Characteristics of patients with oral squamous cell carcinoma (OSCC), or hepatic metastasis derived from carcinoma of the gallbladder (CGB), are summarized in Table 2 below.

Table 2 Characteristics of tumor patients

| sample ID | Tumor location | Sex | Stage | Metastases | Sampling date |
|---|---|---|---|---|---|
| 1 | Tongue base | F | T4N0-1M0 | no | 1989 |
| 2 | Mobile tongue | M | T4N0-1M0 | yes | 1991 |
| 3 | Tongue base | M | T4N0-1M0 | no | 1992 |
| 4 | Tongue base | F | T4N0-1M0 | yes | 1992 |
| 5 | Mobile tongue | M | T4N0-1M0 | yes | 1994 |
| 6 | Tongue base | M | T4N0-1M0 | yes | 1995 |
| 189 | Hepatic Metastasis from CGB | F | T3N2M1 | yes | 2015 |

In the above table 2: human cancer tissues from oral squamous cell carcinoma (OSCC) and hepatic metastasis derived from carcinoma gallbladder (CGB) with annotation of OSCC tumor location. Gender (F, female, M, male), staging (Tumor Node Metastasis (TNM) classification) are shown.

Immunohistochemical staining was performed on serial 5 μm deparaffinized tumor sections. For hTNC staining, intrinsic peroxidase was blocked by incubating sections with 3% hydrogen peroxide for 15 min and antigen retrieval was performed in Sodium Citrate (10 mM) buffer pH 6.0 at 95°C. Sections were blocked in 5 % goat serum for 1 h, then incubated ON/4°C with rabbit anti-TNC antibody (#19011, Millipore, 1 μg/mL) or single-domain antibody directed against Tenascin-C (TNC) (2 μg/mL). After PBS rinsing (washed 3 times with 0.1% Triton /PBS for 5 minutes), sections were incubated with biotinylated goat anti-rabbit (Goat anti-Rabbit IgG, HRP-linked Antibody: Cell signaling, 7074S Goat IgG HRP-conjugated antibody, HAF 109 RnD systems) or goat anti-lama antibodies (Anti-lama: invitrogen, A16063) (1 h at RT (i.e 20°C)) then avidin-biotin (PK-4000, VECTASTAIN ABC Kit, Vector Lab, California, USA). Staining was revealed with 3,3'-Diaminobenzidine developing solution (SK-4100, DAB, Vector Lab, California, USA) then sections were stained with hematoxylin. After embedding in aqueous mounting medium, sections were examined using a Zeiss Axio Imager Z2 microscope. Pictures were taken with an AxioCam MRm (Zeiss, Axiovision) camera. The image acquisition setting (microscope, magnification, light intensity, exposure time) was kept constant per experiment and in between genetic conditions. The origin of the tumor sample, patient gender, TNM stage, presence of metastasis and sampling date are depicted in above Table 2.

**k) Boyden chamber transwell chemoretention assay**

Boyden chamber transwell chemoretention assay of DC2.4 dendritic cells towards CCL21 was carried out as described previously [20]. The bottom of the chamber was filled with Dulbecco's Modified Eagle Medium (DMEM) containing human CCL21 (200 ng/mL, catalog number 366-6C-025 R&D Systems, Minneappolis, USA). The lower surface of the transwells was coated with purified horse fibronectin (FN) [37], rat collagen type I (Col I, catalog number 354236 BD Biosciences, FL, USA) or hTNC at a final concentration of 1 $\mu g/cm^2$ and incubated O/N with blocking solution alone or with a single-domain antibody directed against Tenascin-C (TNC) Nb, respectively. DC2.4 (5 x $10^5$) cells resuspended in 150 μL of 1% FBS-complemented DMEM were placed into the top chamber of the transwell system. Cells were incubated for 5 h at 37°C in 5% $CO_2$. After 5 h of incubation at 37°C, cells were fixed and stained for DAPI before cell counting on the lower side of the insert. N = 2 independent experiments, n = 2 wells with 6 individual randomly chosen images per well. Mean ± SEM, Kruskal-Wallis test and Dunn's post-test. *** $p < 0.005$ (relative to Nb condition).

**l) Adhesion assay**

The adhesion assay was carried out on human KRIB osteosarcoma (v-Ki-ras-transformed human osteosarcoma cells; ref. Berlin O, Samid D, Donthineni-Rao R, Akeson W, Amiel D, Woods VL. Development of a novel spontaneous metastasis model of human osteosarcoma transplanted orthotopically into bone of athymic mice. Cancer Res 1993;53:4890-5). and human MES mesangial cells (Sarrab RM et al. Establishment of conditionally immortalized human glomerular mesangial cells in culture, with unique migratory properties. Am J Physiol Renal Physiol 2011. 301(5) :1131-1138.) Precisely, 96 wells plates were coated with 1 μg/cm2 Fibronectin (FN), human Tenascin-C (hTNC) or with FN and hTNC. Single-domain antibody directed against Tenascin-C (TNC) (nanobody) were added at 500 nM after the coating for 1 h at 37 °C. Plates were rinsed with PBS and non-adsorbed plastic surface was blocked with 1 % Bovine Serum Albumin (BSA) for 1 h. After blocking, KRIB osteosarcoma (5 x$10^3$) cells were plated for 3 h at 37 °C in a humidified atmosphere with 5% $CO_2$. After incubation, non adherent cells were removed with PBS washing and spread cells were stained with crystal violet and counted.

**m) Structural modeling of the single-domain antibody directed against Tenascin-C (TNC) (Nb) - TNC interaction**

For the single-domain antibody directed against Tenascin-C (TNC) (Nb) - TNC structural interaction modeling the Rosetta Antibody application ([50], [51]) from "ROSETTA 3.8" was used. Selection of the top 10 Model was done according to Rosetta scoring based on system energy. The structure of the 5$^{th}$ TNC fibronectin type III repeat (TN5) was extracted from the protein data bank deposited under the PDB code: 1TEN. The TN5 structure was refined using the ROSETTA relax application which then was used to map potential interaction sites in the selected single-domain antibody directed against Tenascin-C (TNC) (Nb) through the ZDOCK docking tool, version 3.0.2 (52). Structure and complex interactions were then visualized via the molecular visualization PyMOL software [53].

## II. Results

**1. Generation of an immune VHH library to produce single-domain antibody directed against Tenascin-C (TNC) (hTNC- specific nanobodies)**

An immune response against hTNC had been elicited in the immunized dromedary as previously described [42]. From PBMC, total RNA was extracted and cDNA was prepared as mentioned in the above material and method. This cDNA was used as template to perform the first PCR using primers CALL001 and CALL002 specific for the variable domains of the heavy-chain isotypes (IgG1, IgG2 and IgG3), subsequently leading to the co-amplification of VH and VHH coding domains (Figure 6A). Because of the presence of CH1 domain in conventional antibodies (IgG1) and different hinge size in non-conventional antibodies (HCAbs), three PCR products were observed by agarose gel electrophoresis: the 900, 790 and 720 bp fragments corresponding to the VH-CH1-Hinge-CH2 of the IgG1 and the VHH-Hinge-CH2 exons of the IgG2 and IgG3, respectively. Figure 6A is an image of the obtained agarose gel electrophoresis of the PCR products from the dromedary antibody fragments (using the primers CALL001 and CALL002). As mentioned above, on this figure the upper band of 900 bp corresponds to DNA fragments (VH-CH1-hinge region-part of CH2) of conventional IgG1, whereas the lower band of 700 bp corresponds to DNA fragments (VHH-hinge-region-part of CH2) of non-conventional IgG2 and IgG3 isotypes. Selective only-VHH fragments were successfully amplified using nested PCR specific-primers and cloned in pMECS phagemid (see above). Approximately, the obtained hTNC-specific VHH (single-domain antibody directed against Tenascin-C (TNC)) library was estimated to contain $3\times10^6$ cfu/mL independent clones. The insert size of 19 randomly chosen clones was investigated by PCR. The library insertion rate with a VHH insert of the expected size was 78.94%. A representative aliquot of the TG1 cells harboring the VHH antibody (single-domain antibody) repertoire was rescued with M13KO7 helper phage to produce phage particles expressing the single-domain antibody directed against Tenascin-C (TNC) (nanobodies). Following three rounds of phage display selection on solid-phase coated hTNC, enrichment for hTNC-specific phage particles was observed from the first round of panning onwards (data not shown). 25 periplasmic extracts selected from randomly picked individual clones of biopanning rounds were checked by ELISA. Only clones scoring positively by ELISA without binding to unspecific proteins (AahI and BotI toxins, respectively) were retained (Figure 6E). In total, 8 recombinants clones displaying highest recognition and binding to the hTNC from the sequenced VHHs were selected. Two recombinant clones displayed identical amino-acid sequences (Nb3 and Nb5). The Nb3, Nb4 were selected for further investigations. As illustrated in Figure 1A, all single-domain antibody directed against Tenascin-C (TNC) (rhTNC-specific Nb) sequences (Nb3, Nb4) exhibit the VHH hallmark amino acid residues in the framework-2 region (FR2 Phe42, Glu49, Arg50, and Gly52) and the conserved Trp at position 118 of the anchoring region. According to their common CDR1 sequence, single-domain antibody directed against Tenascin-C (TNC) Nb3 and single-domain antibody directed against Tenascin-C (TNC) Nb4 are most likely derived from the same V-D-J rearrangement and share the B-cell progenitor [33]. The CDR3 residue length within the Nb3 and Nb4 sequences (17 amino-acid residues) was identical and no difference was noticed. The only divergences in Nb3 and Nb4 sequences were observed at position 2 (Leu substituted by Val in FR1), at position 52 (Gly substituted by Ala in CDR2) and at position 92 (Asp substituted by Gly in FR3), respectively, indicating an interaction with a common epitope on TNC. The sequence of single-domain antibody directed against Tenascin-C (TNC) Nb3 is WLQLVESGGGSVQTGGSLRLSCVVSGYTNSIYTLAWFRQAPGKEREGV AAIGSRNGNTYYDASVKDRFTISLDKAKNTVYLQMNDLKPEDTASYYCAA GSSWDLILQAYAYDYWGQGTQVTVSS (SEQ ID NO: 4) and the sequence of single-domain antibody directed against Tenascin-C (TNC) Nb4 is WVQLVESGGGSVQTGGSLRLSCVVSGYTNSIYTLAWFRQAPGKEREGV AAIASRNGNTYYDASVKDRFTISLDKAKNTVYLQMNGLKPEDTASYYCAA GSSWDLILQAYAYDYWGQGTQVTVSS (SEQ ID NO: 5)

**2. Production and purification of single-domain antibody directed against Tenascin-C (TNC) (rhTNC specific nanobodies)**

The production of single-domain antibody directed against Tenascin-C (TNC) was carried out using flask production and Immobilized Metal Affinity Chromatography purification (IMAC), as disclosed in Hmila et al. [54]. Briefly, production of each soluble single-domain antibody directed against Tenascin-C (TNC) (anti-hTNC Nb) was accomplished by transformation of E.coli WK6 strain with the corresponding recombinant phagemid. The amber stop codon located between the VHH insert and the gene III within the pMECS phagemid, resulted in expression of the single-domain antibody directed against Tenascin-C (TNC) (Nanobody) as soluble protein in the periplasm compartment of E.coli, leading to rapid IMAC purification of the single-domain antibody directed against Tenascin-C (TNC) (Nanobody). The Coomassie-stained SDS/PAGE gel revealed the apparent molecular weight at 14 kDa as shown on Figure 1B. The single-domain antibody directed against Tenascin-C (TNC) Nb3 and single-domain antibody directed against Tenascin-C (TNC) Nb4 production yields were estimated ranging 0.6 and 0.8 mg/L, respectively when flask cultured in TB medium. No bands indicative of contaminants or Nb degradation were detected.

During the ELISA experiment, as illustrated and demonstrated on Figure 1C, single-domain antibody directed against Tenascin-C (TNC) Nb4 (5 $\mu$g/mL) displayed a higher ELISA binding titer towards rhTNC (0.5 $\mu$g/mL, $OD_{492}$nm= 1.526), compared to Nb3 (5 $\mu$g/mL, $OD_{492}$nm= 0.913) and to the irrelevant nanobody (anti-Botl toxin Nanobody, 5 $\mu$g/mL, $OD_{492}$nm= 0,118 (not shown)). Furthermore, as illustrated on figure 1D, immunoblotting assays revealed that both single-domain antibodies directed against Tenascin-C (TNC) Nb3 and Nb4 showed a specific recognition of not only the purified rhTNC (100 ng) but also of TNC overexpressed by HEK293/TNC cells. Parental HEK293 cells did not express TNC.

This example clearly demonstrates the production and purification of single-domain antibody directed against Tenascin-C (TNC). This example also clearly demonstrated the binding of single-domain antibody directed against Tenascin-C (TNC) to purified and cell expressed Tenascin-C.

3. **Assessment of single-domain antibody directed against Tenascin-C (TNC) Nb3 and single-domain antibody directed against Tenascin-C (TNC) Nb4 affinities towards TNC by isothermal fluorescence titration**

By Isothermal Fluorescence Titration (IFT) the binding of single-domain antibody directed against Tenascin-C (TNC) Nb3 or single-domain antibody directed against Tenascin-C (TNC) Nb4 to fluorescently tagged mTNC was determined. The binding of single-domain antibody directed against Tenascin-C (TNC) Nb3 or single-domain antibody directed against Tenascin-C (TNC) Nb4 to fluorescently tagged mTNC was carried out until signal saturation and determined the dissociation constant (Kd) as $711x10^{-9}$ M (Nb3) and $537x10^{-9}$ M (Nb4). The results are illustrated on Figure 1E showing the corresponding obtained diagram.

In addition, ELISA assays were performed with single-domain antibody directed against Tenascin-C (TNC) Nb3 and single-domain antibody directed against Tenascin-C (TNC) Nb4 at molar concentrations ranging from $5x10^{-7}$ M to $5x10^{-12}$ M. The corresponding results and diagrams are shown on figure 1F and revealed specific binding with a 50% Effective Concentration (EC50) of both single-domain antibody directed against Tenascin-C (TNC) Nb3 and single-domain antibody directed against Tenascin-C (TNC) Nb4 binding to rhTNC ranging at approximately 10 nM and 5 nM, respectively

These results clearly demonstrate specific binding and high affinity of single-domain antibody directed against Tenascin-C (TNC) to Tenascin-C.

**4. Detection of single-domain antibody directed against Tenascin-C (TNC) (nanobody) binding site in TNC by Negative Electron Microscopy**

The location of the antigenic epitope in rhTNC was investigated by negative electron microscopy where the single-domain antibodies directed against Tenascin-C (TNC) (nanobodies) were coupled to gold beads. Upon incubation of rhTNC with gold beads-bound single-domain antibodies directed against Tenascin-C (TNC) (nanobodies). Figure 2A correspond to the images obtained and black dots on this figure designated with arrows represent identified binding sites of single-domain antibody directed against Tenascin-C (TNC) Nb3 and Nb4 on hTNC (Figure 2A). As shown on figure 2A the gold particles were detected along the length of the rhTNC monomers and quantified them. In addition, the binging pattern on TNC as shown on figure 2B demonstrates a high number of single-domain antibody directed against Tenascin-C (TNC) Nb3 and single-domain antibody directed against Tenascin-C (TNC) Nb4 binding in the middle of the rhTNC monomer. In addition single-domain antibody directed against Tenascin-C (TNC) Nb3 and single-domain antibody directed against Tenascin-C (TNC) Nb4 showed a similar binding pattern.

These results clearly demonstrate that single-domain antibody directed against Tenascin-C (TNC) binds to Tenascin-C and also suggest that both single-domain antibody directed against Tenascin-C (TNC) Nb3 and single-domain antibody directed against Tenascin-C (TNC) Nb4 (nanobodies) recognize the same or overlapping epitope in rhTNC.

**5. Single-domain antibody directed against Tenascin-C (TNC) Nb3 and single-domain antibody directed against Tenascin-C (TNC) Nb4 recognize hTNC in fresh frozen and paraffin-embedded human tissues**

The binding/detection of TNC, in particular human Tenascin-C, by single-domain antibody directed against Tenascin-C (TNC) Nb3 and single-domain antibody directed against Tenascin-C (TNC) Nb4 in paraffin (FFPE) embedded tissues was also investigated. Human colon tissue with ulcerative colitis (UC) was stained with single-domain antibody directed against Tenascin-C (TNC) Nb3 and single-domain antibody directed against Tenascin-C (TNC) Nb4. The results obtained are illustrated on Figure 3A. As shown on this figure, the obtained staining to resembled TNC expression in this disease on the analyzed tissues from human colon sample ([55], [56], [57], (Table 2, Figure 3A). Tissue from human tongue tumors (OSCC) was also stained with the single-domain antibody directed against Tenascin-C (TNC) Nb3 and single-domain antibody directed against Tenascin-C (TNC) Nb4 and with a commercial anti-TNC antibody on an adjacent section. The obtained results are shown on figure 3B and figure 7. As seen on this figure similar staining patterns for TNC reminiscent of tumor matrix tracks TMT that have previously been described [20]. Hepatic metastasis tissues from a patient with carcinoma gallbladder (CGB) were also stained (Table 2) with the single-domain antibody directed against Tenascin-C (TNC) Nb3 and the single-domain antibody directed against Tenascin-C (TNC) Nb4 and observed staining reminiscent of TNC (Figure 3C).

The binding and recognition of TNC by the single-domain antibody directed against Tenascin-C (TNC) Nb3 and the single-domain antibody directed against Tenascin-C (TNC) Nb4 in U87MG glioblastoma xenografted tumors were done by immunofluorescence staining and where it was previously noticed that human TNC was more abundant than murine TNC [53]. As shown on figure 3D, a fibrillar TNC signal was observed in the U87MG tumors that overlapped with that of the B28.13 antibody signal, confirming specificity of the single-domain antibody directed against Tenascin-C (TNC) (nanobodies) for TNC. As the U87MG tumors also express murine TNC but at much lower abundance [53], U87MG tumors with a knockdown for human TNC were stained in the grafted tumor cells. The results are illustrated on figure 3E and no signal/detection.

These results clearly demonstrate that single-domain antibody directed against Tenascin-C (TNC) binds to Tenascin-C in different tissues and also allow to bind to Tenascin-C in pathological tissues such as tumorous and/or metastasis tissues. In addition, these results demonstrate that single-domain antibody directed against Tenascin-C (TNC) clearly binds to human Tenascin-C.

**6. Single-domain antibody directed against Tenascin-C (TNC) Nb4 counteracts the anti-adhesive properties of TNC on a FN/TNC substratum**

By using human osteosarcoma KRIB cells the effect of single-domain antibody directed against Tenascin-C (TNC) Nb4 on cell rounding by TNC on a FN/TNC substratum was studied. Previously was shown that cells are inhibited by TNC to spread on a combined FN/TNC substratum since TNC competed with syndecan-4 binding to FN ([37], [58]). KRIB cells were plated on FN, FN/TNC and TNC, respectively with or without single-domain antibody directed against Tenascin-C (TNC) Nb4. As illustrated on Figure 4 by staining with phalloidin (polymerized actin) and anti-vinculin (focal adhesions), cell spreading on FN and cell rounding on FN/TNC and TNC were respectively confirmed. While cells do not have many actin stress fibers nor focal adhesions on FN, upon addition of Nb4, it was observed that cells spread on FN/TNC in a single-domain antibody directed against Tenascin-C (TNC) Nb4 dose dependent manner with actin stress fibers and focal adhesions that looked similar to those in cells plated on FN (Figures 4A,B, Figure 8). In contrast, as shown on figure 4B, cells remained spread and rounded on FN and TNC, respectively in the absence of single-domain antibody directed against Tenascin-C (TNC) Nb4. Similarly, as shown on figure 4C, both single-domain antibody directed against Tenascin-C (TNC) Nb3 and single-domain antibody directed against Tenascin-C (TNC) Nb4 restored the adhesion of mesangial cells (MES) on a FN/TNC substratum.

These results clearly demonstrate that single-domain antibody directed against Tenascin-C (TNC) allow to improve/restore the adhesion properties of TNC with regards to pathological cells, for example tumorous and/or cancerous cells. These results demonstrate that examples of single-domain antibody directed against Tenascin-C (TNC) according to the invention allow to inhibit and/or reduce the formation of metastasis, for example by restoring the adhesion of pathological cells to TNC, in particular to fibronectin and Tenascin-C.

**7. Single-domain antibody directed against Tenascin-C (TNC) Nb3 and Single-domain antibody directed against Tenascin-C (TNC)Nb4 abolish DC2.4 chemoretention by TNC/CCL21**

Combination with CCL21 TNC immobilized dendritic DC2.4 cells was previously shown [20]. A Boyden chamber transwell migration assay was used to investigate whether single-domain antibody directed against Tenascin-C Nb3 and Single-domain antibody directed against Tenascin-C Nb4 impacted chemoretention by TNC. The lower surface of the insert was coated with FN, collagen I (ColI) or TNC and added single-domain antibody directed against Tenascin-C Nb3 or single-domain antibody directed against Tenascin-C Nb4, respectively and measured DC2.4 cells that migrated towards CCL21 in the lower chamber (Figure 4E). Cells adhering were measured in the presence or absence of the single-domain antibody directed against Tenascin-C Nb3 or single-domain antibody directed against Tenascin-C Nb4 and observed, as shown on Figure 4F, a significant decrease in cell numbers on the coated TNC surface in the presence of single-domain antibody directed against Tenascin-C Nb3 or single-domain antibody directed against Tenascin-C Nb4 whereas no difference was seen with the other matrix coatings (Ctrl).

These results clearly demonstrate that single-domain antibody directed against Tenascin-C (TNC) allow to inhibit and/or reduce the retention of immune cells, in particular dendritic cells due to TNC. Accordingly, these results demonstrate that examples of single-domain antibody directed against Tenascin-C (TNC) according to the invention allow to inhibit the immunosuppressive effect of TNC.

**8. Three dimensional topology of the single-domain antibody directed against Tenascin-C (TNC) Nb3 / TNC (TN5) interaction complex**

A computational structure analysis strategy was carried out in order to identify amino acid residues involved in the interaction of single-domain antibody directed against Tenascin-C (TNC) Nb3 with the fifth FN type III domain of TNC (TN5). The 1f2x (Chain L), 3I95 (chain B), 5ocl (chain G) and 5vak (chain B) structures were used as principal template for single-domain antibody directed against Tenascin-C (TNC) Nb3-frameworks, CDR1, CDR2 and CDR3, respectively. The number of generated models is set to 1000. Top ten model best scores, ranked according to an energy-based scoring were selected from 10 clusters composed of 100 structures per cluster then visually double checked (to detect structural anomalies) using the molecular visualization PyMOL software. The TN5 structure (1TEN) was included in the single-domain antibody directed against Tenascin-C (TNC) Nb3 molecular docking simulation. The positively and negatively charged residues are indicated , whereas the neutral side-chains are indicated in white, showing clearly separated charges on the TN5 surface. Using the docking application/approach. The top ten possible binding sites were generated, ranked according to an energy-based scoring and filtered them to get a unique complex presenting the best molecular orientation with the most stable position, Figure 5A represent the corresponding result. In order to assess the main amino-acid residues involved in the molecular single-domain antibody directed against Tenascin-C (TNC) Nb3-TN5 interaction, the COCOMAPS (bioCOmplexes COntact MAPS) web application server was used. A predicted intermolecular contact map of the Nb3-TN5 complex is illustrated in Figure 5B with a cut-off of 3 Å, predicted binding-site residues in TN5 are as follow: N, D, NQ, S, I highlighting the residues mainly implicated in the complex interaction. The, N, Y (at position 862, 850, 856-857, 859, 860, 837, 858, respectively) interacting with single-domain antibody directed against Tenascin-C (TNC) Nb3 Y, Y, R, S, Y, Y, DYW (at position 26, 31, 44, 99, 108, 110-113, respectively). Interestingly, the CDR2 and CDR3 are shown to dominate the interaction with TN5. The details of residues involved in H bounds as proton donors or acceptors are described in Table 3 below.

Table 3: Amino acid residues generating H-bound interactions between single-domain antibody directed against Tenascin-C (TNC) Nb3 and TN5 with donor and acceptor atoms details.

| Donor | | | | Acceptor | | | | Dist (Å) | |
|---|---|---|---|---|---|---|---|---|---|
| Res1 | N° res1 | Atom 1 | Chain1 | Res2 | N° res2 | Atom2 | Chain2 | Dist H-Bonds | Dist CA-CA |
| ARG | 44 | NH2 | H | ASP | 850 | O | A | 2.17 | 10.2 |
| ASN | 856 | N | A | TYR | 108 | OH | H | 1.87 | 8.54 |
| SER | 859 | N | A | TYR | 108 | O | H | 2.6 | 5.2 |

(continued)

| Donor | | | | Acceptor | | | | Dist (Å) | |
|---|---|---|---|---|---|---|---|---|---|
| Res1 | N° res1 | Atom 1 | Chain1 | Res2 | N° res2 | Atom2 | Chain2 | Dist H-Bonds | Dist CA-CA |
| SER | 859 | OG | A | ASP | 111 | OD1 | H | 3.26 | 4.69 |
| TYR | 112 | OH | H | GLY | 861 | O | A | 2.86 | 6.56 |

In above table 3 Res means Residue, Dist means Distance, Å means Angstrom and CA means Carbon Atom.

These results clearly demonstrate that examples of single-domain antibody directed against Tenascin-C (TNC) according to the invention allow to specifically bind to Tenascin-C. These results also clearly demonstrate that the binding site on Tenascin-C is located within the fifth FN type III domain of TNC (TN5).

**Conclusion**

As demonstrated above, the results clearly demonstrate the development and production of single-domain antibody directed against Tenascin-C (TNC) (nanobodies directed against hTNC).

The results also clearly demonstrate that examples of single-domain antibody directed against Tenascin-C (TNC) according to the invention allows to detect TNC on different type of tissue, for example on FFPE tissues and also advantageously to block TNC functions.

The results also clearly demonstrate that the VHH library generated from dromedary that meets the required quality control standards ([77], [78]) and allowed to isolate single-domain antibody directed against Tenascin-C (TNC) (nanobodies) that specifically recognized TNC. The amino acid sequences of the isolated single-domain antibody directed against Tenascin-C (TNC) revealed identity with human VH sequences of family III; however, the VHH imprints were clearly present ([79], [80]).

The results also clearly demonstrate that examples of single-domain antibody directed against Tenascin-C (TNC) according to the invention (Nb3 and Nb4) bind in the center of the TNC monomeric molecule around TN5 which is also known to bind several soluble factors [81].

The results also clearly demonstrate that examples of single-domain antibody directed against Tenascin-C (TNC) according to the invention (Nb3 and Nb4) recognized specifically Tenascin-C (TNC), for example by immunoblot, ELISA and negative EM imaging and by staining of fresh frozen, PFA and FFPE fixed human tissues. The results also clearly demonstrate that examples of single-domain antibody directed against Tenascin-C (TNC) according to the invention (Nb3 and Nb4) exhibited relevant staining in all tested tissue sections demonstrating their aptitude to recognize native TNC in situ. In silico, 3D modeling of the interaction of Nb3 with TN5 revealed the potential contribution of CDR2 and CDR3 in the interaction with critical hydrophobic amino acid residues in TN5.

TNC is known to be highly de novo expressed in response to pathological stress conditions such as chronic inflammation and tumor malignancies. The results also advantageously demonstrate that examples of single-domain antibody directed against Tenascin-C (TNC) according to the invention (Nb3 and Nb4) bind to TNC expressed within the ECM of inflammatory diseases such as UC and in solid tumors, for example OSCC, CGB metastasis, U87MG xenografts). The results also demonstrated that examples of single-domain antibody directed against Tenascin-C (TNC) according to the invention (Nb3 and Nb4) recognized/bound TNC in FFPE embedded tissues.

The results also clearly support that single-domain antibody directed against Tenascin-C (TNC) according to the invention are suitable for sandwich ELISA assays for detection of TNC in biological sample, for example in body fluids, for example in blood and/or urine.

The results also clearly support that single-domain antibody directed against Tenascin-C (TNC) according to the invention allow diagnosis of disease with high TNC levels, for example such as cancer, and fibrosis, for example in the lung, kidney and liver.

The results also advantageously demonstrate that examples of single-domain antibody directed against Tenascin-C (TNC) according to the invention (Nb3 and Nb4) inhibit TNC-induced cell rounding, which is important in cancer progression. In addition, the results also advantageously demonstrate that examples of single-domain antibody directed against Tenascin-C (TNC) according to the invention (Nb3 and Nb4) interfered with TNC functions in chemoretention.

This example thus clearly demonstrate that examples of single-domain antibodies directed against Tenascin-C (TNC) according to the invention allow to target TNC and/or identify TNC expression in non pathological or pathological biological tissue.

This example thus clearly demonstrate that examples of single-domain antibodies directed against Tenascin-C (TNC) according to the invention allow to detect tumorous cells, and also advantageously, cancerous cells.

This example thus clearly demonstrate that examples of single-domain antibodies directed against Tenascin-C (TNC) according to the invention inhibit and/or reduce metastasis progression, for example by restoring the adhesion of cells in tumorous and/or cancerous tissue.

**List of References**

1. Takeda A, Otani Y, Iseki H, Takeuchi H, Aikawa K, Tabuchi S, et al. Clinical Significance of Large Tenascin-C Spliced Variant as a Potential Biomarker for Colorectal Cancer. World J Surg (2007) 31:388-394. doi:10.1007/s00268-006-0328-6

2. Hancox RA, Allen MD, Holliday DL, Edwards DR, Pennington CJ, Guttery DS, et al. Tumour-associated tenascin-C isoforms promote breast cancer cell invasion and growth by matrix metalloproteinase-dependent and independent mechanisms. Breast Cancer Res (2009) 11: doi:10.1186/bcr2251

3. Alharth AS, Alyami WA. Tenascin-C (TNC) Promotes Breast Cancer Cell Invasion and Proliferation: Functional Effects of TNC Knockdown in Highly Invasive Breast Cancer Cell Lines. Am J Med Biol Res Am J Med Biol Res (2016) 3:55-61. doi:10.12691/ajmbr-3-2-2

4. Shen C, Wang C, Yin Y, Chen H, Yin X, Cai Z, et al. Tenascin-C expression is significantly associated with the progression and prognosis in gastric GISTs: Medicine (Baltimore) (2019) 98:e14045. doi:10.1097/MD.0000000000014045

5. Ishihara A, Yoshida T, Tamaki H, Sakakura T. Tenascin expression in cancer cells and stroma of human breast cancer and its prognostic significance: Clin Cancer Res (1995) 9: 1035-1041.

6. Leins A, Riva P, Lindstedt R, Davidoff MS, Mehraein P, Weis S. Expression of tenascin-C in various human brain tumors and its relevance for survival in patients with astrocytoma. Cancer (2003) 98:2430-2439. doi:10.1002/cncr.11796

7. Chiquet-Ehrismann R, Hagios C, Schenk S. The complexity in regulating the expression of tenascins. BioEssays (1995) 17:873-878. doi:10.1002/bies.950171009

8. Erickson HP, Bourdon MA. Tenascin: An Extracellular Matrix Protein Prominent in Specialized Embryonic Tissues and Tumors. Cell Biol (1989) 5:71-92.

9. Chiquet-Ehrismann R, Orend G, Chiquet M, Tucker RP, Midwood KS. Tenascins in stem cell niches. Matrix Biol (2014) 37:112-123. doi:10.1016/j.matbio.2014.01.007

10. Chiquet-Ehrismann R, Chiquet M. Tenascins: regulation and putative functions during pathological stress: Tenascins in pathological stress. J Pathol (2003) 200:488-499. doi:10.1002/path.1415

11. Midwood KS, Orend G. The role of tenascin-C in tissue injury and tumorigenesis. J Cell Commun Signal (2009) 3:287-310. doi:10.1007/s12079-009-0075-1

12. Saupe F, Schwenzer A, Jia Y, Gasser I, Spenlé C, Langlois B et al. Tenascin-C Downregulates Wnt Inhibitor Dickkopf-1, Promoting Tumorigenesis in a Neuroendocrine Tumor Model. Cell Rep (2013) 5:482-492. doi:10.1016/j.celrep.2013.09.014

13. Sun Z, Velázquez-Ouesada I, Murdamoothoo D, Ahowesso C, Yilmaz A, Spenlé C, et al. Tenascin-C increases lung metastasis by impacting blood vessel invasions. Matrix Biol (2019) 83: 26-47. doi:10.1016/j.matbio.2019.07.001

14. Oskarsson T, Acharyya S, Zhang XH-F, Vanharanta S, Tavazoie SF, Morris PG, et al. Breast cancer cells produce tenascin C as a metastatic niche component to colonize the lungs. Nat Med (2011) 17:867-874. doi:10.1038/nm.2379

15. Lowy CM, Oskarsson T. Tenascin C in metastasis: A view from the invasive front. Cell Adhes Migr (2015) 9:112-124. doi:10.1080/19336918.2015.1008331

16. Langlois B, Saupe F, Rupp T, Arnold C, Van der Heyden M, Orend G, et al. AngioMatrix, a signature of the tumor angiogenic switch-specific matrisome, correlates with poor prognosis for glioma and colorectal cancer patients. Oncotarget (2014) 5 (21):10529-10545. doi:10.18632/oncotarget.2470

17. Bellone M, Caputo S, Jachetti E. Immunosuppression via Tenascin-C. Oncoscience (2015) 2:667. doi:10.18632/oncoscience.210

18. Jachetti E, Caputo S, Mazzoleni S, Brambillasca CS, Parigi SM, Grioni M, et al. Tenascin-C Protects Cancer Stem-like Cells from Immune Surveillance by Arresting T-cell Activation. Cancer Res (2015) 75:2095-2108. doi:10.1158/0008-5472.CAN-14-2346

19. Deligne C, Murdamoothoo D, Gammage AN, Gschwandtner M, Erne W, Loustau T, et al. Matrix-Targeting Immunotherapy Controls Tumor Growth and Spread by Switching Macrophage Phenotype. Cancer Immunol Res (2020) 8:368-382. doi:10.1158/2326-6066.CIR-19-0276

20. Spenlé C, Loustau T, Murdamoothoo D, Erne W, Beghelli-de la Forest Divonne S, Veber R, et al. Tenascin-C Orchestrates an Immune-Suppressive Tumor Microenvironment in Oral Squamous Cell Carcinoma. Cancer Immunol Res (2020) 8:1122-1138. doi:10.1158/2326-6066.CIR-20-0074

21. Hicke BJ, Marion C, Chang Y-F, Gould T, Lynott CK, Parma D, et al. Tenascin-C Aptamers Are Generated Using Tumor Cells and Purified Protein. J Biol Chem (2001) 276:48644-48654. doi:10.1074/jbc.M104651200

22. Zukiel R, Nowak S. Suppression of human brain tumor with interference RNA specific for tenascin-C. Cancer Biol Ther (2006) 5:1002-1007. doi:10.4161/cbt.5.8.2886

23. Rolle K, Nowak S, Wyszko E, Nowak M, Zukiel R, Piestrzeniewicz R, et al. Promising human brain tumors therapy with interference RNA intervention (iRNAi). Cancer Biol Ther (2010) 9:397-407. doi:10.4161/cbt.9.5.10958

24. Rolle K. miRNA Multiplayers in glioma. From bench to bedside. Acta Biochim Pol (2015) 62:353-365. doi:10.18388/abp.2015_1072

25. Grabowska M, Grzeskowiak BF, Szutkowski K, Wawrzyniak D, Gtodowicz P, Barciszewski J, et al. Nano-mediated delivery of doublestranded RNA for gene therapy of glioblastoma multiforme. PLOS ONE (2019) 14:e0213852. doi:10.1371/journal.pone.0213852

26. Brack SS. Tumor-Targeting Properties of Novel Antibodies Specific to the Large Isoform of Tenascin-C. Clin Cancer Res (2006) 12:3200-3208. doi:10.1158/1078-0432.CCR-05-2804

27. Heuveling DA, de Bree R, Vugts DJ, Huisman MC, Giovannoni L, Hoekstra OS, et al. Phase 0 Microdosing PET Study Using the Human Mini Antibody F16SIP in Head and Neck Cancer Patients. J Nucl Med (2013) 54:397-401. doi:10.2967/jnumed.112.111310

28. Aloj L, D'Ambrosio L, Aurilio M, Morisco A, Frigeri F, Caraco' C, et al. Radioimmunotherapy with Tenarad, a 131I-labelled antibody fragment targeting the extra-domain A1 of tenascin-C, in patients with refractory Hodgkin's lymphoma. Eur J Nucl Med Mol Imaging (2014) 41:867-877. doi:10.1007/s00259-013-2658-6

29. Petronzelli F. Improved Tumor Targeting by Combined Use of Two Antitenascin Antibodies. Clin Cancer Res (2005) 11:7137s-7145s. doi:10.1158/1078-0432.CCR-1004-0007

30. Silacci M. Human monoclonal antibodies to domain C of tenascin-C selectively target solid tumors in vivo. Protein Eng Des Sel (2006) 19:471-478. doi:10.1093/protein/gzl033

31. Saerens D, Ghassabeh G, Muyldermans S. Single-domain antibodies as building blocks for novel therapeutics. Curr Opin Pharmacol (2008) 8:600-608. doi:10.1016/j.coph.2008.07.006

32. Hmila I, Saerens D, Ben Abderrazek R, Vincke C, Abidi N, Benlasfar Z, et al. A bispecific nanobody to provide full protection against lethal scorpion envenoming. FASEB J (2010) 24:3479-3489. doi:10.1096/fj.09-148213

33. Conrath KE, Wernery U, Muyldermans S, Nguyen VK. Emergence and evolution of functional heavy-chain antibodies in Camelidae. Dev Comp Immunol (2003) 27:87-103. doi:10.1016/S0145-305X(02)00071-X

34. Chen J, He Q, Xu Y, Fu J, Li Y, Tu Z, et al. Nanobody medicated immunoassay for ultrasensitive detection of cancer biomarker alpha-fetoprotein. Talanta (2016) 147:523-530. doi:10.1016/j.talanta.2015.10.027

35. Ebrahimizadeh W, Mousavi Gargari S, Rajabibazl M, Safaee Ardekani L, Zare H, Bakherad H. Isolation and characterization of protective anti-LPS nanobody against V. cholerae O1 recognizing Inaba and Ogawa serotypes. Appl Microbiol Biotechnol (2013) 97:4457-4466. doi: 10.1007/s00253-012-4518-x

36. De Meyer T, Muyldermans S, Depicker A. Nanobody-based products as research and diagnostic tools. Trends Biotechnol (2014) 32:263-270.

37. Huang W, Chiquet-Ehrismann R, Moyano JV, Garcia-Pardo A, Orend G. Interference of Tenascin-C with Syndecan-4 Binding to Fibronectin Blocks Cell Adhesion and Stimulates Tumor Cell Proliferation. Cancer Res (2001) 61: 8586-8594.

38. Degen M, Brellier F, Kain R, Ruiz C, Terracciano L, Orend G, et al. Tenascin-W is a novel marker for activated tumor stroma in low-grade human breast cancer and influences cell behavior. Cancer Res (2007) 67: 9169-9179.

39. Ehrismann R, Chiquet M, Turner DC. Mode of action of fibronectin in promoting chicken myoblast attachment. The Journal of Biological Chemistry (1981) 256: 4056-4062.

40. Fischer D, Brown-Lüdi M, Schulthess T, Chiquet-Ehrismann R. Concerted action of tenascin-C domains in cell adhesion, anti-adhesion and promotion of neurite outgrowth. Journal of Cell Science (1997)110: 1513-1522.

41. Fischer D, Chiquet-Ehrismann R, Bernasconi C, Chiquet M. A single heparin binding region within fibrinogen-like domain is functional in chick tenascin-C (1995) 270:3378-3384.

42. Vincke C, Gutiérrez C, Wernery U, Devoogdt N, Hassanzadeh-Ghassabeh G, Muyldermans S. "Generation of Single Domain Antibody Fragments Derived from Camelids and Generation of Manifold Constructs," in Antibody Engineering, ed. P. Chames (Totowa, NJ: Humana Press), 145-176. doi:10.1007/978-1-61779-974-7_8

43. Abderrazek RB, Hmila I, Vincke C, Benlasfar Z, Pellis M, Dabbek H, et al. Identification of potent nanobodies to neutralize the most poisonous polypeptide from scorpion venom. Biochem J (2009) 424:263-272. doi:10.1042/BJ20090697

44. Dhaouadi S, Murdamoothoo D, Tounsi A, Erne W, Benabderrazek R, Benlasfar Z, et al. Generation and characterization of dromedary Tenascin-C and Tenascin-W specific antibodies. Biochem Biophys Res Commun (2020) 530:471-478. doi:10.1016/j.bbrc.2020.05.077

45. Gerlza T, Hecher B, Jeremic D, Fuchs T, Gschwandtner M, Falsone A, et al. A Combinatorial Approach to Biophysically Characterise Chemokine-Glycan Binding Affinities for Drug Development. Molecules (2014) 19:10618-10634. doi:10.3390/molecules190710618

46. Westman J, Hansen FC, Olin AI, Mörgelin M, Schmidtchen A, Herwald H. p33 (gC1q Receptor) Prevents Cell Damage by Blocking the Cytolytic Activity of Antimicrobial Peptides. J Immunol (2013) 191:5714-5721. doi:10.4049/jimmunol.1300596

47. Baschong W, Wrigley NG. Small colloidal gold conjugated to fab fragments or to immunoglobulin g as high-resolution labels for electron microscopy: A technical overview. J Electron Microsc Tech (1990) 14:313-323. doi:10.1002/jemt.1060140405

48. Schenk S, Muser J, Vollmer G, Chiquet-Ehrismann R. Tenascin-C in serum: A questionable tumor marker. Int J Cancer (1995) 61:443-449. doi:10.1002/ijc.2910610402

49. Rupp T, Langlois B, Koczorowska MM, Radwanska A, Sun Z, Hussenet T, et al. Tenascin-C Orchestrates Glioblastoma Angiogenesis by Modulation of Pro- and Anti-angiogenic Signaling. Cell Rep (2016) 17:2607-2619. doi:10.1016/j.celrep.2016.11.012

50. Weitzner B, Jeliazkov J, Lyskov S, Marze N, Kuroda D, Frick R et al. Modeling and docking of antibody structures with Rosetta. Nat Protoc (2017) 12: 401-416. doi.org/10.1038/nprot.2016.180

51. Ksouri A, Ghedira K, Ben Abderrazek R, Shankar Gowri BA, Benkahla A, Bishop Tastan O et al. Homology modeling and docking of AahII-Nanobody complexes reveal the epitope binding site on AahII scorpion toxin. Biochemical and Biophysical Research Communications (2018) 496: 1025-1032. doi.org/10.1016/j.bbrc.2018.01.036

52. Pierce BG, Wiehe K, Hwang H, Kim BH. Vreven T, Weng Z. ZDOCK server: interactive docking prediction of protein-protein complexes and symmetric multimers. Bioinformatics (2014) 30:1771-1773.doi.org/10.1093/ bioinformatics/btu097.

53. Schrödinger LLC. 2010. The PyMOL Molecular Graphics System, Version 1.3r1. Portland. Oregon: Schrödinger, LLC.

54. Hmila I, Ben Abdallah R, Saerens D, Benlasfar Z, Conrath K, Ayeb ME, et al. VHH, bivalent domains and chimeric Heavy chain-only antibodies with high neutralizing efficacy for scorpion toxin AahI'. Mol Immunol (2008) 45:3847-3856. doi:10.1016/j.molimm.2008.04.011

55. Spenlé C, Lefebvre O, Lacroute J, Méchine-Neuville A, Barreau F, Blottière HM, et al. The Laminin Response in Inflammatory Bowel Disease: Protection or Malignancy? PLoS ONE (2014) 9:e111336. doi:10.1371/journal.pone.0111336

56. Kawamura T, Yamamoto M, Suzuki K, Suzuki Y, Kamishima M, Sakata M, et al. Tenascin-C Produced by Intestinal Myofibroblasts Promotes Colitis-associated Cancer Development Through Angiogenesis. Inflamm Bowel Dis (2019) 25:732-741. doi:10.1093/ibd/izy368

57. Ning L, Li S, Gao J, Ding L, Wang C, Chen W, et al. Tenascin-C Is Increased in Inflammatory Bowel Disease and Is Associated with response to Infliximab Therapy. BioMed Res Int (2019) 2019:1-9. doi:10.1155/2019/1475705

58. Sun Z, Schwenzer A, Rupp T, Murdamoothoo D, Vegliante R, Lefebvre O, et al. Tenascin-C Promotes Tumor Cell Migration and Metastasis through Integrin α9β1-Mediated YAP Inhibition. Cancer Res (2018) 78:950-961. doi:10.1158/0008-5472.CAN-17-1597

59. Venning FA, Wullkopf L, Erler JT. Targeting ECM Disrupts Cancer Progression. Front Oncol (2015) 5: doi:10.3389/fonc.2015.00224

60. Genova C, Rijavec E, Grossi F. Tumor microenvironment as a potential source of clinical biomarkers in non-small cell lung cancer: can we use enemy territory at our advantage? J Thorac Dis (2017) 9:4300-4304. doi: 10.21037/jtd.2017.10.66

61. Orend G, Chiquet-Ehrismann R. Tenascin-C induced signaling in cancer. Cancer Lett (2006) 244:143-163. doi:10.1016/j.canlet.2006.02.017

62. Midwood KS, Chiquet M, Tucker RP, Orend G. Tenascin-C at a glance. J Cell Sci (2016) 129:4321-4327. doi:10.1242/jcs.190546

63. Akabani G, Reardon DA, Coleman RE, Wong TZ, Metzler SD, Bowsher JE, et al. Dosimetry and Radiographic Analysis of 131I-Labeled Anti$\chi$ Tenascin 81C6 Murine Monoclonal Antibody in Newly Diagnosed Patients with Malignant Gliomas: A Phase II Study. 11.

64. Reardon DA, Zalutsky MR, Bigner DD. Antitenascin-C monoclonal antibody radioimmunotherapy for malignant glioma patients. Expert Rev Anticancer Ther (2007) 7:675-687. doi:10.1586/14737140.7.5.675

65. Schliemann C, Wiedmer A, Pedretti M, Szczepanowski M, Klapper W, Neri D. Three clinical-stage tumor targeting antibodies reveal differential expression of oncofetal fibronectin and tenascin-C isoforms in human lymphoma. Leuk Res (2009) 33:1718-1722. doi:10.1016/j.leukres.2009.06.025

66. Ko HY, Choi K-J, Lee CH, Kim S. A multimodal nanoparticle-based cancer imaging probe simultaneously targeting nucleolin, integrin αvβ3 and tenascin-C proteins. Biomaterials (2011) 32:1130-1138. doi:10.1016/j.biomaterials.2010.10.034

67. Spenlé C, Gasser I, Saupe F, Janssen K-P, Arnold C, Klein A, et al. Spatial organization of the tenascin-C microenvironment in experimental and human cancer. Cell Adhes Migr (2015) 9:4-13. doi:10.1080/19336918.2015.1005452
68. Carnemolla B, Castellani P, Ponassi M, Borsi L, Urbini S, Nicolo G, et al. Identification of a Glioblastoma-Associated Tenascin-C Isoform by a High Affinity Recombinant Antibody. Am J Pathol (1999) 154(5):1345-1352. doi: 10.1016/S0002-9440(10)65388-6
69. Paganelli G, Magnani P, Zito F, Lucignani G, Sudati F, Truci G, et al. Pre-targeted immunodetection in glioma patients: tumour localization and single-photon emission tomography imaging of [99mTc]PnAO-biotin. Eur J Nucl Med (1994) 21:314-321. doi: 10.1007/BF00947966
70. Riva P, Franceschi G, Frattarelli M, Riva N, Guiducci G, Cremonini AM, et al. 1311 radioconjugated antibodies for the locoregional radioimmunotherapy of high-grade malignant glioma--phase I and II study. Acta Oncol Stockh Swed (1999) 38:351-359. doi: 10.1080/028418699431438
71. Catania C, Maur M, Berardi R, Rocca A, Giacomo AMD, Spitaleri G, et al. The tumor-targeting immunocytokine F16-IL2 in combination with doxorubicin: dose escalation in patients with advanced solid tumors and expansion into patients with metastatic breast cancer. Cell Adhes Migr (2015) 9:14-21. doi:10.4161/19336918.2014.983785
72. De Braud FG, Catania C, Onofri A, Pierantoni C, Cascinu S, Maur M, et al. Combination of the immunocytokine F16-IL2 with doxorubicin or paclitaxel in patients with solid tumors: Results from two phase Ib trials. J Clin Oncol (2011) 29:2595-2595. doi:10.1200/jco.2011.29.15_suppl.2595
73. Kovacs JA, Vogel S, Albert JM, Falloon J, Davey RT, Walker RE, et al. Controlled trial of interleukin-2 infusions in patients infected with the human immunodeficiency virus. N Engl J Med (1996) 335:1350-1356. doi:10.1056/NEJM199610313351803
74. Behar G, Siberil S, Groulet A, Chames P, Pugniere M, Boix C, et al. Isolation and characterization of anti-Fc Rill (CD16) llama single-domain antibodies that activate natural killer cells. Protein Eng Des Sel (2007) 21:1-10. doi:10.1093/protein/gzm064
75. Jailkhani N, Ingram JR, Rashidian M, Rickelt S, Tian C, Mak H, et al. Noninvasive imaging of tumor progression, metastasis, and fibrosis using a nanobody targeting the extracellular matrix. Proc Natl Acad Sci (2019) 116:14181-14190. doi:10.1073/pnas.1817442116
76. Hynes, R.O. (2019) Nanobody Based Imaging and Targeting of ECM in Disease and Development. U.S. Patent No 20190225693. Massachusetts, MA :U.S. Patent and Trademark office.
77. Li T, Huang M, Xiao H, Zhang G, Ding J, Wu P, et al. Selection and characterization of specific nanobody against bovine virus diarrhea virus (BVDV) E2 protein. PloS One (2017) 12:e0178469. doi: 10.1371/journal.pone.0178469
78. Wan R, Liu A, Hou X, Lai Z, Li J, Yang N, et al. Screening and antitumor effect of an anti-CTLA-4 nanobody. Oncol Rep (2017) 39(2): 511-518. doi:10.3892/or.2017.6131
79. Lefranc M-P, Pommié C, Ruiz M, Giudicelli V, Foulquier E, Truong L, et al. IMGT unique numbering for immunoglobulin and T cell receptor variable domains and Ig superfamily V-like domains. Dev Comp Immunol (2003) 27:55-77. doi: 10.1016/S0145-305X (02)00039-3
80. Noël F, Malpertuy A, de Brevern AG. Global analysis of VHHs framework regions with a structural alphabet. Biochimie (2016) 131:11-19. doi:10.1016/j.biochi.2016.09.005
81. De Laporte L, Rice JJ, Tortelli F, Hubbell JA. Tenascin C Promiscuously Binds Growth Factors via Its Fifth Fibronectin Type III-Like Domain. PLoS ONE (2013) 8:e62076. doi:10.1371/journal.pone.0062076
82. Saga Y, Tsukamoto T, Jing N, Kusakabe M, Sakakura T. Mouse tenascin: cDNA cloning, structure and temporal expression of isoforms. Gene (1991). 104:177-185. doi.org/10.1016/0378-1119 (91)90248-A
83. https://france.promega.com/products/vectors/protein-expression-vectors/
84. WO 83/004261
85. Merrifield (19962) Proc. Soc. e.g. Boil.21:412; Merrifield (1963) J. Am. Chem. Soc. 85:2149; Tarn et al (1983) J. Am. Chem.Soc. 105:6442)
86. Surasak Jittavisutthikul et al, 2015 Humanized-VHH transbodies that inhibit HCV protease and replication" . 2015 Apr 20;7(4):2030-56
87. Harmsen et al., 2007 Properties, production, and applications of camelid single-domain antibody fragments, 77:13-22, DOI : doi.org/10.1007/s00253-007-1142-2
88. Hultberg et al, 2011, Llama-derived single domain antibodies to build multivalent, superpotent and broadened neutralizing anti-viral molecules 6(4): e17665, DOI : 10.1371/journal.pone.0017665
89. Schepens et al.,2011. "Nanobodies specific for respiratory Syncytial virus fusion protein protect against infection by inhibition of fusion"2011 J Infect Dis. 2011 Dec 1;204 (11):1692-701. doi: 10.1093/infdis/jir622. PMID: 21998474
90. Thys et al, 2010 In vitro antiviral activity of single domain antibody fragments against poliovirus Antiviral Res.2010 Aug;87(2):257-64.doi:10.1016/j.antiviral.2010.05.012. PMID: 20566349
91. Sebastian et al., 2012. Rotavirus A-specific single-domain antibodies produced in baculovirus-infected insect larvae are protective in vivo. BMC Biotech 2012;12:59. doi: 10.1186/1472-6750-12-59. PMCID: PMC3444942 PMID:

22953695

92. Forsman et al, 2008 Llama antibody fragments with cross-subtype human immunodeficiency virus type 1 (HIV-1)-neutralizing properties and high affinity for HIV-1 gp120, Journal of Virology 2008;82(24):12096-081. doi:10.1128/JVI.01379-08

93. Verccruysse et al., 2010 An intrabody based on a llama single-domain antibody targeting the N-terminal α-helical multimerization domain of HIV-1 Rev prevents viral production, J Biol Chem 2010 Jul 9;285(28):21768-80. doi: 10.1074/jbc.M110.112490. PMCID: PMC2898381. PMID: 20406803

94. Bouchet et al., 2011. "Inhibition of the Nef regulatory protein of HIV-1 by a single-domain antibody". Blood 2011,117(13): 3559-68. doi.org/10.1182/blood-2010-07-296749

95. Jahnichen et al, 2010 "CXCR4 nanobodies (VHH-based single variable domains) potently inhibit chemotaxis and HIV-1 replication and mobilize stem cells" Proceedings of the National Academy of Sciences Nov 2010, 107 (47) 20565-20570; DOI: 10.1073/pnas.1012865107

96. Abderrazek, R.B., et al., 2009. Identification of potentnanobodies to neutralize the most poisonous polypeptide from scorpion venom.Biochem. J. 424, 263-272

97. Fei Fei et al. 2017.Strategies for preparing Albumin-based Nanoparticles for Multifunctional Bioimaging and Drug Delivery 2017;7(15):3667-89

98. Oliveira et al,2010.Downregulation of EGFR by a novel multivalent nanobody-liposome platform.2010;145(2):165-75

## Claims

1. An isolated single-domain antibody directed against Tenascin-C (TNC) wherein said single-domain antibody comprises the amino acid sequences:

   i) GYTNSIYT (SEQ ID NO: 1) as variable heavy (VHH) chain complementarity determining region (CDR)1,
   ii) I$X_a$SRNGNT (SEQ ID NO: 2) as variable heavy (VHH) chain complementarity determining region (CDR)2, wherein $X_a$ is G or A
   iii) AAGSSWDLILQAYAYDY (SEQ ID NO: 3) as variable heavy (VHH) chain complementarity determining region (CDR)3.

2. An isolated single-domain antibody directed against Tenascin-C according to claim 1 wherein said isolated single-domain antibody comprise the amino acid sequences selected from the group consisting of WLQLVESGGGSVQTGGSLRLSCVVSGYTNSIYTLAWFRQAPGKEREGV AAIGSRNGNTYYDASVKDRFTISLDKAKNTVYLQMNDLKPEDTASYYCAA GSSWDLILQAYAYDYWGQGTQVTVSS (SEQ ID NO: 4) or WVQLVESGGGSVQTGGSLRLSCVVSGYTNSIYTLAWFRQAPGKEREGV AAIASRNGNTYYDASVKDRFTISLDKAKNTVYLQMNGLKPEDTASYYCAA GSSWDLILQAYAYDYWGQGTQVTVSS (SEQ ID NO: 5).

3. An isolated single-domain antibody directed against Tenascin-C according to claim 1 or 2 wherein said single-domain antibody consist of the amino acid sequence selected from the group consisting of WLQLVESGGGSVQTGGSLRLSCVVSGYTNSIYTLAWFRQAPGKEREGV AAIGSRNGNTYYDASVKDRFTISLDKAKNTVYLQMNDLKPEDTASYYCAA GSSWDLILQAYAYDYWGQGTQVTVSS (SEQ ID NO: 4) or WVQLVESGGGSVQTGGSLRLSCVVSGYTNSIYTLAWFRQAPGKEREGV AAIASRNGNTYYDASVKDRFTISLDKAKNTVYLQMNGLKPEDTASYYCAA GSSWDLILQAYAYDYWGQGTQVTVSS (SEQ ID NO: 5).

4. An immunoconjugate comprising:

   (a) an isolated single-domain antibody directed against Tenascin-C (TNC) according to any one of claim 1 to 3; and
   (b) a conjugating part selected from the group consisting of a detectable marker, drug, toxin, cytokine, radionuclide, enzyme, gold nanoparticle/nanorod, albumin nanoparticle magnetic nanoparticle, transductor, PolyEthylenGlycol (PEG)-Liposome and a combination thereof

**5.** An isolated nucleic acid that encodes an isolated single-domain antibody directed against Tenascin-C according to any one of claims 1-3.

**6.** An expression vector comprising the isolated nucleic acid of claim 5.

**7.** A host cell comprising a nucleic acid according to claim 5 or an expression vector according to claim 6.

**8.** The host cell of claim 7 which is CHO cell.

**9.** A pharmaceutical composition comprising a single-domain antibody directed against Tenascin-C according to any of claims 1 to 3, and a pharmaceutically acceptable carrier.

**10.** A single-domain antibody directed against Tenascin-C according to any of claims 1 to 3 for use as medicament.

**11.** A single-domain antibody directed against Tenascin-C according to any of claims 1 to 3 or an immunoconjugate according to claim 4 for use in the treatment of cancer, fibrosis, inflammation, viral or bacterial infections.

**12.** A method of producing a single-domain antibody directed against Tenascin-C according to any of claims 1 - 3 comprising culturing the host cell according to claim 7 or 8 and recovering single-domain antibody directed against Tenascin-C from the cell culture.

**13.** A single-domain antibody directed against Tenascin-C according to any of claims 1 to 3 or an immunoconjugate according to claim 4, for use in an *in vitro* or *in vivo* diagnostic or imaging method.

**14.** In vitro use of single-domain antibody directed against Tenascin-C according to any of claims 1 to 3 or an immunoconjugate according to claim 4 for detecting a tumor cell in a biological sample.

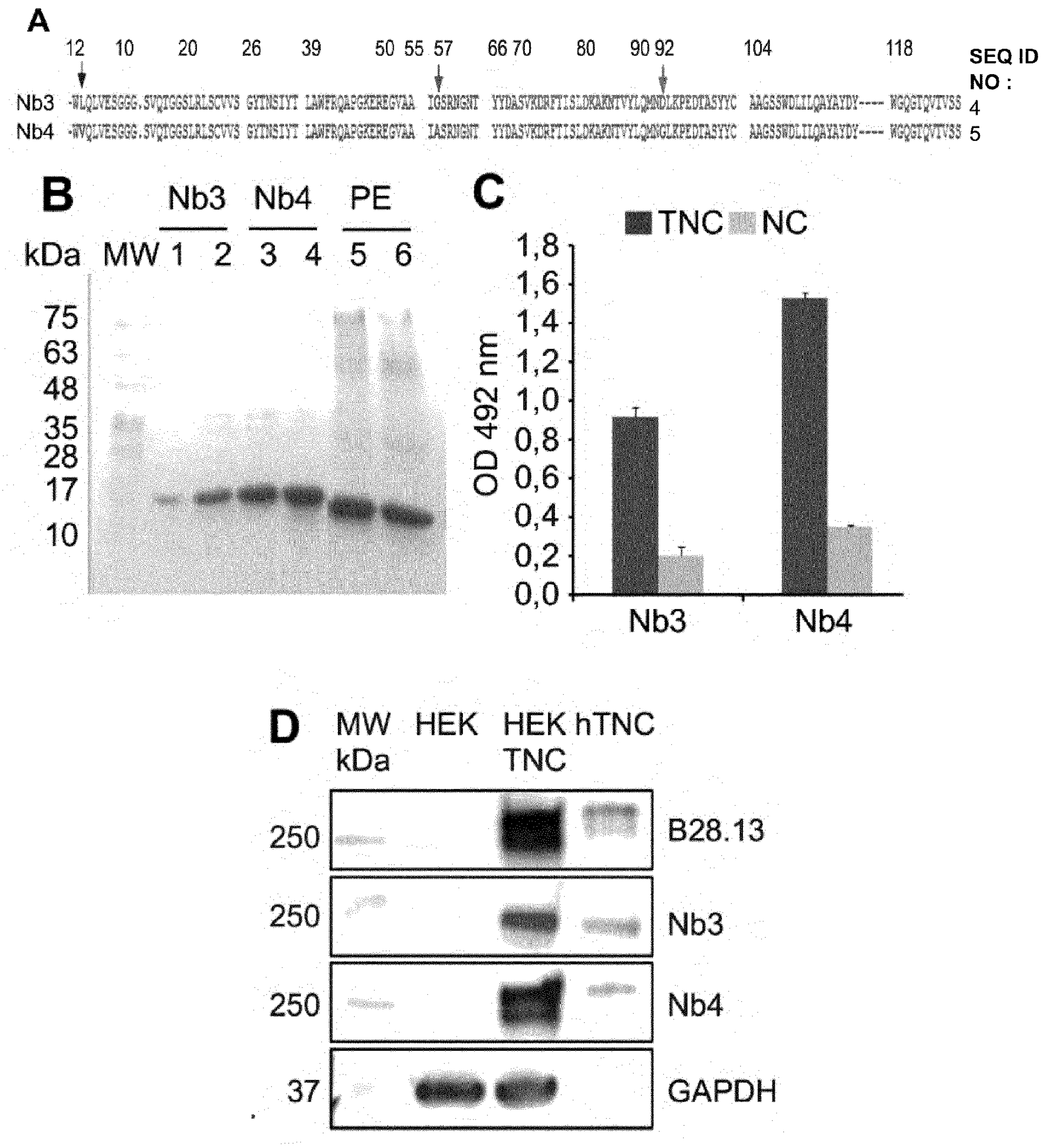
A
12 10 20 26 39 50 55 57 66 70 80 90 92 104 118
SEQ ID NO :
Nb3 -WLQLVESGGG.SVQTGGSLRLSCVVS GYTNSIYT LAWFRQAPGKEREGVAA IGSRNGNT YYDASVKDRFTISLDKAKNTVYLQMNDLKPEDTASYYC AAGSSWDLILQAYAYDY---- WGQGTQVTVSS 4
Nb4 -WVQLVESGGG.SVQTGGSLRLSCVVS GYTNSIYT LAWFRQAPGKEREGVAA IASRNGNT YYDASVKDRFTISLDKAKNTVYLQMNGLKPEDTASYYC AAGSSWDLILQAYAYDY---- WGQGTQVTVSS 5
B
Nb3 Nb4 PE
kDa MW 1 2 3 4 5 6
75
63
48
35
28
17
10
C
TNC NC
OD 492 nm
1,8
1,6
1,4
1,2
1,0
0,8
0,6
0,4
0,2
0,0
Nb3
Nb4
D
MW kDa
HEK
HEK TNC
hTNC
250
250
250
37
B28.13
Nb3
Nb4
GAPDH

FIGURE 1 (1/2)

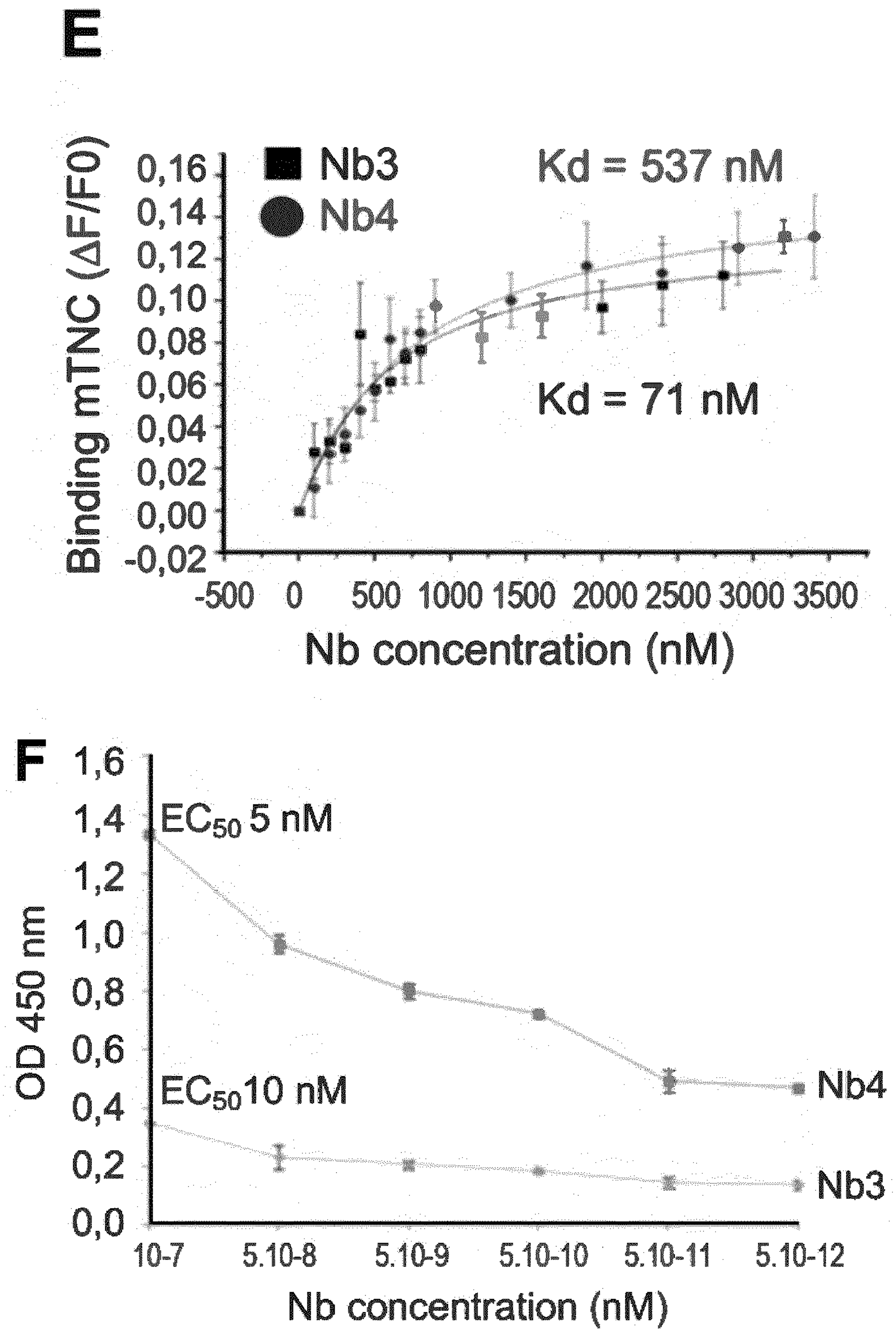
E
Binding mTNC (ΔF/F0)
0,16
0,14
0,12
0,10
0,08
0,06
0,04
0,02
0,00
-0,02
Nb3
Nb4
Kd = 537 nM
Kd = 71 nM
-500 0 500 1000 1500 2000 2500 3000 3500
Nb concentration (nM)
F
OD 450 nm
1,6
1,4
1,2
1,0
0,8
0,6
0,4
0,2
0,0
EC50 5 nM
EC50 10 nM
Nb4
Nb3
10-7 5.10-8 5.10-9 5.10-10 5.10-11 5.10-12
Nb concentration (nM)

FIGURE 1(2/2)

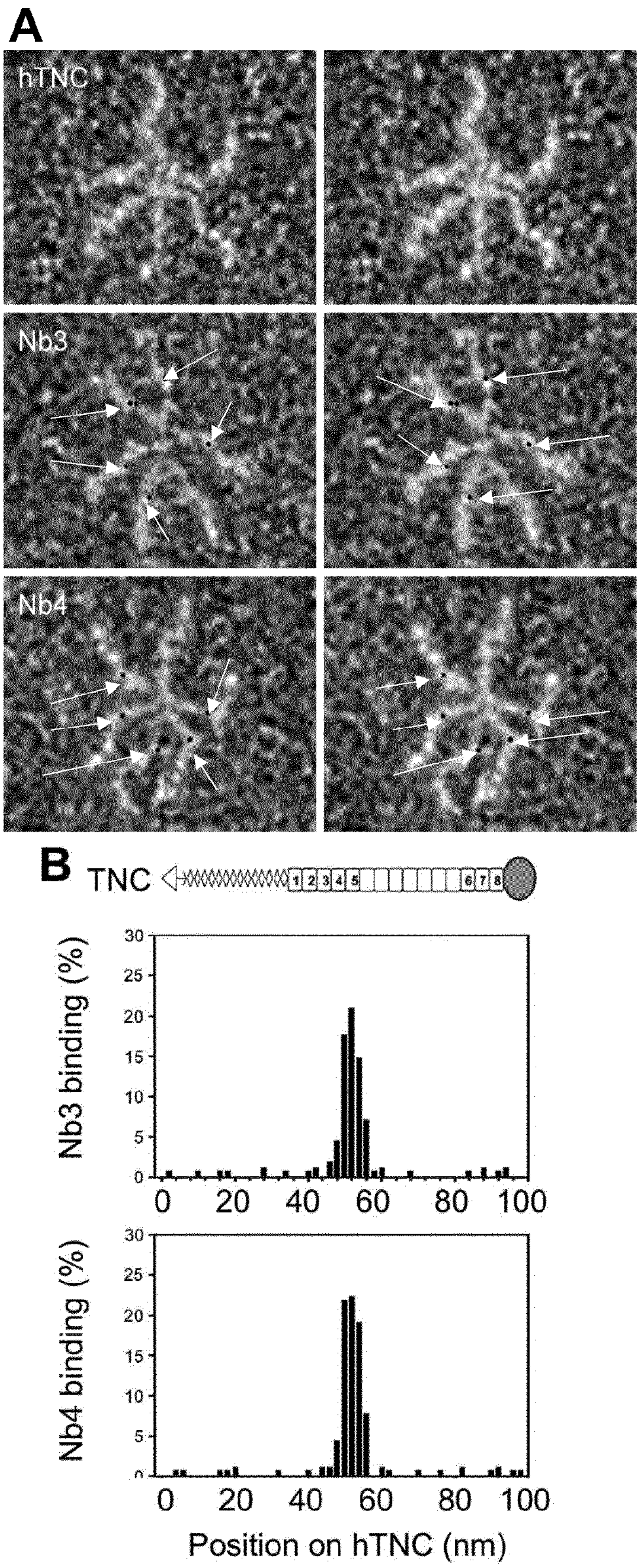
A
hTNC
Nb3
Nb4
B
TNC
1 2 3 4 5 6 7 8
Nb3 binding (%)
0 5 10 15 20 25 30
0 20 40 60 80 100
Nb4 binding (%)
0 5 10 15 20 25 30
0 20 40 60 80 100
Position on hTNC (nm)

FIGURE 2

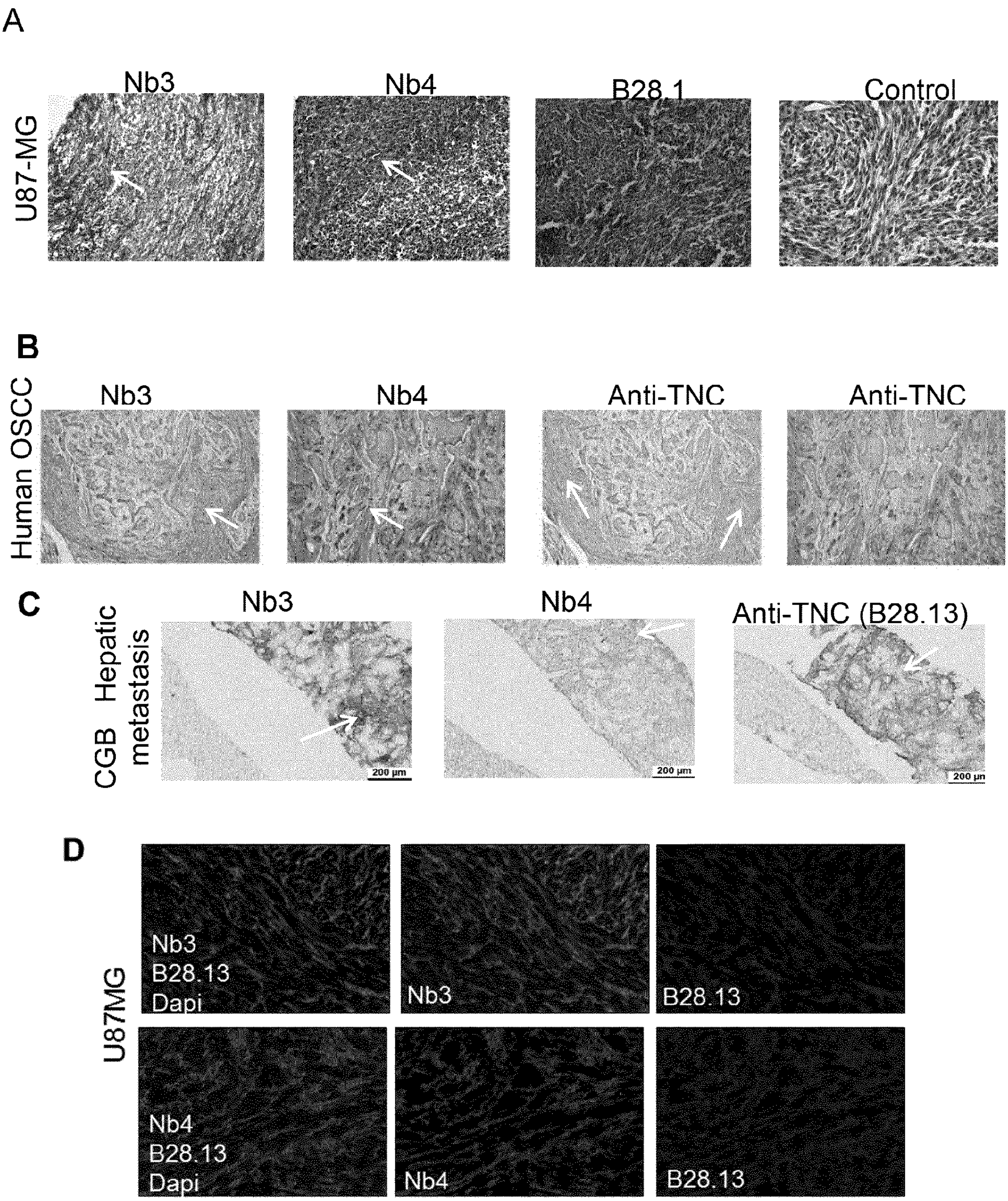
A
U87-MG
Nb3
Nb4
B28.1
Control
B
Human OSCC
Nb3
Nb4
Anti-TNC
Anti-TNC
C
CGB Hepatic metastasis
Nb3
Nb4
Anti-TNC (B28.13)
200 µm
200 µm
200 µm
D
U87MG
Nb3
B28.13
Dapi
Nb3
B28.13
Nb4
B28.13
Dapi
Nb4
B28.13

FIGURE 3 (1/2)

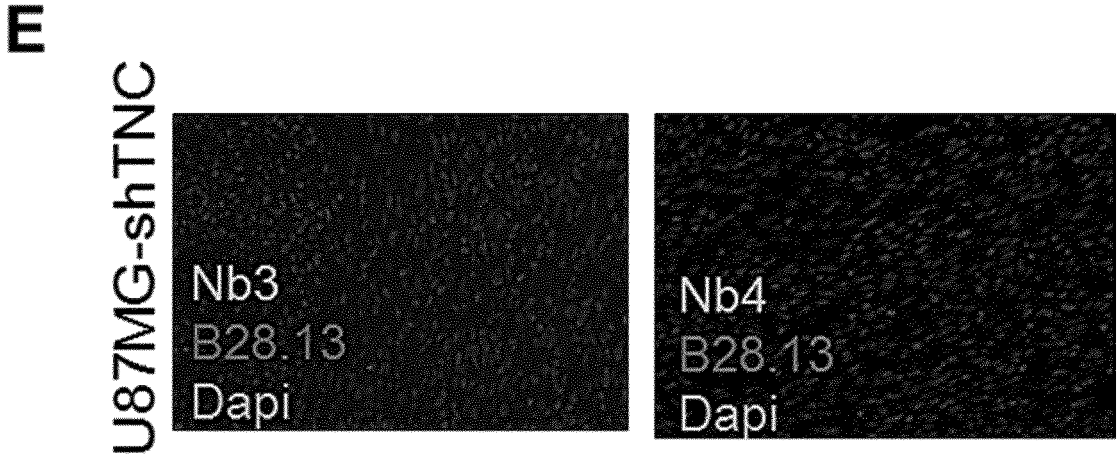


FIGURE 3 (2/2)

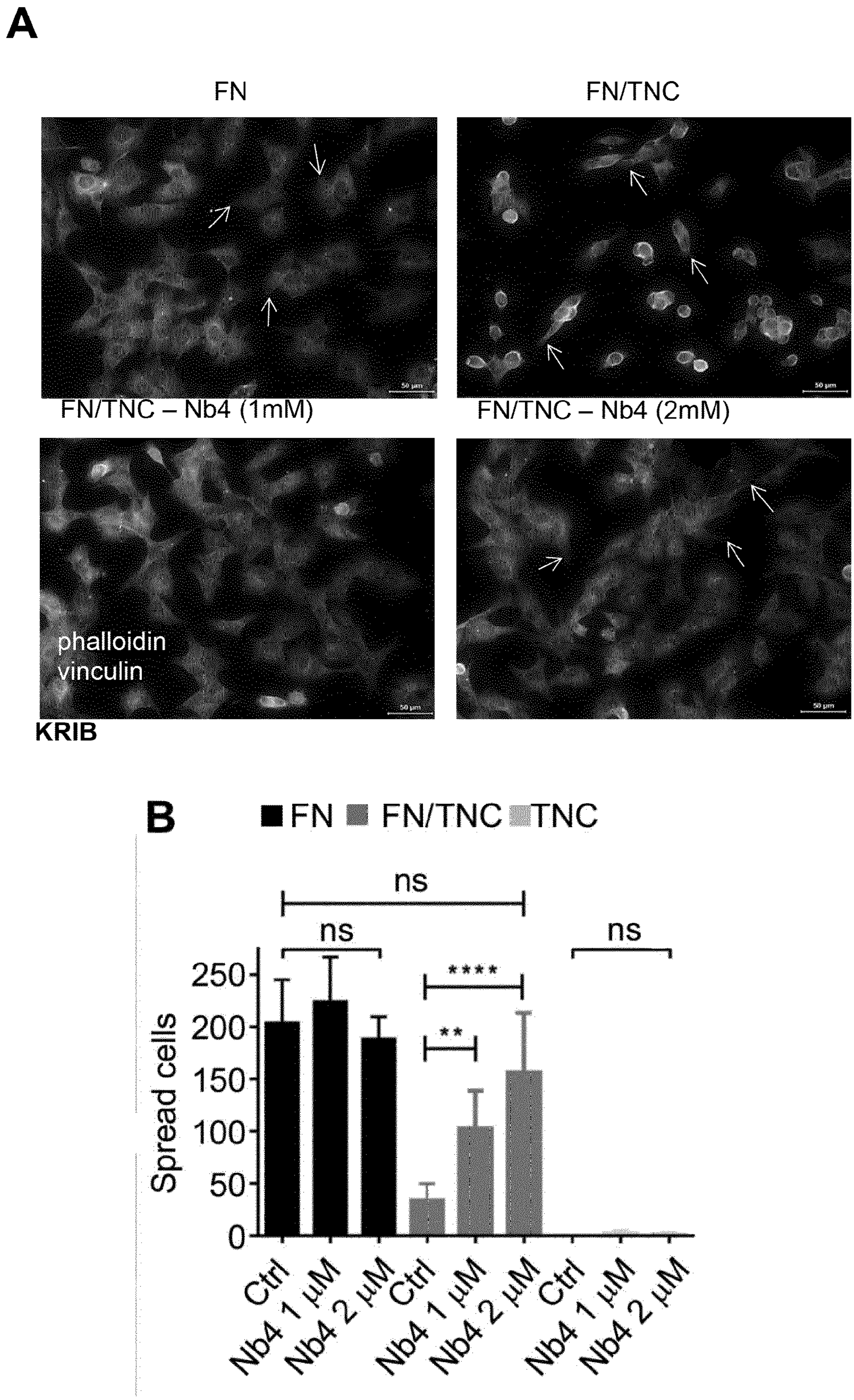
A
FN
FN/TNC
FN/TNC – Nb4 (1mM)
FN/TNC – Nb4 (2mM)
phalloidin
vinculin
KRIB
B
FN
FN/TNC
TNC
ns
ns
****
**
ns
Spread cells
250
200
150
100
50
0
Ctrl
Nb4 1 µM
Nb4 2 µM
Ctrl
Nb4 1 µM
Nb4 2 µM
Ctrl
Nb4 1 µM
Nb4 2 µM

FIGURE 4 (1/3)

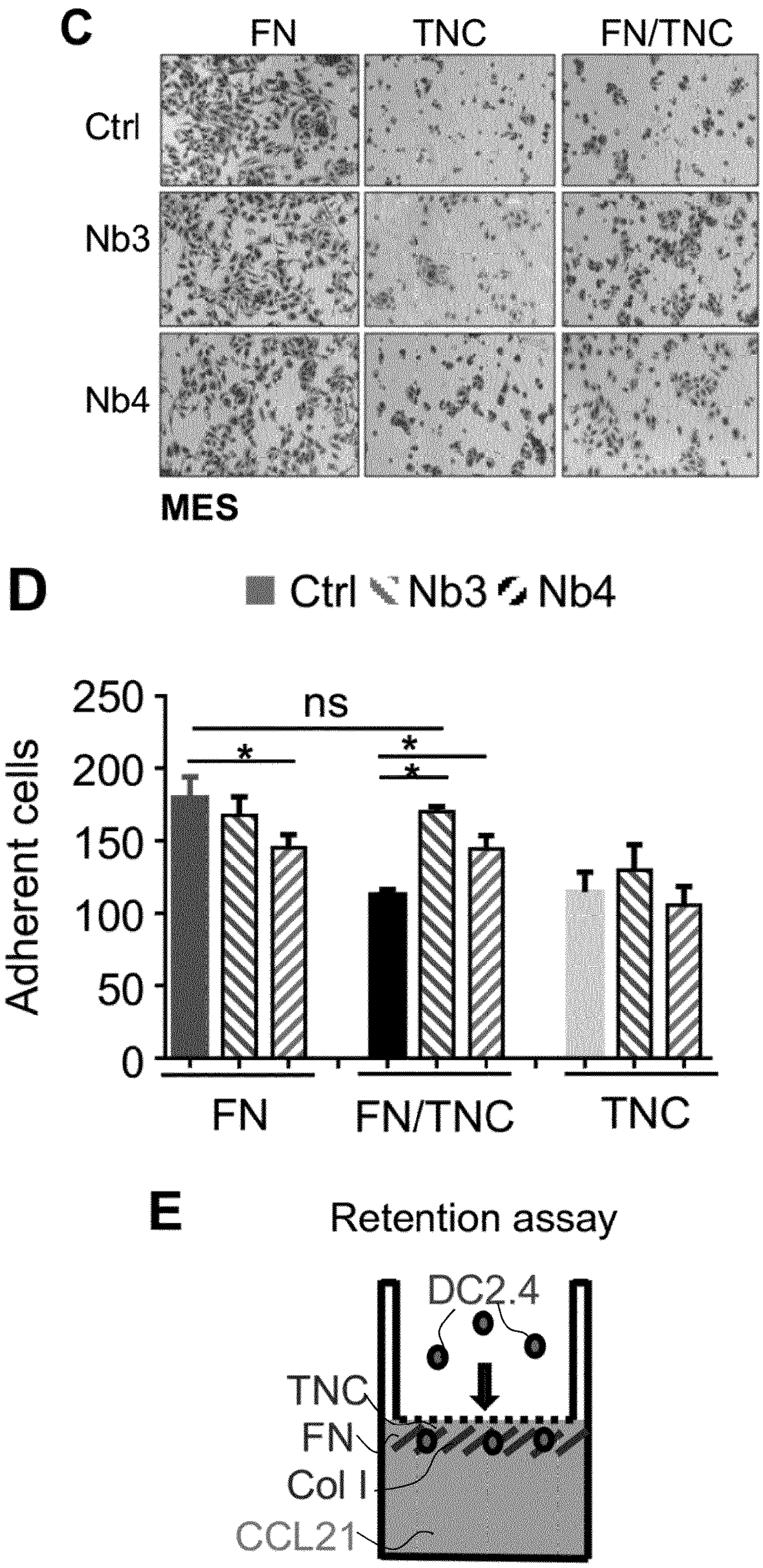
C
FN
TNC
FN/TNC
Ctrl
Nb3
Nb4
MES
D
Ctrl Nb3 Nb4
Adherent cells
250
200
150
100
50
0
ns
*
*
*
FN
FN/TNC
TNC
E
Retention assay
DC2.4
TNC
FN
Col I
CCL21

FIGURE 4 (2/3)

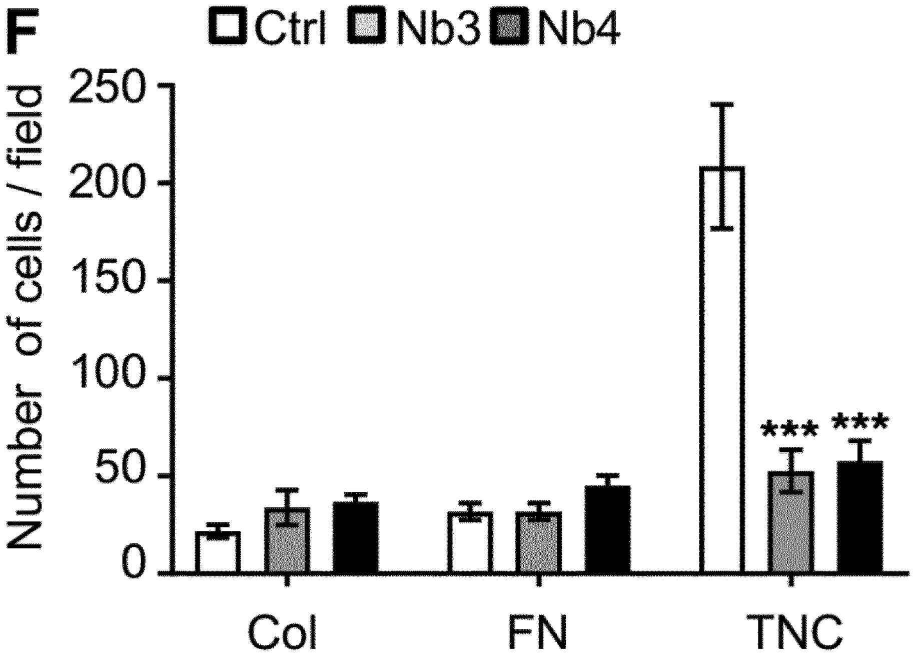
F
Ctrl
Nb3
Nb4
Number of cells / field
250
200
150
100
50
0
***
***
Col
FN
TNC

FIGURE 4 (3/3)

## Figure 5

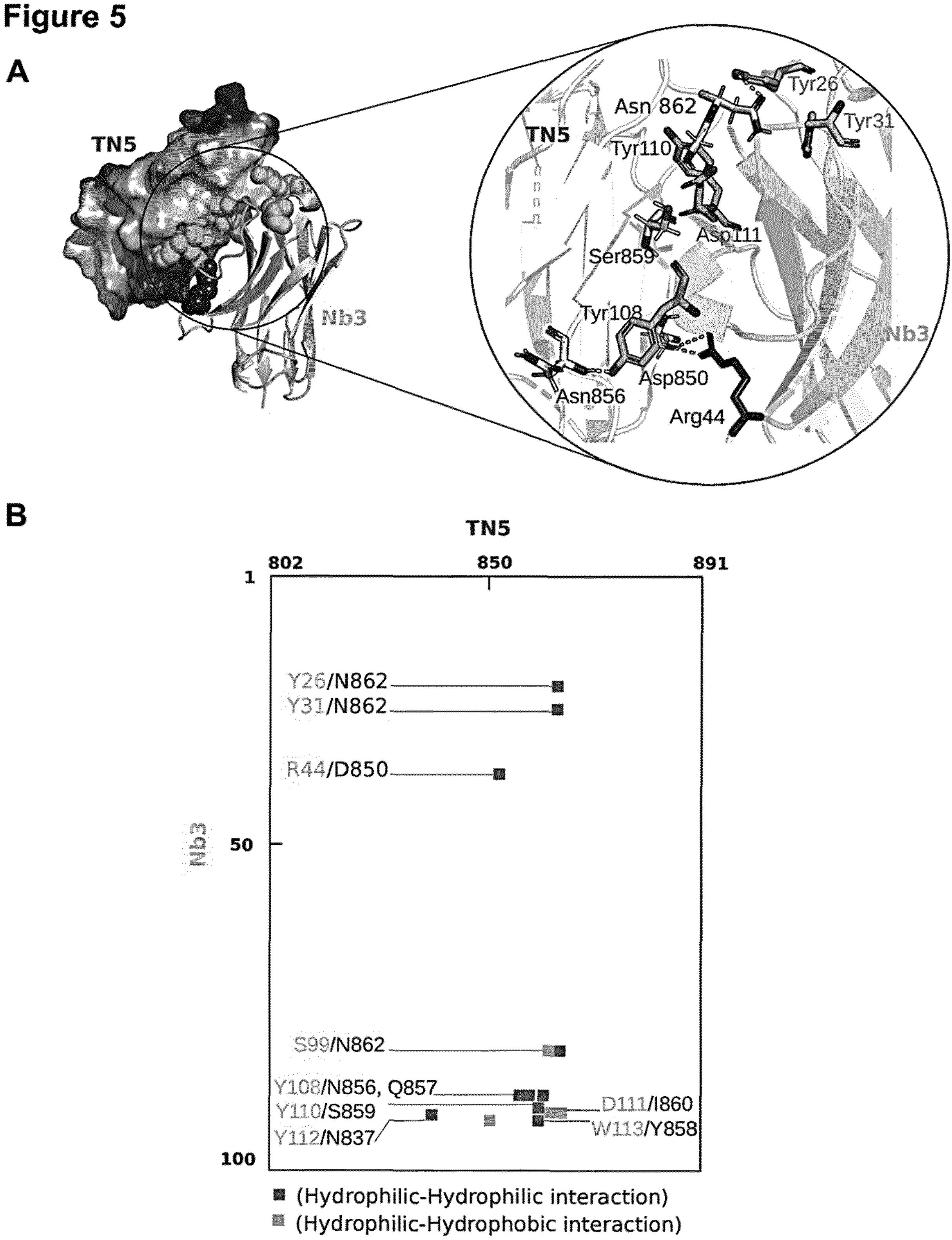


FIGURE 5

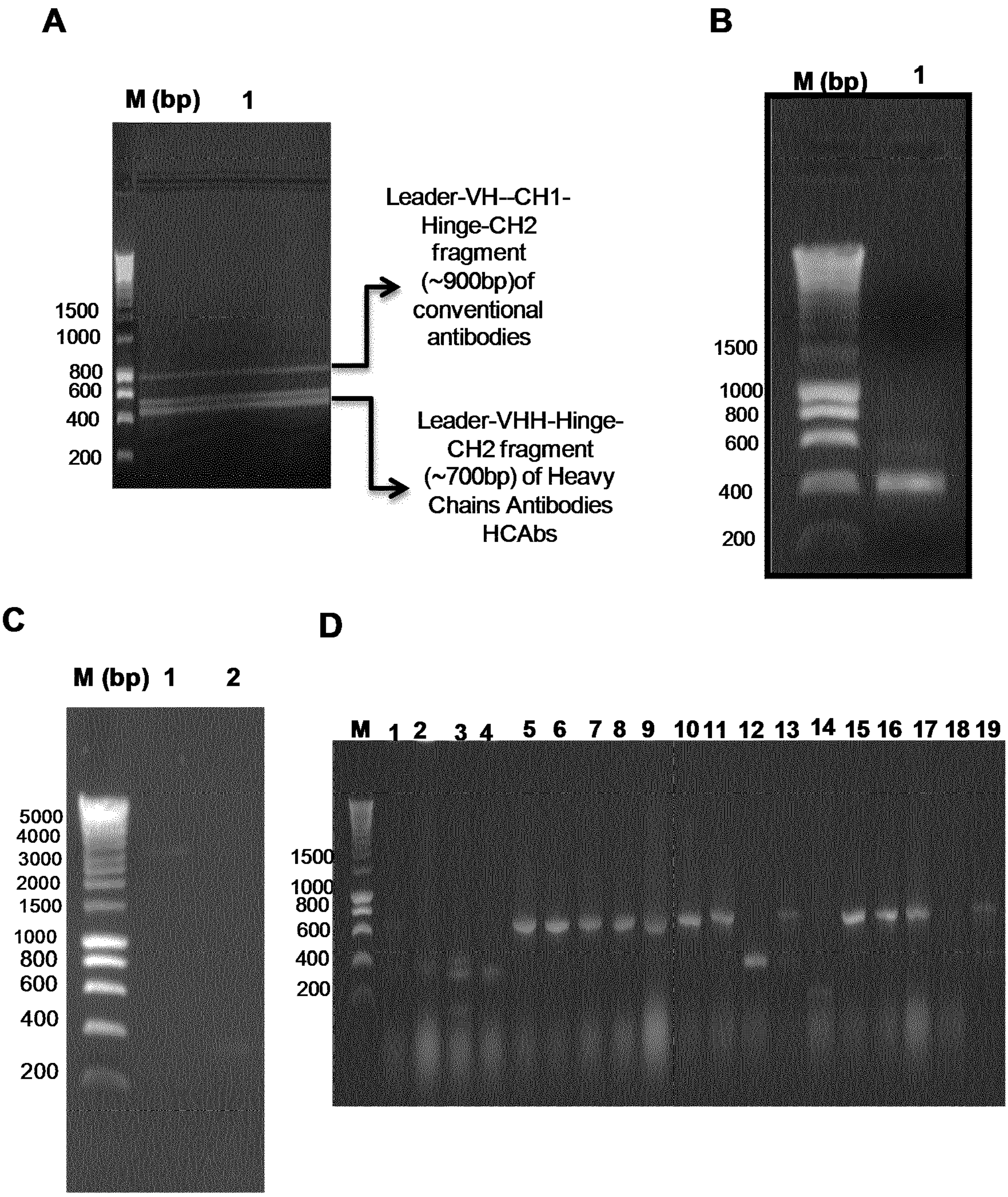
A
M (bp)
1
1500
1000
800
600
400
200
Leader-VH--CH1-Hinge-CH2 fragment (~900bp)of conventional antibodies
Leader-VHH-Hinge-CH2 fragment (~700bp) of Heavy Chains Antibodies HCAbs
B
M (bp)
1
1500
1000
800
600
400
200
C
M (bp)
1
2
5000
4000
3000
2000
1500
1000
800
600
400
200
D
M
1 2 3 4 5 6 7 8 9 10 11 12 13 14 15 16 17 18 19
1500
1000
800
600
400
200

FIGURE 6 (1/2)

E

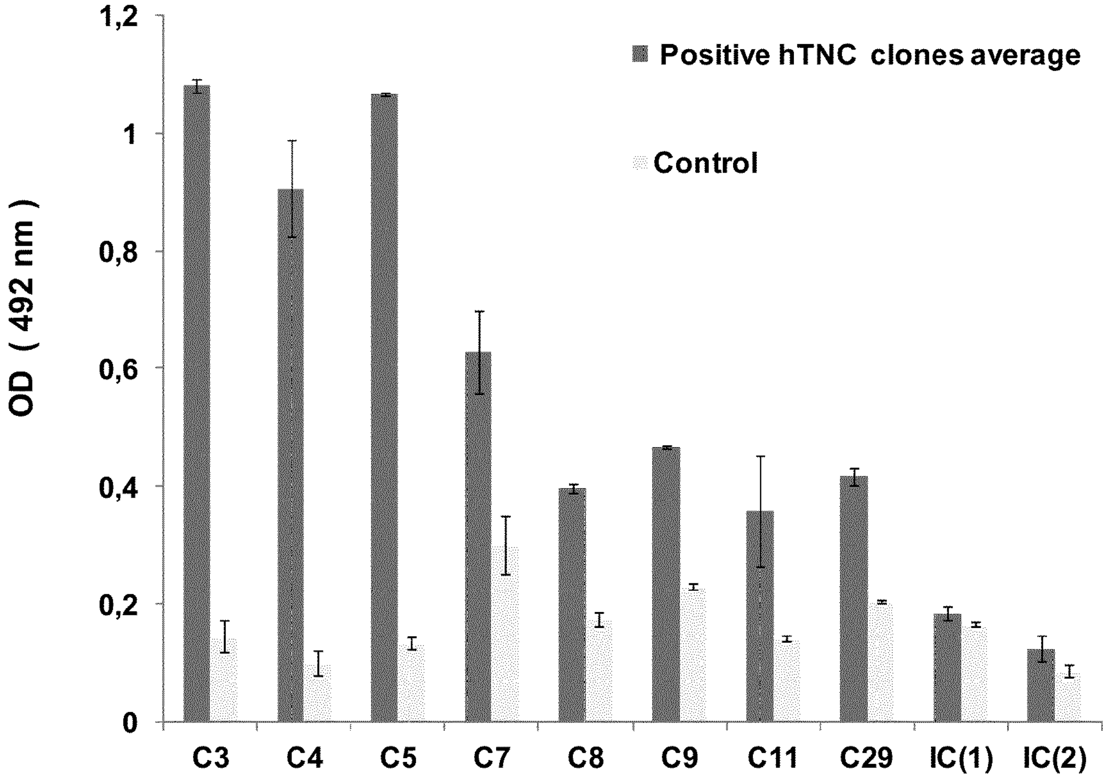


F

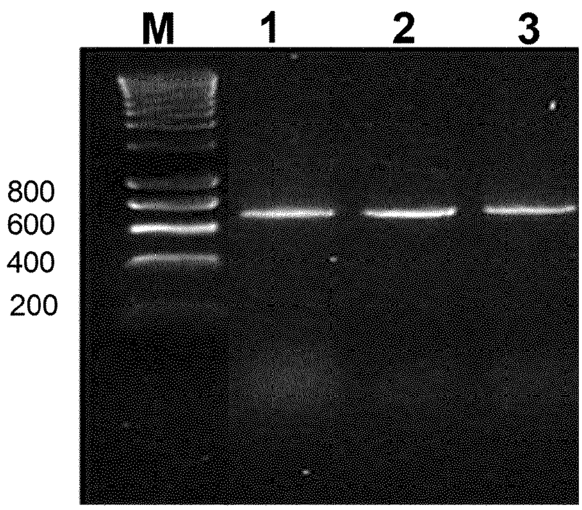


FIGURE 6 (2/2)

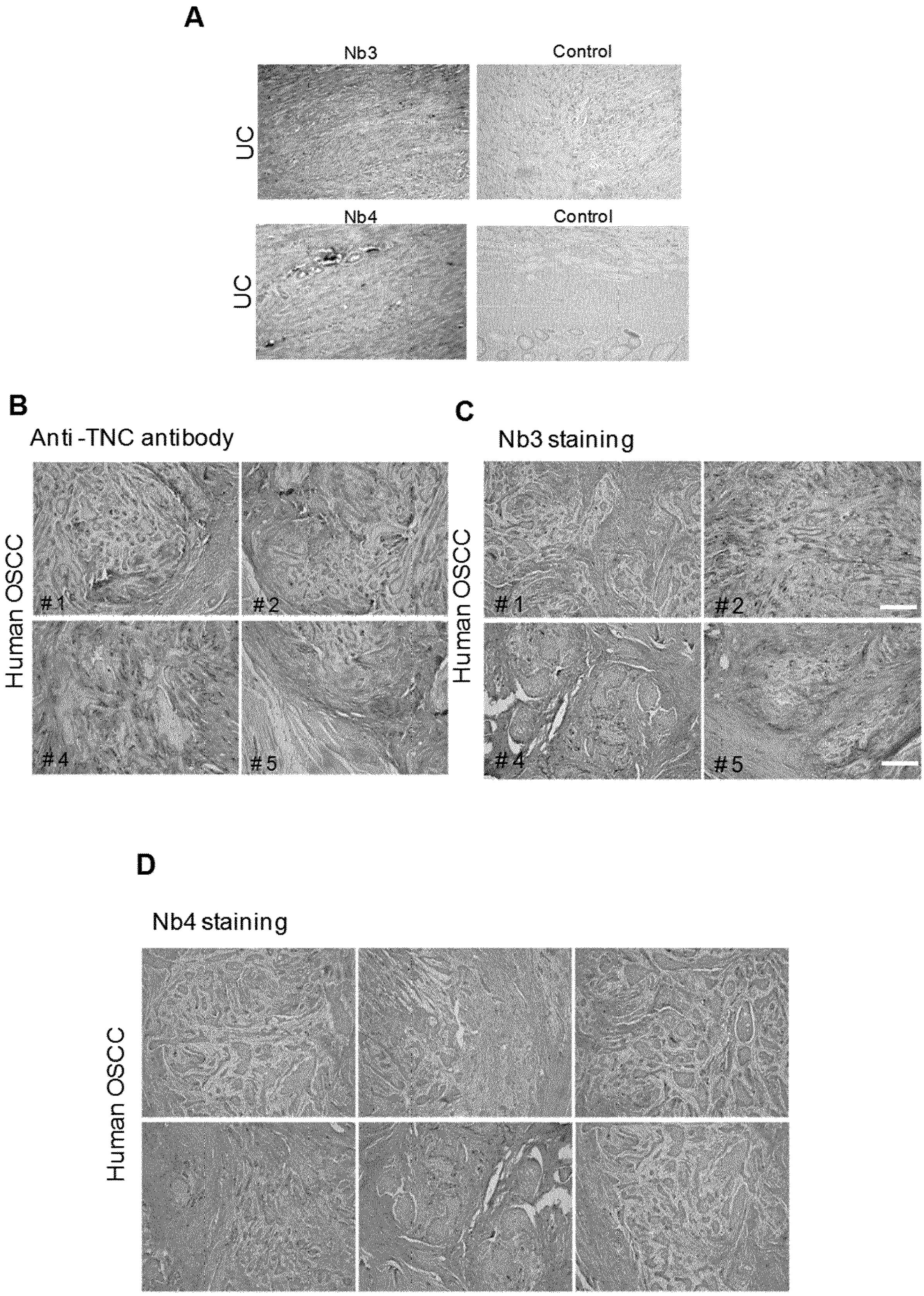
A
Nb3
Control
UC
Nb4
Control
UC
B
Anti -TNC antibody
Human OSCC
#1
#2
#4
#5
C
Nb3 staining
Human OSCC
#1
#2
#4
#5
D
Nb4 staining
Human OSCC

FIGURE 7

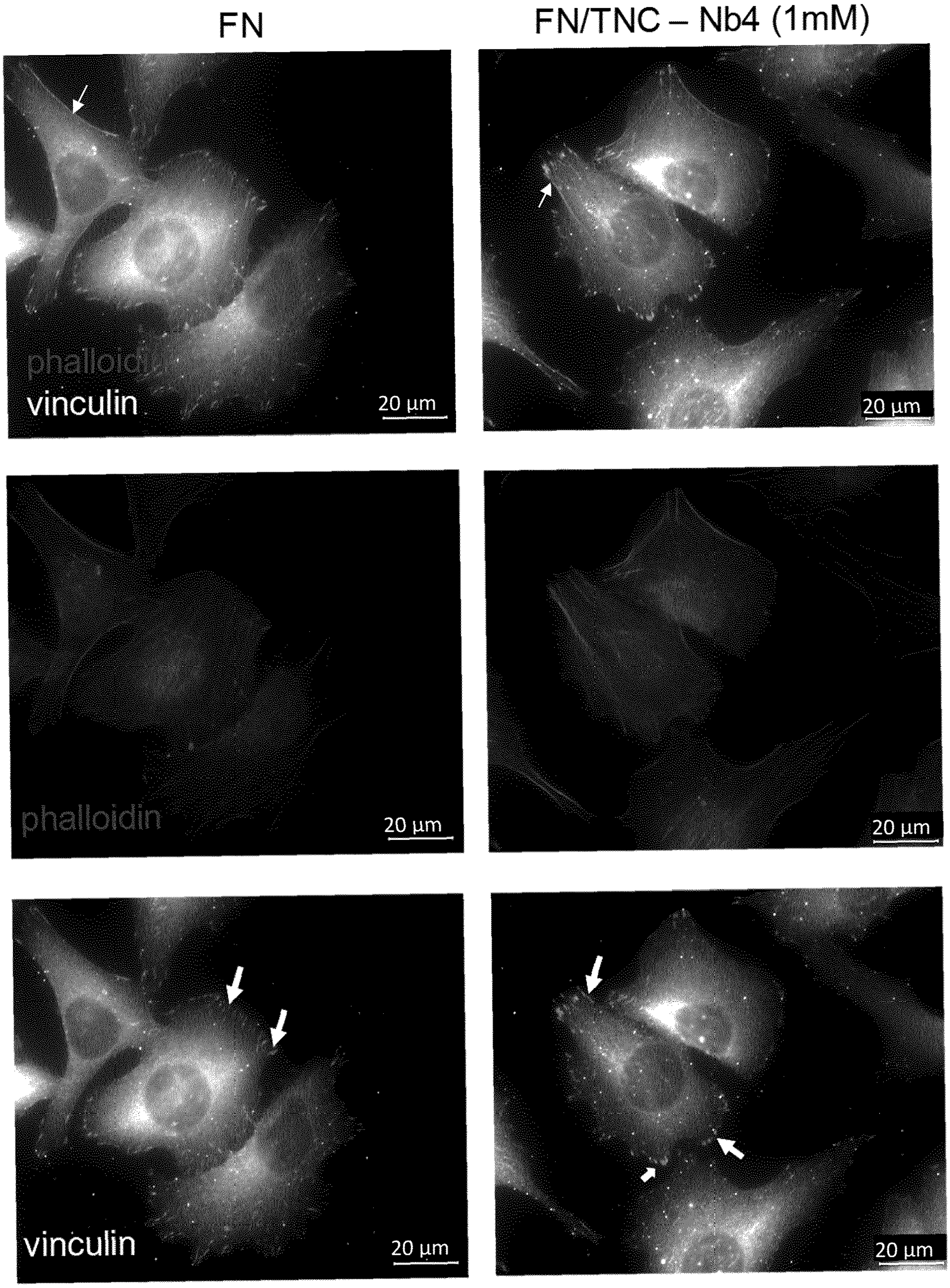
FN
FN/TNC – Nb4 (1mM)
phalloidin
vinculin
20 µm
20 µm
20 µm
phalloidin
20 µm
vinculin
20 µm
20 µm

FIGURE 8

Europäisches Patentamt
European Patent Office
Office européen des brevets

# EUROPEAN SEARCH REPORT

Application Number
EP 21 30 5210

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2019/225693 A1 (HYNES RICHARD O [US] ET AL) 25 July 2019 (2019-07-25)<br>* whole document, especially Example 5; Figure 14 *<br>----- | 11<br>1-10, 12-14 | INV.<br>C07K16/18<br>G01N33/574<br><br>ADD.<br>A61K39/395<br>A61P35/00<br>A61P35/04 |
| T | DHAOUADI SAYDA ET AL: "Novel Human Tenascin-C Function-Blocking Camel Single Domain Nanobodies",<br>FRONTIERS IN IMMUNOLOGY,<br>vol. 12, no. 635166,<br>15 March 2021 (2021-03-15), pages 1-15,<br>XP055824689,<br>DOI: 10.3389/fimmu.2021.635166<br>* the whole document *<br>----- | 1-14 | |
| | | | TECHNICAL FIELDS SEARCHED (IPC) |
| | | | C07K<br>A61K |

The present search report has been drawn up for all claims

1

| Place of search | Date of completion of the search | Examiner |
|---|---|---|
| The Hague | 16 July 2021 | Luyten, Kattie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 30 5210

This annex lists the patent family members relating to the patent documents cited in the above-mentioned European search report. The members are as contained in the European Patent Office EDP file on
The European Patent Office is in no way liable for these particulars which are merely given for the purpose of information.

16-07-2021

| Patent document cited in search report | Publication date | Patent family member(s) | Publication date |
|---|---|---|---|
| US 2019225693 A1 | 25-07-2019 | CN 112218890 A | 12-01-2021 |
| | | EP 3743443 A1 | 02-12-2020 |
| | | JP 2021512071 A | 13-05-2021 |
| | | US 2019225693 A1 | 25-07-2019 |
| | | WO 2019148037 A1 | 01-08-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 83004261 A
- US 4816567 A
- US 5908626 A
- US 20190225693 A


### Non-patent literature cited in the description


- **PARKINSON J1 ; BLAXTER M.** Expressed sequence tags: an overview. *Methods Mol Biol.,* 2009, vol. 533, 1-12
- **MERRIFIELD.** *Proc. Soc. e.g. Boil.,* vol. 21, 412
- **MERRIFIELD.** *J. Am. Chem. Soc.,* 1963, vol. 85, 2149
- **TARN et al.** *J. Am. Chem.Soc.,* 1983, vol. 105, 6442
- **HMILA I et al.** *FASEB J,* 2010, vol. 24, 3479-3489
- **VINCKE C et al.** Antibody Engineering. Humana Press, 145-176
- **ABDERRAZEK RB et al.** *Biochem J,* 2009, vol. 424, 263-272
- **SURASAK JITTAVISUTTHIKUL et al.** *Humanized-VHH transbodies that inhibit HCV protease and replication,* 20 April 2015, vol. 7 (4), 2030-56
- **SCHEPENS et al.** Nanobodies specific for respiratory Syncytial virus fusion protein protect against infection by inhibition of fusion. *2011 J Infect Dis.,* 01 December 2011, vol. 204 (11), 1692-701
- **HULTBERG et al.** *Poliovirus Nanobodies,* 2011
- **SEBASTIAN et al.** Rotavirus A-specific single-domain antibodies produced in baculovirus-infected insect larvae are protective in vivo. *BMC Biotech,* 2012, vol. 2012 (12), 59
- **JAHNICHEN et al.** CXCR4 nanobodies (VHH-based single variable domains) potently inhibit chemotaxis and HIV-1 replication and mobilize stem cells. *Proceedings of the National Academy of Sciences,* November 2010, vol. 107 (47), 20565-20570
- **SHENG et al.** Nanobody-horseradish peroxidase fusion protein as an ultrasensitive probe to detect antibodies against Newcastle disease virus in the immunoassay. *NanoBiotechnol,* 2019, vol. 17, 35
- **BERLIN O ; SAMID D ; DONTHINENI-RAO R ; AKESON W ; AMIEL D ; WOODS VL.** Development of a novel spontaneous metastasis model of human osteosarcoma transplanted orthotopically into bone of athymic mice. *Cancer Res,* 1993, vol. 53, 4890-5
- **SARRAB RM et al.** Establishment of conditionally immortalized human glomerular mesangial cells in culture, with unique migratory properties. *Am J Physiol Renal Physiol,* 2011, vol. 301 (5), 1131-1138
- **TAKEDA A ; OTANI Y ; ISEKI H, TAKEUCHI H ; AIKAWA K ; TABUCHI S et al.** Clinical Significance of Large Tenascin-C Spliced Variant as a Potential Biomarker for Colorectal Cancer. *World J Surg,* 2007, vol. 31, 388-394
- **HANCOX RA ; ALLEN MD ; HOLLIDAY DL ; EDWARDS DR ; PENNINGTON CJ ; GUTTERY DS et al.** Tumour-associated tenascin-C isoforms promote breast cancer cell invasion and growth by matrix metalloproteinase-dependent and independent mechanisms. *Breast Cancer Res,* 2009, 11
- **ALHARTH AS ; ALYAMI WA.** Tenascin-C (TNC) Promotes Breast Cancer Cell Invasion and Proliferation: Functional Effects of TNC Knockdown in Highly Invasive Breast Cancer Cell Lines. *Am J Med Biol Res Am J Med Biol Res,* 2016, vol. 3, 55-61
- **SHEN C ; WANG C ; YIN Y ; CHEN H ; YIN X ; CAI Z et al.** *Tenascin-C expression is significantly associated with the progression and prognosis in gastric GISTs: Medicine (Baltimore),* 2019, vol. 98, e14045
- **ISHIHARA A ; YOSHIDA T ; TAMAKI H ; SAKAKURA T.** *Tenascin expression in cancer cells and stroma of human breast cancer and its prognostic significance: Clin Cancer Res,* 1995, vol. 9, 1035-1041
- **LEINS A ; RIVA P ; LINDSTEDT R ; DAVIDOFF MS ; MEHRAEIN P ; WEIS S.** Expression of tenascin-C in various human brain tumors and its relevance for survival in patients with astrocytoma. *Cancer,* 2003, vol. 98, 2430-2439

- **CHIQUET-EHRISMANN R ; HAGIOS C ; SCHENK S.** The complexity in regulating the expression of tenascins. *BioEssays,* 1995, vol. 17, 873-878
- **ERICKSON HP ; BOURDON MA.** Tenascin: An Extracellular Matrix Protein Prominent in Specialized Embryonic Tissues and Tumors. *Cell Biol,* 1989, vol. 5, 71-92
- **CHIQUET-EHRISMANN R ; OREND G ; CHIQUET M ; TUCKER RP ; MIDWOOD KS.** Tenascins in stem cell niches. *Matrix Biol,* 2014, vol. 37, 112-123
- **CHIQUET-EHRISMANN R ; CHIQUET M.** Tenascins: regulation and putative functions during pathological stress: Tenascins in pathological stress. *J Pathol,* 2003, vol. 200, 488-499
- **MIDWOOD KS ; OREND G.** The role of tenascin-C in tissue injury and tumorigenesis. *J Cell Commun Signal,* 2009, vol. 3, 287-310
- **SAUPE F ; SCHWENZER A ; JIA Y ; GASSER I ; SPENLÉ C ; LANGLOIS B et al.** Tenascin-C Downregulates Wnt Inhibitor Dickkopf-1, Promoting Tumorigenesis in a Neuroendocrine Tumor Model. *Cell Rep,* 2013, vol. 5, 482-492
- **SUN Z ; VELÁZQUEZ-OUESADA I ; MURDAMOOTHOO D ; AHOWESSO C ; YILMAZ A ; SPENLÉ C et al.** Tenascin-C increases lung metastasis by impacting blood vessel invasions. *Matrix Biol,* 2019, vol. 83, 26-47
- **OSKARSSON T ; ACHARYYA S ; ZHANG XH-F ; VANHARANTA S ; TAVAZOIE SF ; MORRIS PG et al.** Breast cancer cells produce tenascin C as a metastatic niche component to colonize the lungs. *Nat Med,* 2011, vol. 17, 867-874
- **LOWY CM ; OSKARSSON T.** Tenascin C in metastasis: A view from the invasive front. *Cell Adhes Migr,* 2015, vol. 9, 112-124
- **LANGLOIS B ; SAUPE F ; RUPP T ; ARNOLD C ; VAN DER HEYDEN M ; OREND G et al.** AngioMatrix, a signature of the tumor angiogenic switch-specific matrisome, correlates with poor prognosis for glioma and colorectal cancer patients. *Oncotarget,* 2014, vol. 5 (21), 10529-10545
- **BELLONE M ; CAPUTO S ; JACHETTI E.** Immunosuppression via Tenascin-C. *Oncoscience,* 2015, vol. 2, 667
- **JACHETTI E ; CAPUTO S ; MAZZOLENI S ; BRAMBILLASCA CS ; PARIGI SM ; GRIONI M et al.** Tenascin-C Protects Cancer Stem-like Cells from Immune Surveillance by Arresting T-cell Activation. *Cancer Res,* 2015, vol. 75, 2095-2108
- **DELIGNE C ; MURDAMOOTHOO D ; GAMMAGE AN ; GSCHWANDTNER M ; ERNE W ; LOUSTAU T et al.** Matrix-Targeting Immunotherapy Controls Tumor Growth and Spread by Switching Macrophage Phenotype. *Cancer Immunol Res,* 2020, vol. 8, 368-382
- **SPENLÉ C ; LOUSTAU T ; MURDAMOOTHOO D ; ERNE W ; BEGHELLI-DE IA FOREST DIVONNE S ; VEBER R et al.** Tenascin-C Orchestrates an Immune-Suppressive Tumor Microenvironment in Oral Squamous Cell Carcinoma. *Cancer Immunol Res,* 2020, vol. 8, 1122-1138
- **HICKE BJ ; MARION C ; CHANG Y-F ; GOULD T ; LYNOTT CK ; PARMA D et al.** Tenascin-C Aptamers Are Generated Using Tumor Cells and Purified Protein. *J Biol Chem,* 2001, vol. 276, 48644-48654
- **ZUKIEL R ; NOWAK S.** Suppression of human brain tumor with interference RNA specific for tenascin-C. *Cancer Biol Ther,* 2006, vol. 5, 1002-1007
- **ROLLE K ; NOWAK S ; WYSZKO E ; NOWAK M ; ZUKIEL R ; PIESTRZENIEWICZ R et al.** Promising human brain tumors therapy with interference RNA intervention (iRNAi). *Cancer Biol Ther,* 2010, vol. 9, 397-407
- **ROLLE K.** miRNA Multiplayers in glioma. From bench to bedside. *Acta Biochim Pol,* 2015, vol. 62, 353-365
- **GRABOWSKA M ; GRZESKOWIAK BF ; SZUTKOWSKI K ; WAWRZYNIAK D ; GTODOWICZ P ; BARCISZEWSKI J et al.** Nano-mediated delivery of doublestranded RNA for gene therapy of glioblastoma multiforme. *PLOS ONE,* 2019, vol. 14, e0213852
- **BRACK SS.** Tumor-Targeting Properties of Novel Antibodies Specific to the Large Isoform of Tenascin-C. *Clin Cancer Res,* 2006, vol. 12, 3200-3208
- **HEUVELING DA ; DE BREE R ; VUGTS DJ ; HUISMAN MC ; GIOVANNONI L ; HOEKSTRA OS et al.** Phase 0 Microdosing PET Study Using the Human Mini Antibody F16SIP in Head and Neck Cancer Patients. *J Nucl Med,* 2013, vol. 54, 397-401
- **ALOJ L ; D'AMBROSIO L ; AURILIO M ; MORISCO A ; FRIGERI F ; CARACO' C et al.** Radioimmunotherapy with Tenarad, a 131I-labelled antibody fragment targeting the extra-domain A1 of tenascin-C, in patients with refractory Hodgkin's lymphoma. *Eur J Nucl Med Mol Imaging,* 2014, vol. 41, 867-877
- **PETRONZELLI F.** Improved Tumor Targeting by Combined Use of Two Antitenascin Antibodies. *Clin Cancer Res,* 2005, vol. 11, 7137s-7145s
- **SILACCI M.** Human monoclonal antibodies to domain C of tenascin-C selectively target solid tumors in vivo. *Protein Eng Des Sel,* 2006, vol. 19, 471-478
- **SAERENS D ; GHASSABEH G ; MUYLDERMANS S.** Single-domain antibodies as building blocks for novel therapeutics. *Curr Opin Pharmacol,* 2008, vol. 8, 600-608

- **HMILA I ; SAERENS D ; BEN ABDERRAZEK R ; VINCKE C ; ABIDI N ; BENLASFAR Z et al.** A bispecific nanobody to provide full protection against lethal scorpion envenoming. *FASEB J,* 2010, vol. 24, 3479-3489
- **CONRATH KE ; WERNERY U ; MUYLDERMANS S ; NGUYEN VK.** Emergence and evolution of functional heavy-chain antibodies in Camelidae. *Dev Comp Immunol,* 2003, vol. 27, 87-103
- **CHEN J ; HE Q ; XU Y ; FU J ; LI Y ; TU Z et al.** Nanobody medicated immunoassay for ultrasensitive detection of cancer biomarker alpha-fetoprotein. *Talanta,* 2016, vol. 147, 523-530
- **EBRAHIMIZADEH W ; MOUSAVI GARGARI S ; RAJABIBAZL M ; SAFAEE ARDEKANI L ; ZARE H ; BAKHERAD H.** Isolation and characterization of protective anti-LPS nanobody against V. cholerae O1 recognizing Inaba and Ogawa serotypes. *Appl Microbiol Biotechnol,* 2013, vol. 97, 4457-4466
- **DE MEYER T ; MUYLDERMANS S ; DEPICKER A.** Nanobody-based products as research and diagnostic tools. *Trends Biotechnol,* 2014, vol. 32, 263-270
- **HUANG W ; CHIQUET-EHRISMANN R ; MOYANO JV ; GARCIA-PARDO A ; OREND G.** Interference of Tenascin-C with Syndecan-4 Binding to Fibronectin Blocks Cell Adhesion and Stimulates Tumor Cell Proliferation. *Cancer Res,* 2001, vol. 61, 8586-8594
- **DEGEN M ; BRELLIER F ; KAIN R ; RUIZ C ; ERRACCIANO L ; OREND G et al.** Tenascin-W is a novel marker for activated tumor stroma in low-grade human breast cancer and influences cell behavior. *Cancer Res,* 2007, vol. 67, 9169-9179
- **EHRISMANN R ; CHIQUET M ; TURNER DC.** Mode of action of fibronectin in promoting chicken myoblast attachment. *The Journal of Biological Chemistry,* 1981, vol. 256, 4056-4062
- **FISCHER D ; BROWN-LÜDI M ; SCHULTHESS T ; CHIQUET-EHRISMANN R.** Concerted action of tenascin-C domains in cell adhesion, anti-adhesion and promotion of neurite outgrowth. *Journal of Cell Science,* 1997, vol. 110, 1513-1522
- **FISCHER D ; CHIQUET-EHRISMANN R ; BERNASCONI C ; CHIQUET M.** A single heparin binding region within fibrinogen-like domain is functional. *chick tenascin-C,* 1995, vol. 270, 3378-3384
- Generation of Single Domain Antibody Fragments Derived from Camelids and Generation of Manifold Constructs. **VINCKE C ; GUTIÉRREZ C ; WERNERY U ; DEVOOGDT N ; HASSANZADEH-GHASSABEH G ; MUYLDERMANS S.** Antibody Engineering. Humana Press
- **ABDERRAZEK RB ; HMILA I ; VINCKE C ; BENLASFAR Z ; PELLIS M ; DABBEK H et al.** Identification of potent nanobodies to neutralize the most poisonous polypeptide from scorpion venom. *Biochem J,* 2009, vol. 424, 263-272
- **DHAOUADI S ; MURDAMOOTHOO D ; TOUNSI A ; ERNE W ; BENABDERRAZEK R ; BENLASFAR Z et al.** Generation and characterization of dromedary Tenascin-C and Tenascin-W specific antibodies. *Biochem Biophys Res Commun,* 2020, vol. 530, 471-478
- **GERLZA T ; HECHER B ; JEREMIC D ; FUCHS T ; GSCHWANDTNER M ; FALSONE A et al.** A Combinatorial Approach to Biophysically Characterise Chemokine-Glycan Binding Affinities for Drug Development. *Molecules,* 2014, vol. 19, 10618-10634
- **WESTMAN J ; HANSEN FC ; OLIN AL ; MÖRGELIN M ; SCHMIDTCHEN A ; HERWALD H.** p33 (gC1q Receptor) Prevents Cell Damage by Blocking the Cytolytic Activity of Antimicrobial Peptides. *J Immunol,* 2013, vol. 191, 5714-5721
- **BASCHONG W ; WRIGLEY NG.** Small colloidal gold conjugated to fab fragments or to immunoglobulin g as high-resolution labels for electron microscopy: A technical overview. *J Electron Microsc Tech,* 1990, vol. 14, 313-323
- **SCHENK S ; MUSER J ; VOLLMER G ; CHIQUET-EHRISMANN R.** Tenascin-C in serum: A questionable tumor marker. *Int J Cancer,* 1995, vol. 61, 443-449
- **RUPP T ; LANGLOIS B ; KOCZOROWSKA MM ; RADWANSKA A ; SUN Z ; HUSSENET T et al.** Tenascin-C Orchestrates Glioblastoma Angiogenesis by Modulation of Pro- and Anti-angiogenic Signaling. *Cell Rep,* 2016, vol. 17, 2607-2619
- **WEITZNER B ; JELIAZKOV J ; LYSKOV S ; MARZE N ; KURODA D ; FRICK R et al.** Modeling and docking of antibody structures with Rosetta. *Nat Protoc,* 2017, vol. 12, 401-416
- **KSOURI A ; GHEDIRA K ; BEN ABDERRAZEK R ; SHANKAR GOWRI BA ; BENKAHLA A ; BISHOP TASTAN O et al.** Homology modeling and docking of AahII-Nanobody complexes reveal the epitope binding site on AahII scorpion toxin. *Biochemical and Biophysical Research Communications,* 2018, vol. 496, 1025-1032
- **PIERCE BG ; WIEHE K ; HWANG H ; KIM BH ; VREVEN T ; WENG Z.** ZDOCK server: interactive docking prediction of protein-protein complexes and symmetric multimers. *Bioinformatics,* 2014, vol. 30, 1771-1773
- The PyMOL Molecular Graphics System. Schrödinger, LLC, 2010
- **HMILA I ; BEN ABDALLAH R ; SAERENS D ; BENLASFAR Z ; CONRATH K ; AYEB ME et al.** VHH, bivalent domains and chimeric Heavy chain-only antibodies with high neutralizing efficacy for scorpion toxin AahI. *Mol Immunol,* 2008, vol. 45, 3847-3856

- **SPENLÉ C ; LEFEBVRE O ; LACROUTE J ; MÉCHINE-NEUVILLE A ; BARREAU F ; BLOTTIÈRE HM et al.** The Laminin Response in Inflammatory Bowel Disease: Protection or Malignancy?. *PLoS ONE,* 2014, vol. 9, e111336
- **KAWAMURA T ; YAMAMOTO M ; SUZUKI K ; SUZUKI Y ; KAMISHIMA M ; SAKATA M et al.** Tenascin-C Produced by Intestinal Myofibroblasts Promotes Colitis-associated Cancer Development Through Angiogenesis. *Inflamm Bowel Dis,* 2019, vol. 25, 732-741
- **NING L ; LI S ; GAO J ; DING L ; WANG C ; CHEN W et al.** Tenascin-C Is Increased in Inflammatory Bowel Disease and Is Associated with response to Infliximab Therapy. *BioMed Res Int,* 2019, vol. 2019, 1-9
- **SUN Z ; SCHWENZER A ; RUPP T ; MURDAMOOTHOO D ; VEGLIANTE R ; LEFEBVRE O et al.** Tenascin-C Promotes Tumor Cell Migration and Metastasis through Integrin $\alpha 9\beta 1$-Mediated YAP Inhibition. *Cancer Res,* 2018, vol. 78, 950-961
- **VENNING FA ; WULLKOPF L ; ERLER JT.** Targeting ECM Disrupts Cancer Progression. *Front Oncol,* 2015, 5
- **GENOVA C ; RIJAVEC E ; GROSSI F.** Tumor microenvironment as a potential source of clinical biomarkers in non-small cell lung cancer: can we use enemy territory at our advantage?. *J Thorac Dis,* 2017, vol. 9, 4300-4304
- **OREND G ; CHIQUET-EHRISMANN R.** Tenascin-C induced signaling in cancer. *Cancer Lett,* 2006, vol. 244, 143-163
- **MIDWOOD KS ; CHIQUET M ; TUCKER RP ; OREND G.** Tenascin-C at a glance. *J Cell Sci,* 2016, vol. 129, 4321-4327
- **REARDON DA ; ZALUTSKY MR ; BIGNER DD.** Antitenascin-C monoclonal antibody radioimmunotherapy for malignant glioma patients. *Expert Rev Anticancer Ther,* 2007, vol. 7, 675-687
- **SCHLIEMANN C ; WIEDMER A ; PEDRETTI M ; SZCZEPANOWSKI M ; KLAPPER W ; NERI D.** Three clinical-stage tumor targeting antibodies reveal differential expression of oncofetal fibronectin and tenascin-C isoforms in human lymphoma. *Leuk Res,* 2009, vol. 33, 1718-1722
- **KO HY ; CHOI K-J ; LEE CH ; KIM S. A.** multimodal nanoparticle-based cancer imaging probe simultaneously targeting nucleolin, integrin $\alpha v\beta 3$ and tenascin-C proteins. *Biomaterials,* 2011, vol. 32, 1130-1138
- **SPENLÉ C ; GASSER I ; SAUPE F ; JANSSEN K-P ; ARNOLD C ; KLEIN A et al.** Spatial organization of the tenascin-C microenvironment in experimental and human cancer. *Cell Adhes Migr,* 2015, vol. 9, 4-13
- **CARNEMOLLA B ; CASTELLANI P ; PONASSI M ; BORSI L ; URBINI S ; NICOLO G et al.** Identification of a Glioblastoma-Associated Tenascin-C Isoform by a High Affinity Recombinant Antibody. *Am J Pathol,* 1999, vol. 154 (5), 1345-1352
- **PAGANELLI G ; MAGNANI P ; ZITO F ; LUCIGNANI G ; SUDATI F ; TRUCI G et al.** Pre-targeted immunodetection in glioma patients: tumour localization and single-photon emission tomography imaging of [99mTc]PnAO-biotin. *Eur J Nucl Med,* 1994, vol. 21, 314-321
- **RIVA P ; FRANCESCHI G ; FRATTARELLI M ; RIVA N ; GUIDUCCI G ; CREMONINI AM et al.** 1311 radioconjugated antibodies for the locoregional radioimmunotherapy of high-grade malignant glioma--phase I and II study. *Acta Oncol Stockh Swed,* 1999, vol. 38, 351-359
- **CATANIA C ; MAUR M ; BERARDI R ; ROCCA A ; GIACOMO AMD ; SPITALERI G et al.** The tumor-targeting immunocytokine F16-IL2 in combination with doxorubicin: dose escalation in patients with advanced solid tumors and expansion into patients with metastatic breast cancer. *Cell Adhes Migr,* 2015, vol. 9, 14-21
- **DE BRAUD FG ; CATANIA C ; ONOFRI A ; PIERANTONI C ; CASCINU S ; MAUR M et al.** Combination of the immunocytokine F16-IL2 with doxorubicin or paclitaxel in patients with solid tumors: Results from two phase Ib trials. *J Clin Oncol,* 2011, vol. 29, 2595-2595
- **KOVACS JA ; VOGEL S ; ALBERT JM ; FALLOON J ; DAVEY RT ; WALKER RE et al.** Controlled trial of interleukin-2 infusions in patients infected with the human immunodeficiency virus. *N Engl J Med,* 1996, vol. 335, 1350-1356
- **BEHAR G ; SIBERIL S ; GROULET A ; CHAMES P ; PUGNIERE M ; BOIX C et al.** Isolation and characterization of anti-Fc RIII (CD16) llama single-domain antibodies that activate natural killer cells. *Protein Eng Des Sel,* 2007, vol. 21, 1-10
- **JAILKHANI N ; INGRAM JR ; RASHIDIAN M ; RICKELT S ; TIAN C ; MAK H et al.** Noninvasive imaging of tumor progression, metastasis, and fibrosis using a nanobody targeting the extracellular matrix. *Proc Natl Acad Sci,* 2019, vol. 116, 14181-14190
- **LI T ; HUANG M ; XIAO H ; ZHANG G ; DING J ; WU P et al.** Selection and characterization of specific nanobody against bovine virus diarrhea virus (BVDV) E2 protein. *PloS One,* 2017, vol. 12, e0178469
- **WAN R ; LIU A ; HOU X ; LAI Z ; LI J ; YANG N et al.** Screening and antitumor effect of an anti-CTLA-4 nanobody. *Oncol Rep,* 2017, vol. 39 (2), 511-518

- **LEFRANC M-P ; POMMIÉ C ; RUIZ M ; GIUDICELLI V ; FOULQUIER E ; TRUONG L et al.** IMGT unique numbering for immunoglobulin and T cell receptor variable domains and Ig superfamily V-like domains. *Dev Comp Immunol,* 2003, vol. 27, 55-77
- **NOËL F ; MALPERTUY A ; DE BREVERN AG.** Global analysis of VHHs framework regions with a structural alphabet. *Biochimie,* 2016, vol. 131, 11-19
- **DE LAPORTE L ; RICE JJ ; TORTELLI F ; HUBBELL JA ; TENASCIN C.** Promiscuously Binds Growth Factors via Its Fifth Fibronectin Type III-Like Domain. *PLoS ONE,* 2013, vol. 8, e62076
- **SAGA Y ; TSUKAMOTO T ; JING N, KUSAKABE M ; SAKAKURA T.** Mouse tenascin: cDNA cloning, structure and temporal expression of isoforms. *Gene,* 1991, vol. 104, 177-185
- **HARMSEN et al.** *Properties, production, and applications of camelid single-domain antibody fragments,* 2007, vol. 77, 13-22
- **HULTBERG et al.** *Llama-derived single domain antibodies to build multivalent, superpotent and broadened neutralizing anti-viral molecules,* 2011, vol. 6 (4), e17665
- **THYS et al.** In vitro antiviral activity of single domain antibody fragments against poliovirus. *Antiviral Res.,* August 2010, vol. 87 (2), 257-64
- **SEBASTIAN et al.** Rotavirus A-specific single-domain antibodies produced in baculovirus-infected insect larvae are protective in vivo. *BMC Biotech,* 2012, vol. 12, 59
- **FORSMAN et al.** Llama antibody fragments with cross-subtype human immunodeficiency virus type 1 (HIV-1)-neutralizing properties and high affinity for HIV-1 gp120. *Journal of Virology,* 2008, vol. 82 (24), 12096-081
- **VERCCRUYSSE et al.** An intrabody based on a llama single-domain antibody targeting the N-terminal $\alpha$-helical multimerization domain of HIV-1 Rev prevents viral production. *J Biol Chem,* 09 July 2010, vol. 285 (28), 21768-80
- **BOUCHET et al.** Inhibition of the Nef regulatory protein of HIV-1 by a single-domain antibody. *Blood,* 2011, vol. 117 (13), 3559-68
- **ABDERRAZEK, R.B. et al.** Identification of potent-nanobodies to neutralize the most poisonous polypeptide from scorpion venom. *Biochem. J.,* 2009, vol. 424, 263-272
- **FEI FEI et al.** *Strategies for preparing Albumin-based Nanoparticles for Multifunctional Bioimaging and Drug Delivery,* 2017, vol. 7 (15), 3667-89
- **OLIVEIRA et al.** *Downregulation of EGFR by a novel multivalent nanobody-liposome platform,* 2010, vol. 145 (2), 165-75